# FASCICULE DE BREVET EUROPEEN

(11) **EP 1 056 771 B1**
(45) Date de publication et mention de la délivrance du brevet: **30.11.2005**
(21) Numéro de dépôt: 99906296.1
(22) Date de dépôt: 24.02.1999
(51) Int. Cl.: C07K 7/06, A61K 38/08, A61K 38/05

(54) **DERIVES DE STREPTOGRAMINES, LEUR PREPARATION ET LES COMPOSITIONS QUI LES CONTIENNENT**
DERIVATE DES STREPTOGRAMINS, IHRE HERSTELLUNG UND SIE ENTHALTENDE ZUBEREITUNGEN
STREPTOGRAMIN DERIVATIVES, PREPARATION METHOD AND COMPOSITIONS CONTAINING SAME

(30) Priorité: 26.02.1998 FR 9802316
(43) Date de publication de la demande: 06.12.2000
(73) Titulaire: Aventis Pharma S.A., 92160 Antony (FR)
(72) Inventeur: DESMAZEAU, Pascal, F-91250 Tigery (FR); DOERFLINGER, Gilles, F-91940 Les Ulis (FR); RIBEILL, Yves, Raleigh, NC 27615 (US); BACQUE, Eric, F-91390 Morsang sur Orge (FR); BARRIERE, Jean-Claude, F-91400 Bures sur Yvette (FR); DUTRUC-ROSSET, Gilles, F-75012 Paris (FR); PUCHAULT, Gérard, F-77139 Marcilly (FR)
(86) Numéro de dépôt international: PCT/FR1999/000409
(87) Numéro de publication internationale: WO 1999/043699

(56) Documents cités:
- WO-A-96/01901
- WO-A-96/04298
- WO-A-96/04299

## Description

La présente invention concerne des dérivés du groupe B des streptogramines de formule générale : 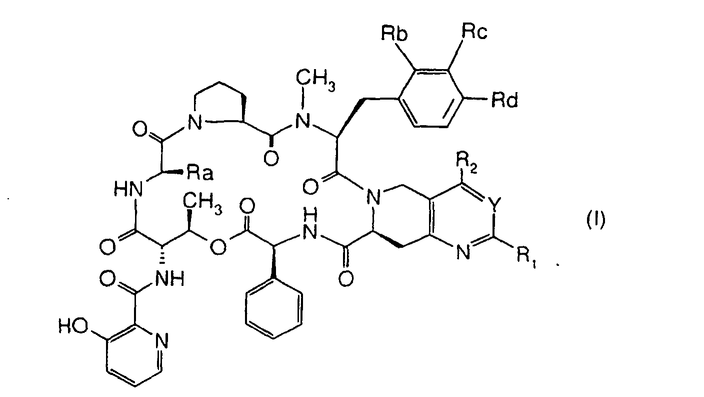 dans laquelle
Y est un atome d'azote ou un radical =CR₃-,
R₁ est un atome d'hydrogène, un radical alcoyle de 1 à 8 carbones, alcényle de 2 à 8 carbones, cycloalcoyle de 3 à 8 carbones, hétérocyclyle saturé ou insaturé de 3 à 8 chaînons, phényle, phényle substitué-par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino- ou un radical NR'R", R' et R" identiques ou différents pouvant être des atomes d'hydrogène ou des radicaux alcoyle de 1 à 3 carbones, ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué - par un radical alcoyle, alcényle de 2 à 8 carbones de cycloalcoyle de 3 à 6 carbones, hétérocyclyle saturé ou insaturé de 4 à 6 chaînons, benzyle, phényle ou phényle substitué tel que défini ci-dessus pour la définition de R₁ -
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R'', n étant un entier de 1 à 4 et R' et R" étant définis comme ci-dessus, ou bien si R₃ est un atome d'hydrogène, R₁ peut être aussi formyle, carboxy, alcoyloxycarbonyle, ou -CONR'R'' pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle, ou un radical NH et R° est un radical alcoyle de 1 à 8 carbones, cycloalcoyle de 3 à 6 carbones, hétérocyclyle saturé ou insaturé de 3 à 8 chainons, hétérocyclylméthyle de 3 à 8 chainons dont la partie hétérocyclyle est attachée au radical méthyle par un atome de carbone. phényle, phényle substitué- par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino- ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle de 1 à 3 carbones,
R₃ est un atome d'hydrogène ou un radical alcoyle, carboxy, alcoyloxycarbonyle ou carbamoyle de structure -CO-NR'R" dans laquelle R' et R" sont définis comme précédemment.
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle de 3 à 5C, et Rd est un radical -NMe-R"' pour lequel R"' représente un radical alcoyle, hydroxyalcoyle de 2 à 4C, ou alcényle de 2 à 8C éventuellement substitué par phényle, cycloalcoyl de 3 à 6C Méthyle, benzyle, benzyle substitué- par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino-, hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué- par un radical alcoyle, alcényle de 2 à 8 carbones, cycloalcoyle de 3 à 6 carbones, hétérocyclyle saturé ou insaturé de 4 à 6 chaînons, phényle, phényle substitué tel que défini ci-avant pour la définition de R₁ ou benzyle-, ou bien R"' représente un radical cyanométhyle, ou -CH₂CORe pour lequel soit Re est -OR'e, R'e étant hydrogène, alcoyle de 1 à 6 carbones, alcényle de 2 à 6 carbones, benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène. Rd est un radical -NHCH₃ ou -N(CH₃)₂ et Rc est un atome de chlore ou de brome, ou représente un radical alcényle de 3 à 5C, si Rd est -N(CH₃)₂,
4) Rb et Rd sont des atomes d'hydrogène et Rc est un atome d'halogène, ou un radical alcoylamino ou dialcoylamino, alcoyloxy, trifluorométhoxy, thioalcoyle, alcoyle de 1 à 6C ou trihalogénométhyle.
5) Rb et Rc sont des atomes d'hygrogène et Rd est un atome d'halogène, ou un radical éthylamino, diéthylamino ou methyl ethyl amino, alcoyloxy ou trifluorométhoxy, alcoylthio, alcoylsulfinyle, alcoylsulfonyle, alcoyle de 1 à 6C, phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle de 1 à 3C, et Rd est un atome d'halogène ou un radical amino, alcoylamino ou dialcoylamino, alcoyloxy ou trifluorométhoxy, thioalcoyle, alcoyle de 1 à 6C ou trihalogenométhyle.
7) Rc est un atome d'hydrogène et Rb et Rd représentent un radical méthyle,
ainsi que leurs sels, qui présentent une activité antibactérienne particulièrement intéressante, seuls ou associés à un dérivé du groupe A des streptogramines.

Dans la formule générale (I) ci-dessus, les atomes d'halogène peuvent être choisis parmi le fluor, le chlore, le brome ou l'iode ; les radicaux alcoyle ou acyle sont droits ou ramifiés et, sauf mention spéciale, contiennent 1 à 4 atomes de carbone. Il en est de même pour les radicaux alcoyle qui seront cités ci-après. Les radicaux alcényle peuvent être également en chaîne droite ou ramifiée.

Par ailleurs. à titre d'exemple, lorsque R' et R" forment ensemble un hétérocycle avec l'atome d'azote auquel ils sont attachés, ce dernier contient 1 ou 2 hétéroatomes et peut être par exemple choisi parmi pyrrolidinyle, pipéridino, morpholino, thiomorpholino, pipérazinyle, méthylpipérazinyle, imidazolidinyle, méthylimidazolidinyle. A titre d'exemple lorsque R₁ ou R° représentent hétérocyclyle, lorsque -NR'R" et/ou R"' sont substitués par hétérocyclyle ou lorsque R"' représente hétérocyclylméthyle, le radical hétérocyclyle contient 1 ou 2 hétéroatomes et peut être par exemple choisi parmi pyridyle, pyrazinyle, pyrimidinyle, thiényle, furyle, imidazolyle, éventuellement substitués ou parmi les hétérocycles cités ci-dessus à titre préférentiel pour -NR'R".

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine^{R} est constituée principalement de pristinamycine IA associée à la pristinamycine IIA.

Un autre antibactérien de la classe des streptogramines : la virginiamycine, a été préparé à partir de *Streptomyces virginiae.* ATCC 13161 [Antibiotics and Chemotherapy. 5. 632 (1955)|. La virginiamycine (Staphylomycine^{R}) est constituée principalement de facteur S associé au facteur M₁.

Des dérivés hémisynthétiques de streptogramines représentés par la structure : 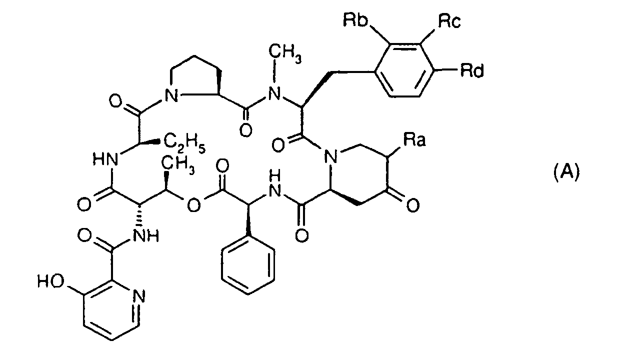 dans laquelle,
Ra est un radical de structure -CH₂R'a pour lequel R'a est un radical du type héterocyclylthio pouvant être substitué ou bien représente un radical de structure =CHR'a pour lequel R'a est un radical alcoylamino, alcoyloxy ou alcoylthio substitués, ou un radical du type héterocyclylamino, héterocyclyloxy, héterocyclylthio pouvant être substitués, Rb et Rc sont des atomes d'hydrogène et Rd est un atome d'hydrogene ou un radical diméthylamino, ou bien
Ra est un atome d'hydrogène et Rb est hydrogène ou méthyle, Rc et Rd sont hydrogène ou divers substituants
ont été décrits dans les brevets ou demandes de brevet EP 133097, EP 248703, EP 770132 et EP 772630. Associés à une composante hémisynthétique du groupe A des streptogramines, ils manifestent une synergie d'action et sont utilisables comme agents antibactériens soit par voie injectable seulement, soit uniquement par voie orale.

Les dérivés de streptogramine de formule générale (I) sont particulièrement intéressants du fait de leur activité puissante à la fois par voie orale et parentérale ce qui leur apporte un avantage indéniable dans le cas notamment de traitements d'infections graves, en milieu hospitalier par voie injectable, suivis d'un traitement ambulatoire par voie orale plus facile à administrer aux patients. Ainsi le praticien ne se trouve plus dans l'obligation de changer le patient de médicament entre la fin du traitement hospitalier et la fin globale du traitement.

Selon l'invention les dérivés de streptogramine pour lesquels Y est un radical =CR₃-et R₃ est autre qu'un radical alcoyle, peuvent être préparés par action d'un énamino ester de formule générale : 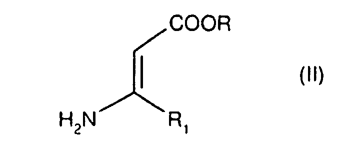 dans laquelle R₁ est défini comme précédemment et R représente le reste d'un ester facilement hydrolysable ou un radical alcoyle, sur le dérivé de 5δ-méthylène pristinamycine correspondant, de formule générale : 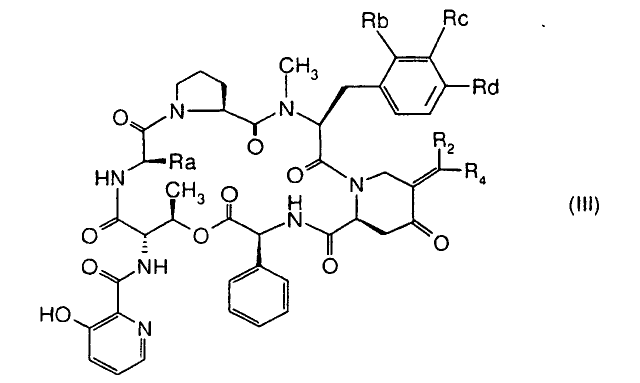 dans laquelle Ra, Rb, Rc et Rd sont définis comme précédemment, R₂ est défini comme précédemment et R₄ est un atome d'hydrogène, ou R₂ représente un atome d'hydrogène et R₄ est un atome d'hydrogène ou un radical dialcoylamino, suivie le cas échéant de la transformation de l'ester obtenu en un acide, puis éventuellement de sa décarboxylation, ou de la transformation de l'acide en un radical carbamoyle selon le dérivé de formule générale (I) souhaité, et/ou suivie le cas échéant de la transformation du dérivé de formule générale (I) pour lequel R₁ est hydroxyméthyle en un dérivé pour lequel R₁ est un radical formyle, puis le cas échéant carboxy, puis le cas échéant alcoyloxycarbonyle ou -CONR'R" et/ou suivie éventuellement de la mono N-déméthylation du dérive de formule générale (I) pour lequel Rd est un radical diméthylamino en un dérivé pour lequel Rd est méthylamino, puis éventuellement suivie de la transformation en un sel lorsqu'ils existent.

On entend par reste d'un ester facilement hydrolysable, par exemple et à titre non limitatif, le reste de l'ester benzylique, méthylique, triméthylsilyléthylique, éthylique, allylique, t.butylique.

La réaction s'effectue généralement dans un solvant organique tel qu'un alcool par exemple (méthanol, éthanol notamment), à une température comprise entre 40°C et la température de reflux du mélange réactionnel.

La transformation en acide, en amide, ou la décarboxylation pour obtenir un dérivé dans lequel R₃ est carboxy, carbamoyle de structure -CO-NR'R" ou un atome d'hydrogène, s'effectuent selon les méthodes connues qui n'altèrent pas le reste de la molécule et plus particulièrement selon les méthodes citées ci-après dans les exemples.

Notamment, lorsque l'on veut obtenir un dérivé de pristinamycine de formule générale (I) pour lequel R₃ est un radical carboxy, on prépare avantageusement l'ester benzylique. L'hydrolyse des esters s'effectue selon les méthodes connues qui n'altèrent pas le reste de la molécule, par exemple les méthodes mentionnées par T.W. Greene Protective Groups in Organic Synthesis, A. Wiley - Interscience Publication (1981), ou par Mc Omie, Protective Groups in Organic Chemistry, Plenum Press (1973). A titre d'exemple, le reste de l'ester benzylique peut être hydrolysé par traitement par le 1,4-cyclohexadiène en présence d'hydroxyde de palladium sur charbon, en milieu alcoolique (méthanol, éthanol par exemple), à une température comprise entre 0 et 60°C.

Lorsque l'on veut préparer un dérivé de formule générale (I) pour lequel R₃ est -CO-NR'R", le produit de formule générale (I) obtenu pour lequel R₃ est carboxy est traité selon les méthodes habituelles de transformation des acides en amides, qui n'altèrent pas le reste de la molécule. Notamment on fait réagir l'amine correspondante sur l'acide en présence d'un agent de condensation (carbodiimide par exemple) à une température comprise entre 0 et 60°C, dans un solvant organique tel qu'un solvant chloré (chloroforme, dichlorométhane par exemple), un amide (diméthylformamide, N-méthylpyrrolidone par exemple).

Lorsque l'on veut obtenir un dérivé de streptogramine de formule générale (I) pour lequel R₃ est un atome d'hydrogène, le produit pour lequel R₃ est carboxy est décarboxylé selon les méthodes habituelles qui n'altèrent pas le reste de la molécule. Notamment on peut opérer selon la méthode décrite par Barton, Tetrahedron. 44(17), 5479-86 (1988), par formation de l'ester de N-hydroxypyridine-2-thione, puis photolyse en présence de terbutylthiol par exemple.

La mono N-déméthylation du dérive de streptogramine de formule générale (I) pour lequel Rd est diméthylamino petit être effectuée selon la méthode décrite dans la demande de brevet EP 821697 par traitement par un periodate en milieu acétique suivi d'un traitement en milieu acide aqueux ou d'un traitement par un agent capable de consommer le formaldéhyde in situ.

La transformation du radical R₁ = hydroxyméthyle en un radical formyle peut s'effectuer par action de l'oxyde de selenium par analogie avec J. Korean Chem. Soc.. 38(7), 537-8 (1994).

La transformation du radical R₁ = formyle en un radical carboxy s'effectue selon les méthodes habituelles qui n'altèrent pas le reste de la molécule. Notamment on peut employer l'oxyde d'argent comme décrit dans Heterocycles 32(10), 1933-40 (1991).

La transformation du radical R₁ = carboxy en un radical alcoyloxycarbonyle s'effectue selon les méthodes habituelles qui n'altèrent pas le reste de la molécule. Notamment comme décrit dans The Chemistry of Acid Derivatives. Part l. page 411, Ed. S. Patai. John Wiley & Sons (1979).

La transformation du radical R₁ = carboxy en un radical carbamoyle de structure -CO-NR'R" s'effectue selon les méthodes habituelles qui n'altèrent pas le reste de la molécule. Notamment on fait réagir l'amine correspondante sur l'acide en présence d'un agent de condensation selon les méthodes classiques de la chimie peptidique : M. Bodanszky, Principles of Peptides Synthesis, Springer Verlag, Berlin - Heidelberg -New-York - Tokyo (1984).

Selon l'invention les dérivés de streptogramine de formule générale (I) pour lesquels Y est un radical =CR₃- et R₃ est un atome d'hydrogène ou un radical alcoyle, peuvent être préparés par action d'un sel de pyridinium de formule générale : 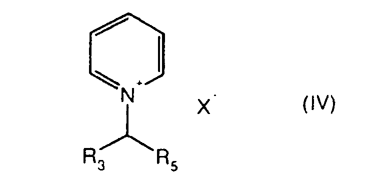 dans laquelle R₃ est défini comme ci-dessus, R₅ est le reste d'une cétone R₁-CO- pour lequel R₁ est défini comme ci-dessus à l'exception de représenter un radical -NR'R", ou représente éventuellement un radical hydroxy protégé ou un radical nitrophényle ou bien R₅ représente le radical cyano pour obtenir un dérivé de streptogramine pour lequel R₁ est un radical amino, et X⁻ est un anion, sur le dérivé de méthylène-5δ pristinamycine correspondant, de formule générale (III) dans laquelle R₄ est un atome d'hydrogène et Ra, Rb, Rc, Rd et R₂ sont définis comme précédemment suivie éventuellement de la libération du radical hydroxy ou le cas échéant de la réduction du radical nitrophényle pour obtenir un dérivé pour lequel R₁ est un radical aminophényle, ou suivie éventuellement de l'action d'une amine de formule générale HNR'R" sur le dérivé de streptogramine de formule générale (I) pour lequel R₁ est halogénométhyle, pour obtenir le dérivé correspondant pour lequel R₁ est un radical -CH₂NR'R", ou suivie le cas échéant de la transformation du dérivé de formule générale (I) pour lequel R₁ est hydroxyméthyle en un dérivé pour lequel R₁ est un radical formyle, puis le cas échéant carboxy, puis le cas échéant alcoyloxycarbonyle ou -CONR'R" et/ou éventuellement de la mono N-déméthylation du dérivé de formule générale (I) pour lequel Rd est un radical diméthylamino en un dérivé pour lequel Rd est méthylamino, puis éventuellement suivie de la transformation en un sel, lorsqu'ils existent.

A titre non limitatif, l'anion X⁻ représente avantageusement un anion halogénure (bromure, chlorure ou iodure par exemple).

La réaction s'effectue généralement en présence d'un sel d'ammonium (acétate d'ammonium par exemple), dans un solvant tel qu'un alcool (méthanol, éthanol par exemple), un nitrile (acétonitrile par exemple), un ester (acétate d'éthyle par exemple) ou une cétone (acétone par exemple), à une température comprise entre 40°C et la température de reflux du mélange réactionnel.

Lorsque le radical R₁ contient un substituant hydroxy, il est préférable de protéger préalablement ce radical selon les méthodes qui n'altèrent pas le reste de la molécule. La protection et la déprotection du radical hydroxy s'effectue selon les méthodes habituelles. Par exemple la protection est effectuée par un radical acétyle ou par tout autre groupement protecteur d'hydroxy dont la mise en place et l'élimination sont mentionnées par exemple par T.W. Greene Protective Groups in Organic Synthesis, A. Wiley - Interscience Publication (1981), ou par Mc Omie, Protective Groups in Organic Chemistry, Plenum Press (1973).

Lorsque l'on veut obtenir un produit pour lequel R₁ est aminophényle, il est préférable de préparer le dérivé nitrophényle correspondant, puis d'effectuer la réduction du radical nitro du dérivé obtenu. Notamment, on peut opérer par réduction en milieu acide (acide chlorhydrique) en présence de fer.

Lorsque l'on veut obtenir le dérivé de streptogramine de formule générale (I) pour lequel R₁ est un radical -CH₂NR'R", on fait agir une amine HNR'R" sur le dérivé correspondant de streptogramine de formule générale (1) pour lequel R₁ est halogénométhyle, en opérant en présence d'une amine tertiaire (triéthylamine, diisopropyléthylamine par exemple) ou d'un excès de l'amine, dans un solvant organique tel qu'un éther (tétrahydrofuranne, dioxanne par exemple), un alcool (méthanol par exemple), un solvant chloré (chloroforme, dichlorométhane par exemple), un nitrile (acétonitrile par exemple) ou le diméthylsulfoxyde à une température comprise entre 40°C et la température de reflux du mélange réactionnel.

La mono N-déméthylation du dérivé de streptogramine de formule générale (I) pour lequel Rd est diméthylamino peut être effectuée selon la méthode décrite dans la demande de brevet EP 821697. La transformation du radical R₁ = hydroxyméthyle en un radical formyle peut s'effectuer par action de l'oxyde de sélenium par analogie avec J. Korean Chem. Soc., 38(7), 537-8 (1994).

La transformation du radical R₁ = formyle en un radical carboxy s'effectue selon les méthodes habituelles qui n'altèrent pas le reste de la molécule. Notamment on peut employer l'oxyde d'argent comme décrit dans Heterocycles 32(10), 1933-40 (1991).

La transformation du radical R₁ = carboxy en un radical alcoyloxycarbonyle s'effectue selon les méthodes habituelles qui n'altèrent pas le reste de la molécule. Notamment comme décrit dans The Chemistry of Acid Derivatives, Part I, page 411, Ed. S. Patai, John Wiley & Sons (1979).

La transformation du radical R₁ = carboxy en un radical carbamoyle de structure -CO-NR'R" s'effectue selon les méthodes habituelles qui n'altèrent pas le reste de la molécule. Notamment on fait réagir l'amine correspondante sur l'acide en présence d'un agent de condensation selon les méthodes classiques de la chimie peptidique : M. Bodanszky, Principles of Peptides Synthesis, Springer Verlag, Berlin - Heidelberg -New-York - Tokyo (1984).

Selon l'invention les dérivés de streptogramine de formule générale (I) pour lesquels Y est un atome d'azote, peuvent être préparés par action d'un sel d'une amidine ou d'un dérivé de l'isourée ou de l'isothiourée de formule générale : 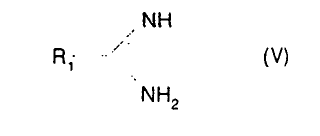 dans laquelle R₁ est défini comme pour la formule générale (I), à l'exception de représenter un radical XR° pour lequel X est sulfonyle ou sulfinyle, ou un radical NR'R" autre qu'amino, sur un dérivé de streptogramine de formule générale (III) pour lequel R₄ est dialcoylamino, puis pour obtenir un dérivé de streptogramine de formule générale (1) pour lequel R₁ est un radical XR° pour lequel X est sulfonyle ou sulfinyle, oxydation du dérivé correspondant pour lequel X est un atome de soufre, puis pour obtenir le dérivé de streptogramine de formule générale (I) pour lequel R₁ est un radical NR'R'', substitution du dérivé sulfonyle obtenu par action de l'amine correspondante HNR'R" et/ou éventuellement pour obtenir un dérivé pour lequel Rd est méthylamino, déméthylation du dérivé de formule générale (I) pour lequel Rd est un radical diméthylamino, puis éventuellement transformation en un sel, lorsqu'ils existent.

La réaction du dérivé de formule générale (V) s'effectue généralement dans un solvant organique comme un amide (diméthylformamide, diméthylacétamide par exemple) ou un nitrile (acétonitrile par exemple), en présence d'une base comme notamment une amine tertiaire (diisopropyléthylamine, triéthylamine par exemple) ou un bicarbonate alcalin (bicarbonate de sodium ou de potassium par exemple), à une température comprise entre 50 et 100°C. La réaction est avantageusement mise en oeuvre au moyen du chlorhydrate, du sulfate ou de l'hydrogénosulfate du dérivé de formule générale (V).

L'oxydation en dérivé sulfinyle ou sulfonyle s'effectue respectivement par traitement par 1 ou 2 équivalents d'Oxone® en milieu acide (par exemple acide sulfurique 0.1 à 2N, de préférence 0,5 à 1N), à une température comprise entre -60 et 60°C. dans un solvant tel qu'un alcool (méthanol, éthanol, i-propanol par exemple). Selon le produit préparé, il peut être éventuellement nécessaire de faire suivre l'opération d'oxydation d'un traitement réducteur de N-oxydes par toute méthode connue et spécifique, qui n'altère pas le reste de la molécule. Notamment il est possible d'opérer par chauffage en présence de fer dans l'acide acétique, ou par traitement par le bisulfite de sodium.

L'opération subséquente de substitution par une amine peut être effectuée par action de l'amine correspondante de formule HNR'R", soit en excès, soit en présence d'une base telle que par exemple un bicarbonate alcalin (bicarbonate de sodium ou de potassium par exemple), en opérant à une température comprise entre 20 et 100°C, dans un solvant organique comme un amide (diméthylformamide, diméthylacétamide par exemple) ou un nitrile (acétonitrile par exemple).

La mono N-déméthylation du dérivé de streptogramine de formule générale (I) pour lequel Rd est diméthylamino peut être effectuée selon la méthode décrite dans la demande de brevet EP 821697.

La transformation du radical R₁ = hydroxyméthyle en un radical formyle peut s'effectuer par action de l'oxyde de sélenium par analogie avec J. Korean Chem. Soc., 38(7), 537-8 (1994).

La transformation du radical R₁ = formyle en un radical carboxy s'effectue selon les méthodes habituelles qui n'altèrent pas le reste de la molécule. Notamment on peut employer l'oxyde d'argent comme décrit dans Heterocycles 32(10). 1933-40 (1991).

La transformation du radical R₁ = carboxy en un radical alcoyloxycarbonyle s'effectue selon les méthodes habituelles qui n'altèrent pas le reste de la molécule. Notamment comme décrit dans The Chemistry of Acid Derivatives, Pan I, page 411, Ed. S. Patai, John Wiley & Sons (1979).

La transformation du radical R = carboxy en un radical carbamoyle de structure -CO-NR'R" s'effectue selon les méthodes habituelles qui n'altèrent pas le reste de la molécule. Notamment on fait réagir l'amine correspondante sur l'acide en présence d'un agent de condensation selon les méthodes classiques de la chimie peptidique : M. Bodanszky, Principles of Peptides Synthesis, Springer Verlag. Berlin - Heidelberg -New-York - Tokyo (1984).

Selon l'invention les dérivés de streptogramine de formule générale (I) pour lesquels Y est un radical =CR₃-, R₁ est un atome d'hydrogène, un radical alcoyle, alcényle, cycloalcoyle, hétérocyclyle aromatique, phényle, phényle substitué, halogénométhyle, hydroxyméthyle, alcoyloxyméthyle, alcoylthiométhyle, alcoylsulfinylméthyle, alcoylsulfonylméthyle, ou -(CH₂)ₙNR'R'', ou bien lorsque R₃ est un atome d'hydrogène, pour lesquels R₁ est formyle, carboxy, alcoyloxycarbonyle, ou -CONR'R" tels que définis précédemment et R₂ est un atome d'hydrogène, peuvent également être préparés par action de la formyl énamine de formule générale : 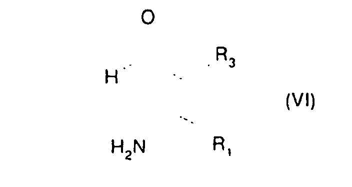 dans laquelle R₁ est un atome d'hydrogène, un radical alcoyle, alcényle, cycloalcoyle, hétérocyclyle aromatique, phényle, phényle substitué, hydroxyméthyle, alcoyloxyméthyle, alcoylthiométhyle, ou -(CH₂)ₙNR'R"et R₃ est défini comme précédemment à l'exception de représenter carboxy, sur un dérivé de streptogramine de formule générale : 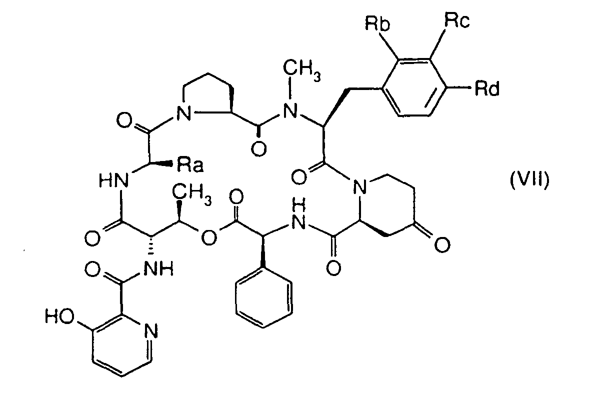 dans laquelle Ra, Rb, Rc et Rd sont définis comme précédemment, suivie le cas échéant de la transformation du dérivé pour lequel R₃ est amide ou ester en un dérivé pour lequel R₃ est carboxy et/ou suivie le cas échéant de l'oxydation du dérivé pour lequel R₁ est alcoylthiométhyle en un dérivé pour lequel R₁ est alcoylsulfinylméthyle ou alcoylsulfonylméthyle, ou suivie le cas échéant de la transformation du dérivé pour lequel R₁ est un radical hydroxyméthyle en un dérivé pour lequel R₁ est halogénométhyle, puis le cas échéant de la transformation du dérivé pour lequel R₁ est halogénométhyle en un dérivé pour lequel R₁ est -CH₂NR'R", ou suivie le cas échéant de la transformation du dérivé de formule générale (I) pour lequel R₁ est hydroxyméthyle en un dérivé pour lequel R₁ est un radical formyle, puis le cas échéant carboxy, alcoyloxycarbonyle et/ou -CONR'R", et/ou éventuellement de la mono N-déméthylation du dérivé de formule générale (I) pour lequel Rd est un radical diméthylamino en un dérivé pour lequel Rd est méthylamino, puis éventuellement suivie de la transformation en un sel, lorsqu'ils existent.

La réaction est mise en oeuvre dans un solvant organique comme un alcool (méthanol, éthanol par exemple) à une température comprise entre 20°C et la température de reflux du mélange réactionnel, en présence d'un donneur d'ammoniac comme par exemple l'acétate d'ammonium.

L'oxydation du radical alcoylthiométhyle en un radical alcoylsulfinylméthyle ou alcoylsulfonylméthyle s'effectue dans les conditions décrites précedemment, par traitement par l'Oxone®.

L'obtention d'un dérivé de streptogramine de formule générale (I) pour lequel R₁ est halogénométhyle à partir d'un dérivé pour lequel R₁ est hydroxyméthyle s'effectue selon les méthodes habituelles. Notamment par action d'un agent d'halogénation comme par exemple le chlorure de thionyle.

La réaction d'une amine HNR'R" sur le dérivé de streptogramine de formule générale (I) pour lequel R₁ est halogénométhyle, s'effectue comme décrit précédemment.

La transformation du radical R₁ = hydroxyméthyle en un radical formyle peut s'effectuer par action de l'oxyde de sélenium par analogie avec J. Korean Chem. Soc., 38(7). 537-8 (1994).

La transformation du radical R₁ = formyle en un radical carboxy s'effectue selon les méthodes habituelles qui n'altèrent pas le reste de la molécule. Notamment on peut employer l'oxyde d'argent comme décrit dans Heterocycles 32(10), 1933-40 (1991).

La transformation du radical R₁ = carboxy en un radical alcoyloxycarbonyle s'effectue selon les méthodes habituelles qui n'altèrent pas le reste de la molécule.

Notamment comme décrit dans The Chemistry of Acid Derivatives, Part I, page 411, Ed. S. Patai, John Wiley & Sons (1979).

La transformation du radical R₁ = carboxy en un radical carbamoyle de structure -CO-NR'R" s'effectue selon les méthodes habituelles qui n'altèrent pas le reste de la molécule. Notamment on fait réagir l'amine correspondante sur l'acide en présence d'un agent de condensation selon les méthodes classiques de la chimie peptidique : M. Bodanszky, Principles of Peptides Synthesis, Springer Verlag, Berlin - Heidelberg -New-York - Tokyo (1984). La transformation directe du radical R₁ = formyle en un radical carbamoyle, peut être effectuée comme décrit dans les exemples.

La mono N-déméthylation du dérivé de streptogramine de formule générale (I) pour lequel Rd est diméthylamino peut être effectuée selon la méthode décrite dans la demande de brevet EP 821697.

Les énamino esters de formule générale (II) sont soit commerciaux, soit peuvent être préparés selon ou par analogie avec les méthodes décrites dans Tetrahedron Letters, 38(3), 443-6 (1997) et FR 2216270.

Le dérivés de méthylène-5δ pristinamycine de formule générale (III) pour lesquels Ra est un radical méthyle, ou pour lesquels Ra est un radical éthyle mais Rb, Rc et Rd n'ont pas simultanément les définitions : « Rb et Rc représentent hydrogène et Rd représente hydrogène ou diméthylamino », peuvent être préparés à partir de la pristinamycine IC, de la virginiamycine S4, de la vernamycine Bδ, de la pristinamycine IB, ou à partir de leurs dérivés ou analogues de formule générale (VII) dans laquelle Ra est défini comme ci-dessus et les substituants Rb, Rc et Rd sont soit définis comme dans la formule générale (I) en 1), à l'exception de représenter simultanément Rb = Rc = hydrogène et Rd = hydrogène ou diméthylamino, lorsque Ra est éthyle, soit sont définis comme pour la formule générale (I) en 2) à 7). en opérant par analogie avec les méthodes décrites dans les demandes européennes EP 133097 ou EP 133098 ou par analogie avec les méthodes décrites ci-après dans les exemples.

Les sels de pyridinium de formule générale (IV) sont préparés selon ou par analogie avec la méthode décrite par F. Kröhnke, Synthesis, (1976) 1-24, ou selon ou par analogie avec les méthodes décrites ci-après dans les exemples.

Les amidines de formule (V) sont soit commerciales soit préparées selon ou par analogie avec la méthode décrite par S. Patai, The Chemistry of amidines and Imidates, Interscience Publication, J. Wiley & Sons, Chap. 7, p. 283 (1975).

Les formyl énamines de formule générale (VI) peuvent être préparés par analogie avec les méthodes décrites dans J. Chem. Soc., Perkin trans 1, 9, 2103 (1984).

Les produits de formule générale (VII) pour lesquels Ra, Rb, Rc et Rd sont définis comme pour la formule générale (1) en 1) sont des streptogramines naturelles du groupe B.
Les composantes naturelles des streptogramines du groupe B leur préparation et leur séparation est effectuée par fermentation et isolement des constituants à partir du môut de fermentation selon ou par analogie avec la méthode décrite par
J. Preud'homme et coll., Bull. Soc. Chim. Fr., vol. 2, 585 (1968) ou selon Antibiotics and Chemotherapy, 5. 632 (1955).
Dans Streptogramine als Modelsysteme für den Kationentransport durch Membranen. Dissertation zur Erlangung des Doktorgrades der Mathematisch-Naturwissenschaftlichen Facultät der Georg-August Universität zu Göttingen, Göttingen 1979, dans Antibiotics III. 521 (1975) et dans Antibiotics of the virginiamycin family, Inhibitors which contain synergistic components. C. Cocito.
Microbiological Reviews, 145-98 (1979) sont également décrites des composantes du groupe B des streptogramines.
Alternativement la préparation des composantes naturelles du groupe B peut être effectuée par fermentation spécifique, comme décrit dans la demande de brevet FR 2 689 518.

Les dérivés de streptogramine de formule générale (VII) pour lesquels Ra, Rb, Rc et Rd sont définis comme pour la formule générale (1) en 3) sont préparés comme décrit dans la demande européenne EP 772630.

Les dérivés de streptogramine de formule générale (VII) pour lesquels Ra, Rb, Rc et Rd sont définis comme pour la formule générale (I) en 4) à 7) sont préparés comme décrit dans la demande européenne EP 770132.

Les dérivés de streptogramine de formule générale (VII) pour lesquels Ra, Rb et Rc sont définis comme pour la formule générale (I) en 5) et Rd est alcoylsulfinyle ou alcoylsulfonyle peuvent être préparées par oxydation du produit correspondant pour lequel Rd est alcoylthio.

Les dérivés de streptogramine de formule générale (VII) pour lesquels Ra, Rb, Rc et Rd sont définis comme pour la formule générale (I) en 2) peuvent être préparés à partir de la pristinamycine IB (Ra = éthyle) ou de la vernamycine Bδ (Ra = méthyle) ou d'un dérivé de streptogramine de formule générale (I) pour lequel Ra, Rb, et Rc sont défini comme en 3) et Rd est -NHCH₃, par action d'un dérivé halogéné de formule générale :

R'''-X (VIII)

dans laquelle R"' est défini comme pour la formule générale (I) en 2) et X est un atome d'iode, de brome ou de chlore, suivie, le cas échéant, de la chloration ou de la bromation du produit obtenu, lorsque l'on veut obtenir un dérivé pour lequel Rc est un atome de chlore ou de brome, après être partis de la pristinamycine IB ou de la vernamycine Bδ.

La réaction s'effectue généralement dans un solvant organique tel qu'un amide (diméthylformamide par exemple), un solvant chloré (chloroforme, dichlorométhane par exemple), un mélange alcool (méthanol, éthanol par exemple) / solvant chlore, un nitrile (acétonitrile par exemple), dans le diméthylsulfoxyde ou la N-méthylpyrrolidone, à une température comprise entre 20 et 100°C, éventuellement en présence d'iodure de sodium ou d'un bicarbonate alcalin (sodium ou potassium). De préférence on opère sous azote. Il est entendu que lorsque le radical R"' contient un radical amino, il est préférable de protéger ce radical préalablement à la réaction. La protection et la déprotection s'effectuent selon les méthodes indiquées dans les références citées précedemment.

Le cas échéant, l'halogénation s'effectue avantageusement par un N-halosuccinimide, dans un solvant organique tel qu'un solvant chloré (dichlorométhane, chloroforme par exemple) ou un nitrile (acétonitrile par exemple), à une température comprise entre 20 et 80°C.

Selon une autre alternative, les dérivés de streptogramine de formule générale (VII) pour lesquels Ra et Rb sont définis comme pour la formule générale (I), Rc est un atome d'hydrogène et Rd est un radical cyanométhyl méthyl amino ou alcoyloxycarbonylméthyl méthyl amino, peuvent être également préparés à partir de la pristinamycine IA (Ra = éthyle) ou de la pristinamycine IC (Ra = méthyle), par action d'un dérivé halogéné de formule générale (VIII) dans laquelle R"' représente cyanométhyle ou alcoyloxycarbonylméthyle.
La réaction s'effectue généralement dans un solvant organique tel qu'un amide (diméthylformamide par exemple) à une température comprise entre 70 et 100°C. De préférence on opère sous azote.

Les dérivés de streptogramines de formule générale (III) pour lesquels Ra est un radical méthyle et Rb, Rc, et Rd sont définis comme dans la formule générale (I) ou pour lesquels Ra est un radical éthyle et Rb, Rc, et Rd sont définis comme dans la formule générale (1) en 2) à 7) ainsi que les dérivés de streptogramine de formule générale (VII) pour lesquels Ra, Rb, Rc et Rd sont définis comme pour la formule générale (I) en 2), à l'exception pour R ''' de représenter éthyle si Rb et Rc sont hydrogène, sont des produits nouveaux.
L'ensemble de ces produits intermédiaires nouveaux peut être représenté par la formule générale : 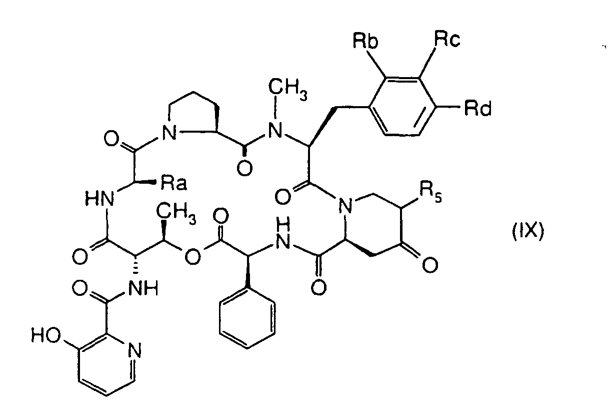 dans laquelle Ra est un radical méthyle et Rb, Rc, et Rd sont définis comme dans la formule générale (I) ou Ra est un radical éthyle et Rb, Rc, et Rd sont définis comme dans la formule générale (I) en 2) à 7) et R₅ représente un radical méthylènyle disubstitué de structure 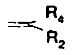 pour lequel R₂ et R₄ sont définis comme précédemment, ou bien dans laquelle Ra, Rb, Rc et Rd sont définis comme pour la formule générale (I) en 2), à l'exception pour R"' de représenter éthyle si Rb et Rc sont hydrogène, et R₅ est un atome d'hydrogène.
Il est entendu que les produits de formule générale (IX) entrent aussi dans le cadre de la présente invention.

Les dérivés de streptogramine de formule générale (I) ou (IX) peuvent être purifiés le cas échéant par des méthodes physiques telles que la cristallisation ou la chromatographie.

Certains des dérivés de streptogramine de formule générale (I) peuvent être transformés à l'état de sels d'addition avec les acides, par les méthodes connues. Il est entendu que ces sels lorsqu'ils existent entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Les dérivés portant un substituant carboxy peuvent être transformés en sels métalliques ou en sels d'addition avec les bases azotées selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhyldrylamine, quinine, choline, arginine, lysine, leucine, dibenzylamine).

Les dérivés de streptogramine selon la présente invention présentent des propriétés antibactériennes et des propriétés synergisantes de l'activité antibactérienne des dérivés de streptogramine du groupe A. Ils sont particulièrement intéressants du fait de leur activité seuls ou associés à des composantes du groupe A des streptogramines et surtout du fait de leur activité à la fois par voie orale et parentérale ce qui ouvre la voie à un traitement relai ambulatoire sans modifier la nature du médicament.

Lorsqu'ils sont associés avec une composante ou un dérivé du groupe A des streptogramines, ces derniers peuvent être notamment choisis selon que l'on désire obtenir une forme administrable par voie orale ou parentérale, parmi les composantes naturelles : prisunamycine IIA. pristinamycine IIB. pristinamycine 11C. pristinamycine IID, pristinamycine IIE, pristinamycine IIF, pristinamyei-ne IIG ou parmi des dérivés d'hémisynthèse tels que décrits dans les brevets ou demandes de brevet US 4590004 et EP 191662 ou encore parmi les dérivés d'hémisynthèse de formule générale : 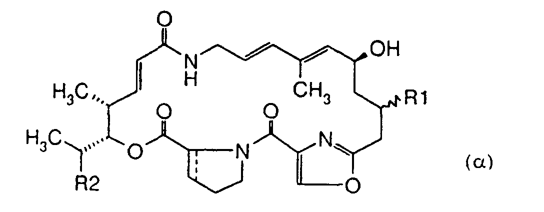 dans laquelle R₁ est un radical -NR'R" pour lequel R' est un atome d'hydrogène ou un radical méthyle, et R" est un atome d'hydrogène, un radical alcoyle, cycloalcoyle, allyle, propargyle, benzyle, ou -OR"', R"' étant un atome d'hydrogène, un radical alcoyle, cycloalcoyle, allyle, propargyle ou benzyle, ou -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double, ainsi que leurs sels.

Il est entendu que les associations des dérivés selon l'invention et des streptogramines du groupe A entrent également dans le cadre de la présente invention.

In vitro, associés à la pristinamycine IIB. les produits de formule générale (I), selon l'invention, se sont montrés actifs à des concentrations comprises entre 0,25 et 16 mg/l sur Staphylococcus aureus 209P. In vivo, sur les infections expérimentales de la souris à Staphylococcus aureus IP 8203, les dérivés de streptogramine de formule générale (I) se sont montrés actifs à des doses comprises entre 15 et 150 mg/kg, par voie orale, associés à la pristinamycine IIB et entre 5 et 150 mg/kg, par voie sous cutanée, associés à la dalfopristine (DC₅₀), [associations 30/70].

Enfin, les produits selon l'invention sont particulièrement intéressants du fait de la faible toxicité observée dans le modèle de septicémie à Staphylococcus aureus IP 8203 chez la souris. L'ensemble des produits, en association 30/70 avec une composante du groupe A, se sont révélés atoxiques à l'exception de quelques uns d'entre eux pour lesquels une faible mortalité a été observée à la dose maximale administrée de 300 mg/kg par voie orale ou sous cutanée, en 2 administrations à 5 heures d'intervalle.

Certains des produits intermédiaires définis par la formule générale (IX) présentent également des propriétés antibactériennes, notamment le sous groupe des dérivés de streptogramine de formule générale (VII). In vivo, sur les infections expérimentales de la souris à Staphylococcus aurcus IP 8203, ils se sont montrés actifs par voie orale associés à la pristinamycine IIB (associations 30/70) à des doses comprises entre 25 et 150 mg/kg.

D'un intérêt particulier sont les produits de formule générale (I) pour lesquels
Y est un atome d'azote ou un radical =CR₃-,
R₁ est un atome d'hydrogène, un radical alcoyle de 1 à 8 carbones, cycloalcoyle de 3 à 8 carbones, hétérocyclyle saturé ou insaturé de 3 à 8 chaînons, phényle, phényle substitué- par un ou plusieurs radicaux amino, alcoylamino ou dialcoylamino ou un radical NR'R", R' et R" identiques ou différents pouvant être des atomes d'hydrogène ou des radicaux alcoyle de 1 à 3 carbones, ou pouvant former ensemble avec l'atome d'azote auquel ils sont attachés, un hétérocycle de 3 à 8 chaînons contenant éventuellement un autre heteroatome choisi parmi l'oxygène le soufre ou l'azote éventuellement substitué par un radical alcoyle,
ou bien lorsque Y est un radical =CR₃-, R₁ peut être aussi halogénométhyle, hydroxyméthyle, alcoylthiométhyle dont la partie alcoyle est éventuellement substituée par NR'R", alcoylsulfinylmethyle, alcoylsulfonylméthyle, acyloxyméthyle, cyclopropylaminométhyle ou -(CH₂)ₙNR'R", n étant un entier de 1 à 4 et R' et R" étant définis comme ci-dessus, ou bien si R₃ est un atome d'hydrogène. R₁ peut être aussi formyle ou -CONR'R" pour lequel R' et R" sont définis comme ci-dessus,
ou bien lorsque Y est un atome d'azote. R₁ peut être aussi un radical -XR² pour lequel X est un atome d'oxygéne ou de soufre, un radical sulfinyle ou sulfonyle, ou un radical NH et R° est un radical alcoyle de 1 à 8 carbones, hétérocyclylméthyle de 3 à 8 chainons dont la partie hétérocyclyle est attachée au radical méthyle par un atome de carbone, ou un radical -(CH₂)ₙNR'R" pour lequel R' et R" sont définis comme ci-dessus et n est un entier de 2 à 4.
R₂ est un atome d'hydrogène ou un radical alcoyle de 1 à 3 carbones,
R₃ est un atome d'hydrogène ou un radical carboxy ou alcoyloxycarbonyle,
Ra est un radical méthyle ou éthyle, et
Rb, Rc et Rd ont les définitions ci-aprés :
- Rb et Rc sont des atomes d'hydrogène et Rd est un atome d'hydrogène ou un radical méthylamino ou diméthylamino,
- Rb est un atome d'hydrogène, Rd est un radical -NHCH₃ ou -N(CH₃)₂ et Rc est un atome de chlore ou de brome.
Et parmi ces produits, plus particulièrement préférés sont les produits de formule générale (I) pour lesquels
Y est un atome d'azote ou un radical =CR₃-,
R₁ est un atome d'hydrogène, un radical alcoyle de 1 à 3 carbones, cycloalcoyle de 3 à 8 carbones, hétérocyclyle saturé ou insaturé de 3 à 8 chainons, phényle, phényle substitué par un radical amino,
ou bien lorsque Y est un radical =CR₃-, R₁ peut être aussi acyloxyméthyle,
ou bien lorsque Y est un atome d'azote. R₁ peut être aussi un radical -XR° pour lequel
X est un atome d'oxygène ou de soufre ou un radical NH et R° est un radical alcoyle de 1 à 4 carbones ou un radical -(CH₂)ₙNR'R" pour lequel R' et R'' identiques ou différents pouvent être des atomes d'hydrogène ou des radicaux alcoyle de 1 à 3 carbones, ou forment ensemble avec l'atome d'azote auquel ils sont attachés, un hétérocycle de 3 à 8 chainons contenant éventuellement un autre hétéroatome choisi parmi l'oxygène le soufre ou l'azote éventuellement substitué par un radical alcoyle, et n est un entier de 2 à 4.
R₂ est un atome d'hydrogène ou un radical alcoyle de 1 à 3 carbones,
R₃ est un atome d'hydrogène ou un radical alcoyloxycarbonyle,
Ra est un radical méthyle ou éthyle, et
Rb, Rc et Rd ont les définitions ci-après :
- Rb et Rc sont des atomes d'hydrogène et Rd est un atome d'hydrogène ou un radical méthylamino ou diméthylamino,
- Rb est un atome d'hydrogène, Rd est un radical -NHCH₃ ou -N(CH₃)₂ et Rc est un atome de chlore.

Et tout particulièrement les produits suivants :
- 2"-méthyl-pyrido [2,3-5γ,5δ] pristinamycine IE ;
- 2"-cyclopropyl-pyrido [2,3-5γ,5δ] pristinamycine IE ;
- pyrido [2,3-5γ,5δ] pristinamycine IE ;
- 2"-éthyl-pyrido [2,3-5γ,5δ] (4ζ-méthylamino) (4ζ-dèsdiméthylamino) pristinamycine IE ;
- 4ε-chloro 2"-(éthyl)-pyrido [2.3-5γ,5δ] (4ζ-méthylamino) (4ζ-désdiméthylamino) pristinamycine IE.

Les dérivés de streptogramine de formule générale (α) sont préparés à partir des composantes de la pristinamycine naturelle de formule générale : 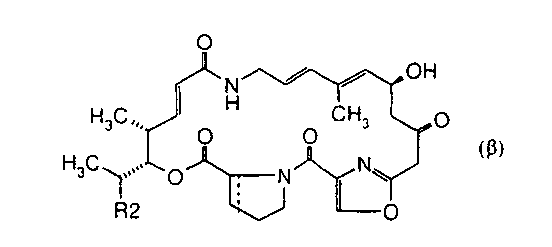 dans laquelle R₂ est défini comme pour la formule générale (α), par action d'une amine de formule générale :

H₂N-R" (γ)

dans laquelle R" est défini comme pour la formule générale (α), suivie de l'action d'un agent réducteur de l'énamine (ou de l'oxime) intermédiaire obtenue, puis, lorsque l'on veut obtenir un dérivé de streptogramine de formule générale (α) pour lequel R' est un radical méthyle, suivie d'une seconde amination réductrice, par action du formaldéhyde ou d'un dérivé générant le formaldéhyde in situ et de la réduction de l'énamine intermédiaire.

L'action de l'amine s'effectue dans un solvant organique comme un alcool (méthanol. éthanol par exemple), un solvant chloré (dichlorométhane, dichloroéthane, chloroforme par exemple), un nitrile (acétonitrile par exemple); la pyridine, à une température comprise entre 0 et 30°C, et éventuellement en présence d'un agent de deshydratation comme par exemple le sulfate de magnésium, le sulfate de sodium ou des tamis moléculaires. De préférence on opère sous atmosphère inerte (argon par exemple). Il est également possible de faire réagir le sel de l'amine.
De préférence, pour préparer des dérivés pour lesquels la liaison --- représente une liaison double, la réaction est effectuée dans un solvant organique comme un nitrile (acétonitrile par exemple) en présence d'un acide, tel qu'un acide organique (acide acétique par exemple) : dans ce cas l'addition d'un agent de deshydratation n'est pas nécessaire.
Lorsque l'on prépare un dérivé de la streptogramine de formule générale (α) pour lequel R" est un radical -OR"', il est possible d'isoler l'oxime intermédiaire de formule générale : 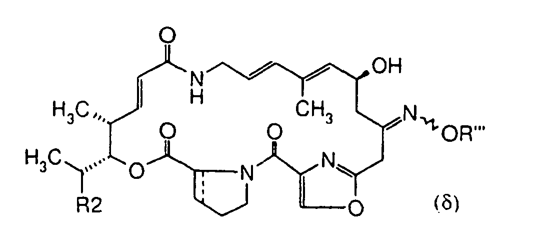 dans laquelle R₂ et R'" sont définis comme pour la formule générale (α), puis de réduire ce produit en un dérivé de formule générale (α) pour lequel R' est un atome d'hydrogène, et éventuellement le mettre en oeuvre dans l'opération- subséquente d'amination-réductrice.

La réduction s'effectue par action d'un agent réducteur, par exemple un borohydrure alcalin (cyanoborohydrure ou triacétoxyborohydrure de sodium par exemple) en présence d'un acide organique (acide acétique par exemple) dans un solvant organique tel que cité ci-dessus pour la réaction d'amination. Le cas échéant, l'opération subséquente d'amination réductrice, destinée à obtenir l'amine disubstituée, s'effectue dans des conditions analogues.

Les exemples suivants donnés à titre non limitatif illustrent la présente invention.

Dans ce qui suit, les exemples A à AF illustrent la préparation des produits intermédiaires, notamment de produits de formule générale (IX). Les exemples 1 à 33 illustrent les dérivés de streptogramine de formule générale (I) selon l'invention.

Dans les exemples qui suivent, les spectres de RMN ont été étudiés dans le deutérochloroforme, la nomenclature utilisée est celle de J.O. Anteunis et coll., Eur. Biochem., 58, 259 (1975) et notamment :

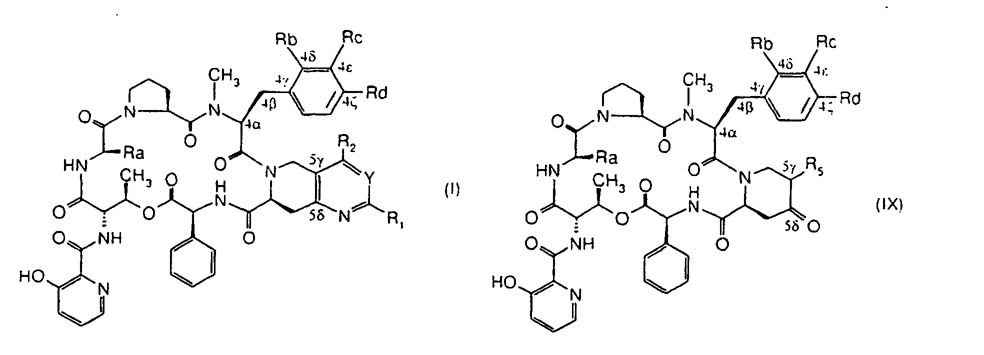

Les chromatographies sur colonne sont réalisées, sauf exception mentionnée, sous pression atmosphérique en utilisant une silice 0,063-0,02 mm. Dans quelques cas précisés, les purifications sont faites par chromatographie-flash en utilisant une silice de 0.04-0.063 mm, ou par chromatographie liquide haute performance (CLHP) sur silice greffée C₈ ou C₁₈.

### PREPARATION DES DERIVES DE FORMULE GENERALE (I)

### Exemple 1

Dans un tricol contenant 20 cm3 de méthanol, on introduit 2 g de 5δ-méthylène-pristinamycine I_{A} et 0,26 g (2.3 mmole) de 3-amino crotonate de méthyle. Le mélange est porté 6 heures au reflux puis on rajoute 0,1 g de 3-amino crotonate de méthyle supplémentaire et le reflux est poursuivi 1 heure. Le mélange réactionnel est concentré à sec à 40°C sous pression réduite (2,7 kPa) pour donner 2,4 g d'un solide jaune qui est purifié par chromatographie sur 30 g de silice [éluant : dichlorométhane/méthanol 95/5 en volumes] pour donner un solide qui est concrété dans 60 cm3 d'un mélange éther-éther de pétrole, filtré puis séché à 40°C sous pression réduite (90 Pa). On obtient ainsi 0,96 g de 3'-méthoxycarbonyl-2"-méthyl-pyrido [2.3-5γ,5δ] pristinamycine IE sous forme d'un solide jaune fondant à 195°C.

Spectre de R.M.N. ¹H (400 MHz. CDCl₃, δ en ppm) : 0,92 (t, J = 7,5 Hz, 3H : CH₃ en 2γ), de 1,20 à 1,40 (mt, 2H : 1H du CH₂ en 3β et 1H du CH₂ en 3γ) ; 1,30 (d, J = 7 Hz, 3H : CH₃ en 1γ); 1,50 (dd, J = 16,5 et 5 Hz, 1H : 1H du CH₂ en 5β) ; de 1,50 à 1,85 (mt : les 3H correspondant a l'autre H du CH₂ en 3γ et au CH₂ en 2β) ; 2,05 (mt, 1H : l'autre H du CH₂ en 3β) : 2.77 (s, 3H : ArCH₃) ; 2.85 (s, 6H : ArN(CH₃)₂) : 2.94 (mt, 1H : 1H du CH₃ en 4β): 3,11 (d, J = 16.5 Hz, 1H : l'autre H du CH₂ en 5β) : de 3,20 à 3.35 (mt, 2H : l'autre H du CH₂ en 4β et 1H du CH₂ en 3δ) ; 3,25 (s, 3H : NCH₃) ; 3,50 (mt, 1H : l'autre H du CH₂ en 3δ) ; 3,90 (mt, 1H : 1H du CH₂ en 5ε) : 3,95 (s, 3H : COOCH₃) ; 4.61 (dd, J = 7 et 4,5 Hz, 1H : CH en 3α) ; 4.80 (mt, 1H : CH en 2α) ; 4,89 (d large, J = 10 Hz, 1H : CH en 1α) ; 5,14 (dd, J = 11 et 5 Hz, 1H : CH en 4α) ; 5.40 (d large, J = 5 Hz, 1H : CH en 5α) ; 5,46 (d, J = 17 Hz. 1H : l'autre H du CH₂ en 5ε) ; 5.60 (d, J = 8.5 Hz, 1H : CH en 6α) ; 5,88 (q large, J = 7 Hz, 1H : CH en 1β) ; 6,33 (d, J = 8 Hz, 2H : H aromatiques en 4ε) ; 6,55 (d, J = 9.5 Hz, 1H : CONH en 2) ; 6,86 (d, J = 8 Hz, 2H : H aromatiques en 4δ) ; de 7.20 à 7.40 (mt : les 5H aromatiques en 6α) ; 7,45 (mt, 2H ; 1' H₄ et 1' H₅) ; 7.89 (s. 1H : H aromatique en ?de 1'N) : 7.95 (s large. 1H : 1'H₆) ; 8,40 (d, J = 10 Hz, 1H : CONH en 1) : 8.66 (d, J = 8,5 Hz, 1H : CONH en 6); 11,64 (s, 1H : OH).

### Exemple 2

Dans un tricol contenant 200 cm3 d'éthanol, on introduit 20,8 g de 50-méthylène-pristinamycine I_{A}, 3,94 g de chlorhydrate de 3,3-diaminoacrylate d'éthyle et 3,3 cm3 de triéthylamine. Le mélange est porté 3 heures au reflux. Après refroidissement, le précipité formé est filtré, repris dans 100 cm3 d'eau et le pH ajusté à 8 par une solution de bicarbonate de sodium puis le produit est extrait par 2 fois 100 cm3 d'acétate d'éthyle. Les phases organiques sont rassemblées, séchées sur sulfate de magnésium, filtrées et concentrées à sec à 40°C sous pression réduite (2,7 kPa) pour donner 22 g d'un solide jaune qui est purifié par chromatographie sur 500 g de silice [éluant : dichlorométhane/méthanol 97,3/2,5 en volumes] pour donner un solide qui est dissous dans 20 cm3 de dichlorométhane puis précipité par addition de 60 cm3 d'éther diisopropylique. Après filtration et séchage à 40°C sous pression réduite (90 Pa) on obtient 1,35 g de 3"-éthoxycarbonyl-2"-amino-pyrido [2,3-5γ,5δ] pristinamycine IE sous forme d'un solide jaune fondant à 190°C.

Spectre de R.M.N. ¹H (400 MHz, CDCl₃, δ en ppm) : 0,86 (t, J = 7,5 Hz, 3H : CH₃ en 2γ) : de 1,15 à 1,30 (mt, 3H : 1H du CH₂ en 3β - 1H du CH₂ en 3γ et 1H du CH₂ en 5β) ; 1.26 (d, J = 7 Hz, 3H : CH₃ en 1γ) ; 1,35 (t, J = 7Hz, 3H : CH₃ de l'éthyle) ; 1.53 (mt, 1H : l'autre H du CH₂ en 3γ) ; 1.61 et 1.70 (2 mts, 1 H chacun : CH₂ en 2β) ; 2,00 (mt, 1H : l'autre H du CH₂ en 3β) ; de 2.75 à 2,95 (mt, 1H : l'autre H du CH₂ en 5β) : 2.84 (s, 6H : ArN(CH₃)₂) ; 2,90 (dd, J = 13 et 5 Hz, 1 H : 1H du CH₂ en 4β) : de 3.10 à 3.25 (mt, 2H : l'autre H du CH₂ en 4β et 1H du CH₂ en 3δ) ; 3,20 (s, 3H : NCH₃) : 3,45 (mt, 1H : l'autre H du CH₂ en 3δ) ; 3.74 (d, J = 17 Hz, 1H : 1H du CH₂ en 5ε) : 4,30 (mt, 2H : COOCH₂ de l'éthyle) : 4.55 (dd, J = 8 et Hz, 1H : CH en 3α) ; 4.77 (mt, 1H : CH en 2α) ; 4,86 (dd, J = 10 et 1,5 Hz, 1H : CH en 1α) ; 5,12 (dd, J = 11 et 5 Hz, 1H : CH en 4α) ; 5,28 (2 d, respectivement J = 6 Hz et J = 17Hz, 1H chacun : CH en 5α et l'autre H du CH₂ en 5ε) ; 5,58 (d, J = 8,5 Hz, 1 H : CH en 6α) ; 5.83 (dq. J = 7 et 1.5 Hz, 1H : CH en 1β) : de 6.00 à 6.50 (mf étalé, 2H : ArNH₂) ; 6,36 (d, J = 8 Hz, 2H : H aromatiques en 4ε) : 6.61 (d, J = 9.5 Hz. 1H : CONH en 2) : 6.84 (d, J = 8 Hz. 2H : H aromatiques en 4δ) : de 7.1 5 a 7.35 (mt : les 5H aromatiques en 6α) : 7.40 (mt. 2H : 1' H₂ et 1' H₅) : 7.78 (s, 1H : H aromatique en γ de l'N) ; 7.89 (d large. J = 4 Hz. 1H : 1' H₆) ; 8,40 (d, J = 10 Hz, 1H : CONH en 1) : 8,60 (d, J = 8,5 Hz, 1H : CONH en 6) ; 11,61 (mf, 1 H : OH).

Le chlorhydrate de 3,3-diaminoacrylate d'éthyle peut être préparé selon H. Meyer et coll., Liebigs Ann. Chem., 1895-1908 (1977).

### Exemple 3

En opérant comme à l'exemple 1 mais à partir de 50 cm3 de méthanol, de 3 de 5δ-méthylène pristinamycine I_{A} et de 0,65 g de 3-amino crotonate de benzyle et chauffage à reflux 3b heures, on obtient après refroidissement du mélange réactionnel à température ambiante et ajout de 50 cm3 d'eau distillée un précipité qui est filtré sur verre fritté puis lavé successivement par 50 cm3 d'eau distillée et 25 cm3 d'éther diisopropylique. Le solide obtenu est dissous à chaud dans 25 cm3 de méthanol et après refroidissement les cristaux formés sont filtrés, lavés par 10 cm3 de méthanol, séchés à 40°C (90 Pa) pour donner 1,2 g de 3"-benzyloxycarbonyl-2"-méthyl-pyrido [2,3-5γ,5δ) pristinamycine IE sous forme d' un solide jaune pâle fondant à 250°C.

Spectre de R.M.N. ¹H (400 MHz. CDCl₃, δ en ppm) : 0,93 (t, J = 7,5 Hz. 3H : CH₃ en 2γ) ; de 1.10 à 1,40 (mt, 2H : 1H du CH₂ en 3β et 1H du CH₂ en 3γ) : 1.30 (d, J = 7 Hz, 3H : CH₃ en 1γ): 1,50 (dd, J = 17 et 5 Hz, 1H : 1H du CH₂ en 5β) ; 1,58 (mt, 1H : l'autre H du CH₂ en 3γ) ; 1,67 et 1.75 (2 mts, 1H chacun : CH₂ en 2β) ; 2,06 (mt, 1H : l'autre H du CH₂ en 3β) ; 2,78 (s, 3H : ArCH₃) ; 2.85 (s, 6H : ArN(CH₃)₂) ; 2,95 (mt, 1H : 1H du CH₂ en 4β) ; 3,10 (d, J = 17 Hz, 1H : l'autre H du CH₂ en 5β) ; de 3,15 à 3.30 (mt, 2H : l'autre H du CH₂ en 4β et 1H du CH₂ en 3δ) ; 3,26 (s, 3H : NCH₃) : 3.50 (mt, 1H : l'autre H du CH₂ en 3δ) ; 3,90 (d, J = 17 Hz, 1H : 1H du CH₂ en 5ε) : 4.62 (dd, J = 8 et 6.5 Hz, 1H : CH en 3α) ; 4.81 (mt, 1H : CH en 2α) ; 4.90 (d large, J = 10 Hz, 1H : CH en 1α) ; 5.15 (dd, J = 11 et 5 Hz, 1H : CH en 4α) ; 5,37 (s. 2H : COOCH₂Ar) ; 5,40 (d, J = 5 Hz, 1H : CH en 5α) ; 5,45 (d, J = 17 Hz, 1H : l'autre H du CH₂ en 5ε) ; 5,61 (d, J = 8.5 Hz, 1H : CH en 6α) ; 5.88 (q large, J = 7 Hz. 1H : CH en 1β) : 6.33 (d, J = 8 Hz, 2H : H aromatiques en 4ε) ; 6.58 (d, J = 9.5 Hz. 1H : CONH en 2) : 6.87 (d. J = 8 Hz, 2H : H aromatiques en 4δ) : de 7.20 à 7.50 (mt : les 12H correspondant aux 5H aromatiques en 6α - au 1' H₄ - au 1' H₅, et aux H aromatiques du benzyloxycarbonyl) ; 7,92 (s, 1H : H aromatique en γ de l'N) ; 7,95 (mt, 1H : 1' H₆) ; 8,41 (d, J = 10 Hz, 1H : CONH en 1) ; 8,68 (d, J = 8,5 Hz, 1H : CONH en 6) ; 11,66 (s, 1H : OH).

Dans un tricol contenant 50 cm3 de méthanol, on introduit sous courant d'azote 1,95 g de 3" -benzyloxycarbonyl-2''-méthyl-pyrido [2,3-5y,5δ] pristinamycine IE puis 1,6 g d'hydroxyde de palladium à 20 % sur charbon et 2 cm3 de 1,4-cyclohexadiène. Le mélange est chauffé à 60°C pendant 30 minutes puis refroidi à température ambiante. Le catalyseur est filtré sur papier filtre Whatman et le filtrat concentré à 45°C sous pression réduite (2,7 kPa) de façon à obtenir un volume final de 5 cm3. On ajoute alors 100 cm3 d'éther diisopropylique et le précipité formé est filtré, lavé par 25 cm3 d'éther diisopropylique puis séché à 40°C sous pression réduite (90 Pa) pour donner 0,95 g de 3"-carboxy-2"-méthyl-pyrido [2,3-5γ,5δ] pristinamycine IE sous forme d'un solide crème fondant à 234°C.

Spectre de R.M.N. ¹H (400 MHz. CDCl₃, δ en ppm) : 0,92 (t, J = 7,5 Hz. 3H : CH₃ en 2₇) : de 1,15 à 1.35 (mt, 2H : 1H du CH₂ en 3β et 1H du CH₂ en 3γ) : 1,32 (d, J = 7 Hz, 3H : CH₃ en 1₇) ; 1,39 (mt, 1 H : 1H du CH₂ en 5β) ; 1,60 (mt, 1H : l'autre H du CH₂ en 3γ) ; 1.71 et 1,80 (2 mts, 1 H chacun : CH₂ en 2β) ; 2.05 (mt, 1H : 1H du CH₂ en 3β) ; 2,67 (s, 3H : ArCH₃) ; 2,78 (s, 6H : ArN(CH₃)₂) ; 2,92 (mt, 1H : 1H du CH₂ en 4β) ; 3,05 (d très large, J = 16 Hz, 1H : l'autre H du CH₂ en 5β) : de 3,15 à 3,35 (mt, 1H : l'autre H du CH₂ en 4β) ; 3,25 (s, 3H : NCH₃) ; 3,48 (mt, 1H : 1H du CH₂ en 3δ) ; 3.57 (mt, 1H : l'autre H du CH₂ en 3δ) ; 4,01 (d, J = 17 Hz, 1H : 1H du CH₂ en 5ε) ; 4,60 (mt, 1H : CH en 3α) : 4.88 (mt, 1H : CH en 2α) : 4,94 (d large, J = 10 Hz, 1H : CH en 1α) ; 5,12 (mt, 1H : CH en 4α) ; 5,40 (mf, 1H : CH en 5α) ; 5.43 (d, J = 17Hz, 1 H : l'autre H du CH₂ en 5ε) ; 5,69 (d, J = 8,5 Hz, 1 H : CH en 6α) 5,88 (q large, J = 7 Hz, 1H : CH en 1β); 6,29 (d, J = 8 Hz, 2H : H aromatiques en 4ε) ; 6,85 (d, J = 8 Hz, 2H : H aromatiques en 4δ) ; 7,13 (d large, 1H : CONH en 2) ; de 7,20 à 7.45 (mt : les 7H correspondant aux 5H aromatiques en 6α - au 1' H₄ et au 1' H₅) : 7.79 (s large. 1H : H aromatique en γ de 1'N) 7,92 (s large. 1H : 1' H₆) : 8.34 (d, J = 10 Hz, 1H : CONH en 1) : 8.65 (d. J = 8,5 Hz, 1H : CONH en 6) : 11.61 (s. 1H : OH).

Le 3-aminocrotonate de benzyle peut être préparé comme décrit par J. Daxoll, J. Chem. Soc., 3802-3808 (1953).

### Exemple 4

En opérant comme à l'exemple 1 mais à partir de 150 cm3 de méthanol, de 20 g de 5δ-méthylène pristinamycine I_{B} et de 0,26 g de 3-amino crotonate de méthyle et après 6 heures de reflux, on obtient 20 g d'un produit jaune qui est purifié par deux chromatographies sucessives sur respectivement 1 kg et 200 g de silice [éluant : dichlorométhane/méthanol 98/2 en volumes], pour donner après séchage à 40°C, sous pression réduite (90 Pa), 13,4 g de 3''-méthoxycarbonyl-2"-méthyl-pyrido [2.3-5γ.5δ] (4ζ-méthylamino) (4ζ-désdiméthylamino) pristinamycine IE sous forme d'un solide jaune fondant à 208°C.

Spectre de R.M.N. ¹H (400 MHz, CDCl₃, δ en ppm) : 0,92 (t, J = 7,5 Hz, 3H : CH₃ en 2γ) : de 1,15 à 1,35 (mt, 2H : 1H du CH₂ en 3β et 1H du CH₂ en 3γ) ; 1,29 (d, J = 7 Hz, 3H : CH₃ en 1γ) : de 1,55 à 1,80 (mt : les 3H correspondant à l'autre H du CH₂ en 3γ et au CH₂ en 2β) ; 1.57 (dd, J = 16 et 5,5 Hz, 1 H : 1H du CH₂ en 5β) ; 2,03 (mt, 1H : l'autre H du CH₂ en 3β) ; 2,67 (s. 3H : ArCH₃) 2,76 (s, 6H : ArNCH₃) : 2,91 (dd. J = 13 et 5 Hz, 1H : 1H du CH₂ en 4β) ; de 3.10 à 3,30 (mt, 2H : l'autre H du CH₂ en 4β et 1H du CH₂ en 3δ) ; 3,13 (d, J = 16 Hz, 1H : l'autre H du CH₂ en 5β); 3.22 (s, 3H : NCH₃) ; 3,49 (mt, 1H : l'autre H du CH₂ en 3δ) ; 3,88 (d, J = 17 Hz, 1H : 1H du CH₂ en 5ε) : 3,92 (s, 3H : COOCH₃) 4,60 (dd, J = 8 et 5.5 Hz, 1H : CH en 3α) ; 4,78 (mt, 1H : CH en 2α) ; 4,87 (d large, J = 10 Hz, 1H : CH en 1α) ; 5,12 (dd, J = 11 et 5 Hz, 1H : CH en 4α) ; 5,38 (d, J = 5,5 Hz, 1H : CH en 5α) ; 5,44 (d, J = 17 Hz, 1H : l'autre H du CH₂ en 5ε) ; 5,61 (d. J = 8,5 Hz, 1H : CH en 6α) ; 5,87 (q large. J = 7 Hz, 1H : CH en 1β) ; 6.18 (d, J = 8 Hz, 2H : H aromatiques en 4ε) ; 6,52 (d large, 1H : CONH en 2) ; 6.79 (d, J = 8 Hz, 2H : H aromatiques en 4δ) ; de 7,15 à 7,35 (mt : les 5H aromatiques en 6α) : 7,42 (mt, 2H : 1' H₄ et 1' H₅) 7,88 (s, 1 H : H aromatique en γ de 1'N) ; 7.92 (mt, 1H : 1' H₆) ; 8,39 (d. J = 10 Hz, 1H : CONH en 1) ; 8,64 (d. J = 8.5 Hz. 1 H : CONH en 6) ; 11.62 (s, 1 H : OH).

### Exemple 5

Dans un tricol contenant 100 cm3 de méthanol, on introduit 3,4 g de 5δ-méthylène pristinamycine I_{A}, 1 g de bromure de 3,3-diméthyl 2-oxo 1-butyl pyridinium puis 3 g d'acétate d'ammonium. Le mélange est porté 3 heures au reflux puis concentré à sec à 40°C sous pression réduite (2.7 kPa). On ajoute alors 100 cm3 d'eau distillée puis le mélange est extrait par 2 fois 100 cm3 d'acétate d'éthyle. Les phases organiques sont décantées, rassemblées, séchées sur sulfate de sodium, filtrées puis concentrées à sec à 40°C sous pression réduite (2,7 kPa) pour donner 3,6 g d'un solide orangé qui est purifié par deux chromatographies successives sur 40 g de silice (éluant : dichlorométhane/méthanol 95/5 en volumes) pour donner un produit qui est repris dans 60 cm3 d'un mélange éther-éther de pétrole. Après filtration et séchage à 40°C sous pression réduite (90 Pa) on obtient 0.64 g de 2"-tert-butyl-pyrido [2,3-5γ,5δ] pristinamycine IE, sous forme d'un solide crème fondant à 196°C.

Spectre de R.M.N. ¹H (400 MHz. (CDCl₃, δ en ppm) : 0,91 (t, J = 7,5 Hz, 3H : CH₃ en 2γ) , de 1,15 à 1,40 (ml. 2H : 1H du CH₂ en 3β et 1H du CH₂) en 3γ); 1.31 (d,J=7 Hz, 3H : CH₃ en 1γ) ; 1,32 (s. 9H : ArC(CH₃)₃); 1,60 (mt, 1H : l'autre H du CH₂ en 3γ) : 1,66 et 1.75 (2 mts : les 2H correspondant au CH₂ en 2β) ; 1,98 (dd. J = 16 et 5.5 Hz, 1H : 1H du CH₂ en 5β) ; 2.0: (mt. 1H : l'autre H du CH₂ en 3β) ; 2.86 (s. 6H : ArN(CH₃)₂); 3.01 (dd, J = 14 et 6.5 Hz, 1H : 1H du CH₂ en 4β) ; de 3,10 à 3.40 (mt, 3H : l'autre H du CH₂ en 4β - l'autre H du CH₂ en 5β et 1H du CH₂ en 3δ) : 3.18 (s. 3H : NCH₃) 3,51 (mt, 1H: l'autre H du CH₂ en 3δ) ; 3,94 (d, J = 17 Hz, 1 H : 1 H du CH₂ en 5ε); 4,58 (t, J = 7,5 Hz, 1 H : CH en 3α) ; 4,81 (mt, 1H : CH en 2α) : 4,90 (d large, J = 10 Hz, 1H : CH en 1α) : de 5.35 à 5.50 (mt, 3H : CH en 4α - l'autre H du CH₂ en 5ε et CH en 5α); 5,65 (d. J = 8.5 Hz, 1H : CH en 6α) ; 5,88 (q large, J = 7 Hz, 1H : CH en 1β) 6.43 (d, J = 8 Hz. 2H : H aromatiques en 4ε) ; 6.75 (d, J = 10 Hz, 1H : CONH en 2) : 6.87 (d, J = 8 Hz, 2H : H aromatiques en 4δ) . 7.12 (d. J = 8 Hz, 1 H : H aromatique en β de 1'N) : de 7.25 à 7,45 (mt : les 8H correspondant aux 5H aromatiques en 6α - à 1'H aromatique en de 1'N - au 1' H₄ et au 1' H₅) ; 7.87 (mt. 1H : 1' H₆) ; 8,49 (d, J = 10 Hz, 1H : CONH en 1); 8,73 (d, J = 8,5 Hz; 1H : CONH en 6) ; 11,70 (s, 1H : OH).

Le bromure de 3,3-diméthyl 2-oxo 1-butyl pyridinium peut être préparé comme décrit par F. Kroencke, Chem. Ber., 69, 921-923 (1936).

### Exemple 6

Dans un tricol contenant 2 litres d'acétone, on introduit 404 g de 5δ-méthylène pristinamycine I_{A}, 78.8 g de chlorure de 1-acétonyl pyridinium puis 354 g d'acétate d'ammonium. Le mélange est porté 1 heure au reflux puis concentré à sec à 40°C sous pression réduite (2.7 kPa). On ajoute alors 10 litres d'eau distillée puis le mélange est extrait par 500 cm3 de dichlorométhane puis par 3 litres d'acétate d'éthyle. Les phases organiques sont décantées, rassemblées, séchées sur sulfate de sodium, filtrées puis concentrées à sec à 40°C sous pression réduite (2,7 kPa) pour donner 205 g d'un solide orangé qui est purifié par chromatographie sur 1 kg de silice (éluant : dichlorométhane/méthanol 98/2 en volumes) pour donner 64,7 g d'un produit qui est repris dans 60 cm3 d'oxyde de diisopropyle puis recristallisé 2 fois dans 100 cm3 de méthanol. Après filtration et séchage à 40°C sous pression réduite (90 Pa), on obtient 23,3 g d'un produit qui est le 2"-méthyl-pyrido [2,3-5γ,5δ] pristinamycine IE sous forme d'un solide jaune fondant à 253°C.

Spectre de R.M.N. ¹H (400 MHz, CDCl₃, δ en ppm) : 0,94 (t, J = 7,5 Hz, 3H : CH₃ en 2γ) : de 1,20 à 1.35 (mt, 2H : 1H du CH₂ en 3β et 1H du CH₂ en 3γ) ; 1.31 (d, J = 7 Hz, 3H : CH₃ en 1γ) : 1,59 (mt, 1H : l'autre H du CH₂ en 3γ) ; de 1,60 à 1,85 (mt : les 2H correspondant au CH₂ en 2β) ; 1,69 (dd, J = 16 et 6 Hz, 1 H : 1 H du CH₂ en 5β) ; 2,06 (mt. 1 H : l'autre H du CH₂ en 3β) ; 2,48 (s, 3H : ArCH₃) 2.86 (s, 6H : ArN(CH₃)₂) ; 2,98 (dd, J = 13,5 et 5,5 Hz, 1 H : 1 H du CH₂ en 4β) ; 3.15 (d. J = 16 Hz, 1H : l'autre H du CH₂ en 5β) ; de 3,15 à 3,30 (mt, 2H : l'autre H du CH₂ en 4β et 1 H du CH₂ en 3δ) : 3,24 (s, 3H : NCH₃) 3,50 (mt, 1H : l'autre H du CH₂ en 3δ) ; 3.92 (d. J = 17 Hz. 1 H : 1 H du CH₂ en -5ε) : 4.61 (dd. J = 8 et 5,5 Hz, 1 H : CH en 3α): 4.81 (mt. 1H : CH en 2α) ; 4,90 (d large, J = 10 Hz. 1H : CH en 1α) : 5.23 (dd, J = 10 et 5,5 Hz, 1H : CH en 4α) ; 5,42 (respectivement d et d large, J = 17 Hz et J = 5,5 Hz, 1H chacun : l'autre H du CH₂ en 5e et CH en 5α) ; 5,63 (d, J = 8,5 Hz, 1H : CH en 6α) ; 5,90 (q large, J = 7 Hz, 1H : CH en 1β) ; 6,36 (d, J = 8 Hz, 2H : H aromatiques en 4ε) ; 6,61 (d, J = 9,5 Hz, 1H : CONH en 2) ; 6,87 (d, J = 8 Hz. 2H : H aromatiques en 4δ) ; 6,96 (d, J = 8 Hz, 1H : H aromatique en β de l'N) ; de 7,20 à 7,40 (mt : les 5H aromatiques en 6α) ; 7,34 (d, J = 8 Hz, 1H : H aromatique en γ de l'N) ; 7,41 (AB limite, 2H : 1' H₄ et 1' H₅) ; 7,92 (mt, 1H : 1' H₆) ; 8.44 (d, J = 10 Hz, 1H : CONH en 1) ; 8,65 (d, J = 8,5 Hz, 1H : CONH en 6) ; 11,66 (s, 1H : OH).

Le chlorure de 1-acétonylpyridinium peut être préparé selon H. Dreser, Arch. Pharm., 232., 183 (1894).

### Exemple 7

En opérant comme à l'exemple 6 mais à partir de 50 g de 5δ-méthylène pristinamycine I_{A} dans 1 litre d'acétone, de 13,7 g de bromure de 1-(2-oxo-butyl) pyridinium, de 44 g d'acétate d'ammonium et chauffage 1 heure au reflux puis ajout de 2,6 g de bromure de 1-(2-oxo-butyl) pyridinium et une heure de reflux supplémentaire on obtient après purification par chromatographie sur 200 g de silice (éluant: dichlorométhane/méthanol 97/3 en volumes), 19,5 g d'un produit qui peut être purifié par cristallisation de la manière suivante. 8 g de ce solide sont dissous à chaud dans un mélange de 30 cm3 de méthanol et de 1 cm3 d'eau distillée. Après refroidissement les cristaux obtenus sont collectés pour donner 3,9 g d'un solide qui est recristallisé dans des conditions analogues. Après filtration et séchage à 40°C sous pression réduite (90 Pa) on obtient 1,7 g de 2"-éthyl-pyrido [2.3-5γ,5δ] pristinamycine IE sous forme d'un solide blanc fondant à 263°C.

Spectre de R.M.N. ¹H (400 MHz, CDCl₃, δ en ppm) : 0,89 (t, J = 7,5 Hz, 3H : CH₃ en 2γ) ; de 1,15 à 1,35 (mt, 2H : 1H du CH₂ en 3β et 1H du CH₂ en 3γ) ; 1,22 (t. J = 7,5 Hz. 3H : CH₃ de l'éthyle) ; 1.28 (d, J = 7 Hz. 3H : CH₃ en 1γ) ; 1.53 (mt, 1 H : l'autre H du CH₂ en 3γ): de 1.60 à 1.80 (mt : les 2H correspondant au CH₂ en 2β) : 1.76 (dd. J = 16 et 5.5 Hz. 1H: 1H du CH₂ en 5β) : 2.00 (mt. 1H: l'autre H du CH₂ en 3β) : 2,72 (q, J = 7,5 Hz, 2H : ArCH₂ de l'éthyle) ; 2,82 (s, 6H : ArN(CH₃)₂ ; 2,94 (dd, J = 13,5 et 5,5 Hz, 1H : 1H du CH₂ en 4β); de 3,10 à 3,25 (mt, 3H : l'autre H du CH₂ en 4β - l'autre H du CH₂ en 5β et 1H du CH₂ en 3δ) ; 3,18 (s, 3H : NCH₃) ; 3,46 (mt, 1H : l'autre H du CH₂ en 3δ) ; 3,90 (d, J = 17 Hz, 1H : 1H du CH₂ en 5ε) ; 4,57 (dd, J = 8 et 5,5 Hz, 1H : CH en 3α) ; 4,75 (mt, 1H : CH en 2α) ; 4,84 (d large, J = 10 Hz, 1H : CH en 1α) ; 5,21 (dd, J = 9 et 5,5 Hz, 1H : CH en 4α) ; 5,38 (d, J = 17 Hz, 1H : l'autre H du CH₂ en 5ε); 5,39 (d large, J = 5,5 Hz, 1 H : CH en 5α) ; 5,60 (d, J = 8,5 Hz, 1H : CH en 6α) ; 5,85 (q large, J = 7 Hz, 1H : CH en 1β) : 6,32 (d, J = 8 Hz, 2H : H aromatiques en 4ε) ; 6,53 (d, J = 9,5 Hz, 1H : CONH en 2) ; 6,82 (d, J = 8 Hz, 2H : H aromatiques en 4δ) : 6,93 (d, J = 8 Hz, 1H : H aromatique en β de 1'N) ; de 7,25 à 7,40 (mt : les 5H aromatiques en 6α) ; 7,29 (d, J = 8 Hz, 1H : H aromatique en γ de 1'N) ; 7,33 (mt, 2H : 1' H₄ et 1' H₅) ; 7,85 (mt, 1H : 1' H₆) : 8,39 (d, J = 10 Hz, 1H : CONH en 1) ; 8,63 (d, J = 8,5 Hz, 1H : CONH en 6) ; 11.62 (s, 1H : OH).

Le bromure de 1-(2-oxo-butyl) pyridinium peut être préparé par analogie avec l'iodure de 1-(2-oxo-butyl) pyridinium comme décrit par R.P. Soni, J. P. Saxena. J. Indian Chem. Soc., 58. 885-887 (1981).

Dans un tricol contenant 150 cm3 d'éthanol on introduit 1g de 1-bromo-2-butanone et 40 cm3 de pyridine et le mélange est chauffé 2 heures au reflux. Après concentration à sec à 40°C sous pression réduite (2,7 kPa), le résidu est repris dans 100 cm3 d'éther diéthylique. Après filtration, lavage par 2 fois 70 cm3 d'éther diéthylique, le précipité est séché pour donner 22 g d'un solide jaune fondant à 181°C.

Spectre de R.M.N. ¹H (250 MHz, (CD₃)₂SO d6, δ en ppm) : 1,06 (t, J = 7 Hz. 3H : CH₃ de l'éthyle) ; 2.70 (q, J = 7 Hz, 2H : COCH₂ de l'éthyle) ; 5.83 (s, 2H : NCH₂CO) ; 8,25 (dd, J = 8 et 5 Hz, 2H : H aromatiques en β de la pyridine) ; 8,69 (t, J = 8 Hz, 2H : H aromatique en γ de la pyridine) : 8,91 (d, J = 5 Hz, 2H : H aromatiques en α de la pyridine).

### Exemple 8

En opérant comme à l'exemple 5 mais à partir de 9,8 g de 5δ-méthylène pristinamycine I_{A} dans 500 cm3 de méthanol, de 2,7 g de bromure de 1-cyclopropylcarbonylméthyl pyridinium, de 8,6 g d'acétate d'ammonium et chauffage 40 minutes au reflux, on obtient après purification par chromatographie sur 150 g de silice (éluant: dichlorométhane/méthanol 97/3 en volumes), 1,1 g de produit qui peut être recristallisé dans 1 cm3 de méthanol bouillant. Après refroidissement les cristaux obtenus sont filtrés puis rincés par 5 cm3 de méthanol pour donner 0,47 g de 2"-cyclopropyl-pyrido [2,3-5γ,5δ] pristinamycine IE sous forme de cristaux blancs fondant à 198°C.

Spectre de R.M.N. ¹H (400 MHz, CDCl₃, δ en ppm) : de 0,80 à 1,00 (mt, 4H : les 2 CH₂ du cyclopropyle) ; 0,91 (t, J = 7,5 Hz, 3H : CH₃ en 2γ) ; de 1,15 à 1,35 (mt, 2H : 1H du CH₂ en 3β et 1H du CH₂ en 3γ) ; 1,30 (d, J = 7 Hz, 3H : CH₃ en 1γ) : de 1,55 à 1,80 (mt : les 2H correspondant au CH₂ en 2β) ; 1.57 (mt, 1H : l'autre H du CH₂ en 3γ) ; 1,68 (dd, J = 16 et 6,5 Hz. 1H : 1H du CH₂ en 5β) ; 1.96 (mt. 1H : ArCH du cyclopropyle) ; 2,04 (mt, 1H : l'autre H du CH₂ en 3β) : 2,86 (s. 6H : ArN(CH₃)₂) ; 2,96 (dd. J = 13 et 6 Hz, 1H : 1H du CH₂ en 4β); 3,10 (d, J = 16 Hz, 1H : l'autre H du CH₂ en 5β) ; de 3,10 à 3,30 (mt, 2H : l'autre H du CH₂ en 4β et 1H du CH₂ en 3δ) ; 3,22 (s, 3H : NCH₃) ; 3,49 (mt, 1 H : l'autre H du CH₂ en 3δ) ; 3.90 (d, J = 17 Hz, 1 H : 1H du CH₂ en 5ε) ; 4,60 (dd, J = 8 et 6 Hz, 1H : CH en 3α) ; 4.79 (mt. 1H : CH en 2α) ; 4,88 (d large, J = 10 Hz, 1H CH en 1α); 5,23 (dd, J = 10 et 6 Hz, 1H : CH en 4α) ; 5,36 (d large, J = 6,5 Hz, 1H : CH en 5α) ; 5,38 (d, J = 17 Hz, 1H : l'autre H du CH₂ en 5ε) ; 5,62 (d. J = 8,5 Hz, 1H : CH en 6α) : 5,88 (q large, J = 7 Hz, 1 H : CH en 1β) ; 6,34 (d, J = 8 Hz, 2H : H aromatiques en 4ε) ; 6.58 (d, J = 9,5 Hz, 1H : CONH en 2) ; de 6,75 à 6.90 (mt, 3H : H aromatiques en 4δ et H aromatique en β de 1'N) ; 7.08 (d. J = 8 Hz, 1H : H aromatique en γ de 1'N) ; de 7,20 à 7,35 (mt : les 5H aromatiques en 6α) : 7,40 (AB limite. 2H : 1' H₄ et 1' H₅) ; 7,91 (mt, 1 H : 1' H₆) ; 8,43 (d, J= 10 Hz. 1H : CONH en 1) : 8.63(d, J=8.5Hz. 1H : CONH en 6) : 11.65 (s. 1H : OH).

Le bromure de 1-cyclopropylcarbonylméthyl pyridinium peut être préparé de la manière suivante :

Dans un tricol contenant 40 cm3 d'éthanol on introduit 2,4 g de 1-bromométhylcyclopropylcétone et 5.8 cm3 de pyridine puis le mélange est chauffé 2 heures à reflux. Après concentration à sec à 40°C sous pression réduite (2,7 kPa), le résidu est repris dans deux fois 30 cm3 d'éther diéthylique. Après filtration, lavage à l'éther diéthylique, le précipité est séché sous pression réduite (90 Pa) pour donner 3,4 g de bromure de 1-cyclopropylcarbonylméthyl pyridinium sous forme d'un solide crème fondant à 160°C.

Spectre de R.M.N. ¹H (300 MHz., (CD₃)₂SO d6, δ en ppm) : 1,08 et 1,16 (2 mts, 2H chacun : les 2 CH₂ du cyclopropane) : 2.34 (mt, 1H : COCH du cyclopropane) ; 6,06 (s, 2H : NCH₂CO) ; 8.24 (dd, J = 8 et 5 Hz, 2H : H aromatiques en β de la pyridine) ; 8,70 (t, J = 8 Hz, 2H : H aromatique en γ de la pyridine) ; 8,96 (d, J = 5 Hz, 2H : H aromatiques en α de la pyridine).

La bromométhylcyclopropylcétone peut être préparée selon V.K. Jinaraj et coll., Ind. J. Chem., Sect. B, 22, 841-45 (1983).

### Exemple 9

En opérant comme à l'exemple 5 mais à partir de 10 g de 5δ-méthylène pristinamycine I_{A} dans 300 cm3 de méthanol, de 2,2 g de bromure de 1-cyanométhyl pyridinium, de 8,5 g d'acétate d'ammomum et chauffage 3 heures au reflux, on obtient après purification par chromatographie sur 70 g de silice (éluant : dichlorométhane méthanol 90/10 en volumes) un produit qui est repurifié 3 fois par la même méthode en changeant la nature de l'éluant (dichlorométhane/méthanol 95/5, puis dichlorométhane/méthanol 97/3 puis dichlorométhane/méthanol 95/5) pour donner 0,16 g de 2"-amino-pyrido [2.3-5γ,5δ] pristinamycine IE sous forme d'un solide blanc fondant à 222°C.

Spectre de R.M.N. ¹H (400 MHz, CDCl₃, δ en ppm) : 0,90 (t, J = 7,5 Hz, 3H : CH₃ en 2γ) ; de 1,15 à 1,35 (mt, 2H : 1H du CH₂ en 3β et 1H du CH₂ en 3γ) ; 1,28 (d, J = 7 Hz, 3H : CH₃ en 1γ) ; de 1,45 à 1,80 (mt, les 4H correspondant à l'autre H du CH₂ en 3γ - à 1H du CH₂ en 5β et au CH₂ en 2β) ; 2,01 (mt, 1 H : l'autre H du CH₂ en 3β) ; de 2,80 à 3,00 (mt, 2H : l'autre H du CH₂ en 5β et 1H du CH₂ en 4β) ; 2,89 (s, 6H : ArN(CH₃)₂) ; de 3,10 à 3,25 (mt, 2H : l'autre H du CH₂ en 4β et 1H du CH₂ en 3δ) ; 3,20 (s, 3H : NCH₃) ; 3,45 (mt, 1 H : l'autre H du CH₂ en 3δ) ; 3.80 (d, J = 17 Hz, 1H : 1H du CH₂ en 5ε) ; 4,27 (mf, 2H: ArNH₂) ; 4,57 (dd, J = 8,5 et 5,5 Hz, 1H : CH en 3α) ; 4,76 (mt, 1H : CH en 2α) ; 4,86 (dd, J = 10 et 1,5 Hz, 1H : CH en 1α); 5.18 (dd, J = 10 et 6 Hz, 1H : CH en 4α) ; 5,27 (d, J = 17 Hz, 1H : l'autre H du CH₂ en 5ε) ; 5,32 (d large, J = 5,5 Hz, 1H : CH en 5α) ; 5,60 (d, J = 8,5 Hz, 1H : CH en 6α) : 5.86 (q large, J = 7 Hz, 1H : CH en 1β); 6,30 (d, J = 8 Hz, 1H : H aromatique β de l'N) ; 6,37 (d, J = 8 Hz, 2H : H aromatiques en 4ε) ; 6,54 (d, J = 9,5 Hz, 1H : CONH en 2) ; 6,83 (d, J = 8 Hz, 2H : H aromatiques en 4δ) : 7,09 (d, J = 8 Hz, 1H: H aromatique en γ de l'N) ; de 7,15 à 7,40 (mt : les 7H correspondant aux 5H aromatiques en 6α - au 1' H₄ et au 1'H₅) ; 7.84 (dd, J = 4 et 1,5 Hz, 1H : 1'H₆) ; 8,38 (d, J = 10 Hz, 1H : CONH en 1) ; 8,55 (d, J = 8,5 Hz, 1H : CONH en 6) ; 11,61 (s, 1H : OH).

### Exemple 10

En opérant comme à l'exemple 5 mais à partir de 30 g de 5δ-méthylène pristinamycine I_{A} dans 300 cm3 de méthanol, de 7,1 g de chlorure de 1-(3-chloro-2-oxo-propyl) pyridinium (à 50 %), de 26 g d'acétate d'ammonium et chauffage 10 minutes au reflux, on obtient après 2 chromatographies successives sur 400 g de silice (éluant : dichlorométhane/méthanol 98/2 en volumes) 1,4 g d'un produit qui est purifié par CLHP sur silice C₈ 10 µm (éluant : eau : acétonitrile : 70/30 en volumes contenant 0,1 % d'acide trifluoroacétique). Les fractions sont rassemblées, l'acétonitrile éliminé à 40°C sous pression réduite (2,7 kPa) et la phase aqueuse ajustée à pH 7 par 3 cm3 d'eau saturée en bicarbonate de sodium. La phase aqueuse est lavée par 2 fois 60 cm3 de dichlorométhane. Les phases organiques sont réunies, séchées sur sulfate de sodium, filtrées puis concentrées à sec sous pression réduite (2,7 kPa) pour donner 0,4 g d'un solide jaune qui est concrété dans- 60 cm3 d'un mélange éther diéthylique-éther de pétrole, filtré puis séché sous pression réduite (90 Pa). On obtient ainsi, 0,3 g de 2"-chlorométhyl-pyrido [2,3-5γ,5δ] pristinamycine IE sous forme d'un solide crème fondant à 194°C.

Spectre de R.M.N. ¹H (400 MHz, CDCl₃, δ en ppm) : 0.92 (t, J = 7,5 Hz, 3H : CH₃ en 2γ) ; de 1,15 à 1,35 (mt, 2H : 1H du CH₂ en 3β et 1H du CH₂ en 3γ) ; 1,30 (d, J = 7 Hz, 3H : CH₃ en 1γ) 1,57 (mt, 1H : l'autre H du CH₂ en 3γ) ; de 1,60 à 1.80 (mt : les 2H correspondant au CH₂ en 2β) ; 1,63 (dd, J = 16 et 6 Hz, 1H : 1H du CH₂ en 5β) ; 2,03 (mt, 1H : l'autre H du CH₂ en 3β) ; 2,85 (s, 6H : ArN(CH₃)₂) ; 2,95 (dd, J = 13 et 5,5 Hz, 1H : 1H du CH₂ en 4β) ; 3,12 (d, J = 16 Hz, 1H : l'autre H du CH₂ en 5β) ; de 3,15 à 3,30 (mt, 2H : l'autre H du CH₂ en 4β et 1H du CH₂ en 3δ) ; 3,23 (s, 3H : NCH₃) ; 3,49 (mt, 1H : l'autre H du CH₂ en 3δ) ; 3,93 (d, J = 17 Hz, 1H : 1H du CH₂ en 5ε) ; 4,58 (AB limite, J = 14 Hz, 2H : ArCH₂Cl) ; de 4,55 à 4,75 (mt, 1H : CH en 3α) ; 4,79 (mt, 1H : CH en 2α) ; 4,88 (d large, J = 10 Hz, 1H : CH en 1α) ; 5.18 (dd, J = 10,5 et 5,5 Hz, 1 H : CH en 4α) ; 5,40 (d large, J = 6 Hz, 1 H : CH en 5α) : 5,46 (d. J = 17 Hz, 1 H : l'autre H du CH₂ en 5ε) ; 5,60 (d, J = 8,5 Hz, 1H : CH en 6α) ; 5,87 (q large, J = 7 Hz : 1H : CH en 1β) : 6,36 (d, J = 8 Hz, 2H : H aromatiques en 4ε) ; 6.55 (d, J = 9,5 Hz, 1 H : CONH en 2) ; 6.86 (d, J = 8 Hz, 2H : H aromatiques en 4δ) : 7,22 (d, J = 8 Hz, 1H : H aromatique en β de 1'N) ; de 7,25 à 7,40 (mt : les 5H aromatiques en 6α) : 7,38 (d, J = 8 Hz, 1 H : H aromatique en γ de 1'N) ; 7,42 (mt, 2H: 1' H₄ et 1' H₅) 7,89 (mt, 1H : 1'H₆). 8,40 (d, J = 10 Hz, 1 H : CONH en 1); 8,68 (d, J = 8,5 Hz, 1 H : CONH en 6) ; 11,64 (s, 1 H : OH).

Le chlorure de 1-(3-chloro-2-oxo-propyl) pyridinium peut être préparé de la manière suivante :

Dans un tricol contenant 800 cm3 d'éther diéthylique on introduit 66,9 g de chlorure de 1,3-dichloroacétone. On ajoute goutte à goutte 28 cm3 de pyridine et on laisse sous agitation la nuit. Le précipité obtenu est filtré, lavé par 2 fois 100 cm3 d'éther diéthylique, puis séche à 40°C sous 90 Pa pour donner 29.2 g de chlorure de 1-(3-chloro-2-oxo-propyl) pyridinium, sous forme de solide crème fondant à 92°C et utilisé tel quel.

### Exemple 11

En opérant comme à l'exemple 6, mais en partant de 36,5 g de 5δ-méthylène pristinamycine I_{A} dans 350 cm3 de méthanol, de 9,6 g de chlorure de 1-(3-acétoxy-2 oxo-propyl) pyridinium, de 32,2 g d'acétate d'ammonium et chauffage 40 minutes au reflux, on obtient un solide qui est chromatographié sur 350 g de silice (éluant : gradiant dichlorométhane/méthanol 100/0 puis 99/1 puis 98/2 puis 96/4 en volumes) pour donner 1,3 g d'un solide jaune. Celui-ci est purifié par CLHP sur silice C₈ 10 µm (éluant : eau/acétonitrile 70/30 en volumes, contenant 0,1 % d'acide trifluoroacétique). Les fractions sont rassemblées, l'acétonitrile éliminé à 40°C, sous pression réduite (2,7 kPa) et le pH de la phase aqueuse ajustée à 7 par addition d'eau saturée en bicarbonate de sodium. La phase aqueuse est extraite par 3 fois 200 cm3 de dichlorométhane. Les phases organiques sont réunies, séchées sur sulfate de magnésium, filtrées et concentrées, à 40°C, sous pression réduite (2,7 kPa), pour donner 0,5 g de 2"-hydroxyméthy!-pyrido [2,3-5γ,5δ] pristinamycine IE sous forme d'un solide blanc fondant à 190°C.

Spectre de R.M.N. ¹H (400 MHz, CDCl₃, δ en ppm) : 0,92 (t, J = 7,5 Hz, 3H : CH₃ en 2γ) ; de 1,20 à 1.35 (mt, 2H : 1H du CH₂ en 3β et 1H du CH₂ en 3γ) ; 1,31 (d, J = 7 Hz. 3H : CH₃ en 1γ) : 1,50(dd,J= 16 et 6 Hz, 1H : 1H du CH₂ en 5β) ; de 1,50 à 1,70 (mt : les 2H correspondant à 1 H du CH₂ en 2β et l'autre H du CH₂ en 3γ) ; 1,75 (mt. 1H : l'autre H du CH₂ en 2β) ; 2,05 (mt, 1 H : l'autre H du CH₂ en 3β) ; 2.82 (s, 6H : ArN(CH₃)₂) ; 2,93 (dd, J = 12 et 5 Hz, 1H : 1H du CH₂ en 4β) ; 3,11 (d, J = 16 Hz, 1H : l'autre H du CH₂ en 5β) ; de 3,15 à 3,30 (mt, 2H : l'autre H du CH₂ en 4β et 1H du CH₂ en 3δ) ; 3,25 (s, 3H : NCH₃) ; 3,48 (mt, 1H : l'autre H du CH₂ en 3δ) ; 3,91 (d. J = 17 Hz, 1H : 1H du CH₂ en 5ε) : 3,94 (mf, 1H : OH) ; 4,61 (dd, J = 8 et 5,5 Hz, 1H : CH en 3α ; 4.67 (s large, 2H : ArCH₂O) . 4.80 (mt. 1H : CH en 2α); 4,89 (d large, J = 10 Hz 1H : CH en 1α) ; 5,14 (dd. J = 12 et 5 Hz, 1H : CH en 4α); 5.37 (d large, J = 6 Hz, 1H : CH en 5α) ; 5,44 (d, J = 17 Hz, 1H : l'autre H du CH₂ en 5ε) ; 5,60 (d, J = 8,5 Hz, 1H :

### Exemple 12

En opérant comme à l'exemple 5 mais à partir de 6 g de 5δ-méthylène pristinamycine I_{A} dans 100 cm3 de méthanol, de 1,9 g de bromure de 1-phénacyl pyridinium, de 5,3 g d'acétate d'ammonium et chauffage 30 minutes au reflux, on obtient après purification par chromatographie sur 90 g de silice [éluant : dichlorométhane/méthanol 95/5 en volumes] un solide qui est repris dans 60 cm3 d'un mélange éther-éther de pétrole. Après filtration et séchage à 40°C sous pression réduite (90 Pa), on obtient 0,8 g de 2"-phényl-pyrido [2,3-5γ,5δ] pristinamycine IE sous forme d'un solide jaune fondant à 212°C.

Spectre de R.M.N. ¹H (400 MHz, CDCl₃, δ en ppm) : 0,93 (t, J = 7,5 Hz, 3H : CH₃ en 2γ) : de 1,20 à 1,40 (mt, 2H : 1H du CH₂ en 3β et 1H du CH₂ en 3γ) ; 1,32 (d, J = 7 Hz, 3H : CH₃ en 1γ) : de 1,50 à 1,85 (mt : les 4H correspondant à 1H du CH₂ en 5β - à l'autre H du CH₂ en 3γ et au CH₂ en 2β) ; 2,06 (mt, 1H : l'autre 1H du CH₂ en 3β) : 2,70 (s, 6H : ArN(CH₃)₂) ; 2,98 (dd, J = 13 et 5,5 Hz, 1H : 1H du CH₂ en 4β) : de 3, 15 à 3,35 (mt, 3H : l'autre H du CH₂ en 5β - l'autre H du CH₂ en 4β et 1H du CH₂ en 3δ): 3,26 (s, 3H : NCH₃) : 3,50 (mt, 1H : l'autre H du CH₂ en 3δ) : 4,00 (d, J = 17 Hz, 1H : 1H du CH₂ en 5ε) ; 4.64 (dd. J = 8 et 5,5 Hz, 1H : CH en 3α) ; 4,82 (mt, 1H : CH en 2α) ; 4,89 (d large, J = 10 Hz, 1H : CH en 1α) ; 5,23 (dd, J = 11 et 5,5 Hz, 1H : CH en 4a) ; 5,46 (d large, J = 5,5 Hz, 1H : CH en 5α) ; 5,50 (d, J = 17 Hz, 1H : l'autre H du CH₂ en 5ε) ; 5,66 (d, J = 8,5 Hz, 1H : CH en 6α) ; 5,90 (q large, J = 7 Hz, 1H : CH en 1β) ; 6,34 (d, J = 8 Hz, 2H : H aromatiques en 4ε) ; 6,60 (d, J = 10 Hz, 1H : CONH en 2) ; 6,88 (d, J = 8 Hz, 2H : H aromatiques en 4δ) ; de 7,25 à 7,50 (mt : les 11H correspondant aux 5H aromatiques en 6α - à 1' H aromatique en γ de 1'N - à 1' H aromatiques en para du phényl - aux H aromatiques en méta du phényl - au 1' H₄ et au 1' H₅) ; 7.56 (d, J = 8 Hz. 1H : H aromatique en β de 1'N) ; 8.00 (mt, 3H : 1' H₆ et H aromatiques en ortho du phényl : 8.45 (d, J = 10 Hz. 1H : CONH en 1) ; 8.58 (d, J = 8.5 Hz. 1H : CONH en 6) : 11.66 s. 1H : OH).

Le bromure de 1-phénacyl pyridinium peut être préparé selon F. Kroencke and H. Timmler, Chem. Ber., 69, 614 (1936).

### Exemple 13

En opérant comme à l'exemple 5 mais à partir de 29,6 g de 5δ-méthylène pristinamycine I_{A} dans 200 cm3 de méthanol, de 10,9 g de bromure de 1-(4-nitrophénacyl) pyridinium et de 26 g d'acétate d'ammonium et chauffage 40 minutes au reflux, on obtient après purification par chromatographie sur 500 g de silice [éluant : dichlorométhane/méthanol 95/5 en volumes] un solide qui est repris dans 60 cm3 d'un mélange éther-éther de pétrole. Après filtration et séchage à 40°C sous pression réduite (90 Pa) on obtient 16 g de 2"-(4-nitrophényl)-pyrido [2,3-5γ,5δ] pristinamycine IE sous forme d'un solide orange fondant à 345°C.

Spectre de R.M.N. ¹H (400 MHz. CDCl₃, δ en ppm) : 0,94 (t, J = 7,5 Hz, 3H : CH₃ en 2γ) ; de 1,20 à 1,40 (mt, 2H : 1H du CH₂ en 3β et 1H du CH₂ en 3γ) ; 1,31 (d, J = 7 Hz, 3H : CH₃ en 1γ) ; de 1,55 à 1.85 (ml : les 4H correspondant à l'autre H du CH₂ en 3γ - à 1H du CH₂ en 5β et au CH₂ en 2β) ; 2,08 (mt, 1H : l'autre H du CH₂ en 3β) ; 2,68 (s, 6H : ArN(CH₃)₂) ; 2,96 (dd, j = 13 et 5 Hz, 1H : 1H du CH₂ en 4β) : de 3,15 à 3,35 (mt, 3H : l'autre H du CH₂ en 5β - l'autre H du CH₂ en 4β et 1H du CH₂ en 3δ) ; 3,26 (s, 3H : NCH₃) ; 3,61(mt, 1H ; l'autre H du CH₂ en 3δ) ; 3,99 (d, J = 17 Hz. 1H : 1H du CH₂ en 5ε) ; 4,64 (dd. J = 7 et 6 Hz, 1H : CH en 3α) ; 4,81 (mt, 1H : CH en 2α); 4,90 (d large, J = 10 Hz, 1H CH en 1α) ; 5,17 (dd, J = 11,5 et 5 Hz, 1H : CH en 4α) ; 5,44 (d large, J = 5 Hz, 1H : CH en 5α) ; 5.53 (d, J = 17 Hz, 1H : l'autre H du CH₂ en 5ε) ; 5,63 (d, J = 8,5 Hz. 1H : CH en 6α) ; 5,88 (q large, J = 7 Hz. 1H : CH en 1β) ; 6,32 (d, J = 8 Hz, 2H : H aromatiques en 4ε) ; 6,58 (d, J = 9,5 Hz. 1H : CONH en 2) ; 6,88 (d, J = 8 Hz, 2H : H aromatiques en 4δ) ; de 7,20 à 7,40 (mt : les 5H aromatiques en 6α) ;de 7,45 à 7,55 (mt. 2H : 1' H₄ et 1' H₅) ; 7,49 (d, J = 8 Hz, 1H : H aromatique en γ de 1'N) ; 7.64 (d, J = 8 Hz, 1H : H aromatique en β de 1'N) ; 7.90 (d large. J = 4 Hz, 1H : 1' H₆) ; 8,20 et 8.31 (2 d, J = 8,5 Hz, 2H chacun : respectivement les H aromatiques en méta du NO₂ et les H aromatiques en ortho du NO₂) : 8.42 (d. J = 10 Hz, 1H : CONH en 1) ; 8,70 (d, J = 8,5 Hz, 1H : CONH en 6) ; 11,66 (s, 1H : OH).

Dans un tricol contenant 90 cm3 d' éthanol et 20 cm3 d'eau distillée, on introduit 9.1g de 2"-(4-nitrophényl)-pyrido [2,3-5γ,5δ] pristinamycine IE puis 50 g de fer en poudre et 1 cm3 d'acide chlorhydrique concentré et on porte au reflux 30 minutes. L'insoluble est éliminé par filtration, lavé par 60 cm3 d'éthanol puis le filtrat est concentré à sec à 40°C sous pression réduite (2,7 kPa). Le résidu obtenu est repris dans 300 cm3 d'eau, le pH amené à 8 par addition de bicarbonate de sodium et la phase aqueuse extraite par 2 fois 100 cm3 de dichlorométhane. Après séchage sur sulfate de sodium, filtration et concentration à sec sous pression réduite on obtient 1 1,5 g d'un solide marron qui est purifié par chromatographie sur 120 g de silice [éluant : dichlorométhane/méthanol 95/5 en volumes]. Le solide obtenu est concrété dans 60 cm3 d'un mélange éther-éther de pétrole, filtré et séché à 40°C sous pression réduite (90 Pa) pour donner 1,5 g de 2"-(4-aminophényl)-pyrido [2,3-5γ,5δ] pristinamycine IE d'un solide jaune fondant à 226°C.

Spectre de R.M.N. ¹H (400 MHz. CDCl₃, δ en ppm) : 0,93 (t, J = 7,5 Hz. 3H : CH₃ en 2γ) ; de 1,15 à 1,40 (mt, 2H : 1H du CH₂ en 3β et 1H du CH₂ en 3γ) ; 1,30 (d, J = 7 Hz, 3H : CH₃ en 1γ) ; de 1,50 à 1,85 (mt : les 4H correspondant à l'autre H du CH₂ en 3γ - à 1H du CH₂ en 5β et au CH₂ en 2β) ; 2.04 (mt, 1H : l'autre H du CH₂ en 3β) ; 2,73 (s, 6H : ArN(CH₃)₂) ; 2,96 (dd, J = 13 et 4,5 Hz, 1H : 1H du CH₂ en 4β) ; de 3,15 à 3,35 (mt, 3H : l'autre H du CH₂ en 5β - l'autre H du CH₂ en 4β et 1 H du CH₂ en 3δ); 3.24 (s, 3H : NCH₃) ; 3.50 (mt, 1H : l'autre H du CH₂ en 3δ) ; 3,80 (mf, 2H : NH₂) ; 3,97 (d, J = 17 Hz, 1H : 1H du CH₂ en 5ε); 4,63 (mt, 1H : CH en 3α) ; 4,81 (mt, 1H : CH en 2α) ; 4,90 (d large, J = 10 Hz, 1H : CH en 1α) ; 5,21 (dd, J = 10 et 4.5 Hz, 1H : CH en 4α) ; 5,42 (mt, 1H : CH en 5α) ; 5,45 (d, J = 17 Hz, 1 H : l'autre H du CH₂ en 5ε) ; 5,64 (d, J = 8.5 Hz, 1H : CH en 6α) ; 5,89 (mt, 1H : CH en 1β) ; 6.33 (d, J = 8 Hz. 2H : H aromatiques en 4ε) ; 6,58 (d, J = 9.5 Hz. 1 H : CONH en 2) ; 6.74 (d, J = 8 Hz. 2H : H aromatiques en ortho du NH₂) ; 6.88 (d. J = 8 Hz. 2H : H aromatiques en 4δ) de 7.20 à 7.50 (mt : les 9H correspondant aux 5H aromatiques en 6aα - au 1'H₄ - au 1' H₅ - à 1'H aromatique en γ de 1'N et à 1'H aromatique en β de 1'N) : 7.82 (d. J = 8 Hz, 2H : H aromatiques en méta du NH₂) ; 7,98 (mf, 1H : 1' H₆) ; 8,44 (d, J = 10Hz, 1H:CONH en 1) ; 8,64 (d, J = 8,5 Hz, 1H : CONH en 6) ; 11,66 (s, 1H : OH).

### Exemple 14'

En opérant comme à l'exemple 5 mais à partir de 10 g de 5δ-méthylène pristinamycine I_{A} dans 100 cm3 de méthanol, de 4 g de bromure de 1-(4-diéthylaminophénacyl) pyridinium et de 9 g d'acétate d'ammonium et chauffage 40 minutes au reflux, on obtient après purification par chromatographie sur 150 g de silice [éluant : dichlorométhane/méthanol 95/5 en volumes] un solide qui est repris dans 60 cm3 d'un mélange éther-éther de pétrole. Après filtration et séchage à 40°C sous pression réduite (90 Pa) on obtient 2,4 g de 2"-(4-diéthylaminophényl)-pyrido [2,3-5γ,5δ] pristinamycine IE sous forme d'un solide jaune fondant à 210°C.

Spectre de R.M.N. ¹H (400 MHz, CDCl₃, δ en ppm) : 0,93 (t, J = 7,5 Hz, 3H : CH₃ en 2γ) ; de 1,15 à 1,40 (mt, 2H : 1H du CH₂ en 3β et 1H du CH₂ en 3γ) ; 1,22 (t. J = 7 Hz, 6H : les 2 CH₃ du diéthylamino); 1,31 (d, J = 7 Hz, 3H : CH₃ en 1γ) ; 1,58 (mt, 1H : l'autre H du CH₂ en 3γ) ; de 1,60 à 1,85 (mt : les 3H correspondant à 1H du CH₂ en 5β et au CH₂ en 2β) ; 2,05 (mt, 1H : l'autre H du CH₂ en 3β) ; 2,75 (s, 6H : ArN(CH₃)₂) ; 2,98 (dd, J = 13 et 5,5 Hz, 1H : 1H du CH₂ en 4β) ; de 3,15 à 3,35 (mt, 3H : l'autre H du CH₂ en 5β - l'autre H du CH₂ en 4β et 1H du CH₂ en 3δ) ; 3,24 (s, 3H : NCH₃) ; 3,42 (mt, 4H : les 2 NCH₂ du diéthylamino) ; 3,50 (mt, 1H : l'autre H du CH₂ en 3δ) ; 3,97 (d, J = 17 Hz, 1H : 1H du CH₂ en 5ε) ; 4,62 (dd, J = 8 et 5,5 Hz, 1H : CH en 3α) ; 4,81 (mt, 1H : CH en 2α) ; 4,90 (d large, J = 10 Hz, 1H : CH en 1α) ; 5,24 (dd, J = 10 et 5,5 Hz. 1H : CH en 4α) ; 5,43 (2 d, respectivement J = 6 Hz et J = 17 Hz, 2H : CH en 5α et l'autre H du CH₂ en 5ε) ; 5,66 (d. J = 8,5 Hz, 1H : CH en 6α) ; 5,89 (q large, J = 7 Hz, 1H : CH en 1β) ; 6,34 (d. J = 8Hz, 2H : H aromatiques en 4ε) ; 6.60 (d. J = 9.5 Hz. 1H : CONH en 2) ; 6,72 (d, J = 8 Hz, 2H : H aromatiques en ortho du diéthylamino) ; 6.87 (d, J = 8 Hz, 2H : H aromatiques en 4δ) ; de 7,20 à 7.40 (mt : les 6H correspondant aux 5H aromatiques en 6α et à 1'H aromatique en γ de 1'N) de 7.40 à 7.50 (mt. 3H : 1' H₄ - 1' H₅ et H aromatique en β de 1'N) : 7.85 (d, J = 8 Hz, 2H : H aromatiques en méta du diethylamino) : 7.98 (mt. 1H : 1' H₅) : 8.44 (d, J = 10 Hz, 1 H : CONH en 1); 8,63 (d, J = 8,5 Hz, 1 H : CONH en 6); 11,67 (s, 1H : OH).

Le bromure de 1-(4-diéthylaminophénacyl) pyridinium peut être préparé de la manière suivante :

Dans un tricol contenant 200 cm3 de tétrahydrofurane on introduit 10 g de bromure de 4-diéthylaminophénacyle puis on ajoute goutte à goutte 15 cm3 de pyridine. L'agitation est poursuivie 90 heures puis le précipité formé est filtré puis lavé par 60 cm3 d'éther diéthylique. Après séchage à 40°C sous pression réduite (90 Pa), on obtient 14,1 g de bromure de 4-diéthylamino phénylpyridinium sous forme d'un solide blanc fondant > 260°C.

Spectre de R.M.N. ¹H (300 MHz. (CD₃)₂SO d6, δ en ppm) : 1,18 (t, J = 7 Hz, 6H : les 2 CH₃ du diéthylamino) ; 3,50 (q, J = 7 Hz, 4H : les 2 NCH₂ du diéthylamino) ; 6,39 (s, 2H : NCH₂COAr) ; 6,84 (d, J = 8 Hz, 2H : H aromatiques en ortho du diéthylamino) ; 7,88 (d, J = 8 Hz, 2H : H aromatiques en méta du diéthylamino) ; 8,28 (dd, J = 8 et 5 Hz, 2H : H aromatiques en β de la pyridine) ; 8,74 (t, J = 8 Hz, 2H : H aromatique en γ de la pyridine) : 9,02 (d, J = 5 Hz, 2H : H aromatiques en α de la pyridine).

### Exemple 15

En opérant comme à l'exemple 5 mais a partir de 5 g de 5δ-méthylène pristinamycine I_{A} dans 75 cm3 de méthanol, de 2.05 g de bromhydrate de bromure de 1-[2-oxo-2-(2-pyridyl)éthyl] pyridinium et de 4,3 g d'acétate d'ammonium et chauffage 3 heures au reflux, on obtient un solide qui est purifié par CLHP préparative sur 400 g de silice Kromasil® C₈ 10 µm [éluant : eau acétonitrile 70/30 en volumes contenant 0.1 % d'acide trifluoroacétique]. Après concentration des fractions pour éliminer l'acétonitrile, la phase aqueuse est neutralisée à pH 7-8 par une solution de bicarbonate de sodium à 10 %. Le précipité obtenu lors de la neutralisation est filtré, repris par 25 cm3 de dichlorométhane sec puis la phase organique séchée sur sulfate de sodium, filtrée et concentrée a sec sous pression réduite pour donner un solide qui est repris dans 10 cm3 d'éther diisopropylique. Après filtration et séchage à 40°C sous pression réduite (90 Pa) on obtient 0,94 g de 2"-(2-pyridyl)-pyrido [2,3-5γ,5δ] pristinamycine IE sous forme d'un solide beige 1,38 g fondant à 190°C.

Spectre de R.M.N. ¹H (400 MHz, CDCl₃, δ en ppm) : 0,93(t, J = 7,5 Hz, 3H : CH₃ en 2γ) ; de 1,20 à 1,40 (mt, 2H : 1H du CH₂ en 3β et 1H du CH₂ en 3γ) ; 1,32 (d, J = 7 Hz, 3H : CH₃ en 1γ) ; 1,58 (mt, 1H : l'autre H du CH₂ en 3γ) ; de 1,60 à 1,85 (mt : les 2H correspondant au CH₂ en 2β) ; 1,66 (dd, J = 16 et 5 Hz, 1H : 1H du CH₂ en 5β) ; 2,07 (mt, 1H : l'autre H du CH₂ en 3β) ; 2,65 (s, 6H : ArN(CH₃)₂) ; 2,96 (dd, J = 13 et 5,5 Hz, 1H : 1H du CH₂ en 4β) ; de 3,15 à 3,35 (mt, 3H : l'autre H du CH₂ en 4β - l'autre H du CH₂ en 5β et 1H du CH₂ en 3δ) ; 3,26 (s, 3H : NCH₃) ; 3,51 (mt, 1H : l'autre H du CH₂ en 3δ) ; 4,00 (d, J = 17 Hz, 1H : 1H du CH₂ en 5ε) ; 4,64 (dd, J = 8 et 6,5 Hz, 1H : CH en 3α) ; 4,82 (mt, 1H : CH en 2α) ; 4,91 (d large, J = 10 Hz, 1H : CH en 1α) ; 5,19 (dd, J = 12 et 5,5 Hz, 1 H : CH en 4α) ; 5,44 (d large, J = 5 Hz, 1 H : CH en 5α) ; 5,52 (d, J = 17 Hz, 1 H : l'autre H du CH₂ en 5ε) ; 5,66 (d, J = 8,5 Hz, 1 H: CH en 6α) ; 5,90 (q large, J = 7 Hz, 1H : CH en 1β) ; 6,31 (d, J = 8 Hz, 2H : H aromatiques en 4ε) ; 6,58 (d, J = 9,5 Hz, 1H : CONH en 2) ; 6,88 (d, J = 8 Hz, 2H : H aromatiques en 4δ) ; de 7,25 à 7,40 (mt : les 6H correspondant aux 5 H aromatiques en 6α et au H₅ de la pyridine) ; de 7,40 à 7,55 (mt, 3H : H aromatique en γ de 1'N - 1' H₅ et 1' H₄) ; 7,78 (t dédoublé, J = 8 et 1,5 Hz, 1H : H₄ de la pyridine) ; 8,02 (d large, J = 4 Hz, 1H : 1' H₆) ; 8,23 (d, J = 8 Hz, 1H : H aromatique en β de 1'N) : 8,42 (mt, 2H : H₃ de la pyridine et CONH en 1) ; 8,66 (d, J = 8,5 Hz, 1H : CONH en 6) ; 8,68 (mt large, 1H : H₆ de la pyridine) ; 11,67 (s, 1H : OH).

Le bromhydrate de bromure de 1-[2-oxo-2-(2-pyridyl)éthyl] pyridinium peut être préparé par analogie avec F. Krönhke et coll., Synthesis, 1-24 (1976) :

Dans un tricol contenant 50 cm3 de tétrahydrofuranne on introduit 5 g de bromydrate de 2-bromoacétylpyridine et 7 cm3 de pyridine. L'agitation est laissée 2 jours à température ambiante puis le précipité formé est filtré, lavé par 30 cm3 de tétrahydrofuranne, puis séché à 40°C sous pression réduite (90 Pa) pour donner 6.9 g de bromhydrate de 1-[2-oxo-2-(2-pyridyl)éthyl] pyridinium sous forme d'un solide beige utilisé tel quel.

Le bromydrate de 2-bromoacétylpyridine peut être préparé comme décrit par J.L. Garcia Ruano et coll., Tetrahedron, 43, 4407-4416 (1987).

### Exemple 16

Dans un tricol contenant 75 cm3 de méthanol on introduit 5 g de 5δ-méthylène pristinamycine I_{A}. 2,1 g de bromhydrate de 1-[2-oxo-2-(3-pyridyl)éthyl] pyridinium et 4,4 d'acétate d'ammonium. Après 1 heure de reflux le mélange réactionnel est concentré de moitié puis jeté sur 200 cm3 d'eau distillée. Le précipité orange apparu est filtré pour donner 3,5 g d'un solide qui est purifié par chromatographie sur 50 g de silice [éluant : dichlorométhane/méthanol 97/3]. Après concentration des fractions on obtient 1 g d'un solide jaune qui est cristallisé dans 30 cm3 de méthanol. On obtient après filtration et séchage à 40°C sous pression réduite (90 Pa), 0,4 g de 2"-(3-pyridyl)-pyrido [2,3-5γ,5δ] pristinamycine IE sous forme d'un solide blanc fondant à 265°C.

Spectre de R.M.N. ¹H (400 MHz, CDCl₃, δ en ppm) : 0,92 (t. J = 7,5 Hz. 3H : CH₃ en 2γ) : de 1,20 à 1,40 (mt, 2H : 1H du CH₂ en 3β et 1H du CH₂ en 3γ) ; 1,31 (d, J = 7 Hz, 3H : CH₃ en 1γ) ; 1,58 (mt, 1H : l'autre H du CH₂ en 3γ) ; de 1,50 à 1,85 (mt : les 3H correspondant au CH₂ en 2β et à 1H du CH₂ en 5β) ; 2,05 (mt, 1H : l'autre H du CH₂ en 3β) ; 2,68 (s, 6H : ArN(CH₃)₂) ; 2,95 (dd, J = 13 et 5,5 Hz, 1H : 1 H du CH₂ en 4β) ; de 3,20 à 3,35 (mt, 3H : l'autre H du CH₂ en 4β - l'autre H du CH₂ en 5β et 1H du CH₂ en 3δ) ; 3,26 (s, 3H : NCH₃) ; 3,49 (mt, 1H : l'autre H du CH₂ en 3δ) ; 3.98 (d, J = 17 Hz, 1H : 1 H du CH₂ en 5ε) 4,61 (dd, J = 8 et 6 Hz, 1H : CH en 3α) ; 4,80 (mt, 1H : CH en 2α) ; 4,89 (d large, J = 10 Hz, 1H : CH en 1α); 5,17 (dd, J = 12 et 5,5 Hz, 1H : CH en 4α) ; 5,43 (d large, J = 5,5 Hz, 1H : CH en 5α) ; 5,49 (d, J = 17 Hz. 1H : l'autre H du CH₂ en 5ε) ; 5,63 (d, J = 8,5 Hz, 1H : CH en 6α) ; 5,88 (q large, J = 7 Hz: 1H : CH en 1β) : 6.30 (d. J = 8 Hz, 2H : H aromatiques en 4ε) : 6,55 (d, J = 9,5 Hz. 1H : CONH en 2) : 6.86 (d,J = 8 Hz, 2H: H aromatiques en 4δ) : de 7,25 à 7,40 (mt : les 6H correspondant aux 5 H aromatiques en 6α - au H₅ de la pyridine) ; de 7,40 à 7,55 (mt, 3H : H aromatique en γ de 1'N - 1' H₅ et 1'H₄) ; 7,58 (d, J = 8 Hz, 1 H : H aromatique en β de 1'N) ; 8,00 (dd, J = 4 et 1,5 Hz, 1 H : 1'H₆) ; 8,31 (dt, J = 8 et 1,5 Hz, 1 H : H₄ de la pyridine) ; 8,40 (d, J = 10 Hz, 1H : CONH en 1) ; 8,63 (dd, J = 5 et 1,5 Hz, 1H : H₆ de la pyridine) ; 8,67 (d, J = 8,5 Hz, 1 H : CONH en 6) ; 9,20 (d, J = 1,5 Hz, 1H : H₂ de la pyridine); 11,64 (s, 1H : OH).

Le bromhydrate de 1-[2-oxo-2-(3-pyridyl)éthyl] pyridinium peut être préparé selon F. Krönhke, Synthesis, 1-24 (1976).

### Exemple 17

Dans un tricol contenant 30 cm3 de dichlorométhane, on introduit 2 g de 2''-éthyl-pyrido [2,3-5γ,5δ] pristinamycine IE 0,17 cm3 d'éthylèneglycol. 2,2 cm3 d'acide acétique et 0,44 g de périodate de tétra n-butylammonium. Le mélange est agité 18 heures à température ambiante puis lavé par 3 fois 20 cm3 d'eau. La phase organique est décantée, séchée sur sulfate de magnésium, filtrée et concentrée à 45°C sous pression réduite (2.7 kPa). Le résidu obtenu est repris par 50 cm3 d'eau et 10 cm3 d'acide sulfurique 0,5N et agité 5 minutes. L'insoluble est éliminé par filtration et la phase aqueuse extraite par 3 fois 30 cm3 d'acétate d'éthyle. La phase aqueuse est ajustée à pH 8 environ avec une solution saturée de bicarbonate de sodium puis extraite par 3 fois 30 cm3 de dichlorométhane. Les phases chlorométhyléniques sont réunies, séchées sur sulfate de sodium, filtrées et concentrées à sec sous pression réduite pour donner 1,7 g de meringue beige qui est purifiée par chromatographie sur 50 g de silice [éluant : dichlorométhane/méthanol 97/3 en volumes]. On obtient ainsi après séchage à 40°C sous 90 Pa, 0.4 g de 2"-éthyl-pyrido [2,3-5γ,5δ] (4ζ-méthylamino) (4ζ-dèsdiméthytamino) pristinamycine IE sous forme de solide crème fondant à 194°C.

Spectre de R.M.N. ¹H (400 MHz. CDCl₃, δ en ppm) : 0.93 (t. J = 7.5 Hz, 3H : CH) en 2γ) : de 1.20 à 1.40 (mt. 2H : 1 H du CH₂ en 3β et 1H du CH₂ en 3γ) : 1,27 (t. J = 7.5 Hz, 3H : CH₃ de l'éthyle) : 1,32 (d. J = 7 Hz. 3H : CH₃ en 1γ) : 1.59 (ml. 1H : l'autre H du CH₂ en 3γ) ; de 1,60 à 1,85 (mt : les 2H correspondant au CH₂ en 2β) ; 1.81 (dd, J = 16 et 5,5 Hz, 1H : 1H du CH₂ en 5β) ; 2,06 (mt, 1H : l'autre H du CH₂ en 3β) ; 2,72 (s, 3H : ArNCH₃) ; 2,77 (q. J = 7,5 Hz, 2H : ArCH₂ de l'éthyle) ; 2,97 (dd, J = 13,5 et 5,5 Hz, 1 H : 1H du CH₂ en 4β) : de 3,15 à 3,30 (mt, 3H : l'autre H du CH₂ en 4β - l'autre H du CH₂ en 5β et 1H du CH₂ en 3δ) : 3,22 (s, 3H : NCH₃) ; 3,51 (mt, 1H: l'autre H du CH₂ en 3δ) ; 3,67 (mf, 1H : ArNH) : 3,93 (d, J = 17 Hz. 1H : 1H du CH₂ en 5ε) ; 4,61 (dd, J = 8 et 6 Hz, 1H : CH en 3α) ; 4,81 (mt. 1H : CH en 2α) ; 4,90 (d large, J = 10 Hz, 1H : CH en 1α) : 5,26 (dd, J = 10 et 5,5 Hz, 1H ; CH en 4α) ; 5,42 (respectivement d large et d. J = 5,5 Hz et J = 17 Hz, 1 H chacun : CH en 5α et l'autre H du CH₂ en 5ε) ; 5,65 (d, J = 8.5 Hz, 1H : CH en 6α) ; 5.90 (q large, J = 7 Hz, 1H : CH en 1β) ; 6,24 (d, J = 8 Hz, 2H : H aromatiques en 4ε) : 6,60 (d, J = 9.5 Hz, 1H : CONH en 2) ; 6,82 (d, J = 8 Hz, 2H : H aromatiques en 4δ) ; 6,99 (d, J = 8 Hz, 1H : H aromatique en β de 1'N) ; de 7,25 à 7,40 (mt : les 5H aromatiques en 6α) ; 7.33 (d, J = 8 Hz, 1H : H aromatique en γ de l'N) ; 7,40 (AB limite, 2H : 1' H₄ et 1' H₅) ; 7,92 (mt, 1H : l' H₆) ; 8,47 (d, J = 10 Hz, 1H : CONH en 1) ; 8,69 (d, J = 8,5 Hz, 1H : CONH en 6) ; 11,67 (s, 1H : OH).

### Exemple 18

En opérant comme à l'exemple 5 mais à partir de 10 g de 5δ-méthylène pristinamycine I_{B} dans 150 cm3 de méthanol, de 4,1 g de bromhydrate de 1-[2-oxo-2-(2-pyridyl)éthyl] pyridinium et 8,7 g d'acétate d'ammonium et chauffage 3 heures au reflux, on obtient 7,5 g d'un solide qui est purifié par CLHP préparative sur 400 g de silice Kromasil® C₈ 10 µm (éluant : eau-acétonitrile 70-30 en volumes contenant 0,1 % d'acide trifluoroacétique). Après concentration des fractions pour éliminer l'acétonitrile, la phase aqueuse est neutralisée à pH 7-8 par une solution de bicarbonate de sodium à 10 %. Le précipité obtenu lors de la neutralisation est filtré, repris par 50 cm3 de dichlorométhane sec puis la phase organique séchée sur sulfate de magnésium, filtrée et concentrée à sec sous pression réduite pour donner un solide qui est repris dans 50 cm3 d'éther diisopropylique. Après filtration et séchage à 40°C sous pression réduite (90 Pa), on obtient 1.12 g de 2"-(2-pyridyl)-pyrido [2,3-5γ,5δ] (4ξ-méthylamino) (4ξ-désdiméthylamino) pristinamycine IE sous forme d'un solide rosé fondant à 200°C.

Spectre de R.M.N. ¹H (400 MHz, CDCl₃, δ en ppm) : 0,93 (t, J = 7.5 Hz, 3H : CH₃ en 2γ) ; de 1,20 à 1,40 (mt, 2H : 1H du CH₂ en 3β et 1H du CH₂ en 3_{γ}) ; 1,32 (d, J = 7 Hz, 3H : CH₃ en 1γ) ; 1,58 (mt, 1H : l'autre H du CH₂ en 3γ) ; de 1,60 à 1,85 (mt, 2H : CH₂ en 2β) ; 1,70 (dd, J = 16 et 5,5 Hz, 1H : 1H du CH₂ en 5β) ; 2,06 (mt, 1H : l'autre H du CH₂ en 3β) ; 2,53 (s, 3H : ArNCH₃) ; 2,94 (dd, J = 13 et 5,5 Hz, 1H : 1H du CH₂ en 4β) ; de 3,15 à 3,30 (mt. 2H : l'autre H du CH₂ en 4β et 1H du CH₂ en 3δ) ; 3,25 (s, 3H : NCH₃) ; 3,29 (d, J = 16 Hz, 1H : l'autre H du CH₂ en 5β) ; 3,50 (mt, 1H : l'autre H du CH₂ en 3δ) ; 3,99 (d, J = 17 Hz, 1H : 1 H du CH₂ en 5ε): 4,62 (dd, J = 8 et 6 Hz, 1H : CH en 3α) ; 4,81 (mt, 1H : CH en 2α) ; 4,90 (d large, J = 10 Hz, 1H : CH en 1α) ; 5,16 (dd, J = 10,5 et 5,5 Hz, 1H : CH en 4α); 5,43 (d large, J = 5,5 Hz, 1H : CH en 5α) ; 5,52 (d, J = 17 Hz, 1H : l'autre H du CH₂ en 5ε) ; 5,67 (d, J = 8.5 Hz, 1 H : CH en 6α) ; 5,90 (q large, J = 7 Hz, 1H : CH en 1β) ; 6,15 (d, J = 8 Hz. 2H : H aromatiques en 4ε) ; 6,58 (d. J = 9.5 Hz. 1H : CONH en 2); 6.81 (d, J = 8 Hz. 2H : H aromatiques en 4δ) ; de 7.25 à 7.40 (mt ; les 6H correspondant aux 5 H aromatiques en 6α et au H₅ de la pyridine) : de 7.40 à 7.55 (mt, 3H : H aromatique en γ de 1'N - 1' H₅ et 1' H₄) ; 7,78 (t dédoublé. J = 8 et 1.5 Hz. 1H : H₄ de la pyridine) : 8.01 (d large, J = 4 Hz, 1H : 1' H₆) ; 8.20 (d. J = s Hz. 1H : H aromatique en β de 1'N) ; 8.38 (d. J = 8 Hz, 1H : H₃ de la pyridine) : 8.46 (d, J = 10 Hz, 1H : CONH en 1) ; 8,66 (d, J = 8,5 Hz, 1H : CONH en 6) : 8.70 (d large. J = 4 Hz, 1H : H₆ de la pyridine) : 11.68 (s. 1H : OH).

Le bromhydrate de 1-[2-oxo-2-(2-pyridyhéthyl] pyridinium peut être préparé selon Krönhke et coll., Synthesis, 1-24 (1976).

### Exemple 19

Dans un tricol contenant 60 cm3 de chlorure de méthylène, on introduit 15 g de 2"-méthyl-pyrido [2.3-5γ.5δ] pristinamycine IE. 1.25 cm3 d'éthylèneglycol, 16.4 cm3 d'acide acétique et 3.33 g de periodate de tétra n-butylammonium. Le mélange est agité 10 heures à température ambiante puis le mélange réactionnel est lavé par 2 fois 50 cm3 d'eau distillée. La phase organique est décantée puis concentrée à sec à 40°C sous pression réduite (2,7 kPa). Le résidu est repris par 100 cm3 d'eau et 200 cm3 d'acide sulfurique 0,5 N puis lavé par 5 fois 100 cm3 d'acétate d'éthyle. La phase aqueuse est décantée, ajustée à pH 7-8 par 200 cm3 d'une solution saturée de bicarbonate de sodium puis extraite par 2 fois 150 cm3 d'acétate d'éthyle. La phase organique est séchée sur sulfate de magnésium puis concentrée à sec (40°C-2,7 kPa) pour donner 32 g d'un solide qui est chromatographié sur 1 kg de silice [éluant : gradient dichlorométhane/méthanol 99/1 à 97.5/2,5]. Après concentration à sec des fractions puis cristallisation dans l'acétate d'éthyle, on obtient après séchage à 40°C sous pression réduite (90 Pa), 4,7 g de 2"-méthyl-pyrido [2,3-5γ,5δ] (4ζ-méthylamino) (4ζ-désdiméthylamino) pristinamycine IE sous forme de cristaux blancs fondant à 244°C.

Spectre de R.M.N. ¹H (400 MHz, CDCl₃, δ en ppm) : 0,92 (t, J = 7,5 Hz, 3H : CH₃ en 2γ) ; de 1,20 à 1,40 (mt, 2H : 1H du CH₂ en 3β et 1H du CH₂ en 3γ) ; 1,31 (d, J = 7 Hz. 3H : CH₃ en 1γ) : 1.57 (mt. 1H : l'autre H du CH₂ en 3γ) ; de 1.60 à 1,85 (mt, 2H : CH₂ en 2β) : 1,73 (dd. J = 16 et 6,5 Hz, 1 H : 1H du CH₂ en 5β) ; 2,05 (mt, 1H : l'autre H du CH₂ en 3β) ; 2.49 (s, 3H : ArCH₃) ; 2, 69 (s, 3H : ArNCH₃) ; 2.95 (dd, J = 13,5 et 5.5 Hz, 1H : 1H du CH₂ en 4β) ; de 3,15 à 3,30 (mt, 2H : l'autre H du CH₂ en 4β et 1H du CH₂ en 3δ); 3,16 (d, J = 16 Hz, 1H : l'autre H du CH₂ en 5β) ; 3,22 (s, 3H : NCH₃) ; 3.49 (mt, 1H : l'autre H du CH₂ en 3δ) ; 3.68 (mf, 1H : ArNH) : 3.91 (d, J = 17 Hz, 1H : 1H du CH₂ en 5ε) ; 4,60 (dd, J = 8 et 5,5 Hz, 1H : CH en 3α) : 4,79 (mt, 1H : CH en 2α) ; 4,88 (dd, J = 10 et 1,5 Hz, 1H : CH en 1α) ; 5,21 (dd, J = 10 et 5.5 Hz. 1H : CH en 4α) ; 5,40 (d, J = 17 Hz, 1H : l'autre H du CH₂ en 5ε) ; 5.41 (d large, J = 5,5 Hz, 1H : CH en 5α) ; 5,63 (d, J = 8,5 Hz, 1H ; CH en 6α) ; 5,88 (dq, J = 7 et 1,5 Hz, 1H : CH en 1β) ; 6,23 (d, J = 8 Hz, 2H : H aromatiques en 4ε) ; 6,58 (d, J = 9.5 Hz, 1H : CONH en 2) ; 6.81 (d, J = 8 Hz, 2H : H aromatiques en 4δ) ; 6,96 (d, J = 8 Hz. 1H : H aromatique en β de 1'N), de 7,20 à 7,40 (mt : les 5H aromatiques en 6α) : 7.33 (d. J = 8 Hz. 1 H : H aromatique en γ de l'N) : 7.40 (AB limite, 2H : l' H₄ et 1' H₅) : 7,91 (mt, 1 H : 1' H₆) ; 8,44 (d, J = 10 Hz, 1 H : CONH en 1) ; 8,65 (d, J = 8,5 Hz, 1H : CONH en 6); 11,65 (s, 1H : OH).

### Exemple 20

Dans un tricol contenant 30 cm3 de tétrahydrofuranne, on ajoute successivement 1,7 g de 2''-chlorométhyl-pyrido [2,3-5γ,5δ] pristinamycine IE et 0,6 cm3 de morpholine puis on porte le mélange au reflux. Après 18 heures, on ajoute 0,3 cm3 de morpholine supplémentaire et 0,3 cm3 de triéthylamine puis on poursuit le reflux 6 heures. Le mélange réactionnel est alors concentré à sec sous pression réduite à 40°C sous 2,7 kPa. Le résidu obtenu est repris par 2 fois 50 cm3 d'eau puis la phase aqueuse est extraite par 2 fois 50 cm3 de dichlorométhane. Les phases organiques sont rassemblées, séchées sur sulfate de sodium, filtrées puis concentrées à sec pour donner 1,3 g de produit qui est purifié par chromatographie sur 80 g de silice [éluant : en gradient dichlorométhane/méthanol de 98/2 à 97/3 en volumes] pour donner 0,3 g d'un solide qui est concrété en mélange avec 0,26 g du même produit provenant d'un autre essai, dans 60 cm3 d'un mélange éther-éther de pétrole (20/80 en volumes). Après filtration et séchage à 40°C sous pression réduite (90 Pa), on obtient 0,3 g de 2"-(N-morpholinométhyl)-pyrido [2,3-5γ,5δ] pristinamycine IE sous forme d'un solide jaune fondant à 189°C.

Spectre de R.M.N. ¹H (400 MHz, CDCl₃, δ en ppm) : 0,92 (t, J = 7,5 Hz, 3H : CH₃ en 2γ) ; de 1,15 à 1,40 (mt, 2H : 1H du CH₂ en 3β et 1H du CH₂ en 3γ) ; 1,29 (d, J = 7 Hz, 3H : CH₃ en 1γ) : de 1,50 à 1,70 (mt : les 2H correspondant à l'autre H du CH₂ en 3γ et à 1H du CH₂ en 2β) : 1,75 (mt, 1H : l'autre H du CH₂ en 2β) ; 1.87 (dd, J = 16 et 6 Hz, 1H : 1H du CH₂ en 5β) ; 2,03 (mt, 1H : l'autre H du CH₂ en 3β) ; 2,50 (mt, 4H : les 2 NCH₂ de la morpholine) ; 2,87 (s, 6H : ArN(CH₃)₂) ; 2.98 (dd. J = 13.5 et 6 Hz, 1H : 1H du CH₂ en 4β) : de 3,10 à 3,35 (mt, 3H : l'autre H du CH₂ en 5β - l'autre H du CH₂ en 4β et 1 H du CH₂ en 3δ) ; 3,22 (s. 3H : NCH₃) : 3,50 (mt, 1 H : l'autre H du CH₂ en 3δ) : 3.59 (s, 2H : ArCH₂N) : 3,74 (mt, 4H : les 2 OCH₂ de la morpholine) : 3.94 (d, J = 17 Hz. 1H : 1H du CH₂ en 5ε) : 4.60 (dd. J = 8 et 5 Hz. 1H : CH en 3α) ; 4.79 (mt, 1H : CH en 2α) ; 4.88 (d large. J = 10 Hz, 1H : CH en 1α) ; 5,29 (dd. J = 9 et 6 Hz, 1H : CH en 4α) ; 5,43 (mt, 1H : CH en 5α) ; 5,45 (d, J = 17 Hz; 1H : l'autre H du CH₂ en 5ε) ; 5,63 (d, J = 8,5 Hz, 1H : CH en 6α) ; 5,90 (q large, J = 7 Hz, 1H : CH en 1β) ; 6,36 (d, J = 8 Hz, 2H : H aromatiques en 4ε) ; 6,58 (d, J = 9,5 Hz, 1H : CONH en 2) ; 6,85 (d, J = 8 Hz, 2H : H aromatiques en 4δ) ; de 7,20 à 7,45 (mt : les 9H correspondant aux 5H aromatiques en 6α - à 1'H aromatique en β de 1'N - à 1'H aromatique en γ de 1'N - au 1' H₅ et au 1' H₄) ; 7,84 (d large, J = 4 Hz, 1H : 1' H₆) ; 8,43 (d, J = 10 Hz, 1H : CONH en 1) ; 8,70 (d, J = 8,5 Hz, 1H : CONH en 6) ; 11,66 (s, 1H : OH).

Le 2"-chlorométhyl-pyrido [2,3-5γ,5δ] pristinamycine IE peut être obtenu comme décrit à l'exemple 10.

### Exemple 21

En opérant comme à l'exemple 20 mais à partir de 50 cm3 de tétrahydrofuranne, de 3,2 g de 2"-chlorométhyl-pyrido [2,3-5γ,5δ] pristinamycine IE et de 1,1 cm3 de N-méthylpipérazine, on obtient après 2 heures de reflux 2,3 g d'un solide qui est purifié par deux chromatographies successives sur 100 g de silice [éluant : dichlorométhane/méthanol 95/5 en volumes] pour donner 0,4 g de 2"-(4-méthyl-1-pipérazinylméthyl)-pyrido [2,3-5γ,5δ] pristinamycine IE sous forme d'un solide jaune fondant à 221°C.

Spectre de R.M.N. ¹H (400 MHz, CDCl₃, δ en ppm) : 0,93 (t, J = 7,5 Hz, 3H : CH₃ en 2γ) ; de 1,20 à 1,40 (mt, 2H : 1H du CH₂ en 3β et 1H du CH₂ en 3γ) ; 1,31 (d, J = 7 Hz, 3H : CH₃ en 1γ) ; de 1,50 à 1,85 (mt : les 4H correspondant à l'autre H du CH₂ en 3γ - au CH₂ en 2β et à 1H du CH₂ en 5β) ; 2,03 (mt, 1H : l'autre H du CH₂ en 3β) ; 2,32 (s, 3H : NCH₃ de la pipérazine) ; de 2,40 à 2,70 (mt, 8H : les 4 NCH₂ de la pipérazine) ; 2,90 (s, 6H : ArN(CH₃)₂) ; 2,99 (dd, J = 13,5 et 6 Hz, 1H : 1H du CH₂ en 4β) ; de 3,15 à 3,35 (mt, 3H : l'autre H du CH₂ en 5β - l'autre H du CH₂ en 4β et 1H du CH₂ en 3δ) ; 3,22 (s, 3H : NCH₃) ; 3,50 (mt, 1H : l'autre H du CH₂ en 3δ) ; 3,62 (s, 2H : ArCH₂N) ; 3,95 (d, J = 17 Hz, 1H : 1H du CH₂ en 5ε) ; 4,60 (dd, J = 7,5 et 5,5 Hz, 1H : CH en 3α) ; 4,80 (mt, 1H : CH en 2α) ; 4,89 (d large, J = 10 Hz, 1H : CH en 1α) ; 5,28 (dd, J = 9 et 6 Hz, 1H : CH en 4α) ; 5,51 (mt, 1H : CH en 5α) ; 5,55 (d, J = 17 Hz, 1H : l'autre H du CH₂ en 5ε) ; 5,65 (d, J = 8,5 Hz, 1H : CH en 6α) ; 5,89 (q large, J = 7 Hz, 1H : CH en 1β) ; 6,37 (d, J = 8 Hz, 2H : H aromatiques en 4ε) ; 6,59 (d, J = 9,5 Hz, 1H : CONH en 2) ; 6,86 (d, J = 8 Hz, 2H : H aromatiques en 4δ) ; de 7,20 à 7,45 (mt : les 9H correspondant aux 5 H aromatiques en 6α - à 1'H aromatique en β de 1'N - à 1'H aromatique en g de 1'N - au 1'H₅ et au 1' H₄) ; 7,87 (d large, J = 4 Hz, 1 H : 1' H₆) ; 8,43 (d, J = 10 Hz, 1 H : CONH en 1) ; 8,70 (d, J = 8,5 Hz, 1 H : CONH en 6) ; 11,64 (mf, 1H : OH).

### Exemple 22

Dans un tricol contenant 30 cm3 de diméthylformamide, on introduit 4,6 g de 5δ-diméthylaminométhylène pristinamycine I_{A}, 1,1 g d'hydrogénosulfate de O-méthylisourée et 1,75 g de bicarbonate de sodium. Le mélange est chauffé à 65°C pendant 18 heures. Après refroidissement on ajoute 100 cm3 d'eau distillée et le produit est extrait par 3 fois 100 cm3 d'acétate d'éthyle. Les phases organiques sont rassemblées, lavées par 200 cm3 de saumure, séchées sur sulfate de magnésium, filtrées et concentrées à sec à 40°C sous pression réduite (2,7 kPa) pour donner 5,05 g d'une huile jaune qui est purifiée par chromatographie sur 90 g de silice [éluant : dichlorométhane/méthanol 97/3 en volumes] pour donner 1,2 g d'un solide. Le solide obtenu est purifié par CLHP sur 450 g de silice C₈ 10 µm (éluant : tampon phosphate pH 2,9 / acétonitrile : 60/40 en volumes). Les fractions sont rassemblées, l'acétonitrile éliminé à 40°C sous pression réduite (2,7 kPa) et la phase aqueuse ajustée à pH 7 par de l'eau saturée en bicarbonate de sodium puis extraite au dichlorométhane. La phase organique est décantée, séchée sur sulfate de magnésium, filtrée et concentrée à 40°C sous pression réduite (2,7 kPa) pour donner un solide qui est trituré dans 10 cm3 d'éther diisopropylique. Après filtration et séchage à 40°C (90 Pa) on obtient 0,40 g de 2"-méthoxy-pyrimido [4,5-5γ,5δ] pristinamycine IE sous forme d'un solide blanc fondant à 195-198°C.

Spectre de R.M.N ¹H (400 MHz, CDCl₃, δ en ppm) : 0,91 (t, J = 7,5 Hz, 3H : CH₃ en 2γ) ; de 1,20 à 1,35 (mt, 3H : 1H du CH₂ en 3β - 1H du CH₂ en 3γ et 1H du CH₂ en 5β) ; 1,31 (d, J = 7 Hz, 3H : CH₃ en 1γ) ; 1,58 (mt, 1H : l'autre H du CH₂ en 3γ) ; de 1,60 à 1,85 (mt : les 2H correspondant au CH₂ en 2β) ; 2,05 (mt, 1H : l'autre H du CH₂ en 3β) ; 2,85 (s, 6H : ArN(CH₃)₂) ; 2,91 (dd, J = 12 et 4,5 Hz, 1H : 1H du CH₂ en 4β) ; 2,93 (d, J = 16,5 Hz, 1H : l'autre H du CH₂ en 5β) ; de 3,15 à 3,30 (mt, 1H : 1H du CH₂ en 3δ) ; 3,21 (t, J = 12 Hz, 1H : l'autre H du CH₂ en 4β) ; 3,25 (s, 3H : NCH₃) ; 3,50 (mt, 1H : l'autre H du CH₂ en 3δ) ; 3,76 (d, J = 17 Hz, 1H : 1H du CH₂ en 5ε) ; 3,95 (s, 3H : ArOCH₃) ; 4,61 (dd, J = 8 et 5,5 Hz, 1H : CH en 3α) ; 4,80 (mt, 1H : CH en 2α) ; 4,88 (dd, J = 10 et 1,5 Hz, 1H : CH en 1α) ; 5,07 (dd, J = 12 et 4,5 Hz, 1H : CH en 4α) ; 5,33 (d large, J = 5,5 Hz, 1H : CH en 5α) ; 5,41 (d, J = 17 Hz, 1H : l'autre H du CH₂ en 5ε) ; 5,64 (d, J = 8,5 Hz, 1H : CH en 6α) ; 5,88 (q dédoublé, J = 7 et 1,5 Hz, 1 H : CH en 1β) ; 6,33 (d, J = 8 Hz, 2H : H aromatiques en 4ε) ; 6,51 (d, J = 10 Hz, 1H : CONH en 2) ; 6,85 (d, J = 8 Hz, 2H : H aromatiques en 4δ) ; de 7,20 à 7,40 (mt : les 5H aromatiques en 6α) ; 7,44 (dd, J = 8,5 et 1,5 Hz, 1H : l' H₄) ; 7,49 (dd, J = 8,5 et 4 Hz, 1H : l' H₅) ; 7,94 (dd, J = 4 et 1,5 Hz, 1H : l' H₆) ; 8,16 (s, 1H : CH=N) ; 8,37 (d, J = 10 Hz, 1H : CONH en 1) ; 8,69 (d, J = 8,5 Hz, 1H : CONH en 6) ; 11,63 (s, 1 H : OH).

### Exemple 23

En opérant comme à l'exemple 22 mais à partir de 12 cm3 de diméthylformamide, de 2,76 g, de 5δ-diméthylaminométhylène pristinamycine I_{A}, de 0,54 g sulfate de S-méthyl isothiouronium et de 0,35 g de bicarbonate de sodium et après 4 heures à 65°C, on obtient après refroidissement, ajout de 100 cm3 d'acétate d'éthyle au mélange réactionnel, lavage de la phase organique par 3 fois 80 cm3 d'eau, décantation de la phase organique qui est séchée sur sulfate de magnésium, filtrée et concentrée à sec à 40°C sous pression réduite (2,7 kPa), 2,5 g d'un solide jaune. Celui-ci est chromatographié sur 200 g de silice [éluant : dichlorométhane/méthanol 95/5 en volumes] pour donner 1,9 g d'un solide qui est purifié par CLHP sur 450 g de silice C₈ 10 µm [éluant eau-acétonitrile 35-65 en volumes contenant 0,1 % d'acide trifluoroacétique]. Les fractions sont rassemblées, l'acétonitrile éliminé à 40°C sous pression réduite (2,7 kPa) et la phase aqueuse ajustée à pH 7-8 par de l'eau saturée en bicarbonate de sodium. Le précipité blanc formé est filtré, lavé par 2 fois 5 cm3 d'éther diisopropylique et séché à 40°C sous 90 Pa pour donner 0,7 g de 2"-méthylthio-pyrimido [4,5-5γ,5δ] pristinamycine IE sous forme d'un solide crème fondant à 197°C.

Spectre de R.M.N. ¹H (400 MHz, CDCl₃, δ en ppm) : 0,91 (t, J = 7,5 Hz, 3H : CH₃ en 2γ) ; de 1,15 à 1,40 (mt, 3H : 1H du CH₂ en 3β - 1H du CH₂ en 3γ et 1H du CH₂ en 5β) ; 1,31 (d, J = 7 Hz, 3H : CH₃ en 1γ); 1,59 (mt, 1H : l'autre H du CH₂ en 3γ) ; 1,67 et 1,76 (2 mts, 1H chacun : CH₂ en 2β) ; 2,06 (mt, 1H : l'autre H du CH₂ en 3β) ; 2,52 (s, 3H : ArSCH₃) ; de 2,80 à 3,00 (mt, 2H : 1H du CH₂ en 4β et l'autre H du CH₂ en 5β) ; 2,88 (s, 6H : ArN(CH₃)₂) ; de 3,15 à 3,35 (mt, 2H : 1H du CH₂ en 3δ et l'autre H du CH₂ en 4β) ; 3,26 (s, 3H : NCH₃) ; 3,50 (mt, 1H : l'autre H du CH₂ en 3δ) ; 3,77 (d, J = 17 Hz, 1H : 1H du CH₂ en 5ε) ; 4,61 (dd, J = 8 et 5,5 Hz, 1H : CH en 3α) ; 4,80 (mt, 1H : CH en 2α) ; 4,89 (d large, J = 10 Hz, 1H : CH en 1α) ; 5,06 (dd, J = 12 et 4,5 Hz, 1H : CH en 4α) ; 5,32 (d large, J = 5,5 Hz, 1H : CH en 5α) ; 5,41 (d, J = 17 Hz, 1H : l'autre H du CH₂ en 5ε) ; 5,65 (d, J = 8,5 Hz, 1H : CH en 6α) ; 5,88 (q large, J = 7 Hz, 1H : CH en 1β) ; 6,35 (d, J = 8 Hz, 2H : H aromatiques en 4ε) ; 6,52 (d, J = 10 Hz, 1H : CONH en 2) ; 6,86 (d, J = 8 Hz, 2H : H aromatiques en 4δ) ; de 7,20 à 7,40 (mt : les 5H aromatiques en 6α) ; 7,47 (d large, J = 8,5 Hz, 1H : l' H₄) ; 7,52 (dd, J = 8, 5 et 4 Hz, 1H : l' H₅) ; 7,96 (d large, J = 4 Hz, 1H : l' H₆) ; 8,18 (s, 1H : CH=N) ; 8,38 (d, J = 10 Hz, 1H : CONH en 1) ; 8,70 (d, J = 8,5 Hz, 1H : CONH en 6) ; 11,64 (s, 1H : OH).

### Exemple 24

Dans un tricol contenant 130 cm3 de méthanol, on introduit 16,2 g de 2"-méthylthio-pyrimido [4,5-5γ,5δ] pristinamycine IE à 95 % de pureté puis on ajoute à 4°C, 551 cm3 d'acide sulfurique 0,5N puis en 6 minutes 19,94 g d'Oxone®. Le mélange est agité 2 heures à 4°C puis 18 heures à température ambiante. Le mélange réactionnel est refroidi à 4°C, dilué par 150 cm3 de dichlorométhane puis le pH ajusté à 3 par une solution de soude diluée. La phase aqueuse est décantée puis lavée par 2 fois 100 cm3 de dichlorométhane. Les phases organiques sont rassemblées, lavées par 50 cm3 d'une solution saturée de chlorure de sodium, séchées et concentrées sous pression réduite (2,7 kPa) de façon à obtenir un volume final de 200 cm3. A la solution chlorométhylènique obtenue placée dans un tricol on ajoute 100 cm3 d'eau distillée et sous forte agitation 3 cm3 d'une solution à 50 % (p/v) de bisulfite de sodium puis une solution saturée de bicarbonate de sodium jusqu'à pH 6. Après décantation, la phase aqueuse est lavée par 2 fois 100 cm3 de dichlorométhane. Les phases organiques sont rassemblées, séchées sur sulfate de sodium, filtrées et concentrées à sec à 40°C sous pression réduite (2,7 kPa) pour donner 15,3 g d'un solide qui est purifié par chromatographie flash [éluant : dichlorométhane/méthanol 95/5 en volumes]. On obtient ainsi 10,2 g de produit sous forme d'un solide jaune qui peut être utilisé tel quel.

Un échantillon analytique peut être obtenu par purification par chromatographie flash [éluant : dichlorométhane/méthanol 98/2 en volumes] de 0,6 g de produit. Après concentration des fractions à 40°C sous pression réduite (2,7 kPa), trituration dans 5 cm3 d'éther diéthylique, filtration et séchage à 50°C (90 Pa), on obtient 0,35 g de 2"-méthylsulfonyl-pyrimido [4,5-5γ,5δ] pristinamycine IE sous forme d'un solide jaune pâle fondant à 214°C.

Spectre de R.M.N. ¹H (400 MHz, CDCl₃, δ en ppm) : 0,92 (t, J = 7,5 Hz, 3H : CH₃ en 2γ) ; de 1,25 à 1,40 (mt, 2H : 1H du CH₂ en 3β - 1H du CH₂ en 3γ) ; 1,32 (d, J = 7 Hz, 3H : CH₃ en 1γ) ; 1,44 (dd, J = 17 et 5,5 Hz, 1H : 1H du CH₂ en 5β) ; de 1,55 à 1,85 (mt : les 3H correspondant au CH₂ en 2β et l'autre H du CH₂ en 3γ) ; 2,08 (mt, 1H : l'autre H du CH₂ en 3β) ; 2,86 (s, 6H : ArN(CH₃)₂) ; 2,95 (dd, J = 12 et 4,5 Hz, 1H: 1H du CH₂ en 4β) ; 3,11 (d, J = 17 Hz, 1H : l'autre H du CH₂ en 5β) ; 3,20 (t, J = 12 Hz, 1H : l'autre H du CH₂ en 4β) ; de 3,20 à 3,35 (mt, 1H : 1H du CH₂ en 3δ) ; 3,27 (s, 6H : NCH₃ et ArSO₂CH₃) ; 3,51 (mt, 1H : l'autre H du CH₂ en 3δ) ; 3,87 (d, J = 17 Hz, 1H : 1H du CH₂ en 5ε) ; 4,61 (dd, J = 7,5 et 6 Hz, 1H : CH en 3α) ; 4, 81 (mt, 1H : CH en 2α) ; 4,91 (d large, J = 10 Hz, 1H : CH en 1α) ; 5,11 (dd, J = 12 et 4,5 Hz, 1H : CH en 4α) ; 5,42 (d large, J = 5,5 Hz, 1H : CH en 5α) ; 5,54 (d, J = 17 Hz, 1H : l'autre H du CH₂ en 5ε) ; 5,63 (d, J = 8,5 Hz, 1H : CH en 6α) ; 5,88 (q large, J = 7 Hz, 1H : CH en 1β) ; 6,34 (d, J = 8 Hz, 2H : H aromatiques en 4ε) ; 6,56 (d, J = 10 Hz, 1H : CONH en 2) ; 6,87 (d, J = 8 Hz, 2H : H aromatiques en 4δ) ; de 7,20 à 7,40 (mt : les 5H aromatiques en 6α) ; 7,49 (d large, J = 8,5 Hz, 1 H : 1'H₄) ; 7,54 (dd, J = 8,5 et 4 Hz, 1H : 1' H₅) ; 7,98 (d large, J = 4 Hz, 1H : 1' H₆) ; 8,41 (d, J = 10 Hz, 1H : CONH en 1) ; 8,53 (s, 1H : CH=N) ; 8,84 (d, J = 8,5 Hz, 1H : CONH en 6) ; 11,65 (s, 1H : OH).

### Exemple 25

Dans un tricol contenant 25 cm3 de dioxanne et 2 g de 2"-(4-méthyl-benzylsulfonyl)-pyrimido [4,5-5γ,5δ] pristinamycine IE, on introduit 1,9 cm3 de pyrrolidine puis on chauffe à 90°C pendant 3 heures. Après concentration à sec du mélange réactionnel à 40°C sous pression réduite (2,7 kPa), le résidu obtenu est chromatographié sur 150 g de silice [éluant : dichlorométhane/méthanol 96/4 en volumes] pour donner 0,46 g d'un solide crème qui est recristallisé dans 10 cm3 de méthanol. Les cristaux sont filtrés, rincés par un minimum de méthanol puis séchés à 40°C sous pression réduite (90 Pa) pour donner 0,32 g de 2"-(1-pyrrolidinyl)-pyrimido [4,5-5γ,5δ] pristinamycine IE sous forme de cristaux blancs fondant à 255°C.

Spectre de R.M.N. ¹H (400 MHz, CDCl₃, δ en ppm) : 0,92 (t, J = 7,5 Hz, 3H : CH₃ en 2γ) ; de 1,20 à 1,40 (mt, 3H : 1H du CH₂ en 3β - 1H du CH₂ en 3γ et 1H du CH₂ en 5β) ; 1,29 (d, J = 7 Hz, 3H : CH₃ en 1γ) ; 1,56 (mt, 1H : l'autre H du CH₂ en 3γ) ; de 1,60 à 1,85 (mt : les 2H correspondant au CH₂ en 2β) ; 1,93 (mt, 4H : les 2 CH₂ de la pyrrolidine) ; 2,03 (mt, 1H : l'autre H du CH₂ en 3β) ; 2,86 (s, 6H : ArN(CH₃)₂) ; 2,88 (d, J = 17,5 Hz, 1H : l'autre H du CH₂ en 5β) ; 2,94 (dd, J = 12 et 4,5 Hz, 1H : 1H du CH₂ en 4β) ; de 3,15 à 3,30 (mt, 2H : l'autre H du CH₂ en 4β et 1H du CH₂ en 3δ) ; 3,23 (s, 3H : NCH₃) ; de 3,45 à 3,60 (mt, 1H : l'autre H du CH₂ en 3δ) ; 3,53 (mt, 4H : les 2 NCH₂ de la pyrrolidine) ; 3,74 (d, J = 17 Hz, 1H : 1H du CH₂ en 5ε) ; 4,61 (dd, J = 8 et 7 Hz, 1H : CH en 3α) ; 4,78 (mt, 1H : CH en 2α) ; 4,86 (d large, J = 10 Hz, 1H : CH en 1α) ; 5,11 (dd, J = 12 et 4,5 Hz, 1H : CH en 4α) ; 5,29 (d, J = 17 Hz, 1H : l'autre H du CH₂ en 5ε) ; 5,31 (mt, 1H : CH en 5α) ; 5,62 (d, J = 8,5 Hz, 1H : CH en 6α) ; 5,87 (q large, J = 7 Hz, 1H : CH en 1β) ; 6,38 (d, J = 8 Hz, 2H : H aromatiques en 4ε) ; 6,55 (d, J = 9,5 Hz, 1H : CONH en 2) ; 6,86 (d, J = 8 Hz, 2H : H aromatiques en 4δ) ; de 7,20 à 7,40 (mt : les 5H aromatiques en 6α) ; 7,43 (AB limite, 2H : 1' H₄ et 1' H₅) ; 7,91 (mt, 1H : 1' H₆) ; 7,99 (s, 1H : CH=N) ; 8,39 (d, J = 10 Hz, 1H : CONH en 1) ; 8,62 (d, J = 8,5 Hz, 1H : CONH en 6) ; 11,64 (s, 1H : OH).

Le 2"-(4-méthyl-benzylsulfonyl)-pyrimido [4,5-5γ,5δ] pristinamycine IE peut-être préparé de la manière suivante :

Dans un tricol contenant 800 cm3 de méthanol et 24,6 g de 2"-(4-méthyl-benzylthio)-pyrimido [4,5-5γ,5δ] pristinamycine IE on ajoute 1 litre d'acide sulfurique 1N. Le mélange est refroidit à 0°C puis 28,4 g d'Oxone® sont ajoutés. L'agitation est maintenue 18 heures à température ambiante puis le mélange est neutralisé par addition lente de bicarbonate de sodium de façon à obtenir un pH de 8 puis extrait par 3 fois 1 litre de dichlorométhane. Les phases organiques sont rassemblées, séchées sur sulfate de magnésium, filtrées et concentré à sec à 45°C sous pression réduite (2,7 kPa) pour donner 30 g d'un solide qui est chromatographié sur 1,2 kg de silice [éluant : dichlorométhane/méthanol/acide acétique, 89/10/1 en volumes]. Après concentration à sec à 45°C sous pression réduite (2,7 kPa) des fractions le produit est trituré dans 100 cm3 d'éther diéthylique, filtré et séché à 40°C sous pression réduite (90 Pa). On obtient ainsi 21,7 g de 2"-(4-méthyl-benzylsulfonyl)-pyrimido [4,5-5γ,5δ] (4ξ-diméthylamino N-oxyde) (4ξ-désdiméthylamino) pristinamycine IE sous forme d'un solide jaune pâle fondant à 247°C.

Spectre de R.M.N. ¹H (400 MHz, CDCl₃, δ en ppm) : 0,91 (t, J = 7,5 Hz, 3H : CH₃ en 2γ) ; 0,99 (dd, J = 17 et 5,5 Hz, 1H : 1H du CH₂ en 5β) ; 1,14 (mt, 1H : 1H du CH₂ en 3β) ; 1,44 (mt, 1H : 1H du CH₂ en 3γ) ; 1,32 (d, J = 7 Hz, 3H : CH₃ en 1γ) ; de 1,55 à 1,75 (mt, 3H : CH₂ en 2β et l'autre H du CH₂ en 3γ) ; 2,07 (mt, 1H : l'autre H du CH₂ en 3β) ; 2,28 (s, 3H : ArCH₃) ; 3,10 (dd, J = 12 et 4 Hz, 1H : 1H du CH₂ en 4β) ; 3,17 (d, J = 17 Hz, 1H : l'autre H du CH₂ en 5β) ; 3,24 (s, 3H : NCH₃) ; 3,27 (t, J = 12 Hz, 1H : l'autre H du CH₂ en 4β) ; 3,47 et 3,58 (2 mts, 1H chacun : CH₂ en 3δ) ; 3,58 et 3,73 (2 s, 3H chacun : ArN(CH₃)₂) ; 3,81 (d, J = 17 Hz, 1H : 1H du CH₂ en 5ε) ; 4,55 (mt, 1H : CH en 3α) ; 4,58 et 4,79 (2 d, J = 14 Hz, 1H chacun : O₂SCH₂Ar) ; 4,84 (mt, 1H : CH en 2α) ; 4,92 (d large, J = 10 Hz, 1H : CH en 1α) ; 5,31 (dd, J = 12 et 4 Hz, 1H : CH en 4α) ; 5,36 (d large, J = 5,5 Hz, 1H : CH en 5α) ; 5,60 (d, J = 17 Hz, 1H : l'autre H du CH₂ en 5ε) ; 5,70 (d, J = 8,5 Hz, 1H : CH en 6α) ; 5,88 (q large, J = 7 Hz, 1H : CH en 1β) ; 6,83 (d, J = 9 Hz, 1H : CONH en 2) ; 7,08 (d,J = 8 Hz, 2H : H aromatiques en ortho du CH₃) ; 7,11 (d, J = 8 Hz, 2H : H aromatiques en 4δ) ; 7,19 (d, J = 8 Hz, 2H : H aromatiques en méta du CH₃) ; de 7,20 à 7,40 (mt : 5H aromatiques en 6α) ; 7,47 (d large, J = 8,5 Hz, 1H : 1' H₄) ; 7,62 (d, J = 8 Hz, 2H : H aromatiques en 4ε); 7,72 (dd, J = 8,5 et 4,5 Hz, 1H : 1' H₅) ; 7,85 (mt, 1H : 1' H₆) ; 8,41 (d, J = 10 Hz, 1H : CONH en 1) ; 8,55 (s, 1H : CH=N) ; 8,75 (d, J = 8,5 Hz, 1H : CONH en 6) ; 11,65 (mf étalé, 1H : OH).

Dans un tricol contenant 50 cm3 d'acide acétique glacial, on introduit 4,8 g de 2"-(4-méthyl-benzylsulfonyl)-pyrimido [4,5-5γ,5δ] (4ζ-diméthylamino N-oxyde) (4ζ-désdiméthylamino) pristinamycine IE et 0,4 g de fer en poudre. Le mélange est chauffé 2 minutes à 60°C, refroidi, neutralisé par addition d'une solution de bicarbonate de sodium à 10 % puis extrait par 100 cm3 de dichlorométhane. Les phases organiques sont rassemblées, séchées sur sulfate de sodium, filtrées et concentrées à sec à 40°C sous pression réduite (2,7 kPa) pour donner 4,35 g d'un solide marron qui est recristallisé dans 50 cm3 d'isopropanol chaud. Après filtration, lavage des cristaux par 10 cm3 d'éther de diisopropyle et séchage à 40°C sous pression réduite (90 kPa) on obtient 2,06 g de 2"-(4-méthyl-benzylsulfonyl)-pyrimido [4,5-5γ,5δ] pristinamycine IE sous forme d'un solide beige fondant à 188°C.

Spectre de R.M.N. ¹H (400 MHz, CDCl₃, δ en ppm) : 0,91 (t, J = 7,5 Hz, 3H : CH₃ en 2γ) ; de 1,20 à 1,40 (mt, 2H : 1H du CH₂ en 3β et 1H du CH₂ en 3γ) ; 1,31 (d, J = 7 Hz, 3H : CH₃ en 1γ) ; 1,45 (dd, J = 17 et 5,5 Hz, 1H : 1H du CH₂ en 5β) ; de 1,55 à 1,75 (mt : les 2H correspondant à 1H du CH₂ en 2β et l'autre H du CH₂ en 3γ) ; 1,74 (mt, 1H : l'autre H du CH₂ en 2β) ; 2,08 (mt, 1H : l'autre H du CH₂ en 3β) ; 2,30 (s, 3H : ArCH₃) ; 2,81 (s, 6H : ArN(CH₃)₂) ; 2,95 (dd, J = 12 et 4,5 Hz, 1H : 1H du CH₂ en 4β) ; 3,01 (d, J = 17 Hz, 1H : l'autre H du CH₂ en 5β) ; 3,19 (t, J = 12 Hz, 1H : l'autre H du CH₂ en 4β) ; de 3,20 à 3,35 (mt, 1H : 1H du CH₂ en 3δ) ; 3,26 (s, 3H : NCH₃) ; 3,51 (mt, 1H : l'autre H du CH₂ en 3δ) ; 3,88 (d, J = 17 Hz, 1H : 1H du CH₂ en 5ε) ; 4,50 et 4,74 (2 d, J = 14 Hz, 1H chacun : O₂SCH₂Ar) ; 4,61 (dd, J = 7,5 et 6 Hz, 1H : CH en 3α) ; 4,80 (mt, 1 H : CH en 2α) ; 4,90 (d large, J = 10 Hz, 1 H : CH en 1α) ; 5,08 (dd, J = 12 et 4,5 Hz, 1H : CH en 4α) ; 5,40 (d large, J = 5,5 Hz; 1H : CH en 5α) ; 5,54 (d, J = 17 Hz, 1H : l'autre H du CH₂ en 5ε) ; 5,66 (d, J = 8,5 Hz, 1H : CH en 6α) ; 5,89 (q large, J = 7 Hz, 1H : CH en 1β) ; 6,29 (d, J = 8 Hz, 2H : H aromatiques en 4ε) ; 6,53 (d, J = 10 Hz, 1H : CONH en 2) ; 6,84 (d, J = 8 Hz, 2H : H aromatiques en 4δ) ; 7,12 (d, J = 8 Hz, 2H : H aromatiques en ortho du CH₃) ; de 7,10 à 7,35 (mt : les 5H aromatiques en 6α) ; 7,20 (d, J = 8 Hz, 2H : H aromatiques en méta du CH₃) ; 7,48 (d large, J = 8,5 Hz, 1H : 1' H₄) ; 7,53 (dd, J = 8,5 et 4 Hz, 1 H : 1' H₅) ; 7,96 (d large, J = 4 Hz, 1H : 1' H₆) 8,39 (d, J = 10 Hz, 1H : CONH en 1) ; 8,50 (s, 1 H : CH=N) ; 8,80 (d, J = 8,5 Hz, 1 H : CONH en 6) ; 11,64 (s, 1H : OH).

Le 2"-(4-méthyl-benzylthio)-pyrimido [4,5-5γ,5δ] pristinamycine IE peut être préparé de la manière suivante :

Dans un tricol contenant 35 cm3 de diméthylformamide, on introduit 4,3 g de 5δ-diméthylaminométhylène pristinamycine I_{A}, 1 g de chlorhydrate de (4-méthylbenzyl) isothiourée puis on ajoute goutte à goutte 1,8 cm3 de N,N-diisopropylamine. Le mélange est chauffé 3 heures à 60°C, refroidi puis dilué par 200 cm3 d'eau distillée. Le précipité formé est filtré pour donner 1 g de produit qui est purifié par CLHP sur 450 g de silice C₈ 10 µm [éluant eau-acétonitrile 50/50 en volumes contenant 0,1 % d'acide trifluoroacétique]. Les fractions sont rassemblées, l'acétonitrile éliminé à 40°C sous pression réduite (2,7 kPa) et la phase aqueuse ajustée à pH 7-8 par de l"eau saturée en bicarbonate de sodium. Le mélange est extrait par 3 fois 80 cm3 de dichlorométhane, les phases organiques sont rassemblées, séchées sur sulfate de magnésium, filtrées, concentrées à sec puis séchées à 40°C sous pression réduite (90 Pa) pour donner 1,09 g de 2"-(4-méthyl-benzylthio)-pyrimido [4,5-5γ,5δ] pristinamycine IE sous forme d'un solide crème fondant à 222°C.

Spectre de R.M.N. ¹H (400 MHz, CDCl₃, δ en ppm) : 0,91 (t, J = 7,5 Hz, 3H : CH₃ en 2γ) ; de 1,15 à 1,35 (mt, 3H : 1H du CH₂ en 3β - 1H du CH₂ en 3γ et 1H du CH₂ en 5β) ; 1,31 (d, J = 7 Hz, 3H : CH₃ en 1γ) ; de 1,55 à 1,80 (mt : les 2H correspondant au CH₂ en 2β) ; 1,59 (mt, 1H : l'autre H du CH₂ en 3γ) ; 2,05 (mt, 1H : l'autre H du CH₂ en 3β) ; 2,32 (s, 3H : ArCH₃) ; 2,86 (s, 6H : ArN(CH₃)₂) ; 2,91 (dd, J = 12 et 4 Hz, 1H : 1H du CH₂ en 4β) ; 2,94 (d, J = 17,5 Hz, 1H : l'autre H du CH₂ en 5β) ; de 3,15 à 3,30 (mt, 1H : 1H du CH₂ en 3δ) ; 3,21 (t, J = 12 Hz, 1H : l'autre H du CH₂ en 4β) ; 3,25 (s, 3H : NCH₃) ; 3,50 (mt, 1 H : l'autre H du CH₂ en 3δ) ; 3,76 (d, J = 17 Hz, 1 H : 1 H du CH₂ en 5ε) ; 4,27 et 4,39 (2 d, J = 13,5 Hz, 1 H chacun : ArSCH₂Ar) ; 4,61 (dd, J = 7,5 et 5,5 Hz, 1H : CH en 3α) ; 4,79 (mt, 1H : CH en 2α) ; 4,88 (d large, J = 10 Hz, 1 H : CH en 1α) ; 5,07 (dd, J = 12 et 4 Hz, 1 H : CH en 4α) ; 5,32 (d large, J = 5,5 Hz, 1 H : CH en 5α) ; 5,39 (d, J = 17 Hz, 1 H : l'autre H du CH₂ en 5ε) ; 5,64 (d, J = 8,5 Hz, 1 H : CH en 6α) ; 5,87 (q large, J = 7 Hz, 1H : CH en 1β) ; 6,33 (d, J = 8 Hz, 2H : H aromatiques en 4ε) ; 6,53 (d, J = 10 Hz, 1H : CONH en 2) ; 6,84 (d, J = 8 Hz, 2H : H aromatiques en 4δ) ; 7,11 (d, J = 8 Hz, 2H : H aromatiques en ortho du CH₃) ; de 7,15 à 7,40 (mt : les 5H aromatiques en 6α) ; 7,32 (d, J = 8 Hz, 2H : H aromatiques en méta du CH₃) ; 7,44 (d large, J = 8,5 Hz, 1H : 1' H₄) ; 7,48 (dd, J = 8, 5 et 4 Hz, 1H : 1'H₅); 7,93 (d large, J = 4 Hz, 1 H : 1' H₆) ; 8,19 (s, 1 H : CH=N) ; 8,38 (d, J = 10 Hz, 1H : CONH en 1) ; 8,70 (d, J = 8,5 Hz, 1H : CONH en 6) ; 11,63 (s, 1H : OH).

### Exemple 26

En opérant comme à l'exemple 25, mais à partir de 40 cm3 de dioxanne, de 2 g de 2"-(4-méthyl-benzylsulfonyl)-pyrimido [4,5-5γ,5δ] pristinamycine IE, de 1,02 cm3 d'azétidine et après 45 minutes de chauffage à 60°C, on obtient après refroidissement un précipité qui est filtré, lavé par 10 cm3 d'éther de diisopropyle puis recristallisé dans 15 cm3 de méthanol pour donner après filtration, séchage à 40°C sous pression réduite (90 Pa), 1,05 g de 2"-(1-azétidinyl)-pyrimido [4,5-5γ,5δ] pristinamycine IE sous forme d'une poudre blanche fondant à 243°C.

Spectre de R.M.N. ¹H (400 MHz, CDCl₃, δ en ppm) : 0,92 (t, J = 7,5 Hz, 3H : CH₃ en 2γ) ; de 1,15 à 1,35 (mt, 3H : 1H du CH₂ en 3β - 1 H du CH₂ en 3γ et 1H du CH₂ en 5β); 1,29 (d, J = 7 Hz, 3H : CH₃ en 1γ) 1,56 (mt, 1H : l'autre H du CH₂ en 3γ) ; 1,65 et 1,72 (2 mts, 1H chacun : CH₂ en 2β) ; 2,05 (mt, 1H : l'autre H du CH₂ en 3β) ; 2,33 (mt, 2H : CH₂ de l'azétidine) ; 2,86 (d, J = 17,5 Hz, 1H : l'autre H du CH₂ en 5β) ; 2,88 (s, 6H : ArN(CH₃)₂) ; 2,92 (dd, J = 12 et 4,5 Hz, 1H : 1H du CH₂ en 4β) ; de 3,10 à 3,35 (mt, 2H : l'autre H du CH₂ en 4β et 1H du CH₂ en 3δ) ; 3,22 (s, 3H : NCH₃) ; 3,48 (mt, 1H : l'autre H du CH₂ en 3δ) ; 3,72 (d, J = 17 Hz, 1H : 1H du CH₂ en 5ε) ; 4,09 (mt, 4H : les 2 NCH₂ de l'azétidine) ; 4,59 (dd, J = 8 et 6 Hz, 1 H : CH en 3α) ; 4,78 (mt, 1 H : CH en 2α) ; 4,88 (d large, J = 10 Hz, 1H : CH en 1α) ; 5,12 (dd, J = 12 et 4,5 Hz, 1 H : CH en 4α) ; 5,29 (d, J = 17 Hz, 1H : l'autre H du CH₂ en 5ε) ; 5,31 (d large J = 6 Hz, 1H : CH en 5α) ; 5,62 (d, J = 8,5 Hz, 1H : CH en 6α) ; 5,87 (q large, J = 7 Hz, 1H : CH en 1β) ; 6,40 (d, J = 8 Hz, 2H : H aromatiques en 4ε) ; 6,55 (d, J = 9,5 Hz, 1H : CONH en 2) ; 6,87 (d, J = 8 Hz, 2H : H aromatiques en 4δ) ; de 7,15 à 7,35 (mt : les 5H aromatiques en 6α) ; 7,42 (AB limite, 2H : 1' H₄ et 1' H₅); 7,90 (mt, 1H : 1' H₆) ; 7,97 (s, 1H : CH=N) ; 8,40 (d, J = 10 Hz, 1H : CONH en 1) ; 8,63 (d, J = 8,5 Hz, 1 H : CONH en 6).

### Exemple 27

En opérant comme à l'exemple 22 mais à partir de 5 cm3 de diméthylformamide, de 1,84 g de 5δ-diméthylaminométhylène pristinamycine I_{A}, de 0,41 g de chlorhydrate de 4-amidinopyridinium, de 0,235 g de bicarbonate de sodium et 4 heures de chauffage à 65°C, on obtient après refroidissement une solution qui est diluée par 40 cm3 d'acétate d'éthyle et 50 cm3 d'eau distillée. Après décantation, la phase aqueuse est lavée par 2 fois 40 cm3 d'acétate d'éthyle, les phases organiques sont réunies puis lavées par 200 cm3 de saumure, séchées sur sulfate de magnésium, filtrées et concentrées à 40°C sous pression réduite (2,7 kPa) pour donner un résidu qui est chromatographié sur 90 g de silice [éluant : dichlorométhane/méthanol 96/4 en volumes]. On obtient 0,535 g d'un produit qui est purifié avec 1,37 g d'un produit identique obtenu par une préparation analogue par CLHP sur 450 g de silice C₈ 10 µm [éluant eau-acétonitrile 35/65 en volumes contenant 0,1 % d'acide trifluoroacétique]. Les fractions sont rassemblées, l'acétonitrile éliminé à 40°C sous pression réduite (2,7 kPa) et la phase aqueuse ajustée à pH 7-8 par de l'eau saturée en bicarbonate de sodium. Le mélange est extrait par 3 fois 100 cm3 de dichlorométhane, les phases organiques sont rassemblées, séchées sur sulfate de magnésium, filtrées, concentrées à sec puis séchées à 40°C sous pression réduite (90 Pa) pour donner 0,73 g d'un solide blanc qui est trituré dans 10 cm3 d'éther de diisopropyle, filtré et séché à 40°C (90 Pa). On obtient ainsi 0,67 g de 2"-(4-pyridyl)-pyrimido[4,5-5γ,5δ] pristinamycine IE sous forme d'un solide blanc fondant à 277°C.

Spectre de R.M.N. ¹H (400 MHz, CDCl₃, δ en ppm) : 0,92 (t, J = 7,5 Hz, 3H : CH₃ en 2γ) ; de 1,20 à 1,40 (mt, 2H : 1H du CH₂ en 3β et 1H du CH₂ en 3γ) ; 1,31 (d, J = 7 Hz, 3H : CH₃ en 1*γ) ;* 1,41 (dd, J = 17 et 6 Hz, 1H : 1H du CH₂ en 5β) ; 1,60 (mt, 1H : l'autre H du CH₂ en 3γ) ; de 1,60 à 1,85 (mt : les 2H correspondant au CH₂ en 2β); 2,07 (mt, 1H : l'autre H du CH₂ en 3β) ; 2,63 (s, 6H : ArN(CH₃)₂) ; 2,92 (dd, J = 12 et 4 Hz, 1H : 1H du CH₂ en 4β) ; 3,10 (d, J = 17 Hz, 1H : l'autre H du CH₂ en 5β) ; de 3,20 à 3,35 (mt, 2H : 1H du CH₂ en 3δ et l'autre H du CH₂ en 4β) ; 3,27 (s, 3H : NCH₃) ; 3,51 (mt, 1H : l'autre H du CH₂ en 3δ) ; 3,88 (d, J = 17 Hz, 1H : 1H du CH₂ en 5ε) ; 4,61 (dd, J = 7,5 et 6 Hz, 1H : CH en 3α) ; 4,80 (mt, 1H CH en 2α) ; 4,88 (d large, J = 10 Hz, 1H : CH en 1α) ; 5,06 (dd, J = 12 et 4 Hz, 1H : CH en 4α) ; 5,41 (d large, J = 6 Hz, 1H : CH en 5α) ; 5,52 (d, J = 17 Hz, 1H : l'autre H du CH₂ en 5ε) ; 5,64 (d, J = 8,5 Hz, 1H : CH en 6α) ; 5,88 (q large, J = 7 Hz, 1H : CH en 1β) ; 6,38 (d, J = 8 Hz, 2H : H aromatiques en 4ε) ; 6,54 (d, J = 10 Hz, 1H : CONH en 2) ; 6,86 (d, J = 8 Hz, 2H : H aromatiques en 4δ) ; de 7,20 à 7,40 (mt : les 5H aromatiques en 6α) ; 7,49 (d large, J = 8,5 Hz, 1H : 1' H₄) ; 7,57 (dd, J = 8,5 et 4 Hz, 1H : 1' H₅) ; 8,04 (d large, J = 4 Hz, 1H : 1' H₆) ; 8,27 (d, J = 5 Hz, 2H : H aromatiques en β de la pyridine) ; 8,38 (d, J = 10 Hz, 1H : CONH en 1) ; 8,48 (s, 1H : CH=N) ; 8,72 (d, J = 8,5 Hz, 1H : CONH en 6) ; 8,75 (d, J = 5 Hz, 2H : H aromatiques en α de la pyridine) ; 11,66 (s, 1H : OH).

### Exemple 28

Dans un tricol contenant 35 cm3 de dimèthylformamide, on introduit 6 g de 5δ-diméthylaminométhylène pristinamycine I_{A}, 1,33 g de chlorhydrate de 2-amidinopyridinium puis on ajoute goutte à goutte 3,4 cm3 de N,N-diisopropylamine. Le mélange est chauffé 4 heures à 65°C, refroidi, puis dilué par 500 cm3 d'eau distillée saturée en chlorure de sodium. Le précipité formé est filtré puis repris par 300 cm3 de dichlorométhane. La solution obtenue est séchée sur sulfate de sodium, filtrée et concentrée à sec sous pression réduite à 40°C (2,7 kPa) pour donner 4,36 g d'un produit qui est purifié par chromatographie sur 220 g de silice [éluant : dichlorométhane/méthanol 95/5 en volumes]. Après concentration à sec des fractions sous pression réduite à 40°C (2,7 kPa), on obtient 3,15 g d'un solide qui est recristallisé dans 20 cm3 d'isopropanol. Les cristaux sont filtrés, lavés par 20 cm3 d'éther diisopropylique puis séchés à 40°C (90 Pa) pour donner 1,08 g de 2"-(2-pyridyl)-pyrimido [4,5-5γ,5δ] pristinamycine IE sous forme d'une poudre blanche fondant à 214°C.

Spectre de R.M.N. ¹H (400 MHz, CDCl₃, δ en ppm) : 0,92 (t, J = 7,5 Hz, 3H : CH₃ en 2γ) ; de 1,15 à 1,35 (mt, 2H : 1H du CH₂ en 3β et 1H du CH₂ en 3γ) ; 1,31 (d, J = 7 Hz, 3H : CH₃ en 1γ) ; 1,51 (dd, J = 17 et 6 Hz, 1H : 1H du CH₂ en 5β) ; de 1,55 à 1,80 (mt : les 2H correspondant au CH₂ en 2β) ; 1,59 (mt, 1H : l'autre H du CH₂ en 3γ) ; 2,06 (mt, 1H : l'autre H du CH₂ en 3β) ; 2,64 (s, 6H : ArN(CH₃)₂) ; 2,93 (dd, J = 12 et 4,5 Hz, 1 H : 1H du CH₂ en 4β) ; 3,13 (d, J = 17 Hz, 1H : l'autre H du CH₂ en 5β) ; de 3,15 à 3,30 (mt, 1H : 1H du CH₂ en 3δ) ; 3,22 (t, J = 12 Hz, 1 H : l'autre H du CH₂ en 4β) ; 3,26 (s, 3H : NCH₃) ; 3,51 (mt, 1H : l'autre H du CH₂ en 3δ) ; 3,89 (d, J = 17 Hz, 1H : 1H du CH₂ en 5ε) ; 4,60 (dd, J = 8 et 6 Hz, 1H : CH en 3α) ; 4,80 (mt, 1H : CH en 2α) ; 4,90 (d large, J = 10 Hz, 1H : CH en 1α) ; 5,12 (dd, J = 12 et 4,5 Hz, 1H : CH en 4α) ; 5,42 (d large, J = 6 Hz, 1H : CH en 5α) ; 5,52 (d, J = 17 Hz, 1H : l'autre H du CH₂ en 5ε) ; 5,65 (d, J = 8,5 Hz, 1H : CH en 6α) ; 5,87 (q large, J = 7 Hz, 1H : CH en 1β) ; 6,28 (d, J = 8 Hz, 2H : H aromatiques en 4ε) ; 6,56 (d, J = 10 Hz, 1H : CONH en 2) ; 6,85 (d, J = 8 Hz, 2H : H aromatiques en 4δ) ; de 7,15 à 7,40 (mt : les 5H aromatiques en 6α) ; 7,35 (mt, 1H : H en 5 de la pyridine) ; 7,46 (d large, J = 8,5 Hz, 1H : l' H₄) ; 7,51 (dd, J = 8,5 et 4 Hz, 1H : l' H₅) ; 7,82 (t dédoublé, J = 8 et 1,5 Hz, 1H : H en 4 de la pyridine) ; 7,99 (d large, J = 4 Hz, 1H : l' H₆) ; 8,41 (d, J = 10 Hz, 1H : CONH en 1) ; 8,47 (d, J = 8 Hz, H en 3 de la pyridine) ; 8,56 (s, 1H : CH=N) ; 8,72 (d, J = 8,5 Hz, 1H : CONH en 6) ; 8,82 (d large, J = 5 Hz, 1H : H en 6 de la pyridine) ; 11,65 (s, 1H : OH).

### Exemple 29

En opérant comme à l'exemple 22 mais à partir de 4 cm3 de diméthylformamide, de 0,92 g de 5δ-diméthylaminométhylène pristinamycine I_{A}, de 0,22 g chlorhydrate de benzamidine et de 0,12 g de bicarbonate de sodium et après 4 heures à 60°C, on obtient après refroidissement, ajout de 50 cm3 d'eau distillée et de 20 cm3 d'acétate d'éthyle au mélange réactionnel, lavage de la phase aqueuse par 2 fois 20 cm3 d'acétate d'éthyle, décantation de la phase organique qui est séchée sur sulfate de magnésium, filtrée et concentrée à sec à 40°C sous pression réduite (2,7 kPa), 1 g d'un résidu qui est chromatographié sur 170 g de silice [éluant : dichlorométhane/méthanol 96/4 en volumes]. Après concentration à sec à 40°C sous pression réduite (2,7 kPa) des fractions, le produit est trituré dans 10 cm3 d'éther diisopropylique, filtré et séché à 40°C sous pression réduite (90 Pa) pour donner 0,49 g de 2"-phényl-pyrimido [4,5-5γ,5δ] pristinamycine IE sous forme d'une poudre blanche fondant à 201°C.

Spectre de R.M.N. ¹H (400 MHz, CDCl₃, δ en ppm) : 0,92 (t, J = 7,5 Hz, 3H : CH₃ en 2γ) ; de 1,15 à 1,35 (mt, 2H : 1H du CH₂ en 3β et 1H du CH₂ en 3γ) ; 1,31 (d, J = 7 Hz, 3H : CH₃ en 1γ) ; 1,40 (dd, J=17 et 6Hz, 1H : 1H du CH₂ en 5β) ; 1,59 (mt, 1H : l'autre H du CH₂ en 3γ) ; 1,65 (mt : 1H correspodant à 1H du CH₂ en 2β); 1,74 (mt, 1H : l'autre H du CH₂ en 2β) ; 2,06 (mt, 1H : l'autre H du CH₂ en 3β) ; 2,64 (s, 6H : ArN(CH₃)₂) ; 2,93 (dd, J = 12 et 4 Hz, 1H : 1H du CH₂ en 4β) ; 3,09 (d, J = 17 Hz, 1H : l'autre H du CH₂ en 5β) ; de 3,15 à 3,30 (mt, 2H : 1H du CH₂ en 3δ et l'autre H du CH₂ en 4β) ; 3,27 (s, 3H : NCH₃) ; 3,50 (mt, 1H : l'autre H du CH₂ en 3δ) ; 3,87 (d, J = 17 Hz, 1H : 1H du CH₂ en 5ε) ; 4,61 (dd, J = 8 et 6 Hz, 1H : CH en 3α) ; 4,81 (mt, 1H : CH en 2α) ; 4,90 (dd, J = 10 et 1,5 Hz, 1H : CH en 1α); 5,10 (dd, J = 12 et 4 Hz, 1H : CH en 4α) ; 5,41 (d large, J = 6 Hz, 1H : CH en 5α) ; 5,49 (d, J = 17 Hz, 1H : l'autre H du CH₂ en 5ε) ; 5,65 (d, J = 8,5 Hz, 1H : CH en 6α) ; 5,90 (q dédoublé, J = 7 et 1,5Hz, 1H : CH en 1β) ; 6,30 (d, J = 8 Hz, 2H : H aromatiques en 4ε) ; 6,56 (d, J = 10 Hz, 1H : CONH en 2) ; 6,87 (d, J = 8 Hz, 2H : H aromatiques en 4δ) ; de 7,20 à 7,40 (mt : les 5H aromatiques en 6α) ; de 7.40 à 7.50 (mt, 3H : H aromatiques en para et méta du phényle) ; 7,48 (dd, J = 8,5 et 1,5 Hz, 1H : 1' H₄) ; 7,56 (dd, J = 8,5 et 4 Hz, 1H : 1' H₅) ; 8,03 (dd, J = 4 et 1,5 Hz, 1H : 1' H₆) ; de 8,35 à 8,45 (mt, 3H : H aromatiques en ortho du phényle et CONH en 1) ; 8,44 (s, 1H : CH=N) ; 8,70 (d, J = 8,5 Hz, 1H : CONH en 6) ; 11,66 (s, 1H : OH).

### Exemple 30

En opérant comme à l'exemple 22 mais à partir de 10 cm3 de diméthylformamide, de 2 g de 5δ-diméthylaminométhylène pristinamycine I_{A}, de 0,59 g de dichlorhydrate de 3-aminobenzamidine et de 0,47 g de bicarbonate de sodium et après 4 heures à 60°C, on obtient après refroidissement, ajout de 50 cm3 d'eau distillée et de 40 cm3 d'acétate d'éthyle au mélange réactionnel, lavage de la phase aqueuse par 2 fois 40 cm3 d'acétate d'éthyle, décantation de la phase organique qui est séchée sur sulfate de magnésium, filtrée et concentrée à sec à 40°C sous pression réduite (2,7 kPa), un résidu qui est chromatographié sur 200 g de silice [éluant : dichlorométhane/méthanol 96/4 en volumes] pour donner 1,06 g d'un solide. Celui-ci est purifié par CLHP sur 450 g de silice C₈ 10 µm [éluant eau-acétonitrile 65/35 en volumes contenant 0,1 % d'acide trifluoroacétique], les fractions sont rassemblées, l'acétonitrile éliminé à 40°C sous pression réduite (2,7 kPa) et la phase aqueuse ajustée à pH 7-8 par de l'eau saturée en bicarbonate de sodium. Le précipité formé est filtré, lavé par de l'éther de diisopropyle, séché à 40°C sous pression réduite (90 Pa) pour donner 0,31 g de 2"-(3-aminophényl)-pyrimido [4,5-5γ,5δ] pristinamycine IE sous forme d'un solide jaune pâle fondant à 212°C.

Spectre de R.M.N. ¹H (400 MHz, CDCl₃, δ en ppm : 0,93 (t, J = 7,5 Hz, 3H : CH₃ en 2γ) ; de 1,20 à 1,40 (mt, 2H : 1H du CH₂ en 3β et 1H du CH₂ en 3γ) ; 1,31 (d, J = 7 Hz, 3H : CH₃ en 1γ) ; 1,43 (dd, J = 17 et 6 Hz, 1H : 1H du CH₂ en 5β) ; de 1,50 à 1,75 (mt : les 2H correspondant à l'autre H du CH₂ en 3γ et à 1H du CH₂ en 2β) ; 1,76 (mt, 1H : l'autre H du CH₂ en 2β) ; 2,08 (mt, 1H : l'autre H du CH₂ en 3β) ; 2,69 (s, 6H : ArN(CH3)2) ; 2,94 (dd, J = 12,5 et 4,5 Hz, 1H : 1H du CH₂ en 4β) 3,10 (d, J = 17 Hz, 1H : l'autre H du CH₂ en 5β) ; de 3,15 à 3,35 (mt, 2H : 1H du CH₂ en 3δ et l'autre H du CH₂ en 4β) ; 3,28 (s, 3H : NCH₃) ; 3,51 (mt, 1 H : l'autre H du CH₂ en 3δ) ; 3,76 (s large, 2H : ArNH₂) 3,88 (d, J = 17 Hz, 1H : 1H du CH₂ en 5ε) ; 4,63 (dd, J = 8 et 6 Hz, 1H : CH en 3α) ; 4,82 (mt, 1H : CH en 2α) ; 4,90 (d large, J = 10 Hz, 1 H : CH en 1α) ; 5,11 (dd, J = 12,5 et 4,5 Hz, 1H : CH en 4α) ; 5,41 (d large, J = 6 Hz, 1H : CH en 5α) ; 5,49 (d, J = 17 Hz, 1H : l'autre H du CH₂ en 5ε) ; 5,66 (d, J = 8,5 Hz, 1H : CH en 6α) ; 5,90 (q large, J = 7 Hz, 1H : CH en 1β) ; 6,31 (d, J = 8 Hz, 2H : H aromatiques en 4ε) ; 6,55 (d, J = 10 Hz, 1H : CONH en 2) ; 6,80 (dd, J = 8 et 1,5 Hz, 1H : H aromatique en 4 du 3-aminophényl) ; 6,85 (d, J = 8 Hz, 2H : H aromatiques en 4δ) ; de 7,20 à 7,40 (mt : les 6H correspondant aux 5H aromatiques en 6α et à 1' H aromatique en 5 du 3-aminophényl) ; 7,49 (d large, J = 8,5 Hz, 1H : 1' H₄) ; 7,55 (dd, J = 8,5 et 4 Hz, 1H : 1'H₅); 7,75 (s large, 1H : H aromatique en 2 du 3-aminophényl); 7,82 (d large, 1H : H aromatique en 6 du 3-aminophényl) ; 8,03 (dd, J = 4 et 1,5 Hz, 1H : 1' H₆) ; 8,40 (d, J = 10 Hz, 1H : CONH en 1) ; 8,42 (s, 1 H : CH=N) ; 8,70 (d, J = 8,5 Hz, 1H : CONH en 6) ; 11,66 (s, 1H : OH).

### Exemple 31

En opérant comme à l'exemple 22 mais à partir de 45 cm3 de diméthylformamide, de 5 g de 5δ-diméthylaminométhylène pristinamycine I_{B}, de 0,64 g de sulfate de S-méthyl isothiouronium et de 0,77 g de bicarbonate de sodium et après 18 heures à 60°C, on obtient après refroidissement, ajout de 200 cm3 d'eau distillée et de 150 cm3 d'acétate d'éthyle au mélange réactionnel, lavage de la phase aqueuse par 2 fois 150 cm3 d'acétate d'éthyle, décantation de la phase organique qui est lavée par 250 cm3 d'eau distillée, séchée sur sulfate de magnésium, filtrée et concentrée à sec à 40°C sous pression réduite (2,7 kPa), 3,16 g d'un résidu qui est chromatographié sur 250 g de silice [éluant : dichlorométhane/méthanol 95/5 en volumes] pour donner 1,2 g d'un solide. Celui-ci est purifié par CLHP sur 450 g de silice C₈ 10 µm [éluant eau-acétonitrile 65/35 en volumes contenant 0,1 % d'acide trifluoroacétique], les fractions sont rassemblées, l'acétonitrile éliminé à 40°C sous pression réduite (2,7 kPa), la phase aqueuse ajustée à pH 7-8 par de l'eau saturée en bicarbonate de sodium et extraite par 2 fois 100 cm3 de dichlorométhane. La phase organique est décantée, séchée sur sulfate de magnésium, filtrée, puis concentrée à sec puis séchée à 40°C sous pression réduite (90 Pa) pour donner 0,45 g de 2"-méthylthio-pyrimido [4,5-5γ,5δ] (4ζ-méthylamino) (4ζ-désdiméthylamino) pristinamycine IE sous forme d'un solide jaune pâle fondant à 282°C.

Spectre de R.M.N. ¹H (400 MHz, CDCl₃, δ en ppm) : 0,91 (t, J = 7,5 Hz, 3H : CH₃ en 2γ) ; de 1,20 à 1,40 (mt, 3H : 1H du CH₂ en 3β - 1H du CH₂ en 3γ et 1H du CH₂ en 5β) ; 1,32 (d, J = 7 Hz, 3H : CH₃ en 1γ) ; 1,58 (mt, 1H : l'autre H du CH₂ en 3γ) ; de 1,60 à 1,85 (mt : les 2H correspondant au CH₂ en 2β) ; 2,06 (mt, 1H : l'autre H du CH₂ en 3β) ; 2,64 (s, 3H : ArSCH₃) ; 2,77 (s, 3H : ArNCH₃) ; 2,89 (dd, J = 12 et 4,5 Hz, 1 H : 1H du CH₂ en 4β) ; 2,97 (d, J = 17,5 Hz, 1H : l'autre H du CH₂ en 5β) ; 3,20 (t, J = 12 Hz, 1H : l'autre H du CH₂ en 4β) ; de 3,20 à 3,35 (mt, 1H : 1H du CH₂ en 3δ) ; 3,25 (s, 3H : NCH₃) ; 3,50 (mt, 1H : l'autre H du CH₂ en 3δ) ; de 3,65 à 3,85 (mf étalé, 1H : ArNH) ; 3,75 (d, J = 17 Hz, 1H : 1H du CH₂ en 5ε) ; 4,61 (dd, J = 8 et 5,5 Hz, 1H : CH en 3α) ; 4,80 (mt, 1H : CH en 2α) ; 4,88 (d large, J = 10 Hz, 1H : CH en 1α) ; 5,03 (dd, J = 12 et 4,5 Hz, 1H : CH en 4α) ; 5,32 (d large, J = 6 Hz, 1H : CH en 5α) ; 5,39 (d, J = 17 Hz, 1H : l'autre H du CH₂ en 5ε) ; 5,65 (d, J = 8,5 Hz, 1H : CH en 6α) ; 5,88 (q large, J = 7 Hz, 1H : CH en 1β) ; 6,18 (d, J = 8 Hz, 2H : H aromatiques en 4ε) ; 6,51 (d, J = 10 Hz, 1H : CONH en 2) ; 6,78 (d, J = 8 Hz, 2H : H aromatiques en 4δ) ; de 7,20 à 7,40 (mt : les 5H aromatiques en 6α) ; 7,46 (d large, J = 8,5 Hz, 1H : 1'H₄) ; 7,50 (dd, J = 8,5 et 4 Hz, 1H : 1' H₅) ; 7,94 (d large, J = 4 Hz, 1H: 1' H₆) ; 8,17 (s, 1H : CH=N) ; 8,38 (d, J = 10 Hz, 1H : CONH en 1) ; 8,67 (d, J = 8,5 Hz, 1H : CONH en 6) ; 11,62 (s, 1H : OH).

### Exemple 32

Dans un ballon contenant 0,4 cm3 de dichlorométhane, on introduit 97 mg de 2"-(1-pyrrolidinyl-pyrimido[4,5-5γ,5δ] pristinamycine IE, 5,4 mg d'éthylèneglycol, 65 mg d'acide acétique et 20 mg de periodate de tétra n-butylammonium. Le mélange est agité 4 heures à température ambiante puis le mélange réactionnel est repris par 8 cm3 d'eau et 4 cm3 de dichlorométhane. La phase organique est décantée, lavée par 4 fois 8 cm3 d'eau distillée, décantée, séchée puis concentration à sec à 40°C sous pression réduite (2,7 kPa) pour donner 70 mg d'un solide qui est purifié par chromatographie flash avec 210 mg d'un produit identique provenant d'une préparation analogue sur 15 g de silice [éluant : dichlorométhane/méthanol 97/3 en volumes] pour donner après concentration à sec des fractions, trituration dans 4 cm3 d'éther diéthylique, filtration et séchage à 20°C sous pression réduite (90 Pa), 98 mg de 2"-(1-pyrrolidinyl)-pyrimido [4,5-5γ,5δ] (4ζ-méthylamino) (4ζ-désdiméthylamino) pristinamycine IE sous forme d'une poudre crème fondant à 222°C.

Spectre de R.M.N. ¹H (400 MHz, CDCl₃, δ en ppm) : 0,92 (t, J = 7,5 Hz, 3H : CH₃ en 2γ) ; de 1,20 à 1,40 (mt, 2H : 1H du CH₂ en 3β et 1H du CH₂ en 3y) ; 1,31 (d, J = 7 Hz, 3H : CH₃ en 1γ) ; 1,48 (dd, J = 17 et 6 Hz, 1H : 1H du CH₂ en 5β) ; de 1,50 à 1,85 (mt : les 3H correspondant à l'autre H du CH₂ en 3γ et au CH₂ en 2β) ; 1,95 (mt, 4H : les 2 CH₂ de la pyrrolidine) ; 2,04 (mt, 1H : l'autre H du CH₂ en 3β) ; 2,62 (s, 3H : ArNCH₃) ; 2,91 (dd, J = 12,5 et 4,5 Hz, 1H : 1H du CH₂ en 4β) ; 2,92 (d, J = 17,5 Hz, 1H : l'autre H du CH₂ en 5β) ; de 3,15 à 3,35 (mt, 2H : l'autre H du CH₂ en 4β et 1H du CH₂ en 3δ) ; 3,22 (s, 3H : NCH₃) ; de 3,45 à 3,65 (mt, 5H : l'autre H du CH₂ en 3δ et les 2 NCH₂ de la pyrrolidine) ; 3,73 (d, J = 17 Hz, 1H : 1H du CH₂ en 5ε) ; 4,60 (dd, J = 6,5 et 5,5 Hz, 1H : CH en 3α) ; 4,78 (mt, 1H : CH en 2α) ; 4,88 (d large, J = 10 Hz, 1H : CH en 1α) ; 5,14 (dd, J = 12 et 4,5 Hz, 1H : CH en 4α) ; 5,29 (d, J = 17 Hz, 1H : l'autre H du CH₂ en 5ε) ; 5,31 (mf, 1H : CH en 5α) ; 5,64 (d, J = 8,5 Hz, 1H : CH en 6α) ; 5,87 (q large, J = 7 Hz, 1H : CH en 1β) ; 6,28 (d, J = 8 Hz, 2H : H aromatiques en 4ε) ; 6,56 (d, J = 9,5 Hz, 1H : CONH en 2) ; 6,82 (d, J = 8 Hz, 2H : H aromatiques en 4δ) ; de 7,20 à 7,40 (mt : les 5H aromatiques en 6α) ; 7,42 (AB limite, 2H : l' H₄ et 1' H₅) ; 7,90 (mt, 1H : l' H₆) ; 7,98 (s, 1H : CH=N) ; 8,42 (d, J = 10 Hz, 1H : CONH en 1) ; 8,62 (d, J = 8,5 Hz, 1H : CONH en 6) ; 11,65 (s, 1H : OH).

### Exemple 33

Dans un tricol contenant 400 cm3 de méthanol, on introduit 30 g de pristinamycine I_{A}, 2,42 g de 3-aminoacroléine puis 25,8 g d'acétate d'ammonium. Le mélange est porté 3 jours au reflux puis dilué par 1 litre d'eau distillée. Le précipité obtenu est filtré, séché, puis chromatographié sur 1 kg de silice (éluant : dichlorométhane/méthanol 98/2 en volumes). Le solide obtenu est purifié par CLHP sur silice C₈ 10 µm (éluant : eau : acétonitrile 70/30 contenant 0,1 % d'acide trifluoroacétique. Les fractions sont rassemblées, l'acétonitrile éliminé à 40°C sous pression réduite (2,7 kPa) et la phase aqueuse ajustée à pH 8 par 3 cm3 d'eau saturée en bicarbonate de sodium. Le précipité obtenu est filtré, rincé par 10 cm3 d'eau distillée puis 10 cm3 d'éther diéthylique pour donner après séchage à 40°C sous pression réduite (90 Pa), 0,45 g de pyrido [2,3-5γ,5δ] pristinamycine IE sous forme d'un solide blanc vers 170-180°C (déc.)

Spectre de R.M.N. 1H (400 MHz, CDCl₃, δ en ppm) : 0,92 (t, J = 7,5 Hz, 3H : CH₃ en 2γ) ; de 1,20 à 1,40 (mt, 2H : 1H du CH₂ en 3β et 1H du CH₂ en 3γ) ; 1,34 (d, J = 7 Hz, 3H : CH₃ en 1γ) ; de 1,55 à 1,85 (mt, 4H : l'autre H du CH₂ en 3γ - CH₂ en 2β et 1H du CH₂ en 5β) ; 2,04 (mt, 1H : l'autre H du CH₂ en 3β) ; 2,91 (s, 6H : ArN(CH₃)₂) ; 2,95 (dd, J = 12 et 5 Hz, 1H : 1H du CH₂ en 4β) ; 3,17 (t, J = 12 Hz, 1H : l'autre H du CH₂ en 4β) ; 3,24 (s, 3H : NCH₃) ; 3,30 (mt, 1H : 1H du CH₂ en 3δ) ; 3,43 (d large, J = 17 Hz, 1H : l'autre H du CH₂ en 5β) ; 3,52 (mt, 1H : l'autre H du CH₂ en 3δ) ; 3,91 (d, J = 17 Hz, 1H : 1H du CH₂ en 5ε) ; 4,58 (dd, J = 7 et 5,5 Hz, 1H : CH en 3α) ; 4,81 (mt, 1H : CH en 2α) ; 4,87 (dd, J = 10 et 1,5 Hz, 1H : CH en 1α) ; 5,13 (dd, J = 12 et 5 Hz, 1H : CH en 4α) ; 5,43 (mt, 1H : CH en 5α) ; 5,46 (d, J = 17 Hz, 1H : l'autre H du CH₂ en 5ε) ; 5,65 (d, J = 8,5 Hz, 1 H : CH en 6α) ; 5,87 (dq, J = 7 et 1,5 Hz, 1H : CH en 1β) ; 6,40 (d, J = 8 Hz, 2H : H aromatiques en 4ε) ; 6,58 (d, J = 9,5 Hz, 1H : CONH en 2) ; 6,85 (d, J = 8 Hz, 2H : H aromatiques en 4δ) ; de 7,20 à 7,40 (mt : les 5H aromatiques en 6α) ; de 7,30 à 7,45 (mt, 1H : H aromatique en β de 1'N) ; 7,43 (d, J = 8 Hz, 1H : 1'H₄) ; 7,56 (dd, J = 8 et 4 Hz, 1H : 1' H₅) ; 7,61 (mt, 1 H : H aromatique en γ de 1'N) ; 8,13 (mt, 1H : 1' H₆) ; 8,38 (d, J = 4 Hz, 1 H : H aromatique en α de 1'N) ; 8,42 (d, J = 10 Hz, 1 H : CONH en 1) ; 8,69 (d, J = 8,5 Hz, 1H : CONH en 6) ; 11,59 (s, 1H : OH).

La 3-amino acroléine peut être préparée suivant R.P. Thummel & D.K. Kohli, J. Org. Chem., 42, 2742-2747 (1977).

### Exemple 34

En opérant comme à l'exemple 6, mais à partir de 11,4 g de 5δ-méthylène pristinamycine I_{A} dans 200 cm³ d'acétone, de 3,8 g de bromure de 1-(2-oxo-pentyl) pyridinium, de 10 g d'acétate d'ammonium et chauffage 3 heures au reflux, on obtient un solide qui est chromatographié sur 100 g de silice (éluant : acétonitrile) puis par CLHP sur 450 g de silice C₈ 10µm (éluant : eau-acétonitrile 70/30 en volumes, contenant 0,1 % d'acide trifluoroacétique). Les fractions sont rassemblées, l'acétonitrile éliminé à 40°C sous pression réduite (2,7 kPa) et le pH de la phase aqueuse ajusté à 8 par addition d'eau saturée en bicarbonate de sodium. Le précipité est filtré, lavé par 20 cm3 d'eau distillée et séché à 40°C sous pression réduite (90 Pa), pour donner 0,8 g de 2"-propyl-pyrido [2,3-5γ,5δ] pristinamycine I_{E} sous forme d'un solide blanc fondant à 172°C.

Spectre de RMN ¹H (400 MHz, CDCl₃): 0,93 (t, J = 7,5 Hz, 3H : CH₃ en 2γ) ; 0,98 (t, J = 7,5 Hz, 3H : CH₃ du propyle) ; de 1,20 à 1,35 (mt, 2H : 1H du CH₂ en 3β et 1H du CH₂ en 3γ) ; 1,30 (d, J = 7 Hz, 3H : CH₃ en 1γ) ; 1,58 (mt, 1H : l'autre H du CH₂ en 3γ) ; de 1,50 à 1,90 (mt : les 3H correspondant au CH₂ en 2β et à 1H du CH₂ en 5β) ; 1,70 (mt, 2H : CH₂ central du propyle) ; 2,03 (mt, 1H : l'autre H du CH₂ en 3β) ; 2,68 (t, J = 8 Hz, 2H : ArCH₂ du propyle) ; 2,84 (s, 6H : ArN(CH₃)₂) ; 2,97 (dd, J = 13 et 5,5 Hz, 1H : 1H du CH₂ en 4β) ; de 3,15 à 3,35 (mt, 3H : l'autre H du CH₂ en 5β - l'autre H du CH₂ en 4β et 1H du CH₂ en 3δ) ; 3,21 (s, 3H : NCH₃) ; 3,49 (mt, 1H : l'autre H du CH₂ en 3δ) ; 3,92 (d, J = 17 Hz, 1H : 1H du CH₂ en 5ε) ; 4,60 (dd, J = 8 et 5,5 Hz, 1H : CH en 3α) ; 4,79 (mt, 1H : CH en 2α) ; 4,87 (dd, J = 10 et 1 Hz, 1H : CH en 1α) ; 5,28 (dd, J = 10 et 5,5 Hz, 1H : CH en 4α) ; de 5,35 à 5,50 (mt, 2H : CH en 5α et l'autre H du CH₂ en 5ε) ; 5,63 (d, J = 8 Hz, 1H : CH en 6α) ; 5,88 (q dédoublé, J = 7 et 1 Hz, 1H : CH en 1β) ; 6,36 (d, J = 8 Hz, 2H : H aromatiques en 4ε) ; 6,58 (d, J = 9,5 Hz, 1H : CONH en 2) ; 6,84 (d, J = 8 Hz, 2H : H aromatiques en 4δ) ; 6,94 (d, J = 8 Hz, 1H : H aromatique en β de 1'N) ; de 7,20 à 7,45 (mt : les 8H correspondant aux 5 H aromatiques en 6α - à 1'H aromatique en γ de 1'N - au 1' H₅ et au 1' H4) ; 7,86 (dd, J = 4 et 1 Hz, 1H : 1'H₆) ; 8,43 (d, J = 10 Hz, 1H : CONH en 1) ; 8,66 (d, J = 8 Hz, 1H : CONH en 6) ; 11,64 (s, 1H : OH).

Le bromure de 1-(2-oxo-pentyl) pyridinium peut être préparé par analogie avec l'iodure de 1-(2-oxo-pentyl) pyridinium comme décrit par R.P. SONI, J. P. SAXENA, J. Indian Chem. Soc., 58, 885-887 (1981).

Dans un tricol contenant 25 cm3 d'éthanol, on introduit 3,8 g de 1-bromo-2-pentanone et 9,2 cm3 de pyridine puis le mélange est chauffé 3 heures au reflux. Après concentration à sec à 40°C sous pression réduite (2,7 kPa), le résidu est repris dans 200 cm3 d'éther diisopropylique. Après filtration, lavage par 50 cm3 d'éther diéthylique, le précipité est séché pour donner 3,8 g d'un solide jaune pâle de pureté 80 % fondant à 72°C et utilisé tel quel.

La 1-bromo-2-pentanone peut-être préparée selon H.J. HA , Synth. Commun., 24, 2557, (1994)

### Exemple 35

En opérant comme à l'exemple 6, mais à partir de 30 g de 5δ-méthylène pristinamycine I_{A} dans 200 cm3 d'acétone, de 10 g de bromure de 1-(3-méthyl-2-oxo-butyl) pyridinium, de 26,3 g d'acétate d'ammonium et chauffage 3 heures au reflux, on obtient 34 g d'un solide qui est purifié par 2 chromatographies successives sur 1 kg de silice (éluant: chlorure de méthylène-acétonitrile-eau : 96/2/2 en volumes) puis sur 700 g de silice (éluant : gradient chlorure de méthylène puis chlorure de méthylène-méthanol-acétonitrile : 99/0,5/0,5 à 98/1/1 en volumes). Après 2 recristallisations dans le méthanol, on obtient 4,3 g de produit dont 2 g sont purifiés par CLHP sur 450 g de silice C₈ 10µm (éluant : eau-acétonitrile 70/30 en volumes, contenant 0, 1 % d'acide trifluoroacétique). Les fractions sont rassemblées, l'acétonitrile éliminé à 40°C sous pression réduite (2,7 kPa) et le pH de la phase aqueuse ajusté à 8 par addition d'eau saturée en bicarbonate de sodium. Le précipité est filtré, lavé par 20 cm3 d'eau puis par 20 cm3 d'éther diisopropylique. Après recristallisation dans 15 cm3 de méthanol, filtration, lavage par 10 cm3 de méthanol et 10 cm3 d'éther diisopropylique puis séchage à 40°C, sous pression réduite (90 Pa), on obtient 0,75 g de 2"-isopropyl-pyrido [2,3-5γ,5δ] pristinamycine I_{E} sous forme d'aiguilles blanches fondant à 263°C.

Spectre de RMN ¹H (400 MHz, CDCl₃) : 0,92 (t, J = 7,5 Hz, 3H : CH₃ en 2γ) ; de 1,20 à 1,35 (mt, 11H : 1H du CH₂ en 3β - 1H du CH₂ en 3γ - CH₃ en 1γ et les 2 CH₃ de l'isopropyle) ; 1,58 (mt, 1H : l'autre H du CH₂ en 3γ) ; 1,66 et 1,75 (2 mts, 1H chacun : CH₂ en 2β) ; 1,89 (mt : 1H correspondant à 1H du CH₂ en 5β) ; 2,03 (mt, 1H : l'autre H du CH₂ en 3β) ; 2,85 (s, 6H : ArN(CH₃)₂) ; de 2,95 à 3,05 (mt, 1 H : ArCH de l'isopropyle) ; 2,99 (dd, J = 14 et 6,5 Hz, 1H : 1H du CH₂ en 4β) ; de 3,15 à 3,30 (mt, 3H : l'autre H du CH₂ en 5β - l'autre H du CH₂ en 4β et 1H du CH₂ en 3δ) ; 3,20 (s, 3H : NCH₃) ; 3,49 (mt, 1H : l'autre H du CH₂ en 3δ) ; 3,93 (d, J = 17 Hz, 1H : 1H du CH₂ en 5ε) ; 4,60 (dd, J = 7,5 et 6 Hz, 1H : CH en 3α) ; 4,79 (mt, 1H : CH en 2α) ; 4,88 (d large, J = 10 Hz, 1H : CH en 1α) ; 5,32 (dd, J = 9 et 6,5 Hz, 1H : CH en 4α) ; de 5,40 à 5,50 (mt, 2H : CH en 5α et l'autre H du CH₂ en 5ε) ; 5,64 (d, J = 8 Hz, 1H : CH en 6α) ; 5,88 (q large, J = 7 Hz, 1 H : CH en 1β) ; 6,39 (d, J = 8 Hz, 2H : H aromatiques en 4ε) ; 6,60 (d, J = 9,5 Hz, 1H : CONH en 2) ; 6,85 (d, J = 8 Hz, 2H : H aromatiques en 4δ) ; 6,99 (d, J = 8 Hz, 1H : H aromatique en β de l'N) ; de 7,20 à 7,40 (mt : les 8H correspondant aux 5 H aromatiques en 6α - à 1'H aromatique en γ de 1'N - au 1' H₅ et au 1' H₄) ; 7,85 (d large, J = 4 Hz, 1H : 1' H₆) ; 8,44 (d, J = 10 Hz, 1H : CONH en 1) ; 8,69 (d, J = 8 Hz, 1H : CONH en 6) ; 11,66 (s, 1H : OH).

Le bromure de 1-(3-méthyl-2-oxo-butyl) pyridinium peut être préparé comme décrit par J. P. SAXENA, J. Indian Chem. Soc., 68, 99-100 (1991).

### Exemple 36

En opérant comme à l'exemple 5, mais à partir d'1,5 litre d'acétonitrile, 100 g de 5δ-méthylène pristinamycine I_{A}, 27,1 g de chlorure de 1-(3-chloro-2-oxo-propyl) pyridinium, 88 g d'acétate d'ammonium et reflux 5 heures, on obtient un solide qui est purifié par deux chromatographies successives sur 1,5 kg et 100 g de silice (éluant : chlorure de méthylène-méthanol 97/3 en volumes). Les fractions contenant le produit attendu sont concentrées pour donner un solide qui est purifié par CLHP sur 450 g de silice C₈ 10µm (éluant : eau-acétonitrile 70/30 en volumes, contenant 0,1 % d'acide trifluoro acétique). Les fractions sont rassemblées, l'acétonitrile éliminé à 40°C sous pression réduite (2,7 kPa) et le pH de la phase aqueuse ajusté à 8 par addition d'eau saturée en bicarbonate de sodium. La phase aqueuse est extraite par 2 fois 50 cm3 de chlorure de méthylène. Les phases organiques sont réunies, séchées sur sulfate de sodium, filtrées, concentrées sous pression réduite (45°C, 2,7 kPa) et le solide obtenu est repris par 20 cm3 d'éther diéthylique. Après filtration puis séchage à 40°C sous pression réduite (90 Pa), on obtient 0,5 g de 2"-acétoxyméthyl-pyrido [2,3-5γ,5δ] pristinamycine I_{E} sous forme d'un solide crème fondant à 206°C.

Spectre de RMN ¹H (400 MHz, CDCl₃) : 0,91 (t, J = 7,5 Hz, 3H : CH₃ en 2γ) ; 1,25 (mt, 2H : 1H du CH₂ en 3β et 1H du CH₂ en 3γ) ; 1,30 (d, J = 7 Hz, 3H : CH₃ en 1γ) ; 1,58 (mt, 1H : l'autre H du CH₂ en 3γ) ; de 1,60 à 1,85 (mts : les 3H correspondant au CH₂ en 2β et à 1H du CH₂ en 5β) ; 2,03 (mt, 1H : l'autre H du CH₂ en 3β) ; 2,14 (s, 3H : OCOCH₃) ; 2,84 (s, 6H : ArN(CH₃)₂) ; 2,96 (dd, J = 13 et 5,5 Hz, 1H : 1H du CH₂ en 4β) ; 3,14 (d, J = 16,5 Hz, 1H : l'autre H du CH₂ en 5β) ; de 3,15 à 3,30 (mt, 2H : l'autre H du CH₂ en 4β et 1H du CH₂ en 3δ) ; 3,22 (s, 3H : NCH₃) ; 3,49 (mt, 1H : l'autre H du CH₂ en 3δ) ; 3,93 (d, J = 17 Hz, 1H : 1H du CH₂ en 5ε) ; 4,60 (dd, J = 8 et 5,5 Hz, 1H : CH en 3α) ; 4,79 (mt, 1H : CH en 2α) ; 4,88 (dd, J = 10 et 1 Hz, 1H : CH en 1α) ; 5,07 et 5,18 (2d, J = 13 Hz, 1H chacun : ArCH₂OCO) ; de 5,15 à 5,25 (mt, 1H : CH en 4α) ; 5,40 (d large, J = 5,5 Hz, 1H : CH en 5α) ; 5,45 (d, J = 17 Hz, 1H : l'autre H du CH₂ en 5ε) ; 5,60 (d, J = 8 Hz, 1H : CH en 6α) ; 5,88 (q dédoublé, J = 7 et 1 Hz, 1H : CH en 1β) ; 6,34 (d, J = 8 Hz, 2H : H aromatiques en 4ε) ; 6,56 (d, J = 9,5 Hz, 1H : CONH en 2) ; 6,85 (d, J = 8 Hz, 2H : H aromatiques en 4δ) ; 7,15 (d, J = 8 Hz, 1H : H aromatique en β de 1'N) ; de 7,20 à 7,35 (mt : les 5H correspondant aux H aromatiques en 6α) ; 7,36 (d, J = 8 Hz, 1H : H aromatique en γ de l'N) ; 7,40 (mt, 2H : 1' H₅ et 1' H₄) ; 7,89 (mt, 1H : 1' H₆) ; 8,40 (d, J = 10 Hz, 1H : CONH en 1) ; 8,68 (d, J = 8 Hz, 1H : CONH en 6) ; 11,65 (s, 1H : OH).

### Exemple 37

En opérant comme à l'exemple 20, mais à partir de 40 cm3 d'acétonitrile, de 2 g de 2"-chlorométhyl-pyrido [2,3-5γ,5δ] pristinamycine I_{E}, de 1,5 cm3 de cyclopropylamine et 0,34 g d'iodure de potassium, on obtient, après 24 heures de reflux, 2,2 g d'une meringue qui est purifiée par deux chromatographies successives sur 60 g de silice (éluant : chlorure de méthylène-méthanol 95/5 en volumes). Les-fractions sont rassemblées, séchées sur sulfate de sodium, filtrées et concentrées à 40°C, sous pression réduite (2,7 kPa); la meringue obtenue est délitée dans 30 cm3 d'éther diéthylique. Après filtration et séchage à 40°C sous pression réduite (90 Pa), on obtient 0,55 g de 2"-cyclopropylaminométhyl-pyrido [2,3-5γ,5δ] pristinamycine I_{E} sous forme d'un solide jaune fondant à 184°C.

Spectre de RMN ¹H (400 MHz, CDCl₃) : de 0,35 à 0,50 (mt, 4H : CH₂CH₂ du cyclopropane) ; 0,92 (t, J = 7,5 Hz, 3H : CH₃ en 2γ) ; 1,26 (mt, 2H : 1H du CH₂ en 3β et 1H du CH₂ en 3γ); 1,30 (d, J = 7 Hz, 3H : CH₃ en 1γ) ; 1,58 (mt, 1H : l'autre H du CH₂ en 3γ) ; de 1,60 à 1,85 (mt : les 2H correspondant au CH₂ en 2β) ; 1,78 (dd, J = 16 et 6,5 Hz, 1H : 1H du CH₂ en 5β) ; 2,03 (mt, 1H : l'autre H du CH₂ en 3β) ; 2,16 (mt, 1 H : CH du cyclopropane) ; 2,86 (s, 6H : ArN(CH₃)₂) ; 2,97 (dd, J = 13,5 et 6 Hz, 1H : 1H du CH₂ en 4β) ; de 3,15 à 3,30 (mt, 2H : l'autre H du CH₂ en 4β - l'autre H du CH₂ en 5β et 1H du CH₂ en 3δ) ; 3,22 (s, 3H : NCH₃) ; 3,49 (mt, 1H : l'autre H du CH₂ en 3δ) ; 3,88 (s, 2H : ArCH₂N) ; 3,94 (d, J = 17 Hz, 1H : 1H du CH₂ en 5ε) ; 4,60 (dd, J = 8 et 5,5 Hz, 1H : CH en 3α) ; 4,79 (mt, 1H : CH en 2α) ; 4,88 (dd, J = 10 et 1 Hz, 1H : CH en 1α) ; 5,26 (dd, J = 10 et 6 Hz, 1H : CH en 4α) ; de 5,40 à 5,50 (mt, 2H : CH en 5α et l'autre H du CH₂ en 5ε) ; 5,62 (d, J = 8 Hz, 1H : CH en 6α) ; 5,88 (q dédoublé, J = 7 et 1 Hz, 1 H : CH en 1β) ; 6,36 (d, J = 8 Hz, 2H : H aromatiques en 4ε) ; 6,57 (d, J = 9,5 Hz, 1H : CONH en 2) ; 6,85 (d, J = 8 Hz, 2H : H aromatiques en 4δ) ; 7,10 (d, J = 8 Hz, 1H : H aromatique en β de 1'N) ; de 7,20 à 7,35 (mt : les 6H correspondant aux 5H aromatiques en 6α et à 1'H aromatique en γ de 1'N) ; 7,39 (AB limite, 2H : 1' H₅ et 1' H₄) ; 7,87 (dd, J = 4 et 2 Hz, 1H : 1' H₆) ; 8,42 (d, J = 10 Hz, 1H : CONH en 1) ; 8,67 (d, J = 8 Hz, 1H : CONH en 6) ; 11,65 (mf, 1H : OH).

### Exemple 38

En opérant comme à l'exemple 20, mais à partir de 1,5 g de 2"-chlorométhyl-pyrido [2,3-5γ,5δ] pristinamycine I_{E} dans 30 cm3 d'acétonitrile, 0,5 cm3 de diéthylamine, de 0,26 g d'iodure de potassium et après 6 heures de reflux à 45°C, on obtient 1,35 g de produit qui est purifié par CLHP sur 450 g de silice C₈ 10µm (éluant : eau-acétonitrile 60/40 en volumes, contenant 0,1% d'acide trifluoroacétique). Les fractions sont rassemblées et l'acétonitrile éliminé à 40°C sous pression réduite (2,7 kPa). La phase aqueuse est amenée à pH 8 par addition d'eau saturée en bicarbonate de sodium puis extrait par 300 cm3 d'acétate d'éthyle. La phase oganique est décantée, séchée sur sulfate de sodium, filtrée puis concentrée sous pression réduite (45°C, 2,7 kPa), pour donner un solide qui est cristallisé dans 30 cm3 de méthanol. Après filtration, lavage par 50 cm3 d'éther diisopropylique et séchage à 40°C, sous pression réduite (90 Pa), on obtient 0,4 g de 2"-N-diéthylaminométhyl-pyrido [2,3-5γ,5δ] pristinamycine I_{E} sous forme d'un solide blanc cotonneux fondant à 264°C.

Spectre de RMN ¹H (400 MHz, CDCl₃) : 0,92 (t, J = 7,5 Hz, 3H CH₃ en 2γ) ; 1,04 (t, J = 7 Hz, 6H : CH₃ du diéthylamino) ; de 1,20 à 1,35 (mt, 2H : 1H du CH₂ en 3β et 1H du CH₂ en 3γ) ; 1,30 (d, J = 7 Hz, 3H : CH₃ en 1γ) ; 1,58 (mt, 1H : l'autre H du CH₂ en 3γ) ; de 1,60 à 1,80 (mt : les 2H correspondant au CH₂ en 2β) ; 1,85 (dd, J = 16,5 et 5,5 Hz, 1H : 1H du CH₂ en 5β) ; 2,03 (mt, 1H : l'autre H du CH₂ en 3β) ; 2,55 (q, J = 7 Hz, 4H : les 2 NCH₂ du diéthylamino) ; 2,85 (s, 6H : ArN(CH₃)₂) ; 2,98 (dd, J = 14 et 6 Hz, 1H : IH du CH₂ en 4β) ; de 3,15 à 3,30 (mt, 3H : l'autre H du CH₂ en 4β - l'autre H du CH₂ en 5β et 1H du CH₂ en 3δ) ; 3,21 (s, 3H : NCH₃) ; 3,50 (mt, 1H : l'autre H du CH₂ en 3δ) ; 3,66 (s, 2H : ArCH₂N) ; 3,94 (d, J = 17 Hz, 1H : 1H du CH₂ en 5ε) ; 4,60 (dd, J = 7,5 et 5,5 Hz, 1H : CH en 3α) ; 4,79 (mt, 1H : CH en 2α) ; 4,88 (d large, J = 10 Hz, 1H : CH en 1α) ; 5,30 (dd, J = 9 et 6 Hz, 1H : CH en 4α) ; 5,42 (d large, J = 5,5 Hz, 1H : CH en 5α) ; 5,43 (d, J = 17 Hz, 1H : l'autre H du CH₂ en 5ε) ; 5,64 (d, J = 8,5 Hz, 1H : CH en 6α) ; 5,88 (q large, J = 7 Hz, 1H : CH en 1β) ; 6,37 (d, J = 8 Hz, 2H : H aromatiques en 4ε) ; 6,58 (d, J = 9,5 Hz, 1H : CONH en 2) ; 6,85 (d, J = 8 Hz, 2H : H aromatiques en 4δ) ; de 7,20 à 7,40 (mt : les 9H correspondant aux 5H aromatiques en 6α - à l'H aromatique en β de 1'N - à 1'H aromatique en γ de 1'N - au 1' H₄ et au 1' H₅) ; 7,85 (d large, J = 4 Hz, 1H : 1' H₆) ; 8,43 (d, J = 10 Hz, 1H : CONH en 1) ; 8,68 (d, J = 8,5 Hz, 1H : CONH en 6) ; 11,65 (s large, 1H : OH).

La 2"-chlorométhyl-pyrido [2,3-5γ,5δ] pristinamycine I_{E} peut être obtenue comme décrit à l'exemple 10.

### Exemple 39

En opérant comme à l'exemple 6, mais en partant de 5,6 g de 5δ-méthylène virginiamycine S dans 100 cm3 d'acétonitrile, de 1,15 g de chlorure de 1-acétonyl pyridinium, de 5,17 g d'acétate d'ammonium et chauffage 4 heures à reflux, on obtient une huile rouge qui est chromatographiée sur 500 g de silice (éluant : chlorure de méthylène-méthanol 98/2 en volumes) pour donner 2,1 g de meringue jaune. Celle-ci est purifiée par CLHP sur 450 g de silice C₈ 10µm (éluant : eau-acétonitrile 65/35 en volumes, contenant 0,1 % d'acide trifluoroacétique). Les fractions sont rassemblées, l'acétonitrile éliminé à 40°C sous pression réduite (2,7 kPa), le pH de la phase aqueuse ajusté à 7 par addition d'eau saturée en bicarbonate de sodium; le précipité obtenu est filtré, lavé par 20 cm3 d'eau puis 20 cm3 d'éther diéthylique. Après filtration et séchage à 40°C, sous pression réduite (90 Pa), on obtient 0,39 g de 2"-méthyl-pyrido [2,3-5γ,5δ] 5γ-déoxy virgiamycine S sous forme d'un solide blanc fondant à 176°C.

La 5δ-méthylène virginiamycine S peut être obtenue comme décrite.

Spectre de RMN ¹H (400 MHz, CDCl₃) : 0,93 (t, J = 7,5 Hz, 3H : CH₃ en 2γ) ; 1,27 (mt, 2H : 1H du CH₂ en 3β et 1H du CH₂ en 3γ) ; 1,31 (d, J = 7 Hz, 3H : CH₃ en 1γ) ; de 1,50 à 1,85 (mt : les 4H correspondant à l'autre H du CH₂ en 3γ - au CH₂ en 2β et 1H du CH₂ en 5β) 2,04 (mt, 1H : l'autre H du CH₂ en 3β) ; 2,50 (s, 3H : ArCH₃) ; 3,07 (dd, J = 13 et 6 Hz, 1H : 1H du CH₂ en 4β) ; de 3,15 à 3,35 (mt, 3H : l'autre H du CH₂ en 4β - l'autre H du CH₂ en 5β et 1H du CH₂ en 3δ) ; 3,22 (s, 3H : NCH₃) ; 3,51 (mt, 1H : l'autre H du CH₂ en 3δ) ; 3,92 (d, J = 17,5 Hz, 1H : 1H du CH₂ en 5ε) ; 4,58 (dd, J = 8 et 6,5 Hz, 1H : CH en 3α) ; 4,80 (mt, 1H : CH en 2α) ; 4,87 (d large, J = 10 Hz, 1H : CH en 1α) ; 5,34 (dd, J = 10 et 6 Hz, 1H : CH en 4α) ; de 5,35 à 5,45 (mt, 2H : l'autre H du CH₂ en 5ε et CH en 5α) ; 5,64 (d, J = 8 Hz, 1H : CH en 6α) ; 5,88 (q large, J = 7 Hz, 1H : CH en 1β) ; 6,56 (d, J = 9,5 Hz, 1H : CONH en 2) ; de 6,95 à 7,40 (mt : les 13H correspondant aux 5H aromatiques en 6α - aux 5H aromatiques en 4β - à 1'H aromatique en γ de 1'N - au 1' H₄ et au 1' H₅) ; 6,96 (d, J = 8 Hz, 1H : H aromatique en β de 1'N) ; 7,81 (d large, J = 4 Hz, 1H : 1' H₆) ; 8,42 (d, J = 10 Hz, 1H : CONH en 1) ; 8,64 (d, J = 8 Hz, 1H : CONH en 6) ; 11,65 (s, 1H : OH).

### Exemple 40

En opérant par analogie avec l'exemple 15, mais à partir de 4ε-chloro 5δ-méthylène pristinamycine IA, on obtient la 4 ε-chloro 2"-(2-pyridyl)-pyrido [2,3-5γ,5δ] pristinamycine I_{E} sous forme d'un solide blanc fondant à 194°C.

Spectre de RMN ¹H (400 MHz, CDCl₃) : 0,93 (t, J = 7,5 Hz, 3H : CH₃ en 2γ) ; de 1,20 à 1,35 (mt, 2H : 1H du CH₂ en 3β et 1H du CH₂ en 3γ) ; 1,31 (d, J = 7 Hz, 3H : CH₃ en 1γ) ; 1,58 (mt, 1H : l'autre H du CH₂ en 3γ) ; de 1,60 à 1,85 (mt : les 3H correspondant au CH₂ en 2β et à 1H du CH₂ en 5β) ; 2,04 (mt, 1H : l'autre H du CH₂ en 3β) ; 2,56 (s, 6H : ArN(CH₃)₂) 2,99 (dd, J = 13 et 5,5 Hz, 1H : 1H du CH₂ en 4β); de 3,20 à 3,35 (mt, 2H : l'autre H du CH₂ en 4β et 1H du CH₂ en 3δ) ; 3,23 (s, 3H : NCH₃) ; 3,36 (d, J = 16,5 Hz, 1H : l'autre H du CH₂ en 5β) ; 3,51 (mt, 1H : l'autre H du CH₂ en 3δ) ; 4,01 (d, J = 17 Hz, 1 H : 1 H du CH₂ en 5ε) ; 4,60 (dd, J = 7,5 et 6 Hz, 1H : CH en 3α) ; 4,81 (mt, 1H : CH en 2α) ; 4,91 (dd, J = 10 et 1 Hz, 1H : CH en 1α); 5,26 (dd, J = 10 et 5,5 Hz, 1H : CH en 4α) ; 5,46 (d large, J = 5,5 Hz, 1H : CH en 5α); 5,53 (d, J = 17 Hz, 1H : l'autre H du CH₂ en 5ε) ; 5,63 (d, J = 8,5 Hz, 1H : CH en 6α); 5,89 (q dédoublé, J = 7 et 1 Hz, 1H : CH en 1β) ; 6,47 (d, J = 8 Hz, 1H : H aromatique en 4ε) ; 6,57 (d, J = 9,5 Hz, 1H : CONH en 2) ; 6,77 (dd, J = 8 et 2 Hz, 1H : H aromatique en 4δ en para du Cl) ; 7,13 (d, J = 2 Hz, 1H : H aromatique en 4δ en ortho du Cl) ; de 7,20 à 7,40 (mt : les 6H correspondant aux 5H aromatiques en 6α et au H₅ de la pyridine) ; 7,41 (d large, J = 8 Hz, 1H : 1' H₄) ; 7,50 (d, J = 8 Hz, 1H : H aromatique en γ de l'N) ; 7,53 (dd, J = 8 et 4,5 Hz, 1H : 1' H₅) ; 7,74 (t dédoublé, J = 8 et 1,5 Hz, 1H : H₄ de la pyridine) ; 7,90 (d large, J = 4,5 Hz, 1H : 1' H₆) ; 8,24 (d, J = 8 Hz, 1H : H aromatique en P de 1'N) ; 8,37 (d, J = 8 Hz, 1H : H₃ de la pyridine) ; 8,38 (d, J = 10 Hz, 1H : CONH en 1) ; 8,64 (d, J = 8,5 Hz, 1H : CONH en 6); 8,67 (d large, J = 4,5 Hz, 1H : H₆ de la pyridine) ; 11,67 (s, 1H : OH).

### Exemple 41

En opérant par analogie avec l'exemple 18, mais à partir de 4ε-chloro 5δ-méthylène pristinamycine IA, on obtient la 4ε-chloro 2"-(2-pyridyl)-pyrido [2,3-5γ,5δ] (4ξ-méthylamino) (4ξ-désdiméthylamino) pristinamycine I_{E}, sous forme d'un solide blanc fondant à 204°C.

Spectre de RMN ¹H (400 MHz, CDCl₃) : 0,93 (t, J = 7,5 Hz, 3H : CH₃ en 2γ) ; 1,27 (mt, 2H : 1H du CH₂ en 3β et 1H du CH₂ en 3γ) ; 1,31 (d, J = 7 Hz, 3H : CH₃ en 1γ) ; 1,59 (mt, 1H : l'autre H du CH₂ en 3γ) ; 1,67 et 1,75 (2 mts, 1H chacun : CH₂ en 2β) ; 1,84 (dd, J = 16,5 et 6 Hz, 1H : 1H du CH₂ en 5β) ; 2,04 (mt, 1H : l'autre H du CH₂ en 3β) ; 2,53 (s, 3H : ArNCH₃) ; 2,94 (dd, J = 13,5 et 6 Hz, 1H : 1H du CH₂ en 4β) ; de 3,15 à 3,30 (mt, 2H : l'autre H du CH₂ en 4β et 1H du CH₂ en 3δ) ; 3,22 (s, 3H : NCH₃) ; 3,35 (d, J = 16,5 Hz, 1H : l'autre H du CH₂ en 5β) ; 3,49 (mt, 1H : l'autre H du CH₂ en 3δ) ; 4,00 (d, J = 17 Hz, 1H : 1H du CH₂ en 5ε) ; 4,60 (dd, J = 8 et 7 Hz, 1H : CH en 3α) ; 4,79 (mt, 1H : CH en 2α) ; 4,89 (d large, J = 10 Hz, 1H : CH en 1α); 5,19 (dd, J = 10 et 6 Hz, 1H : CH en 4α) ; de 5,45 à 5,55 (mt, 2H : CH en 5α et l'autre H du CH₂ en 5ε) ; 5,64 (d, J = 8,5 Hz, 1H : CH en 6α) ; 5,88 (q large, J = 7 Hz, 1H : CH en 1β) ; 6,09 (d, J = 8 Hz, 1H : H aromatique en 4ε) ; 6,57 (d, J = 9,5 Hz, 1H : CONH en 2) ; 6,75 (d large, J = 8 Hz, 1H : H aromatique en 4δ en para du CI) ; 6,95 (s large, 1H : H aromatique en 4δ en ortho du Cl) ; de 7,20 à 7,40 (mt : les 6H correspondant aux 5H aromatiques en 6α et au H₅ de la pyridine) ; 7,41 (d large, J = 8 Hz, 1H : 1 ' H₄) ; 7,48 (d, J = 8 Hz, 1H : H aromatique en γ de 1'N) ; 7,51 (dd, J = 8 et 4 Hz, 1H : 1 ' H₅) ; 7,77 (t large, J = 8 Hz, 1H : H₄ de la pyridine) ; 7,97 (d large, J = 4 Hz, 1H : 1' H₆) ; 8,19 (d, J = 8 Hz, 1H : H aromatique en β de 1'N); de 8,3 5 à 8,45 (mt, 2H : H₃ de la pyridine et CONH en 1) ; 8,63 (d, J = 8,5 Hz, 1H : CONH en 6) ; 8,67 (d large, J = 4,5 Hz, 1H : H₆ de la pyridine) ; 11,67 (s, 1H : OH).

### Exemple 42

En opérant par analogie avec l'exemple 7, mais à partir de 4ε-chloro 5δ-méthylène pristinamycine IA, on obtient la 4ε-chloro 2"-éthyl-pyrido [2,3-5γ,5δ] pristinamycine I_{E} sous forme d'un solide blanc fondant à 184°C.

Spectre de RMN ¹H (400 MHz, CDCl₃) : 0,91 (t, J = 7,5 Hz, 3H : CH₃ en 2γ) ; de 1,20 à 1,40 (mt, 2H : 1H du CH₂ en 3β et 1H du CH₂ en 3γ) ; 1,27 (t, J = 7,5 Hz, 3H : CH₃ de l'éthyle) ; 1,30 (d, J = 7 Hz, 3H : CH₃ en 1γ) ; 1,59 (mt, 1H : l'autre H du CH₂ en 3γ) ; 1,67 et 1,75 (2 mts, 1H chacun : CH₂ en 2β) ; 1,90 (dd, J = 16 et 5,5 Hz, 1H : 1H du CH₂ en 5β) ; 2,04 (mt, 1H : l'autre H du CH₂ en 3β) ; 2,72 (s, 6H : ArN(CH₃)₂) ; 2,77 (mt, 2H : ArCH₂ de l'éthyle) ; 3,01 (dd, J = 14 et 7 Hz, 1 H : 1 H du CH₂ en 4β) ; de 3,15 à 3,25 (mt, 1 H : l'autre H du CH₂ en 4β) ; 3,19 (s, 3H : NCH₃) ; de 3,25 à 3,35 (mt, 1H : 1H du CH₂ en 3δ) ; 3,33 (d, J = 16 Hz, 1H : l'autre H du CH₂ en 5β) ; 3,51 (mt, 1H : l'autre H du CH₂ en 3δ) ; 3,95 (d, J = 17,5 Hz, 1H : 1H du CH₂ en 5ε) ; 4,57 (t large, J = 6,5 Hz, 1H : CH en 3α) ; 4,78 (mt, 1H : CH en 2α) ; 4,89 (d large, J = 10 Hz, 1H : CH en 1α) ; 5,34 (mt, 1H : CH en 4α) ; 5,41 (d, J = 17,5 Hz, 1H : l'autre H du CH₂ en 5ε) ; 5,47 (d large, J = 5,5 Hz, 1H : CH en 5α) ; 5,61 (d, J = 8,5 Hz, 1H : CH en 6α) ; 5,88 (q large, J = 7 Hz, 1H : CH en 1β) ; 6,59 (mt, 2H : H aromatique en 4ε et CONH en 2) ; 6,79 (d large, J = 8 Hz, 1H : H aromatique en 4δ en para du Cl) ; 6,98 (d, J = 8 Hz, 1H : H aromatique en β de 1'N) ; 7,06 (s large, 1H : H aromatique en 4δ en ortho du Cl) ; de 7,20 à 7,40 (mt : les 8H correspondant aux 5H aromatiques en 6α - à 1'H aromatique en γ de 1'N - au 1 ' H₄ et au 1' H₅) 7,82 (d large, J = 4 Hz, 1H : 1' H₆) ; 8,38 (d, J = 10 Hz, 1H : CONH en 1) ; 8,64 (d, J = 8,5 Hz, 1H : CONH en 6) ; 11,67 (s, 1H : OH).

### Exemple 43

En opérant par analogie avec l'exemple 17, mais à partir de 4ε-chloro 5δ-méthylène pristinamycine IA, on obtient la 4ε-chloro 2"-(éthyl)-pyrido [2,3-5γ,5δ] (4ζ-méthylamino) (4ζ-désdiméthylamino) pristinamycine I_{E}, sous forme d'un solide blanc fondant à 186°C.

Spectre de RMN ¹H (400 MHz, CDCl₃) : 0,92 (t, J = 7,5 Hz, 3H : CH₃ en 2γ) ; de 1,20 à 1,35 (mt, 2H : 1H du CH₂ en 3β et 1H du CH₂ en 3γ) ; 1,28 (t, J = 7,5 Hz, 3H : CH₃ de l'éthyle) ; 1,31 (d, J = 7 Hz, 3H : CH₃ en 1γ) ; 1,58 (mt, 1H : l'autre H du CH₂ en 3γ) ; de 1,60 à 1,85 (mt : les 2H correspondant au CH₂ en 2β) ; 1,93 (dd, J = 16 et 6 Hz, 1H : 1H du CH₂ en 5β) ; 2,03 (mt, 1H : l'autre H du CH₂ en 3β) ; 2,76 (mt, 2H : ArCH₂ de l'éthyle) ; 2,77 (s, 3H : ArNCH₃) ; 2,96 (dd, J = 14 et 6,5 Hz, 1H : 1H du CH₂ en 4β) ; de 3,10 à 3,30 (mt, 2H : l'autre H du CH₂ en 4β et 1H du CH₂ en 3δ) ; 3,19 (s, 3H : NCH₃) ; 3,30 (d, J = 16 Hz, 1H : l'autre H du CH₂ en 5β) ; 3,50 (mt, 1H : l'autre H du CH₂ en 3δ) ; 3,95 (d, J = 17 Hz, 1H : 1H du CH₂ en 5ε) ; 4,21 (mf, 1H : ArNH) ; 4,60 (dd, J = 7,5 et 5,5 Hz, 1H : CH en 3α) ; 4,79 (mt, 1H : CH en 2α) ; 4,88 (dd, J = 10 et 1 Hz, 1H : CH en 1α) ; 5,28 (dd, J = 9 et 6,5 Hz, 1H : CH en 4α) ; 5,42 (d, J = 17 Hz, 1H : l'autre H du CH₂ en 5ε) ; 5,46 (d large, J = 6 Hz, 1H : CH en 5α) ; 5,63 (d, J = 8 Hz, 1H : CH en 6α) ; 5,89 (q dédoublé, J = 7 et 1 Hz, 1H : CH en 1β) ; 6,19 (d, J = 8 Hz, 1H : H aromatique en 4ε) ; 6,57 (d, J = 9,5 Hz, 1H : CONH en 2) ; 6,77 (dd, J = 8 et 1,5 Hz, 1H : H aromatique en 4δ en para du Cl) ; 6,94 (d, J = 1,5 Hz, 1H : H aromatique en 4δ en para du Cl) ; 6,98 (d, J = 8 Hz, 1H : H aromatique en β de l'N) ; de 7,20 à 7,45 (mt : les 6H correspondant aux 5H aromatiques en 6α et à l'H aromatique en γ de 1'N) ; 7,37 (AB limite, 2H : 1' H₄ et 1' H₅) ; 7,84 (dd, J = 4 et 1,5 Hz, 1H : l' H₆) ; 8,40 (d, J = 10 Hz, 1H : CONH en 1) ; 8,67 (d, J = 8 Hz, 1H : CONH en 6) ; 11,66 (s, 1H : OH).

### Exemple 44

En opérant par analogie avec l'exemple 6, mais à partir de 4ε-chloro 5δ-méthylène pristinamycine IA, on obtient la 4ε-chloro 2"-méthyl-pyrido [2,3-5γ,5δ] pristinamycine I_{E} sous forme de poudre jaune fondant à 210°C.

Spectre de RMN ¹H (400 MHz, CDCl₃) : 0,93 (t, J = 7,5 Hz, 3H : CH₃ en 2γ) ; de 1,20 à 1,40 (mt, 2H : 1H du CH₂ en 3β et 1H du CH₂ en 3γ) ; 1,29 (d,J = 7 Hz, 3H : CH₃ en 1γ) ; de 1,50 à 1,80 (mt : les 3H correspondant à l'autre H du CH₂ en 3γ et au CH₂ en 2β) ; 1,81 (dd, J = 16 et 5,5 Hz, 1H : 1H du CH₂ en 5β) ; 2,04 (mt, 1H : l'autre H du CH₂ en 3β) ; 2,48 (s, 3H : ArCH₃) ; 2,74 (s, 6H : ArN(CH₃)₂ ; 3,02 (dd, J = 14 et 6,5 Hz, 1H : 1H du CH₂ en 4β) ; de 3,15 à 3,30 (mt, 2H : l'autre H du CH₂ en 4β et 1H du CH₂ en 3δ) ; 3,20 (s, 3H : NCH₃) ; 3,28 (d, J = 16 Hz, 1H : l'autre H du CH₂ en 5β) ; 3,51 (mt, 1H : l'autre H du CH₂ en 3δ) ; 3,96 (d, J = 17 Hz, 1H : 1H du CH₂ en 5ε) ; 4,59 (dd, J = 7,5 et 7 Hz, 1H : CH en 3α) ; 4,80 (mt, 1H : CH en 2α) 4,90 (d large, J = 10 Hz, 1H : CH en 1α) ; 5,31 (dd, J = 9 et 6,5 Hz, 1H : CH en 4α) ; 5,40 (d, J = 17 Hz, 1H : l'autre H du CH₂ en 5ε) ; 5,47 (d large, J = 5,5 Hz, 1 H : CH en 5α) ; 5,61 (d, J = 8 Hz, 1H : CH en 6α) ; 5,89 (q large, J = 7 Hz, 1H : CH en 1β) ; 6,45 (d, J = 8 Hz, 1H : H aromatique en 4ε) ; 6,59 (d, J = 9,5 Hz, 1H : CONH en 2) ; 6,80 (d large, J = 8 Hz, 1 H : H aromatique en 4δ en para du Cl) ; 6,97 (d, J = 8 Hz, 1H : H aromatique en β de 1'N) ; 7,10 (s large, 1H : H aromatique en 4δ en ortho du Cl) ; de 7,20 à 7,35 (mt : les 6H correspondant aux 5H aromatiques en 6α et à l'H aromatique en γ de l'N) ; 7,37 (d large, J = 8 Hz, 1H : l' H₄) ; 7,42 (dd, J = 8 et 4,5 Hz, 1H : 1' H₅) ; 7,84 (d large, J = 4,5 Hz, 1H : 1' H₆) ; 8,37 (d, J = 10 Hz, 1H : CONH en 1) ; 8,61 (d, J = 8 Hz, 1H : CONH en 6) ; 11,68 (s, 1H : OH).

### Exemple 45

Dans un tricol contenant 70 cm3 de dioxane, on introduit 4 g de 2"-hydroxyméthyl-pyrido [2,3-5γ,5δ] pristinamycine I_{E}, 0,48 g d'oxyde de sélénium et on porte à reflux 1 heure. Le mélange réactionnel est filtré sur Celite® et le filtrat concentré sous pression réduite à 45°C (2,7 kPa) pour donner 5,7 g d'une meringue marron qui est purifiée par 2 chromatographies successives sur 60 g de silice (éluant : chlorure de méthylène-méthanol 97/3 en volume). Les fractions sont rassemblées et concentrées sous pression réduite à 45°C (2,7 kPa). Le solide obtenu est agité dans 30 cm3 d'éther diéthylique, filtré et séché à 40°C, sous pression réduite (90 Pa), pour donner 0,76 g de 2"-formyl-pyrido [2,3-5γ,5δ] pristinamycine I_{E} sous forme d'un solide blanc fondant à 202°C.

Spectre de RMN ¹H (400 MHz, CDCl₃) : 0,92 (t, J = 7,5 Hz, 3H : CH₃ en 2γ) ; de 1,20 à 1,35 (mt, 2H : 1H du CH₂ en 3β et 1H du CH₂ en 3γ) ; 1,31 (d, J = 7 Hz, 3H : CH₃ en 1γ) ; de 1,50 à 1,75 (mt : les 3H correspondant à l'autre H du CH₂ en 3γ - à 1H du CH₂ en 2β et à 1H du CH₂ en 5β) ; 1,74 (mt, 1H : l'autre H du CH₂ en 2β) ; 2,05 (mt, 1H : l'autre H du CH₂ en 3β) ; 2,79 (s, 6H : ArN(CH₃)₂) ; 2,95 (dd, J = 13 et 5 Hz, 1H : 1H du CH₂ en 4β) ; de 3,15 à 3,30 (mt, 3H : l'autre H du CH₂ en 4β - l'autre H du CH₂ en 5β et 1H du CH₂ en 3δ) ; 3,26 (s, 3H : NCH₃) ; 3,50 (mt, 1H : l'autre H du CH₂ en 3δ) ; 3,97 (d, J = 18 Hz, 1H : 1H du CH₂ en 5ε); 4,62 (dd, J = 7,5 et 6 Hz, 1H : CH en 3α) ; 4,81 (mt, 1H : CH en 2α) ; 4,89 (d large, J = 10 Hz, 1H : CH en 1α) ; 5,14 (dd, J = 11 et 5 Hz, 1H : CH en 4α) ; 5,44 (d large, J = 5,5 Hz, 1H : CH en 5α) ; 5,52 (d, J = 18 Hz, 1H : l'autre H du CH₂ en 5ε) ; 5,62 (d, J = 8,5 Hz, 1H: CH en 6α) ; 5,88 (q large, J = 7 Hz, 1H : CH en 1β) ; 6,29 (d, J = 8 Hz, 2H : H aromatiques en 4ε) ; 6,56 (d, J = 9,5 Hz, 1H : CONH en 2) ; 6,86 (d, J = 8 Hz, 2H : H aromatiques en 4δ) ; de 7,20 à 7,40 (mt : les 5H correspondant aux H aromatiques en 6α) ; 7,45 (d large, J = 8 Hz, 1H : 1' H₄) ; de 7,45 à 7,55 (mt, 2H : 1' H₅ et H aromatique en γ de 1'N) ; 7,77 (d, J = 8 Hz, 1H : H aromatique en β de 1'N) ; 8,00 (d large, J = 4 Hz, 1H : 1' H₆) ; 8,40 (d, J = 10 Hz, 1H : CONH en 1) ; 8,69 (d, J = 8,5 Hz, 1H : CONH en 6) ; 9,97 (s, 1H : COH) ; 11,65 (s, 1H : OH).

La 2"-hydroxyméthyl-pyrido [2,3-5γ,5δ] pristinamycine I_{E} peut être obtenue comme décrit à l'exemple 11.

### Exemple 46

Dans un tricol contenant 100 cm3 de dioxane, on introduit 1,7 g de 2"-formyl-pyrido [2,3-5γ,5δ] pristinamycine I_{E}, 1,4 g d'acétate d'ammonium et on porte à reflux 1 heure. Le mélange réactionnel est concentré sous pression réduite puis repris par 100 cm3 d'eau et 100 cm3 de chlorure de méthylène. Après décantation, séchage de la phase organique sur sulfate de sodium, filtration et concentration à sec on obtient 1,5 g d'un solide qui est chromatographié sur 60 g de silice (éluant : chlorure de méthylène-méthanol 97/3 en volume). Les fractions sont rassemblées puis concentrées sous pression réduite à 45°C (2,7 kPa). Le solide obtenu est repris par 30 cm3 d'éther diéthylique puis filtré et séché à 40°C, sous pression réduite (90 Pa), pour donner 0,32 g de 2"-carbamoyl-pyrido [2,3-5γ,5δ] pristinamycine I_{E} sous forme de solide orange fondant à 226°C.

Spectre de RMN ¹H (400 MHz, CDCl₃) : 0,92 (t, J = 7,5 Hz, 3H : CH₃ en 2γ) ; 1,26 (mt, 2H : 1H du CH₂ en 3β et 1H du CH₂ en 3γ) ; 1,30 (d, J = 7 Hz, 3H : CH₃ en 1γ) ; 1,43 (dd, J = 16,5 et 5,5 Hz, 1H : 1H du CH₂ en 5β) ; de 1,50 à 1,70 (mt : les 2H correspondant à l'autre H du CH₂ en 3γ et à 1H du CH₂ en 2β) ; 1,75 (mt, 1H : l'autre H du CH₂ en 2β) ; 2,06 (mt, 1H : l'autre H du CH₂ en 3β) ; 2,79 (s, 6H : ArN(CH3)₂); 2,93 (dd, J = 12,5 et 4,5 Hz, 1H : 1H du CH₂ en 4β) ; 3,08 (d, J = 16,5 Hz, 1H : l'autre H du CH₂ en 5β) ; de 3,15 à 3,30 (mt, 2H : l'autre H du CH₂ en 4β et 1H du CH₂ en 3δ) ; 3,26 (s, 3H : NCH₃) ; 3,49 (mt, 1H : l'autre H du CH₂ en 3δ) ; 3,94 (d, J = 17,5 Hz, 1H : 1H du CH₂ en 5ε) ; 4,62 (dd, J = 8 et 6,5 Hz, 1H : CH en 3α) ; 4,81 (mt, 1H : CH en 2α) ; 4,89 (d large, J = 10 Hz, 1H : CH en 1α) ; 5,09 (dd, J = 11,5 et 4,5 Hz, 1H : CH en 4α) ; 5,39 (d large, J = 5,5 Hz, 1H : CH en 5α) ; 5,48 (d, J = 5 Hz, 1H : 1H du CONH₂) ; 5,51 (d, J = 17,5 Hz, 1H : l'autre H du CH₂ en 5ε) ; 5,60 (d, J = 8,5 Hz, 1H : CH en 6α) ; 5,88 (q large, J = 7 Hz, 1H : CH en 1β) ; 6,28 (d, J = 8 Hz, 2H : H aromatiques en 4ε) ; 6,57 (d, J = 9,5 Hz, 1H : CONH en 2) ; 6,87 (d, J = 8 Hz, 2H : H aromatiques en 4δ) ; de 7,20 à 7,40 (mt : les 5H correspondant aux H aromatiques en 6α) ; 7,46 (d large, J = 8 Hz, 1H : 1' H₄) ; de 7,50 à 7,60 (mt, 2H : 1' H₅ et H aromatique en γ de 1'N) ; 7,78 (d, J = 5 Hz, 1H : l'autre H du CONH₂) ; 7,99 (mt, 1H : 1' H₆) ; 8,00 (d, J = 8 Hz, 1H : H aromatique en β de 1'N) ; 8,3 8 (d, J = 10 Hz, 1H : CONH en 1) ; 8,67 (d, J = 8,5 Hz, 1H : CONH en 6) ; 11,67 (s, 1H : OH).

La 2"-formyl-pyrido [2,3-5γ,5δ] pristinamycine I_{E} peut être obtenue comme décrit à l'exemple 45.

### Exemple 47

En opérant comme à l'exemple 22, mais à partir de 40 cm3 d'acétonitrile, de 4,6 g de 5δ-diméthylaminométhylène pristinamycine I_{A}, de 0,38 g d'acétamidine et chauffage 12 heures à 60°C, on obtient, après concentration à sec du mélange réactionnel à 45°C (2,7 kPa), un solide qui est chromatographié sur 400 g de silice (éluant : chlorure de méthylène-méthanol 97/3 en volumes). Les fractions sont réunies, séchées sur sulfate de sodium, filtrées et concentrées à 45°C sous pression réduite (2,7 kPa). Le solide obtenu est cristallisé dans 10 cm3 de méthanol puis filtré et séché à 40°C (90Pa), pour donner 0,4 g de 2"-méthyl-pyrimido [4,5-5γ,5δ] pristinamycine I_{E} sous forme de cristaux blancs fondant à 265°C.

Spectre de RMN ¹H (400 MHz, CDCl₃ avec ajout de (CD₃)₂SO d6) : 0,79 (t, J = 7,5 Hz, 3H : CH₃ en 2γ) ; de 1,05 à 1,20 (mt, 2H : 1H du CH₂ en 3β et 1 H du CH₂ en 3γ) ; 1,14 (d, J = 7 Hz, 3H : CH₃ en 1γ) ; 1,24 (dd, J = 17 et 5,5 Hz, 1H : 1H du CH₂ en 5β) ; 1,46 (mt, 1H : l'autre H du CH₂ en 3γ) ; 1,52 et 1,62 (2 mts, 1H chacun : CH₂ en 2β) ; 1,95 (mt, 1H : l'autre H du CH₂ en 3β) ; 2,51 (s, 3H : ArCH₃) ; 2,74 (s, 6H : ArN(CH₃)₂) ; de 2,75 à 2,85 (mt, 1H 1H du CH₂ en 4β) ; 2,83 (d, J = 17 Hz, 1H : l'autre H du CH₂ en 5β) ; de 3,05 à 3,20 (mt, 2H : l'autre H du CH₂ en 4β et 1H du CH₂ en 3δ) ; 3,13 (s, 3H : NCH₃) ; 3,37 (mt, 1H : l'autre H du CH₂ en 3δ) ; 3,70 (d, J = 17 Hz, 1H : 1H du CH₂ en 5ε), 4,48 (dd, J = 8 et 6 Hz, 1H : CH en 3α) ; 4,65 (mt, 1H : CH en 2α) ; 4,75 (d large, J = 10 Hz, 1 H : CH en 1α) ; 4,94 (dd, J = 11,5 et 5 Hz, 1 H : CH en 4α) ; 5,24 (d large J = 5,5 Hz, 1 H : CH en 5α) ; 5,30 (d, J = 17 Hz, 1H : l'autre H du CH₂ en 5ε) ; 5,45 (d, J = 8Hz, 1H : CH en 6α) ; 5,72 (q large, J = 7 Hz, 1H : CH en 1β) ; 6,20 (d, J = 8 Hz, 2H : H aromatiques en 4ε) ; 6,54 (d, J = 9,5 Hz, 1H : CONH en 2) ; 6,72 (d, J = 8 Hz, 2H : H aromatiques en 4δ) ; de 7,05 à 7,30 (mt : les 5H correspondant aux H aromatiques en 6α) ; 7,32 (d large, J = 8 Hz, 1H : 1'H₄) ; 7,37 (dd, J = 8 et 4 Hz, 1H : 1' H₅) ; 7,81 (d large, J = 4 Hz, 1 H : 1' H₆) ; 8,17 (s, 1H : CH=N) ; 8,22 (d, J = 10 Hz, 1H : CONH en 1) ; 8,56 (d, J = 8 Hz, 1 H : CONH en 6) ; 11,52 (s, 1H : OH).

### Exemple 48

En opérant comme à l'exemple 22, mais à partir de 40 cm3 de diméthylformamide, de 1,84 g de 5δ-diméthylaminométhylène pristinamycine I_{A}, de 0,41 g de chlorhydrate de 2-pyrazine carboxamidine, de 1 cm3 de diisopropylamine, le mélange réactionnel est chauffé 12 heures à 65°C. On ajoute 0,16 g de chlorhydrate de 2-pyrazine carboxamidine et on poursuit le chauffage 24 heures supplémentaires. Après traitement et concentration à sec du mélange réactionnel à 45°C (2,7 kPa), on obtient 2,1 g de solide qui est chromatographié sur 100 g de silice (éluant : chlorure de méthylène-méthanol 97/3 en volumes). Les fractions sont réunies, séchées sur sulfate de sodium, filtrées et concentrées à 45°C sous pression réduite (2,7 kPa). Le solide obtenu est cristallisé dans 10 cm3 de méthanol, filtré, lavé par 2 fois 5 cm3 d'éther diisopropylique puis séché à 40°C (90Pa), pour donner 0,49 g de 2"-(2-pyrazinyl)-pyrimido [4,5-5γ,5δ] pristinamycine I_{E} sous forme de cristaux jaunes fondant à 254°C.

Spectre de RMN ¹H (400 MHz, CDCl₃) : 0,93 (t, J = 7,5 Hz, 3H : CH₃ en 2γ) ; de 1,25 à 1,40 (mt, 2H : 1H du CH₂ en 3β et 1H du CH₂ en 3γ) ; 1,31 (d, J = 7 Hz, 3H : CH₃ en 1γ) ; 1,51 (dd, J = 17 et 6 Hz, 1H : 1H du CH₂ en 5β) ; de 1,55 à 1,65 (mt : 1H correspondant à l'autre H du CH₂ en 3γ) ; 1,67 et 1,75 (2 mts, 1H chacun : CH₂ en 2β) ; 2,08 (mt, 1H : l'autre H du CH₂ en 3β) ; 2,64 (s, 6H : ArN(CH₃)₂) ; 2,94 (dd, J = 12 et 5 Hz, 1H: 1H du CH₂ en 4β) ; 3,18 (d, J = 17 Hz, 1H : l'autre H du CH₂ en 5β) ; de 3,20 à 3,35 (mt, 1H : 1H du CH₂ en 3δ et l'autre H du CH₂ en 4β) ; 3,26 (s, 3H : NCH₃) ; 3,51 (mt, 1H : l'autre H du CH₂ en 3δ) ; 3,90 (d, J = 17,5 Hz, 1H : 1 H du CH₂ en 5ε) ; 4,61 (dd, J = 8 et 6 Hz, 1H : CH en 3α) ; 4,81 (mt, 1H : CH en 2α) ; 4,90 (dd, J = 10 et 1 Hz, 1H : CH en 1α) ; 5,12 (dd, J = 12 et 5 Hz, 1H : CH en 4α) ; 5,45 (d large, J = 6 Hz, 1H : CH en 5α) ; 5,53 (d, J = 17,5 Hz, 1H : l'autre H du CH₂ en 5ε) ; 5,65 (d, J = 8,5 Hz, 1H : CH en 6α) ; 5,89 (q dédoublé, J = 7 et 1 Hz, 1H : CH en 1β) ; 6,29 (d, J = 8 Hz, 2H : H aromatiques en 4ε) ; 6,55 (d, J = 10 Hz, 1H : CONH en 2) ; 6,87 (d, J = 8 Hz, 2H : H aromatiques en 4δ) ; de 7,20 à 7,40 (mt : les 5H correspondant aux H aromatiques en 6α) ; 7,48 (d large, J = 8,5 Hz, 1 H : 1' H₄) ; 7,53 (dd, J = 8,5 et 4 Hz, 1 H : 1' H₅) ; 8,02 (d large, J = 4 Hz, 1H : 1' H₆) ; 8,41 (d, J = 10 Hz, 1H : CONH en 1) ; 8,58 (s, 1H : CH=N) ; 8,67 (d, J = 2 Hz, 1H : H en 5 de la pyrazine) ; 8,74 (d, J = 8,5 Hz, 1H : CONH en 6) ; 8,77 (dd, J = 2 et 1,5 Hz, 1H : H en 6 de la pyrazine) ; 9,68 (d, J = 1,5 Hz, 1H : H en 3 de la pyrazine) ; 11,65 (s, 1H : OH).

### Exemple 49

En opérant comme à l'exemple 22, mais à partir de 40 cm3 de diméthylformamide, de 3 g de 5δ-diméthylaminométhylène pristinamycine I_{A}, de 0,74 g de chlorhydrate de 1H-pyrazole carboxamidine, de 2 cm3 de diisopropyléthylamine, le mélange réactionnel est chauffé 4 heures à 65°C. Après traitement et concentration à sec du mélange réactionnel à 45°C (2,7 kPa), on obtient 2,4 g d'un solide qui est chromatographié sur 160 g de silice (éluant : chlorure de méthylène-méthanol 96/4 en volumes). Les fractions sont réunies, séchées sur sulfate de sodium, filtrées et concentrées à 45°C sous pression réduite (2,7 kPa). La meringue obtenue est cristallisée dans 10 cm3 d'isopropanol. Après filtration, lavage et séchage à 40°C (90Pa), on obtient 0,41 g de 2"-(1-pyrazolyl)-pyrimido [4,5-5γ,5δ] pristinamycine I_{E} sous forme de cristaux jaunes fondant à 197°C.

Spectre de RMN ¹H (400 MHz, CDCl₃) : 0,93 (t, J = 7,5 Hz, 3H : CH₃ en 2γ) ; de 1.20 à 1,35 (mt, 2H : 1H du CH₂ en 3β et 1H du CH₂ en 3γ) ; 1,31 (d, J = 7 Hz, 3H : CH₃ en 1γ) ; 1,45 (dd, J = 17 et 6 Hz, 1H : 1H du CH₂ en 5β); de 1,55 à 1,65 (mt : 1H correspondant à l'autre H du CH₂ en 3γ) ; 1,67 et 1,75 (2 mts, 1H chacun : CH₂ en 2β) ; 2,07 (mt, 1H : l'autre H du CH₂ en 3β) ; 2,67 (s, 6H : ArN(CH₃)₂) ; 2,93 (dd. J = 12 et 4,5 Hz, 1H : 1H du CH₂ en 4β) ; 3,10 (d, J = 17 Hz, 1H : l'autre H du CH₂ en 5β) ; de 3,15 à 3,30 (mt, 1H : 1H du CH₂ en 3δ et l'autre H du CH₂ en 4β) ; 3.27 (s, 3H : NCH₃) ; 3,51 (mt, 1H : l'autre H du CH₂ en 3δ) ; 3,84 (d, J = 17 Hz. 1H : 1H du CH₂ en 5ε) ; 4,62 (dd, J = 8 et 6 Hz, 1H : CH en 3α) ; 4,81 (mt, 1H : CH en 2α) ; 4,89 (d large, J = 10 Hz, 1H : CH en 1α) ; 5,07 (dd, J = 12 et 4,5 Hz, 1H : CH en 4α) ; 5,41 (d large, J = 6 Hz, 1H : CH en 5α); 5,48 (d, J = 17 Hz, 1H : l'autre H du CH₂ en 5ε) ; 5.67 (d, J = 8.5 Hz. 1H : CH en 6α) ; 5,89 (q large, J = 7 Hz, 1H : CH en 1β) ; 6,29 (d, J = 8 Hz, 2H : H aromatiques en 4ε) ; 6,48 (d large. J =2 Hz, 1H : H en 4 du pyrazole) ; 6,53 (d, J = 10 Hz, 1H : CONH en 2) ; 6,87 (d, J = 8 Hz, 2H : H aromatiques en 4δ) : de 7.20 à 7,40 (mt : les 5H correspondant aux H aromatiques en 6α): 7.48 (d large. J = 8.5 H₇. 1H : 1' H₄) : 7.53 (dd, J = 8.5 et 4 Hz. 1H : 1' H₅); 7.80 is large, 1H : H en 3 du pyrazole) ; 8.00 (d large. J = 4 Hz. 1H : 1'H₆): 8.38 (d. J = 10 Hz, 1H : CONH en 1) ; 8,38 (s, 1H : CH=N) ; 8,54 (d, J = 2 Hz, 1H : H en 5 du pyrazole) ; 8,71 (d, J = 8,5 Hz, 1H : CONH en 6) ; 11,65 (s, 1H : OH).

### Exemple 50

En opérant comme à l'exemple 22, mais à partir de 40 cm3 de diméthylformamide, de 1,84 g de 5δ-diméthylaminométhylène pristinamycine I_{A}, de 0,60 g de chlorhydrate de S-(2-morpholinoéthyl) isothiouronium, de 1 cm3 de diisopropylamine et chauffage la nuit à 65°C, on obtient, après traitement et concentration à sec du mélange réactionnel à 45°C (2,7 kPa), 1,5 g de solide jaune qui est purifié par deux chromatographies successives avec respectivement 100 g et 200 g de silice (éluant : chlorure de méthylène-méthanol 97/3 en volumes). Les fractions sont réunies, séchées sur sulfate de sodium, filtrées et concentrées à 45°C sous pression réduite (2,7 kPa). Le solide obtenu est repris par 10 cm3 d'éther diisopropylique. Après filtration, lavage et séchage à 40°C (90 Pa), on obtient 0,51 g de 2"-(2-morpholinoéthylthio)-pyrimido [4,5-5γ,5δ] pristinamycine I_{E} sous forme d'un solide blanc cassé fondant à 187°C.

Spectre de R.M.N. ¹H (400 MHz, CDCl₃) : 0,92 (t, J = 7,5 Hz, 3H : CH₃ en 2γ) ; de 1,15 à 1,40 (mt, 3H : 1H du CH₂ en 3β - 1H du CH₂ en 3γ et 1H du CH₂ en 5β) ; 1,31 (d, J = 7 Hz, 3H : CH₃ en 1γ) ; de 1,50 à 1,70 (mt : les 2H correspondant à l'autre H du CH₂ en 3γ et à 1H du CH₂ en 2β) ; 1,75 (mt, 1H : l'autre H du CH₂ en 2β) ; 2,04 (mt, 1H : l'autre H du CH₂ en 3β) ; 2,53 (mf, 4H : les 2 NCH₂ de la morpholine) ; 2,69 (t, J = 7,5 Hz, 2H : NCH₂) de 2,80 à 2,95 (mt, 2H : l'autre H du CH₂ en 5β et 1H du CH₂ en 4β) ; 2,90 (s, 6H : ArN(CH₃)₂) ; de 3,15 à 3,35 (mt, 4H : ArSCH₂ - l'autre H du CH₂ en 4β et 1H du CH₂ en 3δ) ; 3,27 (s, 3H : NCH₃) ; 3,50 (mt, 1H : l'autre H du CH₂ en 3δ) ; de 3,65 à 3,80 (mt, 5H : les 2 OCH₂ de la morpholine et 1H du CH₂ en 5ε) ; 4,60 (dd, J = 8 et 6 Hz, 1H : CH en 3α) ; 4,80 (mt, 1H : CH en 2α) ; 4,89 (d large, J = 10 Hz, 1H : CH en 1α) ; 5,08 (dd, J = 11,5 et 5 Hz, 1H : CH en 4α) ; 5,33 (d large, J = 5,5 Hz, 1H : CH en 5α) ; 5,40 (d, J = 17 Hz, 1H : l'autre H du CH₂ en 5ε) ; 5,65 (d, J = 8,5 Hz, 1H : CH en 6α) ; 5,88 (q large, J = 7 Hz, 1H : CH en 1β) ; 6,35 (d, J = 8 Hz, 2H : H aromatiques en 4ε) ; 6,52 (d, J = 10 Hz, 1H -CONH en 2) ; 6,85 (d, J = 8 Hz, 2H : H aromatiques en 4δ) ; de 7,15 à 7,40 (mt : les 5H correspondant aux H aromatiques en 6α) ; 7,45 (d large, J = 8,5 Hz, 1H : 1' H₄) ; 7,49 (dd, J = 8, 5 et 4 Hz, 1H : 1' H₅) ; 7,92 (d large, J = 4 Hz, 1H : 1' H₆) ; 8,16 (s, 1H : CH=N) ; 8,37 (d, J = 10 Hz, 1H : CONH en 1) ; 8,70 (d, J = 8,5 Hz, 1H : CONH en 6) ; 11,64 (s, 1H : OH).

Le dichlorhydrate de S-(2-morpholinoéthyl) isothiouronium peut être préparé selon DOHERTY Chem. Soc., 79, 5667-70, (1957) ou CLINTON J.Am. Chem. Soc., 70, 950,(1948).

### Exemple 51

En opérant comme à l'exemple 22, mais à partir de 50 cm3 de diméthylformamide, de 2 g de 5δ-diméthylaminométhylène pristinamycine I_{A}, de 0,67 g de chlorhydrate de S-(4-pyridylméthyl) isothiouronium, de 1,5 cm3 de diisopropyléthylamine et chauffage à 65°C pendant 48 heures, on obtient, après traitement et concentration à sec du mélange réactionnel à 45°C (2,7 kPa), un solide qui est chromatographié sur 40 g de silice (éluant : chlorure de méthylène-méthanol 98/2 en volumes) puis par CLHP sur 450 g de silice C₈ 10µm (éluant : eau-acétonitrile 72,5/27,5 en volumes, contenant 0,1 % d'acide trifluoroacétique). Les fractions sont rassemblées, l'acétonitrile éliminé à 40°C sous pression réduite (2,7 kPa) puis le pH de la phase aqueuse ajusté à 7-8 par addition d'eau saturée en bicarbonate de sodium. Le précipité obtenu est filtré, séché à 40°C sous 90Pa, pour donner 0,22 g de 2"-(4-pyridylméthylthio)-pyrimido [4,5-5γ,5δ] pristinamycine I_{E} sous forme d'un solide blanc fondant à 195°C.

Spectre de RMN ¹H (400 MHz, CDCl₃) : 0,91 (t, J = 7,5 Hz, 3H : CH₃ en 2γ) ; de 1,20 à 1,40 (mt, 3H : 1 H du CH₂ en 3β - 1 H du CH₂ en 3γ et 1 H du CH₂ en 5β) ; 1,31 (d, J = 7 Hz, 3H : CH₃ en 1γ) ; de 1,50 à 1,75 (mt : les 2H correspondant à 1 H du CH₂ en 2β et l'autre H du CH₂ en 3γ) ; 1,74 (mt, 1H : l'autre H du CH₂ en 2β) ; 2,05 (mt, 1H : l'autre H du CH₂ en 3β) ; 2,83 (s, 6H : ArN(CH₃)₂) ; de 2,90 à 3,00 (mt, 2H : 1H du CH₂ en 4β et l'autre H du CH₂ en 5β) ; de 3,15 à 3,30 (mt, 2H : 1H du CH₂ en 3δ et l'autre H du CH₂ en 4β) ; 3,26 (s, 3H : NCH₃) ; 3,50 (mt, 1H : l'autre H du CH₂ en 3δ) ; 3,76 (d, J = 17 Hz, 1 H : 1 H du CH₂ en 5ε) ; 4,27 et 4,39 (2 d, J = 15 Hz, 1 H chacun : ArSCH₂Ar) ; 4,61 (dd, J = 7,5 et 5,5 Hz, 1 H : CH en 3α) ; 4,79 (mt, 1 H : CH en 2α) ; 4,87 (d large, J = 10 Hz, 1 H : CH en 1α) ; 5,07 (dd, J = 12 et 4,5 Hz, 1 H : CH en 4α) ; 5,33 (d large, J = 5,5 Hz, 1H : CH en 5α) ; 5,39 (d, J = 17 Hz, 1H : l'autre H du CH₂ en 5ε) ; 5,64 (d, J = 8 Hz, 1H : CH en 6α) ; 5,87 (q large, J = 7 Hz, 1H : CH en 1β) ; 6,33 (d, J = 8 Hz, 2H : H aromatiques en 4ε) ; 6,53 (d, J = 10 Hz, 1H : CONH en 2) ; 6,84 (d, J = 8 Hz, 2H : H aromatiques en 4δ) ; de 7,20 à 7,40 (mt : les 7H correspondant aux H aromatiques en 6α et aux H en β de la pyridine) ; 7,45 (d large, J = 8,5 Hz, 1H : 1' H₄) ; 7,48 (dd, J = 8, 5 et 4 Hz, 1 H : 1'H₅) ; 7,93 (d large, J = 4 Hz, 1H : 1' H₆) ; 8,18 (s, 1H : CH=N) ; 8,36 (d, J = 10 Hz, 1H : CONH en 1) ; 8,52 (d, J = 6 Hz, 2H : H en α de la pyridine) ; 8,72 (d, J = 8 Hz, 1H : CONH en 6) ; 11,63 (s, 1H : OH).

### Exemple 52

Dans un tricol contenant 100 cm3 d'acétonitrile et 5 g de 2"-méthylsulfonyl-pyrimido [4,5-5γ,5δ] pristinamycine I_{E}, on introduit 25 cm3 d'eau, 1,1 g de métapériodate de sodium puis 21 mg de trichlorure de ruthénium et on laisse sous agitation 12 heures. On ajoute à nouveau 0,55 g de periodate de sodium supplémentaire et laisse sous agitation 4 heures. Au mélange réactionnel on ajoute alors 25 cm3 d'eau 1,25 g de thiosulfate de sodium puis 250 cm3 de chlorure de méthylène et 150 cm3 d'eau. La phase organique est décantée, séchée sur sulfate de magnésium, filtrée et concentrée à sec à 40°C sous pression réduite (2,7kPa). Le solide obtenu est délité dans l'éther diéthylique pour donner 3,57 g d'un solide qui est purifié par 2 chromatographies flash respectivement sur 250 g et 70 g de silice (éluant : chlorure de méthylène-méthanol 95/5 puis 97/3 en volumes). Les fractions sont réunies, séchées sur sulfate de magnésium, filtrées et concentrées à 45°C sous pression réduite (2,7 kPa), pour donner, après séchage à 40°C (90Pa), 0,60 g de 2"-méthylsulfonyl-pyrimido [4,5-5γ,5δ] (4ζ-méthylamino) (4ζ-désdiméthylamino) pristinamycine I_{E}.

Spectre de RMN ¹H (400 MHz, CDCl₃) : 0,91 (t, J = 7,5 Hz, 3H : CH₃ en 2γ) ; de 1,20 à 1,40 (mt, 3H : 1H du CH₂ en 3β - 1H du CH₂ en 3γ et 1H du CH₂ en 5β) ; 1,31 (d, J = 7 Hz, 3H : CH₃ en 1γ); de 1,50 à 1,70 (mt : les 2H correspondant à 1H du CH₂ en 2β et l'autre H du CH₂ en 3γ) ; 1,74 (mt, 1H : l'autre H du CH₂ en 2β) ; 2,07 (mt, 1H : l'autre H du CH₂ en 3β) ; 2,82 (s, 3H : ArNCH₃) ; 2,88 (dd, J = 12 et 4 Hz, 1H : 1H du CH₂ en 4β) ; 3,10 (d , J = 18 Hz, 1H : l'autre H du CH₂ en 5β) ; 3,16 (t, J = 12 Hz, 1H : l'autre H du CH₂ en 4β) ; de 3,20 à 3,30 (mt, 1H : 1H du CH₂ en 3δ) ; 3,25 (s, 3H : NCH₃) ; 3,33 (s, 3H : SO₂CH₃) ; 3,50 (mt, 1H : l'autre H du CH₂ en 3δ) ; 3,82 (d, J = 17,5 Hz, 1H : 1H du CH₂ en 5ε) ; 4,12 (mf, 1H : ArNH) ; 4,60 (dd, J = 8 et 5,5 Hz, 1H : CH en 3α) ; 4,80 (mt, 1H : CH en 2α) ; 4,90 (dd, J = 10 et 1 Hz, 1H : CH en 1α) ; 4,97 (dd, J = 12 et 4 Hz, 1H : CH en 4α) ; 5,4 (d large, J = 6,5 Hz, 1H : CH en 5α) ; 5,55 (d, J = 17,5 Hz, 1H : l'autre H du CH₂ en 5ε); 5,65 (d, J = 8,5 Hz, 1H : CH en 6α) ; 5,87 (q dédoublé, J = 7 et 1 Hz, 1H : CH en 1β) ; 6,05 (d, J = 8 Hz, 2H : H aromatiques en 4ε) ; 6,53 (d, J = 10 Hz, 1H : CONH en 2) ; 6,72 (d, J = 8 Hz, 2H : H aromatiques en 4δ) ; de 7,15 à 7,40 (mt : les 5H correspondant aux H aromatiques en 6α) ; 7,49 (dd, J = 8,5 et 1,5 Hz, 1H : 1'H₄) ; 7,56 (dd, J = 8,5 et 4 Hz, 1H : 1' H₅) ; 8,00 (dd, J = 4 et 1,5 Hz, 1H : 1'H₆) ; 8,38 (d, J = 10 Hz, 1H : CONH en 1) ; 8,50 (s, 1H : CH=N) ; 8,73 (d, J = 8,5 Hz, 1H : CONH en 6) ; 11,63 (s, 1H : OH).

La 2"-méthylsulfonyl)-pyrimido [4,5-5γ,5δ] pristinamycine I_{E} peut être obtenu comme décrit à l'exemple 24.

### Exemple 53

Dans un tricol contenant 10 cm3 de diméthylformamide, on ajoute 0,146 ml de 2-diéthylaminoéthanethiol, 47 mg d'hydrure de sodium puis, goutte à goutte, 1 g de sel de potassium de 2 "-méthylsulfonyl-pyrimido [4,5-5γ,5δ] pristinamycine I_{E} dans 10 cm3 de diméthylformamide. On laisse une heure sous agitation à 20°C. Le mélange réactionnel est versé sur 100 cm3 d'eau et on ajoute 10 cm3 d'acide chlorhydrique 0,1N jusqu'à pH 7 puis 40 cm3 de chlorure de méthylène. La phase aqueuse est décantée et extraite par 4 fois 40 cm3 de chlorure de méthylène. Les phases organiques sont rassemblées, séchées sur sulfate de magnésium, filtrées, puis concentrées sous pression réduite. Le solide obtenu est délité dans l'éther pour donner, après filtration, 0,64 g de solide qui est purifié par chromatographie flash (éluant : chlorure de méthylène-méthanol 97/3 en volumes). Les fractions sont réunies, séchées sur sulfate de magnésium, filtrées et concentrées à 45°C sous pression réduite (2,7 kPa), pour donner, après séchage à 40°C (90Pa), 0,23 g de 2"-diéthylaminoéthylthio-pyrimido [4,5-5γ,5δ] pristinamycine I_{E}.

Spectre de RMN ¹H (400 MHz, CDCl₃) : 0,90 (t, J = 7,5 Hz, 3H : CH₃ en 2γ) ; 1,06 (mf, 6H : les 2 CH₃ de la diéthylamine) ; de 1,20 à 1,35 (mt, 3H : 1H du CH₂ en 3β - 1H du CH₂ en 3γ et 1H du CH₂ en 5β) ; 1,30 (d, J = 7 Hz, 3H : CH₃ en 1γ) ; de 1,50 à 1,65 (mt : les 1H correspondant à l'autre H du CH₂ en 3γ) ; 1,65 et 1,74 (2 mts, 1H chacun : CH₂ en 2β) ; 2,05 (mt, 1H : l'autre H du CH₂ en 3β) ; 2,58 (mf, 4H : les 2 NCH₂ de la diéthylamine) ; 2,79 (mt, 2H : NCH₂) ; de 2,85 à 3,00 (mt, 2H : 1H du CH₂ en 4β et l'autre H du CH₂ en 5β) ; 2,88 (s, 6H : ArN(CH₃)₂) ; de 3,10 à 3,30 (mt, 4H : l'autre H du CH₂ en 4β - 1H du CH₂ en 3δ et ArSCH₂) ; 3,26 (s, 3H : NCH₃) ; 3,50 (mt, 1H : l'autre H du CH₂ en 3δ) ; 3,76 (d, J = 17,5 Hz, 1H : 1H du CH₂ en 5ε) ; 4,60 (dd, J = 7,5 et 5,5 Hz, 1H : CH en 3α) ; 4,80 (mt, 1H : CH en 2α) ; 4,89 (dd, J = 10 et 1Hz, 1H : CH en 1α) ; 5,07 (dd, J = 12 et 4,5 Hz, 1H : CH en 4α) ; 5,33 (d large, J = 5,5 Hz, 1H : CH en 5α) ; 5,38 (d, J = 17,5 Hz, 1H : l'autre H du CH₂ en 5ε); 5,65 (d, J = 8 Hz, 1H : CH en 6α) ; 5,88 (q dédoublé, J = 7 et 1 Hz, 1H : CH en 1β) ; 6,34 (d, J = 8 Hz, 2H : H aromatiques en 4ε) ; 6,53 (d, J = 10 Hz, 1H : CONH en 2) ; 6,85 (d, J = 8 Hz, 2H : H aromatiques en 4δ) ; de 7,20 à 7,40 (mt : les 5H correspondant aux H aromatiques en 6α) ; 7,46 (dd, J = 8,5 et 1,5 Hz, 1H : 1' H₄) ; 7,49 (dd, J = 8,5 et 4 Hz, 1H : 1' H₅) ; 7,92 (dd, J = 4 et 1,5 Hz, 1H : 1' H₆) ; 8,15 (s, 1H : CH=N) ; 8,38 (d, J = 10 Hz, 1H : CONH en 1) ; 8,68 (d, J = 8 Hz, 1H : CONH en 6) ; 11,64 (mf, 1H : OH).

Le sel de potassium de la 2"-méthylsulfonyl-pyrimido [4,5-5γ,5δ] pristinamycine I_{E} peut être préparé de la façon suivante :

Dans un ballon placé sous argon contenant 150 cm3 d'acétone et 10 g de 2"-méthylsulfonyl-pyrimido [4,5-5γ,5δ] pristinamycine I_{E}, on ajoute 1,4 g de bicarbonate de potassium et on laisse agiter la nuit. La précipité crème est filtré, lavé plusieurs fois à l'acétone et à l'éther diéthylique puis filtré, séché sous pression réduite, pour donner 7,4 g de sel de potassium du 2"-méthylsulfonyl-pyrimido [4,5-5γ,5δ] pristinamycine I_{E} utilisé tel quel.

La 2"-méthylsulfonyl-pyrimido [4,5-5γ,5δ] pristinamycine I_{E} peut être obtenu comme décrit à l'exemple 24.

### Exemple 54

Dans un autoclave contenant 37 cm3 de diméthylformamide, on ajoute 3,7 g de sel de potassium de 2"-méthylsulfonyl-pyrimido [4,5-5γ,5δ] pristinamycine I_{E} et 2,2 cm3 d'une solution de méthylamine 8M dans l'éthanol et on chauffe 8 heures à 80°C. Le mélange réactionnel est concentré à sec à 50°C, sous pression réduite 2,7 kPa), pour donner 4,1 g d'un résidu orange qui est repris par 20 cm3 d'eau et 15 cm3 d'acétate d'éthyle. La phase organique est décantée, lavée par 2 fois 10 cm3 d'eau, séchée sur sulfate de magnésium, filtrée et concentrée à sec à 35°C sous pression réduite (2,7 kPa). On obtient ainsi 0,4 g d'un solide beige qui est purifié par chromatographie flash sur 40 g de silice (éluant : chlorure de méthylène-méthanol 96/4 en volumes). Les fractions sont réunies, séchées sur sulfate de magnésium, filtrées et concentrées à 45°C sous pression réduite (2,7 kPa), pour donner 0,16 g d'un solide qui est cristallisé dans 3,2 cm3 d'un mélange acétonitrile-eau (50/50 en volumes). Après filtration puis séchage à 40°C (90Pa), on obtient 0,13 g de 2"-méthylamino-pyrimido [4,5-5γ,5δ] pristinamycine I_{E} sous forme de cristaux beige fondant à 210-220°C.

Spectre de RMN ¹H (400 MHz, CDCl₃) : 0,92 (t, J = 7,5 Hz, 3H : CH₃ en 2γ) ; de 1,15 à 1,35 (mt, 3H : 1H du CH₂ en 3β - 1H du CH₂ en 3γ et 1H du CH₂ en 5β) ; 1,30 (d, J = 7 Hz, 3H : CH₃ en 1γ) ; de 1,50 à 1,70 (mt : les 2H correspondant à l'autre H du CH₂ en 3γ et à 1H du CH₂ en 2β) ; 1,74 (mt, 1H : l'autre H du CH₂ en 2β) ; 2,04 (mt, 1H : l'autre H du CH₂ en 3β) ; de 2,80 à 3,00 (mt, 2H : 1H du CH₂ en 4β et l'autre H du CH₂ en 5β) ; 2,88 (s, 6H : ArN(CH₃)₂) ; 2,97 (d, J = 5 Hz, 3H : ArNCH₃) ; de 3,15 à 3,30 (mt, 2H : l'autre H du CH₂ en 4β et 1H du CH₂ en 3δ) ; 3,25 (s, 3H : NCH₃) ; 3,48 (mt, 1H : l'autre H du CH₂ en 3δ) ; 3,72 (d, J = 17 Hz, 1H : 1H du CH₂ en 5ε) ; 4,60 (dd, J = 8 et 7 Hz, 1H : CH en 3α) ; 4,80 (mt, 1H : CH en 2α) ; de 4,85 à 4,95 (mt, 2H : CH en 1α et ArNH) ; 5,10 (dd, J = 10,5 et 4 Hz, 1H : CH en 4α) ; 5,30 (mt, 1H : CH en 5α) ; 5,31 (d, J = 17 Hz, 1H : l'autre H du CH₂ en 5ε) ; 5,64 (d, J = 8 Hz, 1H : CH en 6α) ; 5,88 (q large, J = 7 Hz, 1H : CH en 1β) ; 6,40 (d, J = 8 Hz, 2H : H aromatiques en 4ε) ; 6,54 (d, J = 10 Hz, 1H : CONH en 2) ; 6,87 (d, J = 8 Hz, 2H : H aromatiques en 4δ) ; de 7,15 à 7,40 (mt : les 5H correspondant aux H aromatiques en 6α) ; 7,44 (AB limite, 2H : 1' H₄ et 1' H₅) 7,91 (d large, J = 4 Hz, 1H : 1' H₆) ; 7,97 (s, 1H : CH=N) ; 8,38 (d, J = 10 Hz, 1 H : CONH en 1) ; 8,64 (d, J = 8 Hz, 1H : CONH en 6) ; 11,65 (s, 1H : OH).

Le sel de potassium du 2"-méthylsulfonyl-pyrimido [4,5-5γ,5δ] pristinamycine I_{E} peut est préparé selon l'exemple 53.

### Exemple 55

En opérant par analogie avec l'exemple 32 mais à partir de 20 cm3 de chlorure de méthylène, de 2,3 g de 2"-(2-pyridyl)-pyrimido [4,5-5γ,5δ] pristinamycine I_{E} de 0,2 g d'éthylène glycol, de 2,35 g d'acide acétique, de 0,48 g de periodate de tétra-n-butylammonium et agitation 12 heures, on obtient 3,4 g d'un produit brut qui est dissous dans 70 cm3 d'acide sulfurique 0,5N. On extrait par 3 fois 50 cm3 d'acétate d'éthyle. Après traitement et concentration, on obtient 1,58 g de solide jaune qui est purifié par deux chromatographies successives, respectivement sur 100 g et 30 g de silice (éluant : chlorure de méthylène-méthanol 95/5 puis chlorure de méthylène-acétonitrile-méthanol : 86/8/6 en volumes). Les fractions sont réunies, séchées sur sulfate de magnésium, filtrées puis concentrées à 45°C sous pression réduite (2,7 kPa). Le solide obtenu est repris par 10 cm3 d'éther diisopropylique, filtré, lavé par 10 cm3 d'éther diisopropylique puis séché à 40°C, sous pression réduite (90 Pa), pour donner 0,52 g de 2"-(2-pyridyl)-pyrimido [4,5-5γ,5δ] (4ζ-méthylamino) (4ζ-désdiméthylamino) pristinamycine I_{E} sous forme d'un solide jaune pâle et fondant à 209°C.

Spectre de RMN ¹H (400 MHz, CDCl₃) : 0,93 (t, J = 7,5 Hz, 3H : CH₃ en 2γ) ; de 1,25 à 1,40 (mt, 2H : 1H du CH₂ en 3β et 1H du CH₂ en 3γ) ; 1,32 (d, J = 7 Hz, 3H : CH₃ en 1γ) ; 1,52 (dd, J = 18 et 6 Hz, 1H : 1H du CH₂ en 5β) ; 1,62 (mt, 1H : l'autre H du CH₂ en 3γ) ; de 1,60 à 1,85 (mt : les 2H correspondant au CH₂ en 2β) ; 2,08 (mt, 1H : l'autre H du CH₂ en 3β) ; 2,58 (s, 3H : ArNCH₃) ; 2,92 (dd, J = 12 et 4,5 Hz, 1H : 1H du CH₂ en 4β) ; de 3,15 à 3,30 (mt, 3H : l'autre H du CH₂ en 5β - 1H du CH₂ en 3δ et l'autre H du CH₂ en 4β) ; 3,27 (s, 3H : NCH₃) ; 3,51 (mt, 1H : l'autre H du CH₂ en 3δ) ; 3,89 (d, J = 17 Hz, 1H : 1H du CH₂ en 5ε) ; 4,63 (dd, J = 8 et 6 Hz, 1H : CH en 3α) ; 4,82 (mt, 1H : CH en 2α) ; 4,90 (dd, J = 10 et 1 Hz, 1H : CH en 1α) ; 5,10 (dd, J = 11 et 4,5 Hz, 1H : CH en 4α) ; 5,42 (d large, J = 6 Hz, 1H : CH en 5α) ; 5,53 (d, J = 17 Hz, 1H : l'autre H du CH₂ en 5ε) ; 5,69 (d, J = 8,5 Hz, 1H : CH en 6α) ; 5,89 (q dédoublé, J = 7 et 1 Hz, 1H : CH en 1β) ; 6,16 (d, J = 8 Hz, 2H : H aromatiques en 4ε) ; 6,56 (d, J = 10 Hz, 1H : CONH en 2) ; 6,79 (d, J = 8 Hz, 2H : H aromatiques en 4δ) ; de 7,20 à 7,40 (mt : les 5H correspondant aux H aromatiques en 6α) ; 7,40 (dd large, J = 8 et 5 Hz, 1 H : H en 5 de la pyridine) ; 7,48 (dd, J = 8,5 et 1 Hz, 1H : 1' H₄) ; 7,53 (dd, J = 8,5 et 4 Hz, 1H : 1' H₅) ; 7,85 (t dédoublé, J = 8 et 2 Hz, 1H : H en 4 de la pyridine) ; 8,01 (dd, J = 4 et 1 Hz, 1H : 1' H₆) ; 8,43 (d, J = 10 Hz, 1H : CONH en 1) ; 8,46 (d large, J = 8 Hz, H en 3 de la pyridine) ; 8,56 (s, 1H : CH=N) ; 8,71 (d, J = 8,5 Hz, 1H : CONH en 6) ; 8,84 (d large, J = 5 Hz, 1H : H en 6 de la pyridine) ; 11,64 (s, 1H : OH).

La 2"-(2-pyridyl)-pyrimido [4,5-5γ,5δ] pristinamycine I_{E} peut être obtenu comme décrit à l'exemple 28.

### Exemple 56

Dans un tricol contenant 70 cm3 de tétrahydrofurane et 100 cm3 d'acide chlorhydrique 0,1N, on ajoute 3,9 g de 2"-azido-pyrimido [4,5-5γ,5δ] pristinamycine I_{E} et 2,16 g de triphénylphosphine et on laisse sous agitation la nuit. La mélange réactionnel est concentré à sec à 40°C sous pression réduite (2,7 kPa); le résidu gommeux est repris par 50 cm3 d'eau et 100 cm3 d'acide chlorhydrique 0,1N et on extrait par 3 fois 80 cm3 de chlorure de méthylène. Après décantation, la phase aqueuse est neutralisée par addition d'eau saturée en bicarbonate de sodium et on extrait par 3 fois 100 cm3 de chlorure de méthylène. Les phases organiques sont réunies, séchées sur sulfate de magnésium, filtrées et concentrées à 45°C sous pression réduite, pour donner 3,5 g d'un solide jaune qui est purifié par chromatographie sur 300 g de silice (éluant : chlorure de méthylène-méthanol : 96/4 en volumes). Les fractions sont réunies, séchées sur sulfate de magnésium, filtrées et concentrées à 45°C, sous pression réduite (2,7 kPa), pour donner un solide jaune qui est recristallisé dans 40 cm3 d'isopropanol. Après filtration, lavage par 10 cm3 d'isopropanol et séchage à 40°C sous pression réduite, on obtient 0,97 g de 2"-amino-pyrimido [4,5-5γ,5δ] pristinamycine I_{E} sous forme d'une poudre jaune pâle fondant à 214°C.

La 2"-azido-pyrimido [4,5-5γ,5δ] pristinamycine I_{E} peut être préparée comme à l'exemple 25 mais à partir de 250 cm3 de diméthylformamide, de 10 g de 2"-(4-méthyl-benzènesulfonyl)-pyrimido [4,5-5γ,5δ] pristinamycine I_{E} de 2,42 g d'azoture de sodium et chauffage à 65°C pendant trois jours. Après concentration à sec du mélange réactionnel, on ajoute 400 cm3 d'eau saturée en chlorure de sodium. Le précipité jaune orangé apparu est filtré puis repris par 200 cm3 de chlorure de méthylène. Après décantation, séchage sur sulfate de magnésium, filtration et concentration à sec à 40°C sous pression réduite (2,7 kPa), on obtient un solide qui est purifié par chromatographie sur 150 g de silice (éluant : chlorure de méthylène-méthanol 96/4 en volumes) pour donner, après concentrationdes fractions 3,9 g de 2"-azido-pyrimido [4,5-5γ,5δ] pristinamycine I_{E} sous forme d'un solide orangé utilisé comme tel.

Spectre de RMN ¹H (400 MHz, CDCl₃) : 0,92 (t, J = 7,5 Hz, 3H : CH₃ en 2γ) ; de 1,15 à 1,35 (mt, 3H : 1H du CH₂ en 3β - 1H du CH₂ en 3γ et 1H du CH₂ en 5β) ; 1,30 (d, J = 7 Hz, 3H : CH₃ en 1γ) ; 1,56 (mt, 1H : l'autre H du CH₂ en 3γ) ; de 1,60 à 1,80 (mt : les 2H correspondant au CH₂ en 2β) ; 2,04 (mt, 1H : l'autre H du CH₂ en 3β) ; 2,81 (d, J = 17,5 Hz, 1H : l'autre H du CH₂ en 5β) ; de 2,85 à 2,95 (mt, 1 H : 1H du CH₂ en 4β) ; 2,89 (s, 6H : ArN(CH₃)₂) ; de 3,15 à 3,30 (mt, 2H : l'autre H du CH₂ en 4β et 1H du CH₂ en 3δ) ; 3,25 (s, 3H : NCH₃) ; 3,49 (mt, 1H : l'autre H du CH₂ en 3δ) ; 3,71 (d, J = 17Hz, 1H : 1H du CH₂ en 5ε) ; 4,60 (dd, J = 8 et 6 Hz, 1H : CH en 3α) ; 4,80 (mt, 1H : CH en 2α) ; 4,86 (s, 2H : ArNH₂) ; 4,88 (d large, J = 10 Hz, 1H : CH en 1α) ; 5,08 (dd, J = 11,5 et 5 Hz, 1H : CH en 4α) ; 5,31 (mt, 1H : CH en 5α) ; 5,33 (d, J = 17 Hz, 1H : l'autre H du CH₂ en 5ε) ; 5,64 (d, J = 8,5 Hz, 1 H : CH en 6α) ; 5,88 (q large, J = 7 Hz, 1 H : CH en 1β) ; 6,40 (d, J = 8 Hz, 2H : H aromatiques en 4ε) ; 6,54 (d, J = 10 Hz, 1 H : CONH en 2) ; 6,86 (d, J = 8 Hz, 2H : H aromatiques en 4δ) ; de 7,20 à 7,35 (mt : les 5H correspondant aux H aromatiques en 6α) ; 7,42 (dd, J = 8 et 1,5 Hz, 1H : 1' H₄) ; 7,45 (dd, J = 8 et 4 Hz, 1H : 1' H₅) ; 7,89 (dd, J = 4 et 1,5 Hz, 1 H : 1' H₆) ; 7,97 (s, 1 H : CH=N) ; 8,36 (d, J = 10 Hz, 1 H : CONH en 1) ; 8,64 (d, J = 8,5 Hz, 1 H : CONH en 6) ; 11,65 (s, 1 H : OH).

La 2"-(4-méthyl-benzènesulfonyl)-pyrimido [4,5-5γ,5δ] pristinamycine I_{E} peut être obtenu comme décrit à l'exemple 25.

### Exemple 57

Dans un tricol placé sous courant d'azote et contenant 10 cm3 d'acétonitrile, on ajoute 1 g de 2"-hydroxyméthyl-pyrido [2,3-5γ,5δ] pristinamycine I_{E} et 0,155 cm3 de chlorure de thionyle. On laisse sous agitation 30 minutes et on ajoute 0,9 cm3 de triéthylamine. Après avoir filtré le chlorhydrate de triéthylamine formé, on ajoute une solution du sel de sodium du 2-diéthylaminoéthanethiol (obtenue après 30 minutes d'agitation à partir de 0,324 cm3 de diéthylaminoéthanethiol et de 102 mg d'hydrure de sodium dans 20 cm3 d'acétonitrile). Après chauffage à 50°C pendant 3 heures, l'insoluble est éliminé par filtration puis lavé par 20 cm3 d'acétonitrile. Le filtrat est concentré à sec sous pression réduite (45°C-2,7 kPa) puis le résidu est repris par 50 cm3 de chlorure de méthylène et 50 cm3 d'eau. La phase organique est décantée, lavée par 25 cm3 d'eau, séchée sur sulfate de sodium puis filtrée pour donner, après concentration à sec, 1,1 g d'un résidu qui est chromatographié sur 50 g de silice (éluant: gradiant chlorure de méthylène-méthanol 98/2 à 90/10 en volumes) pour donner 150 mg de produit qui est purifié par CLHP sur 450 g de silice C₈ 10µm (éluant : eau-acétonitrile 70/30 en volumes, contenant 0,1 % d'acide trifluoroacétique). Les fractions sont rassemblées puis l'acétonitrile éliminé à 40°C sous pression réduite (2,7 kPa). La phase aqueuse est amenée à pH 7-8 par addition d'eau saturée en bicarbonate de sodium puis extraite par 2 fois 25 cm3 de chlorure de méthylène. La phase organique est séchée sur sulfate de sodium, filtrée puis concentrée à sec, pour donner 30 mg de 2"-diéthylaminoéthylthiométhyl-pyrido [2,3-5γ,5δ] pristinamycine I_{E} sous forme d'un solide jaune fondant à 132°C.

Spectre de RMN ¹H (400 MHz, CDCl₃) : 0,92 (t, J = 7,5 Hz, 3H : CH₃ en 2γ) ; 1,02 (t, J = 7 Hz, 6H : CH₃ du diéthylamino) ; de 1,20 à 1,35 (mt, 2H : 1H du CH₂ en 3β et 1H du CH₂ en 3γ) ; 1,29 (d, J = 7 Hz, 3H : CH₃ en 1γ) ; 1,57 (mt, 1H : l'autre H du CH₂ en 3γ) ; de 1,60 à 1,80 (mt : les 2H correspondant au CH₂ en 2β) ; 1,88 (d très large, J = 16,5 Hz, 1H : 1H du CH₂ en 5β) ; 2,03 (mt, 1H : l'autre H du CH₂ en 3β) ; de 2,45 à 2,65 (mf, 4H : NCH₂ du diéthylamino) ; de 2,60 à 2,75 (mt, 4H : SCH₂CH₂N) ; 2,84 (s, 6H : ArN(CH₃)₂) ; 2,98 (dd, J = 13,5 et 6 Hz, 1H : 1H du CH₂ en 4β) ; de 3,10 à 3,30 (mt, 3H : l'autre H du CH₂ en 4β - l'autre H du CH₂ en 5β et 1H du CH₂ en 3δ) ; 3,20 (s, 3H : NCH₃) ; 3,49 (mt, 1H : l'autre H du CH₂ en 3δ) ; 3,79 (s, 2H : ArCH₂S) ; 3,94 (d, J = 17 Hz, 1H : 1H du CH₂ en 5ε) ; 4,60 (dd, J = 8 et 5,5 Hz, 1H : CH en 3α) ; 4,79 (mt, 1H : CH en 2α) ; 4,87 (dd, J = 10 et 1 Hz, 1H : CH en 1α) ; 5,28 (dd, J = 9 et 6 Hz, 1H : CH en 4α) ; 5,44 (d large, J = 5,5 Hz, 1H : CH en 5α) ; 5,44 (d, J = 17 Hz, 1H : l'autre H du CH₂ en 5ε) ; 5,60 (d, J = 8 Hz, 1H : CH en 6α) ; 5,87 (q dédoublé, J = 7 et 1 Hz, 1H : CH en 1β) ; 6,36 (d, J = 8 Hz, 2H : H aromatiques en 4ε) ; 6,57 (d, J = 10 Hz, 1H : CONH en 2) ; 6,84 (d, J = 8 Hz, 2H : H aromatiques en 4δ) ; 7,20 (d, J = 8 Hz, 1H : H aromatique en β de l'N) ; de 7,20 à 7,40 (mt : les 8H correspondant aux 5H aromatiques en 6α - à l'H aromatique en γ de 1'N - au 1' H₄ et au 1' H₅) ; 7,83 (dd, J = 4 et 1 Hz, 1H : 1' H₆) ; 8,40 (d, J = 10 Hz, 1H : CONH en 1) ; 8,67 (d, J = 8 Hz, 1H : CONH en 6) ; 11,65 (mf étalé, 1H : OH).

La 2"-hydroxyméthyl-pyrido [2,3-5γ,5δ] pristinamycine I_{E} peut être obtenue comme décrit à l'exemple 11.

### Exemple 58

- 4ε-chloro 2"-tert-butyl-pyrido [2,3-5γ,5δ] pristinamycine IE
- 2"-tert-butyl-pyrido [2,3-5γ,5δ] (4-ζ-méthylamino) (4-ζ-désdiméthylamino) pristinamycine IE
- 4ε-chloro 2"-tert-butyl-pyrido [2,3-5γ,5δ] (4-ζ-méthylamino) (4-ζ-désdiméthylamino) pristinamycine IE
- 4ε-chloro 2"-amino-pyrido [2,3-5γ,5δ] pristinamycine IE
- 2"- amino -pyrido [2,3-5γ,5δ] (4-ζ-méthylamino) (4-ζ-désdiméthylamino) pristinamycine IE
- 4ε-chloro 2"- amino -pyrido [2,3-5γ,5δ] (4-ζ-méthylamino) (4-ζ-désdiméthylamino) pristinamycine IE
- 4ε-chloro 3"-méthoxycarbonyl-2 "-méthyl-pyrido [2,3-5γ,5δ] pristinamycine IE
- 4ε-chloro 2"-phényl-pyrido [2,3-5γ,5δ] pristinamycine IE
- 2"- phényl -pyrido [2,3-5γ,5δ] (4-ζ-méthylamino) (4-ζ-désdiméthylamino) pristinamycine IE
- 4ε-chloro 2"- phényl -pyrido [2,3-5γ,5δ] (4-ζ-méthylamino) (4-ζ-désdiméthylamino) pristinamycine IE
- 4ε-chloro 2"-(4-aminophényl)-pyrido [2,3-5γ,5δ] pristinamycine IE
- 2"-(4-aminophényl)-pyrido [2,3-5γ,5δ] (4-ζ-méthylamino) (4-ζ-désdiméthylamino) pristinamycine IE
- 4ε-chloro 2"-(4-aminophényl)-pyrido [2,3-5γ,5δ] (4-ζ-méthylamino) (4-ζ-désdiméthylamino) pristinamycine IE
- 4ε-chloro 2"-(4-diéthylaminophényl)-pyrido [2,3-5γ,5δ] pristinamycine iE
- 2 "-(4-diéthylaminophényl)-pyrido [2,3-5γ,5δ] (4-ζ-méthylamino) (4-ζ-désdiméthylamino) pristinamycine IE
- 4ε-chloro 2"-(4-diéthylaminophényl)-pyrido [2,3-5γ,5δ] (4-Ç-méthylamino) (4-ζ-désdiméthylamino) pristinamycine IE
- 4ε-chloro -pyrido [2,3-5γ,5δ] pristinamycine IE
- pyrido [2,3-5γ,5δ] (4-ζ-méthylamino) (4-ζ-désdiméthylamino) pristinamycine IE
- 4ε-chloro pyrido [2,3-5γ,5δ] (4-ζ-méthylamino) (4-ζ-désdiméthylamino) pristinamycine IE
- 4ε-chloro 2"-chlorométhyl-pyrido [2,3-5γ,5δ] pristinamycine IE
- 2"-chlorométhyl-pyrido [2,3-5γ,5δ] (4-ζ-méthylamino) (4-ζ-désdiméthylamino) pristinamycine IE
- 4ε-chloro 2"-chlorométhyl-pyrido [2,3-5γ,5δ] (4-ζ-méthylamino) (4-ζ-désdiméthylamino) pristinamycine IE
- 4ε-chloro 3"-méthoxycarbonyl-2"- méthyl -pyrido [2,3-5γ,5δ] (4-ζ-méthylamino) (4-ζ-désdiméthylamino) pristinamycine IE
- 4ε-chloro 2"-(2-pyridyl)-pyrido [2,3-5γ,5δ] pristinamycine IE
- 4ε-chloro 2"-morpholinométhyl-pyrido [2,3-5γ,5δ] pristinamycine IE
- 2"-morpholinométhyl-pyrido [2,3-5γ,5δ] (4-ζ-méthylamino) (4-ζ-désdiméthylamino) pristinamycine IE
- 4ε-chloro 2"-morpholinométhyl-pyrido [2,3-5γ,5δ] (4-ζ-méthylamino) (4-ζ-désdiméthylamino) pristinamycine IE
- 4ε-chloro 2"-(3-pyridyl)-pyrido [2,3-5γ,5δ] pristinamycine IE
- 2" -(3-pyridyl)-pyrido [2,3-5γ,5δ] (4-ζ-méthylamino) (4-ζ-désdiméthylamino) pristinamycine IE
- 4ε-chloro 2"-(3-pyridyl)-pyrido [2,3-5γ,5δ] (4-ζ-méthylamino) (4-ζ-désdiméthylamino) pristinamycine IE
- 4ε-chloro 2''-(4-méthyl-1-pipérazinylméthyl)-pyrido [2,3-5γ,5δ] pristinamycine IE
- 2"-(4-méthyl-1-pipérazinylméthyl)-pyrido [2,3-5γ,5δ] (4-ξ-méthylamino) (4-ξ-désdiméthylamino) pristinamycine IE
- 4ε-chloro 2"-(4-méthyl-1-pipérazinylméthyl)-pyrido [2,3-5γ,5δ] (4-ξ-méthylamino) (4-ξ-désdiméthylamino) pristinamycine IE
- 4ε-chloro 2"-méthyl-pyrido [2,3-5γ,5δ] (4-ξ-méthylamino) (4-ξ-désdiméthylamino) pristinamycine IE
- 4ε-chloro 2''-cyclopropyl-pyrido [2,3-5γ,5δ] pristinamycine IE
- 2"-cyclopropyl-pyrido [2,3-5γ,5δ] (4-ξ-méthylamino) (4-ξ-désdiméthylamino) pristinamycine IE
- 4ε-chloro 2"-cyclopropyl-pyrido [2,3-5γ,5δ] (4-ξ-méthylamino) (4-ξ-désdiméthylamino) pristinamycine IE
- 4ε-chloro 2"-hydroxyméthyl-pyrido [2,3-5γ,5δ] pristinamycine IE
- 2"-hydroxyméthyl-pyrido [2,3-5γ,5δ] (4-ξ-méthylamino) (4-ξ-désdiméthylamino) pristinamycine IE
- 4ε-chloro 2"-hydroxyméthyl-pyrido [2,3-5γ,5δ] (4-ξ-méthylamino) (4-ξ-désdiméthylamino) pristinamycine IE
- 4ε-chloro 2"-propyl-pyrido [2,3-5γ,5δ] pristinamycine IE
- 2"-propyl-pyrido [2,3-5γ,5δ] (4-ξ-méthylamino) (4-ξ-désdiméthylamino) pristinamycine IE
- 4ε-chloro 2"-propyl-pyrido [2,3-5γ,5δ] (4-ζ-méthylamino) (4-ζ-désdiméthylamino) pristinamycine IE
- 4ε-chloro 2"-isopropyl-pyrido [2,3-5γ,5δ] pristinamycine IE
- 2"-isopropyl-pyrido [2,3-5γ,5δ] (4-ζ-méthylamino) (4-ζ-désdiméthylamino) pristinamycine IE
- 4ε-chloro 2"-isopropyl-pyrido [2,3-5γ,5δ] (4-Ç-méthylamino) (4-ζ-désdiméthylamino) pristinamycine IE
- 4ε-chloro 2"-acétoxyméthyl-pyrido [2,3-5γ,5δ] pristinamycine IE
- 2"-acétoxymétyl-pyrido [2,3-5γ,5δ] (4-ζ-méthylamino) (4-ζ-désdiméthylamino) pristinamycine IE
- 4ε-chloro 2"-acétoxyméthyl-pyrido [2,3-5γ,5δ] (4-ζ-méthylamino) (4-ζ-désdiméthylamino) pristinamycine IE
- 4ε-chloro 2"-cyclopropylaminométhyl-pyrido [2,3-5γ,5δ] pristinamycine IE
- 2"-cyclopropylaminométyl-pyrido [2,3-5γ,5δ] (4-ζ-méthylamino) (4-ζ-désdiméthylamino) pristinamycine IE
- 4ε-chloro 2",3"-diméthyl-pyrido [2,3-5γ,5δ] (4-ζ-méthylamino) (4-ζ-désdiméthylamino) pristinamycine IE
- 4ε-chloro 2"-éthoxycarbonyl-pyrido [2,3-5γ,5δ] pristinamycine IE
- 2"-éthoxycarbonyl-pyrido [2,3-5γ,5δ] (4-ζ-méthylamino) (4-ζ-désdiméthylamino) pristinamycine IE
- 4ε-chloro 2"-éthoxycarbonyl-pyrido [2,3-5γ,5δ] (4-Ç-méthylamino) (4-ζ-désdiméthylamino) pristinamycine IE
- PIA Cl: 4ε-chloro 2"-(N-diéthylaminométhyl)-pyrido [2,3-5γ,5δ] pristinamycine IE
- PIB : 2 "-(N-diéthylaminométyl)-pyrido [2,3-5γ,5δ] (4-ζ-méthylamino) (4-ζ-désdiméthylamino) pristinamycine IE
- PIB Cl: 4ε-chloro 2"-(N-diéthylaminométhyl)-pyrido [2,3-5γ,5δ] (4-ζ-méthylamino) (4-ζ-désdiméthylamino) pristinamycine IE
- PIA CI: 4ε-chloro 2"-carbamoyl-pyrido [2,3-5γ,5δ] pristinamycine IE
- PIB : 2"-carbamoyl-pyrido [2,3-5γ,5δ] (4-ζ-méthylamino) (4-ζ-désdiméthylamino) pristinamycine IE
- PIB Cl: 4ε-chloro 2"-carbamoyl-pyrido [2,3-5γ,5δ] (4-ζ-méthylamino) (4-ζ-désdiméthylamino) pristinamycine IE
- PIA CI: 4ε-chloro 2"-diéthylaminoéthylthiométhyl-pyrido [2,3-5γ,5δ] pristinamycine IE
- PIB : 2"-diéthylaminoéthylthiométhyl-pyrido [2,3-5γ,5δ] (4-ζ-méthylamino) (4-ζ-désdiméthylamino) pristinamycine IE
- PIB Cl: 4ε-chloro 2"-diéthylaminoéthylthiométhyl-pyrido [2,3-5γ,5δ] (4-ζ-méthylamino) (4-ζ-désdiméthylamino) pristinamycine IE
- PIA Cl: 4ε-chloro 2"- (morpholinoéthylthiométhyl)-pyrido [2,3-5γ,5δ] pristinamycine IE
- PIB : 2"-(morpholinoéthylthiométhyl-pyrido [2,3-5γ,5δ] (4-ζ-méthylamino) (4-ζ-désdiméthylamino) pristinamycine IE
- 4ε-chloro 2"-(morpholinoéthylthiométhyl-pyrido [2,3-5γ,5δ] (4-ζ-méthylamino) (4-ζ-désdiméthylamino) pristinamycine IE
- 4ε-chloro 2"- (1-pyrrolidinoéthylthiométhyl)-pyrido [2,3-5γ,5δ] pristinamycine IE
- 2"-(1-pyrrolidinoéthylthiométhyl-pyrido [2,3-5γ,5δ] (4-ζ-méthylamino) (4-ζ-désdiméthylamino) pristinamycine IE
- 4ε-chloro 2"-(1-pyrrolidinoéthylthiométhyl)-pyrido [2,3-5γ,5δ] (4-ζ-méthylamino) (4-ζ-désdiméthylamino) pristinamycine IE
- 4ε-chloro 2"- (pipéridinoéthylthiométhyl)-pyrido [2,3-5γ,5δ] pristinamycine IE
- 2"-(pipéridinoéthylthiométhyl-pyrido [2,3-5γ,5δ] (4-ζ-méthylamino) (4-ζ-désdiméthylamino) pristinamycine IE
- 4ε-chloro 2"-(pipéridinoéthylthiométhyl)-pyrido [2,3-5γ,5δ] (4-ζ-méthylamino) (4-ζ-désdiméthylamino) pristinamycine IE
- 4ε-bromo 2"-tert-butyl-pyrido [2,3-5γ,5δ] pristinamycine IE
- 4ε-bromo 2"-tert-butyl-pyrido [2,3-5γ,5δ] (4ζ-méthylamino) (4ζ-désdiméthylamino) pristinamycine IE
- 4ε-allyl 2"-tert-butyl-pyrido [2,3-5γ,5δ] pristinamycine IE
- 4ε-allyl 2"-tert-butyl-pyrido [2,3-5γ,5δ] (4ζ-méthylamino) (4ζ -désdiméthylamino) pristinamycine IE
- 4ε-(2-méthyl prop-ène 1-yl) 2"-tert-butyl-pyrido [2,3-5γ,5δ] pristinamycine IE
- 4ε-(2-méthyl prop-ène 1-yl) 2"-tert-butyl-pyrido [2,3-5γ,5δ] (4ζ-méthylamino) (4ζ-désdiméthylamino) pristinamycine IE
- 2"-tert-butyl-pyrido [2,3-5γ,5δ] (4ζ-diéthylamino) (4ζ-désdiméthylamino) pristinamycine IE
- 2"-tert-butyl-pyrido [2,3-5γ,5δ] (4ζ-N-méthyl, N-allylamino) (4ζ-désdiméthylamino) pristinamycine IE
- 2"-tert-butyl-pyrido [2,3-5γ,5δ] (4ζ-N-méthyl, N-éthylamino) (4ζ-désdiméthylamino) pristinamycine IE
- 2"-tert-butyl-pyrido [2,3-5γ,5δ] (4ζ-N-méthyl, N-propylamino) (4ζ-désdiméthylamino) pristinamycine IE
- 2"-tert-butyl-pyrido [2,3-5γ,5δ] (4ζ-N-méthyl, N-(4-pyridylméthyl) amino) (4ζ-désdiméthylamino) pristinamycine IE
- 2"-tert-butyl-pyrido [2,3-5γ,5δ] (4ζ-N-méthyl, N-(3-pyridylméthyl) amino) (4ζ-désdiméthylamino) pristinamycine IE
- 2"-tert-butyl-pyrido [2,3-5γ,5δ] (4ζ-méthyl) (4ζ-désdiméthylamino) pristinamycine IE
- 2"-tert-butyl-pyrido [2,3-5y,5δ] (4ζ-tert-butyl) (4ζ-désdiméthylamino) pristinamycine IE
- 4ε-bromo 2"-amino-pyrido [2,3-5γ,5δ] pristinamycine IE
- 4ε-bromo 2"-amino-pyrido [2,3-5γ,5δ] (4Ç-méthylamino) (4ζ-désdiméthylamino) pristinamycine IE
- 4ε-allyl 2"-amino-pyrido [2,3-5γ,5δ] pristinamycine IE
- 4ε-allyl 2 "-amino-pyrido [2,3-5γ,5δ] (4ζ-méthylamino) (4ζ-désdiméthylamino) pristinamycine IE
- 4ε-(2-méthyl prop-ène 1-yl) 2"-amino-pyrido [2,3-5γ,5δ] pristinamycine IE
- 4ε-(2-méthyl prop-ène 1-yl) 2"-amino-pyrido [2,3-5γ,5δ] (4ζ-méthylamino) (4ζ-désdiméthylamino) pristinamycine IE
- 2"-amino-pyrido [2,3-5γ,5δ] (4ζ-diéthylamino) (4ζ-désdiméthylamino) pristinamycine IE
- 2"-amino-pyrido [2,3-5γ,5δ] (4ζ-N-méthyl, N-allylamino) (4ζ-désdiméthylamino) pristinamycine IE
- 2"-amino-pyrido [2,3-5γ,5δ] (4ζ-N-méthyl, N-éthylamino) (4ζ-désdiméthylamino) pristinamycine IE
- 2"-amino-pyrido [2,3-5γ,5δ] (4ζ-N-méthyl, N-propylamino) (4ζ-désdiméthylamino) pristinamycine IE
- 2"-amino-pyrido [2,3-5γ,5δ] (4ζ-N-méthyl, N-(4-pyridylméthyl) amino) (4ζ-désdiméthylamino) pristinamycine IE
- 2"-amino-pyrido [2,3-5γ,5δ] (4ζ-N-méthyl, N-(3-pyridylméthyl) amino) (4ζ-désdiméthylamino) pristinamycine IE
- 2''-amino-pyrido [2,3-5γ,5δ] (4ζ-méthyl) (4ζ-désdiméthylamino) pristinamycine IE
- 2"-amino-pyrido [2,3-5γ,5δ] (4ζ-tert-butyl) (4ζ-désdiméthylamino) pristinamycine IE
- 4ε-bromo 3 "-méthoxycarbonyl-2"méthyl-pyrido [2,3-5γ,5δ] pristinamycine IE
- 4ε-bromo 3 "-méthoxycarbonyl-2"méthyl-pyrido [2,3-5γ,5δ] (4ζ-méthylamino) (4ζ-désdiméthylamino) pristinamycine IE
- 4ε-allyl 3 "-méthoxycarbonyl-2"méthyl-pyrido [2,3-5γ,5δ] pristinamycine IE
- 4ε-allyl 3"-méthoxycarbonyl-2"méthyl-pyrido [2,3-5γ,5δ] (4ζ-méthylamino) (4ζ-désdiméthylamino) pristinamycine IE
- 4ε-(2-méthyl prop-ène 1-yl) 3 "-méthoxycarbonyl-2"méthyl-pyrido [2,3-5γ,5δ] pristinamycine IE
- 4ε-(2-méthyl prop-ène 1-yl) 3 "-méthoxycarbonyl-2"méthyl-pyrido [2,3-5γ,5δ] (4ζ-méthylamino) (4ζ-désdiméthylamino) pristinamycine IE
- 3"-méthoxycarbonyl-2"méthyl-pyrido [2,3-5γ,5δ] (4ζ-diéthylamino) (4ζ-désdiméthylamino) pristinamycine IE
- 3"-méthoxycarbonyl-2"méthyl-pyrido [2,3-5γ,5δ] (4ζ-N-méthyl, N-allylamino) (4ζ-désdiméthylamino) pristinamycine IE
- 3 "-méthoxycarbonyl-2"méthyl-pyrido [2,3-5γ,5δ] (4ζ-N-méthyl, N-éthylamino) (4ζ-désdiméthylamino) pristinamycine IE
- 3 "-méthoxycarbonyl-2"méthyl-pyrido [2,3-5γ,5δ] (4ζ-N-méthyl, N-propylamino) (4ζ-désdiméthylamino) pristinamycine IE
- 3 "-méthoxycarbonyl-2"méthyl-pyrido [2,3-5γ,5δ] (4ζ-N-méthyl, N-(4-pyridylméthyl) amino) (4ζ-désdiméthylamino) pristinamycine IE
- 3 "-méthoxycarbonyl-2"méthyl-pyrido [2,3-5γ,5δ] (4ζ-N-méthyl, N-(3-pyridylméthyl) amino) amino)pyridylméthyl)amino) (4ζ-désdiméthylamino) pristinamycine IE
- 3"-méthoxycarbonyl-2"méthyl-pyrido [2,3-5γ,5δ] (4ξ-méthyl) (4ξ-désdiméthylamino) pristinamycine IE
- 3"-méthoxycarbonyl-2"méthyl-pyrido [2,3-5γ,5δ] (4ξ-tert-butyl) (4ξ-désdiméthylamino) pristinamycine IE
- 4ε-bromo 2"-phényl-pyrido [2,3-5γ,5δ] pristinamycine IE
- 4ε-bromo 2"-phényl-pyrido [2,3-5γ,5δ] (4ξ-méthylamino) (4ξ-désdiméthylamino) pristinamycine IE
- 4ε-allyl 2"-phényl-pyrido [2,3-5γ,5δ] pristinamycine IE
- 4ε-allyl 2"-phényl-pyrido [2,3-5γ,5δ] (4ξ-méthylamino) (4ξ-désdiméthylamino) pristinamycine IE
- 4ε-(2-méthyl prop-ène 1-yl) 2"-phényl-pyrido [2,3-5γ,5δ] pristinamycine IE
- 4ε-(2-méthyl prop-ène 1-yl) 2"-phényl-pyrido [2,3-5γ,5δ] (4ξ-méthylamino) (4ξ-désdiméthylamino) pristinamycine IE
- 2"-phényl-pyrido [2,3-5γ,5δ] (4ξ-diéthylamino) (4ξ-désdiméthylamino) pristinamycine IE
- 2"-phényl-pyrido [2,3-5γ,5δ] (4ξ-N-méthyl, N-allylamino) (4ξ-désdiméthylamino) pristinamycine IE
- 2"-phényl-pyrido [2,3-5γ,5δ] (4ξ-N-méthyl, N-éthylamino) (4ξ-désdiméthylamino) pristinamycine IE
- 2"-phényl-pyrido [2,3-5γ,5δ] (4ξ-N-méthyl, N-propylamino) (4ξ-désdiméthylamino) pristinamycine IE
- 2"-phényl-pyrido [2,3-5γ,5δ] (4ξ-N-méthyl, N-(4-pyridylméthyl) amino) amino)pyridylméthyl)amino) (4ξ-désdiméthylamino) pristinamycine IE
- 2"-phényl-pyrido [2,3-5γ,5δ] (4ξ-N-méthyl, N-(3-pyridylméthyl) amino) (4ξ-désdiméthylamino) pristinamycine IE
- 2"-phényl-pyrido [2,3-5γ,5δ] (4ξ-méthyl) (4ξ-désdiméthylamino) pristinamycine IE
- 2"-phényl-pyrido [2,3-5γ,5δ] (4ξ-tert-butyl) (4ξ-désdiméthylamino) pristinamycine IE
- 4ε-bromo 2"-(4-aminophényl)-pyrido [2,3-5γ,5δ] pristinamycine IE
- 4ε-bromo 2"-(4-aminophényl)-pyrido [2,3-5γ,5δ] (4ξ-méthylamino) (4ξ-désdiméthylamino) pristinamycine IE
- 4ε-allyl 2"-(4-aminophényl)-pyrido [2,3-5γ,5δ] pristinamycine IE
- 4ε-allyl 2"-(4-aminophényl)-pyrido [2,3-5γ,5δ (4ζ-méthylamino) (4ζ-désdiméthylamino) pristinamycine IE
- 4ε-(2-méthyl prop-ène 1-yl) 2"-(4-aminophényl)-pyrido [2,3-5γ,5δ] pristinamycine IE
- 4ε-(2-méthyl prop-ène 1-yl) 2"-(4-aminophényl)-pyrido [2,3-5γ,5δ] (4ζ-méthylamino) (4ζ-désdiméthylamino) pristinamycine IE
- 2"-(4-aminophényl)-pyrido [2,3-5γ,5δ] (4ζ-diéthylamino) (4ζ-désdiméthylamino) pristinamycine IE
- 2"-(4-aminophényl)-pyrido [2,3-5γ,5δ] (4ζ-N-méthyl, N-allylamino) (4ζ-désdiméthylamino) pristinamycine IE
- 2"-(4-aminophényl)-pyrido [2,3-5γ,5δ] (4ζ-N-méthyl, N-éthylamino) (4ζ-désdiméthylamino) pristinamycine IE
- 2"-(4-aminophényl)-pyrido [2,3-5γ,5δ] (4ζ-N-méthyl, N-propylamino) (4ζ-désdiméthylamino) pristinamycine IE
- 2"-(4-aminophényl)-pyrido [2,3-5γ,5δ] (4ζ-N-méthyl, N-(4-pyridylméthyl) amino) (4ζ-désdiméthylamino) pristinamycine IE
- 2"-(4-aminophényl)-pyrido [2,3-5γ,5δ] (4ζ-N-méthyl, N-(3-pyridylméthyl) amino) (4ζ-désdiméthylamino) pristinamycine IE
- 2"-(4-aminophényl)-pyrido [2,3-5γ,5δ] (4ζ-méthyl) (4ζ-désdiméthylamino) pristinamycine IE
- 2"-(4-aminophényl)-pyrido [2,3-5γ,5δ] (4ζ-tert-butyl) (4ζ-désdiméthylamino) pristinamycine IE
- 4ε-bromo 2"-(4-diéthylaminophényl)-pyrido [2,3-5γ,5δ] pristinamycine IE
- 4ε-bromo 2"-(4-diéthylaminophényl)-pyrido [2,3-5γ,5δ] (4ζ-méthylamino) (4ζ-désdiméthylamino) pristinamycine IE
- 4ε-allyl 2"-(4-diéthylaminophényl)-pyrido [2,3-5γ,5δ] pristinamycine IE
- 4ε-allyl 2"-(4-diéthylaminophényl)-pyrido [2,3-5γ,5δ] (4ζ-méthylamino) (4ζ-désdiméthylamino) pristinamycine IE
- 4ε-(2-méthyl prop-ène 1-yl) 2"-(4-diéthylaminophényl)-pyrido [2,3-5ε,5δ] pristinamycine IE
- 4ε-(2-méthyl prop-ène 1-yl) 2"-(4-diéthylaminophényl)-pyrido [2,3-5γ,5δ] (4ζ-méthylamino) (4ζ-désdiméthylamino) pristinamycine IE
- 2"-(4-diéthylaminophényl)-pyrido [2,3-5γ,5δ] (4ζ-diéthylamino) (4ζ-désdiméthylamino) pristinamycine IE
- 2"-(4-diéthylaminophényl)-pyrido [2,3-5γ,5δ] (4ζ-N-méthyl, N-allylamino) (4ζ-désdiméthylamino) pristinamycine IE
- 2"-(4-diéthylaminophényl)-pyrido [2,3-5γ,5δ] (4ζ-N-méthyl, N-éthylamino) (4ζ-désdiméthylamino) pristinamycine IE
- 2"-(4-diéthylaminophényl)-pyrido [2,3-5γ,5δ] (4ζ-N-méthyl, N-propylamino) (4ζ-désdiméthylamino) pristinamycine IE
- 2"-(4-diéthylaminophényl)-pyrido [2,3-5γ,5δ] (4ζ-N-méthyl, N-(4-pyridylméthyl) amino) (4ζ-désdiméthylamino) pristinamycine IE
- 2"-(4-diéthylaminophényl)-pyrido [2,3-5γ,5δ] (4ζ-N-méthyl, N-(3-pyridylméthyl) amino) (4ζ-désdiméthylamino) pristinamycine IE
- 2"-(4-diéthylaminophényl)-pyrido [2,3-5γ,5δ] (4ζ-méthyl) (4ζ-désdiméthylamino) pristinamycine IE
- 2"-(4-diéthylaminophényl)-pyrido [2,3-5γ,5δ] (4ζ-tert-butyl) (4ζ-désdiméthylamino) pristinamycine IE
- 4ε-bromo -pyrido [2,3-5γ,5δ] pristinamycine IE
- 4ε-bromo -pyrido [2,3-5γ,5δ] (4ζ-méthylamino) (4ζ-désdiméthylamino) pristinamycine IE
- 4ε-allyl -pyrido [2,3-5γ,5δ] pristinamycine IE
- 4ε-allyl-pyrido [2,3-5γ,5δ] (4ζ-méthylamino) (4ζ-désdiméthylamino) pristinamycine IE
- 4ε-(2-méthyl prop-ène 1-yl) -pyrido [2,3-5γ,5δ] pristinamycine IE
- 4ε-(2-méthyl prop-ène 1-yl) -pyrido [2,3-5γ,5δ] (4Ç-méthylamino) (4Ç-désdiméthylamino) pristinamycine IE
- pyrido [2,3-5γ,5δ] (4ζ-diéthylamino) (4ζ-désdiméthylamino) pristinamycine IE
- pyrido [2,3-5γ,5δ] (4ζ-N-méthyl, N-allylamino) (4ζ-désdiméthylamino) pristinamycine IE
- pyrido [2,3-5γ,5δ] (4ζ-N-méthyl, N-éthylamino) (4ζ-désdiméthylamino) pristinamycine IE
- pyrido [2,3-5γ,5δ] (4ζ-N-méthyl, N-propylamino) (4ζ-désdiméthylamino) pristinamycine IE
- pyrido [2,3-5γ,5δ] (4ζ-N-méthyl, N-(4-pyridylméthyl) amino) (4ζ-désdiméthylamino) pristinamycine IE
- pyrido [2,3-5γ,5δ] (4ζ-N-méthyl, N-(3-pyridylméthyl) amino) (4ζ-désdiméthylamino) pristinamycine IE
- pyrido [2,3-5γ,5δ] (4ζ-méthyl) (4ζ-désdiméthylamino) pristinamycine IE
- pyrido [2,3-5γ,5δ] (4ζ-tert-butyl) (4ζ-désdiméthylamino) pristinamycine IE
- 4ε-bromo 2"-chlorométhyl-pyrido [2,3-5γ,5δ] pristinamycine IE
- 4ε-bromo 2"-chlorométhyl-pyrido [2,3-5γ,5δ] (4ζ-méthylamino) (4ζ-désdiméthylamino) pristinamycine IE
- 4ε-allyl 2"-chlorométhyl-pyrido [2,3-5γ,5δ] pristinamycine IE
- 4ε-allyl 2"-chlorométhyl-pyrido [2,3-5γ,5δ] (4ζ-méthylamino) (4ζ-désdiméthylamino) pristinamycine IE
- 4ε-(2-méthyl prop-ène 1-yl) 2"-chlorométhyl-pyrido [2,3-5γ,5δ] pristinamycine IE
- 4ε-(2-méthyl prop-ène 1-yl) 2"-chlorométhyl-pyrido [2,3-5γ,5δ] (4ζ-méthylamino) (4ζ-désdiméthylamino) pristinamycine IE
- 2"-chlorométhyl-pyrido [2,3-5γ,5δ] (4ζ-diéthylamino) (4ζ-désdiméthylamino) pristinamycine IE
- 2"-chlorométhyl-pyrido [2,3-5γ,5δ] (4ζ-N-méthyl, N-allyl) (4ζ-désdiméthylamino) pristinamycine IE
- 2"-chlorométhyl-pyrido [2,3-5γ,5δ] (4ζ-N-méthyl, N-éthylamino) (4ζ-désdiméthylamino) pristinamycine IE
- 2"-chlorométhyl-pyrido [2,3-5γ,5δ] (4ζ-N-méthyl, N-propylamino) (4ζ-désdiméthylamino) pristinamycine IE
- 2"-chlorométhyl-pyrido [2,3-5γ,5δ] (4ζ-N-méthyl, N-(4-pyridylméthyl) amino) (4ζ-désdiméthylamino) pristinamycine IE
- 2"-chlorométhyl-pyrido [2,3-5γ,5δ] (4ζ-N-méthyl, N-(3-pyridylméthyl) amino) (4ζ-désdiméthylamino) pristinamycine IE
- 2"-chlorométhyl-pyrido [2,3-5γ,5δ] (4ζ-méthyl) (4ζ-désdiméthylamino) pristinamycine IE
- 2"-chlorométhyl-pyrido [2,3-5γ,5δ] (4ζ-tert-butyl) (4ζ-désdiméthylamino) pristinamycine IE
- 4ε-bromo 2"-(2-pyridyl)-pyrido [2,3-5γ,5δ] pristinamycine IE
- 4ε-bromo 2"-(2-pyridyl)-pyrido [2,3-5γ,5δ] (4ζ-méthylamino) (4ζ-désdiméthylamino) pristinamycine IE
- 4ε-allyl 2"-(2-pyridyl)-pyrido [2,3-5γ,5δ] pristinamycine IE
- 4ε-allyl 2"-(2-pyridyl)-pyrido [2,3-5γ,5δ] (4ζ-méthylamino) (4ζ-désdiméthylamino) pristinamycine IE
- 4ε-(2-méthyl prop-ène 1-yl) 2"-(2-pyridyl)-pyrido [2,3-5γ,5δ] pristinamycine IE
- 4ε-(2-méthyl prop-ène 1-yl) 2"-(2-pyridyl)-pyrido [2,3-5γ,5δ] (4ζ-méthylamino) (4ζ-désdiméthylamino) pristinamycine IE
- 2"-(2-pyridyl)-pyrido [2,3-5γ,5δ] (4ζ-diéthylamino) (4ζ-désdiméthylamino) pristinamycine IE
- 2"-(2-pyridyl)-pyrido [2,3-5γ,5δ] (4ζ-N-méthyl, N-allylamino) (4ζ-désdiméthylamino) pristinamycine IE
- 2"-(2-pyridyl)-pyrido [2,3-5γ,5δ] (4ζ-N-méthyl, N-éthylamino) (4ζ-désdiméthylamino) pristinamycine IE
- 2"-(2-pyridyl)-pyrido [2,3-5γ,5δ] (4ζ-N-méthyl, N-propylamino) (4ζ-désdiméthylamino) pristinamycine IE
- 2"-(2-pyridyl)-pyrido [2,3-5γ,5δ] (4ζ-N-méthyl, N-(4-pyridylméthyl) amino) (4ζ-désdiméthylamino) pristinamycine IE
- 2"-(2-pyridyl)-pyrido [2,3-5γ,5δ] (4ζ-N-méthyl, N-(3-pyridylméthyl) amino) (4ζ-désdiméthylamino) pristinamycine IE
- 2"-(2-pyridyl)-pyrido [2,3-5γ,5δ] (4ζ-méthyl) (4ζ-désdiméthylamino) pristinamycine IE
- 2"-(2-pyridyl)-pyrido [2,3-5γ,5δ] (4ζ-tert-butyl) (4ζ-désdiméthylamino) pristinamycine IE
- 4ε-bromo 2"-morpholinométhyl-pyrido [2,3-5γ,5δ] pristinamycine IE
- 4ε-bromo 2"- morpholinométhyl-pyrido [2,3-5γ,5δ] (4ζ-méthylamino) (4ζ-désdiméthylamino) pristinamycine IE
- 4ε-allyl 2"- morpholinométhyl-pyrido [2,3-5γ,5δ] pristinamycine IE
- 4ε-allyl 2"- morpholinométhyl -pyrido [2,3-5γ,5δ] (4ζ-méthylamino) (4ζ-désdiméthylamino) pristinamycine IE
- 4ε-(2-méthyl prop-ène 1-yl) 2"- morpholinométhyl-pyrido [2,3-5γ,5δ] pristinamycine IE
- 4ε-(2-méthyl prop-ène 1-yl) 2"- morpholinométhyl-pyrido [2,3-5γ,5δ] (4ζ-méthylamino) (4ζ-désdiméthylamino) pristinamycine IE
- 2"- morpholinométhyl-pyrido [2,3-5γ,5δ] (4ζ-diéthylamino) (4ζ-désdiméthylamino) pristinamycine IE
- 2"- morpholinométhyl-pyrido [2,3-5γ,5δ] (4ζ-N-méthyl, N-allylamino) (4ζ-désdiméthylamino) pristinamycine IE
- 2"- morpholinométhyl-pyrido [2,3-5γ,5δ] (4ζ-N-méthyl, N-éthylamino) (4ζ-désdiméthylamino) pristinamycine IE
- 2"- morpholinométhyl-pyrido [2,3-5γ,5δ] (4ζ-N-méthyl, N-propylamino) (4ζ-désdiméthylamino) pristinamycine IE
- 2"- morpholinométhyl-pyrido [2,3-5γ,5δ] (4ζ-N-méthyl, N-(4-pyridylméthyl) amino) (4ζ-désdiméthylamino) pristinamycine IE
- 2"- morpholinométhyl-pyrido [2,3-5γ,5δ] (4Ç-N-méthyl, N-(3-pyridylméthyl) amino) (4ζ-désdiméthylamino) pristinamycine IE
- 2"- morpholinométhyl-pyrido [2,3-5γ,5δ] (4ζ-méthyl) (4ζ-désdiméthylamino) pristinamycine IE
- 2"- morpholinométhyl-pyrido [2,3-5γ,5δ] (4ζ-tert-butyl) (4ζ-désdiméthylamino) pristinamycine IE
- 4ε-bromo 2"-(3-pyridyl)-pyrido [2,3-5γ,5δ] pristinamycine IE
- 4ε-bromo 2"-(3-pyridyl)-pyrido [2,3-5γ,5δ] (4ζ-méthylamino) (4ζ-désdiméthylamino) pristinamycine IE
- 4ε-allyl 2"-(3-pyridyl)-pyrido [2,3-5γ,5δ] pristinamycine IE
- 4ε-allyl 2"-(3-pyridyl)-pyrido [2,3-5γ,5δ] (4ζ-méthylamino) (4ζ-désdiméthylamino) pristinamycine IE
- 4ε-(2-méthyl prop-ène 1-yl) 2"-(3-pyridyl)-pyrido [2,3-5γ,5δ] pristinamycine IE
- 4ε-(2-méthyl prop-ène 1-yl) 2"-(3-pyridyl)-pyrido [2,3-5γ,5δ] (4ζ-méthylamino) (4ζ-désdiméthylamino) pristinamycine IE
- 2"-(3-pyridyl)-pyrido [2,3-5γ,5δ] (4ζ-diéthylamino) (4ζ-désdiméthylamino) pristinamycine IE
- 2"-(3-pyridyl)-pyrido [2,3-5γ,5δ] (4ζ-N-méthyl, N-allylamino) (4ζ-désdiméthylamino) pristinamycine IE
- 2"-(3-pyridyl)-pyrido [2,3-5γ,5δ] (4ζ-N-méthyl, N-éthylamino) (4ζ-désdiméthylamino) pristinamycine IE
- 2"-(3-pyridyl)-pyrido [2,3-5γ,5δ] (4ζ-N-méthyl, N-propylamino) (4ζ-désdiméthylamino) pristinamycine IE
- 2"-(3-pyridyl)-pyrido [2,3-5γ,5δ] (4ζ-N-méthyl, N-(4-pyridylméthyl) amino) (4ζ-désdiméthylamino) pristinamycine IE
- 2"-(3-pyridyl)-pyrido [2,3-5γ,5δ] (4ζ-N-méthyl, N-(3-pyridylméthyl) amino) (4ζ-désdiméthylamino) pristinamycine IE
- 2"-(3-pyridyl)-pyrido [2,3-5γ,5δ] (4ζ-méthyl) (4ζ-désdiméthylamino) pristinamycine IE
- 2"-(3-pyridyl)-pyrido [2,3-5γ,5δ] (4ζ-tert-butyl) (4ζ-désdiméthylamino) pristinamycine IE
- 4ε-bromo 2"-(4-méthyl-1-pipérazinylméthyl)-pyrido [2,3-5γ,5δ] pristinamycine IE
- 4ε-bromo 2"-(4-méthyl-1-pipérazinylméthyl)-pyrido [2,3-5γ,5δ] (4ζ-méthylamino) (4ζ-désdiméthylamino) pristinamycine IE
- 4ε-allyl 2"-(4-méthyl-1-pipérazinylméthyl)-pyrido [2,3-5γ,5δ] pristinamycine IE
- 4ε-allyl 2"-(4-méthyl-1-pipérazinylméthyl)-pyrido [2,3-5γ,5δ](4ζ-méthylamino) (4ζ**-**désdiméthylamino) pristinamycine IE
- 4ε-(2-méthyl prop-ène 1-yl) 2"-(4-méthyl-1-pipérazinylméthyl)-pyrido [2,3-5γ,5δ] pristinamycine IE
- 4ε-(2-méthyl prop-ène 1-yl) 2"-(4-méthyl-1-pipérazinylméthyl)-pyrido [2,3-5γ,5δ] (4ζ-méthylamino) (4ζ-désdiméthylamino) pristinamycine IE
- 2"-(4-méthyl-1-pipérazinylméthyl)-pyrido [2,3-5γ,5δ] (4ζ-diéthylamino) (4ζ-désdiméthylamino) pristinamycine IE
- 2"-(4-méthyl-1-pipérazinylméthyl)-pyrido [2,3-5γ,5δ] (4ζ-N-méthyl, N-allylamino) (4ζ-désdiméthylamino) pristinamycine IE
- 2"-(4-méthyl-1-pipérazinylméthyl)-pyrido [2,3-5γ,5δ] (4ζ-N-méthyl, N-éthylamino) (4ζ-désdiméthylamino) pristinamycine IE
- 2"-(4-méthyl-1-pipérazinylméthyl)-pyrido [2,3-5γ,5δ] (4ζ-N-méthyl, N-propylamino) (4ζ-désdiméthylamino) pristinamycine IE
- 2"-(4-méthyl-1-pipérazinylméthyl)-pyrido [2,3-5γ,5δ] (4ζ-N-méthyl, N-(4-pyridylméthyl) amino) (4ζ-désdiméthylamino) pristinamycine IE
- 2"-(4-méthyl-1-pipérazinylméthyl)-pyrido [2,3-5γ,5δ] (4ζ-N-méthyl, N-(3-pyridylméthyl) amino) (4ζ-désdiméthylamino) pristinamycine IE
- 2"-(4-méthyl-1-pipérazinylméthyl)-pyrido [2,3-5γ,5δ] (4ζ-méthyl) (4ζ-désdiméthylamino) pristinamycine IE
- 2"-(4-méthyl-1-pipérazinylméthyl)-pyrido [2,3-5γ,5δ] (4ζ-tert-butyl) (4ζ-désdiméthylamino) pristinamycine IE
- 4ε-bromo 2"-éthyl-pyrido [2,3-5γ,5δ] pristinamycine IE
- 4ε-bromo 2"-éthyl-pyrido [2,3-5γ,5δ] (4ζ-méthylamino) (4ζ-désdiméthylamino) pristinamycine IE
- 4ε-allyl 2"-éthyl-pyrido [2,3-Sγ,5δ] pristinamycine IE
- 4ε-allyl 2"-éthyl-pyrido [2,3-5γ,5δ] (4ζ-méthylamino) (4ζ-désdiméthylamino) pristinamycine IE
- 4ε-(2-méthyl prop-ène 1-yl) 2"-éthyl-pyrido [2,3-5γ,5δ] pristinamycine IE
- 4ε-(2-méthyl prop-ène 1-yl) 2"-éthyl-pyrido [2,3-5γ,5δ] (4ζ-méthylamino) (4ζ-désdiméthylamino) pristinamycine IE
- 2"-éthyl-pyrido [2,3-5γ,5δ] (4ζ-diéthylamino) (4ζ-désdiméthylamino) pristinamycine IE
- 2"-éthyl-pyrido [2,3-5γ,5δ] (4ζ-N-méthyl, N-allylamino) (4ζ-désdiméthylamino) pristinamycine IE
- 2"-éthyl-pyrido [2,3-5γ,5δ] (4ζ-N-méthyl, N-éthylamino) (4ζ-désdiméthylamino) pristinamycine IE
- 2"-éthyl-pyrido [2,3-5γ,5δ] (4ζ-N-méthyl, N-propylamino) (4ζ-désdiméthylamino) pristinamycine IE
- 2"-éthyl-pyrido [2,3-5γ,5δ] (4ζ-N-méthyl, N-(4-pyridylméthyl) amino) (4ζ-désdiméthylamino) pristinamycine IE
- 2"-éthyl-pyrido [2,3-5γ,5δ] (4ζ-N-méthyl, N-(3-pyridylméthyl) amino) (4ζ-désdiméthylamino) pristinamycine IE
- 2"-éthyl-pyrido [2,3-5γ,5δ] (4ζ-méthyl) (4ζ-désdiméthylamino) pristinamycine IE
- 2"-éthyl-pyrido [2,3-5γ,5δ] (4ζ-tert-butyl) (4ζ-désdiméthylamino) pristinamycine IE
- 4ε-bromo 2"-cyclopropyl-pyrido [2,3-5γ,5δ] pristinamycine IE
- 4ε-bromo 2"-cyclopropyl-pyrido [2,3-5γ,5δ] (4ζ-méthylamino) (4ζ-désdiméthylamino) pristinamycine IE
- 4ε-allyl 2"-cyclopropyl-pyrido [2,3-5γ,5δ] pristinamycine IE
- 4ε-allyl 2"-cyclopropyl-pyrido [2,3-5γ,5δ] (4ζ-méthylamino) (4ζ-désdiméthylamino) pristinamycine IE
- 4ε-(2-méthyl prop-ène 1-yl) 2"-cyclopropyl-pyrido [2,3-5γ,5δ] pristinamycine IE
- 4ε-(2-méthyl prop-ène 1-yl) 2"-cyclopropyl-pyrido [2,3-5γ,5δ] (4ζ-méthylamino) (4ζ-désdiméthylamino) pristinamycine IE
- 2"-cyclopropyl-pyrido [2,3-5γ,5δ] (4ζ-diéthylamino) (4ζ-désdiméthylamino) pristinamycine IE
- 2"-cyclopropyl-pyrido [2,3-5γ,5δ] (4ζ-N-méthyl, N-allylamino) (4ζ-désdiméthylamino) pristinamycine IE
- 2"-cyclopropyl-pyrido [2,3-5γ,5δ] (4ζ-N-méthyl, N-éthylamino) (4ζ-désdiméthylamino) pristinamycine IE
- 2"-cyclopropyl-pyrido [2,3-5γ,5δ] (4ζ-N-méthyl, N-propylamino) (4ζ-désdiméthylamino) pristinamycine IE
- 2"-cyclopropyl-pyrido [2,3-5γ,5δ] (4ζ-N-méthyl, N-(4-pyridylméthyl) amino) (4ζ-désdiméthylamino) pristinamycine IE
- 2"-cyclopropyl-pyrido [2,3 -5γ,5δ] (4ζ-N-méthyl, N-(3-pyridylméthyl) amino) (4ζ-désdiméthylamino) pristinamycine IE
- 2"-cyclopropyl-pyrido [2,3-5γ,5δ] (4ζ-méthyl) (4ζ-désdiméthylamino) pristinamycine IE
- 2"-cyclopropyl-pyrido [2,3-5γ,5δ] (4ζ-tert-butyl) (4ζ-désdiméthylamino) pristinamycine IE
- 4ε-bromo 2"-hydroxyméthyl-pyrido [2,3-5γ,5δ] pristinamycine IE
- 4ε-bromo 2"-hydroxyméthyl-pyrido [2,3-5γ,5δ] (4ζ-méthylamino) (4ζ-désdiméthylamino) pristinamycine IE
- 4ε-allyl 2"-hydroxyméthyl-pyrido [2,3-5γ,5δ] pristinamycine IE
- 4ε-allyl 2"-hydroxyméthyl-pyrido [2,3-5γ,5δ] (4ζ-méthylamino) (4ζ-désdiméthylamino) pristinamycine IE
- 4ε-(2-méthyl prop-ène 1-yl) 2"-hydroxyméthyl-pyrido [2,3-5γ,5δ] pristinamycine IE
- 4ε-(2-méthyl prop-ène 1-yl) 2"-hydroxyméthyl-pyrido [2,3-5γ,5δ] (4ζ-méthylamino) (4ζ-désdiméthylamino) pristinamycine IE
- 2"-hydroxyméthyl-pyrido [2,3-5γ,5δ] (4ζ-diéthylamino) (4ζ-désdiméthylamino) pristinamycine IE
- 2"-hydroxyméthyl-pyrido [2,3-5γ,5δ] (4ζ-N-méthyl, N-allylamino) (4ζ-désdiméthylamino) pristinamycine IE
- 2"-hydroxyméthyl-pyrido [2,3-5γ,5δ] (4ζ-N-méthyl, N-éthylamino) (4ζ-désdiméthylamino) pristinamycine IE
- 2"-hydroxyméthyl-pyrido [2,3-5γ,5δ] (4ζ-N-méthyl, N-propylamino) (4ζ-désdiméthylamino) pristinamycine IE
- 2"-hydroxyméthyl-pyrido [2,3-5γ,5δ] (4ζ-N-méthyl, N-(4-pyridylméthyl) amino) (4ζ-désdiméthylamino) pristinamycine IE
- 2"-hydroxyméthyl-pyrido [2,3-5γ,5δ] (4ζ-N-méthyl, N-(3-pyridylméthyl) amino) (4ζ-désdiméthylamino) pristinamycine IE
- 2"-hydroxyméthyl-pyrido [2,3-5γ,5δ] (4ζ-méthyl) (4ζ-désdiméthylamino) pristinamycine IE
- 2"-hydroxyméthyl-pyrido [2,3-5γ,5δ] (4ζ-tert-butyl) (4ζ-désdiméthylamino) pristinamycine IE
- 4ε-bromo 2"-propyl-pyrido [2,3-5γ,5δ] pristinamycine IE
- 4ε-bromo 2"-propyl-pyrido [2,3-5γ,5δ] (4ζ-méthylamino) (4ζ-désdiméthylamino) pristinamycine IE
- 4ε-allyl 2"-propyl-pyrido [2,3-5γ,5δ] pristinamycine IE
- 4ε-allyl 2"-propyl-pyrido [2,3-5γ,5δ] (4ζ-méthylamino) (4ζ-désdiméthylamino) pristinamycine IE
- 4ε-(2-méthyl prop-ène 1-yl) 2"-propyl-pyrido [2,3-5y,50] pristinamycine IE
- 4ε-(2-méthyl prop-ène 1-yl) 2"-propyl-pyrido [2,3-5γ,5δ] (4ζ-méthylamino) (4ζ-désdiméthylamino) pristinamycine IE
- 2"-propyl-pyrido [2,3-5γ,5δ] (4ζ-diéthylamino) (4ζ-désdiméthylamino) pristinamycine IE
- 2"-propyl-pyrido [2,3-5γ,5δ] (4ζ-N-méthyl, N-allylamino) (4ζ-désdiméthylamino) pristinamycine IE
- 2"-propyl-pyrido [2,3-5γ,5δ] (4ζ-N-méthyl, N-éthylamino) (4ζ-désdiméthylamino) pristinamycine IE
- 2"-propyl-pyrido [2,3-5γ,5δ] (4ζ-N-méthyl, N-propylamino) (4ζ-désdiméthylamino) pristinamycine IE
- 2"-propyl-pyrido [2,3-5γ,5δ] (4ζ-N-méthyl, N-(4-pyridylméthyl) amino) (4ζ-désdiméthylamino) pristinamycine IE
- 2 "-propyl-pyrido [2,3-5γ,5δ] (4ζ-N-méthyl, N-(3-pyridylméthyl) amino) (4ζ-désdiméthylamino) pristinamycine IE
- 2"-propyl-pyrido [2,3-5γ,5δ] (4ζ-méthyl) (4ζ-désdiméthylamino) pristinamycine IE
- 2"-propyl-pyrido [2,3-5γ,5δ] (4ζ-tert-butyl) (4ζ-désdiméthylamino) pristinamycine IE
- 4ε-bromo 2"-isopropyl-pyrido [2,3-5γ,5δ] pristinamycine IE
- 4ε-bromo 2"-isopropyl-pyrido [2,3-5γ,5δ] (4ζ-méthylamino) (4ζ-désdiméthylamino) pristinamycine IE
- 4ε-allyl 2"-isopropyl-pyrido [2,3-5γ,5δ] pristinamycine IE
- 4ε-allyl 2"-isopropyl-pyrido [2,3-5γ,5δ] (4ζ-méthylamino) (4ζ-désdiméthylamino) pristinamycine IE
- 4ε-(2-méthyl prop-ène 1-yl) 2"-isopropyl-pyrido [2,3-5γ,5δ] pristinamycine IE
- 4ε-(2-méthyl prop-ène 1-yl) 2"-isopropyl-pyrido [2,3-5γ,5δ] (4ζ-méthylamino) (4ζ-désdiméthylamino) pristinamycine IE
- 2"-isopropyl-pyrido [2,3-5γ,5δ] (4ζ-diéthylamino) (4ζ-désdiméthylamino) pristinamycine IE
- 2"-isopropyl-pyrido [2,3-5γ,5δ] (4ζ-N-méthyl, N-allylamino) (4ζ-désdiméthylamino) pristinamycine IE
- 2"-isopropyl-pyrido [2,3-5γ,5δ] (4ζ-N-méthyl, N-éthylamino) (4ζ-désdiméthylamino) pristinamycine IE
- 2"-isopropyl-pyrido [2,3-5γ,5δ] (4ζ-N-méthyl, N-propylamino) (4ζ-désdiméthylamino) pristinamycine IE
- 2"-isopropyl-pyrido [2,3-5γ,5δ] (4ζ-N-méthyl, N-(4-pyridylméthyl) amino) (4ζ-désdiméthylamino) pristinamycine IE
- 2"-isopropyl-pyrido [2,3-5γ,5δ] (4ζ-N-méthyl, N-(3-pyridylméthyl) amino) (4ζ-désdiméthylamino) pristinamycine IE
- 2"-isopropyl-pyrido [2,3-5γ,5δ] (4ζ-méthyl) (4ζ-désdiméthylamino) pristinamycine IE
- 2"-isopropyl-pyrido [2,3-5γ,5δ] (4ζ-tert-butyl) (4ζ-désdiméthylamino) pristinamycine IE
- 4ε-bromo 2"-acétoxyméthylméthyl-pyrido [2,3-5γ,5δ] pristinamycine IE
- 4ε-bromo 2"-acétoxyméthylméthyl-pyrido [2,3-5γ,5δ] (4ζ-méthylamino) (4ζ-désdiméthylamino) pristinamycine IE
- 4ε-allyl 2"-acétoxyméthylméthyl-pyrido [2,3-5γ,5δ] pristinamycine IE
- 4ε-allyl 2 "-acétoxyméthylméthyl-pyrido [2,3-5γ,5δ] (4ζ-méthylamino) (4ζ-désdiméthylamino) pristinamycine IE
- 4ε-(2-méthyl prop-ène 1-yl) 2"-acétoxyméthyl-pyrido [2,3-5γ-5δ] pristinamycine IE
- 4ε-(2-méthyl prop-ène 1-yl) 2"-acétoxyméthyl-pyrido [2,3-5γ-5δ] (4ζ-méthylamino) (4ζ-désdiméthylamino) pristinamycine IE
- 2"-acétoxyméthyl-pyrido [2,3-5γ,5δ] (4ζ-diéthylamino) (4ζ-désdiméthylamino) pristinamycine IE
- 2"-acétoxyméthyl-pyrido [2,3-5γ,5δ] (4ζ-N-méthyl, N-allylamino) (4ζ-désdiméthylamnino) pristinamycine IE
- 2"-acétoxyméthyl-pyrido [2,3-5γ,5δ] (4ζ-N-méthyl, N-éthylamino) (4ζ-désdiméthylamino) pristinamycine IE
- 2"-acétoxyméthyl-pyrido [2,3-5γ,5δ] (4ζ-N-méthyl, N-propylamino) (4ζ-désdiméthylamino) pristinamycine IE
- 2"-acétoxyméthyl-pyrido [2,3-5γ,5δ] (4ζ-N-méthyl, N-(4-pyridylméthyl) amino) (4ζ-désdiméthylamino) pristinamycine IE
- 2"-acétoxyméthyl-pyrido [2,3-5γ,5δ] (4ζ-N-méthyl, N-(3-pyridylméthyl) amino) (4ζ-désdiméthylamino) pristinamycine IE
- 2"-acétoxyméthyl-pyrido [2,3-5γ,5δ] (4ζ-méthyl) (4ζ-désdiméthylamino) pristinamycine IE
- 2"-acétoxyméthyl-pyrido [2,3-5γ,5δ] (4ζ-tert-butyl) (4ζ-désdiméthylamino) pristinamycine IE
- 4ε-bromo 2"-cyclopropylaminométhyl-pyrido [2,3-5γ,5δ] pristinamycine IE
- 4ε-bromo 2"-cyclopropylaminométhyl-pyrido [2,3-5γ,5δ] (4ζ-méthylamino) (4ζ-désdiméthylamino) pristinamycine IE
- 4ε-allyl 2"-cyclopropylaminométhyl-pyrido [2,3-5γ,5δ] pristinamycine IE
- 4ε-allyl 2"-cyclopropylaminométhyl-pyrido [2,3-5γ,5δ] (4ζ-méthylamino) (4ζ-désdiméthylamino) pristinamycine IE
- 4ε-(2-méthyl prop-ène 1-yl) 2"-cyclopropylaminométhyl-pyrido [2,3-5γ,5δ] pristinamycine IE
- 4ε-(2-méthyl prop-ène 1-yl) 2"-cyclopropylaminométhyl-pyrido [2,3-5γ,5δ](4ζ-méthylamino) (4ζ-désdiméthylamino) pristinamycine IE
- 2"-cyclopropylaminométhyl-pyrido [2,3-5γ,5δ] (4ζ-diéthylamino) (4ζ-désdiméthylamino) pristinamycine IE
- 2"-cyclopropylaminométhyl-pyrido [2,3-5γ,5δ] (4ζ-N-méthyl, N-allylamino) (4ζ-désdiméthylamino) pristinamycine IE
- 2"-cyclopropylaminométhyl-pyrido [2,3-5γ,5δ] (4ζ-N-méthyl, N-éthylamino) (4ζ-désdiméthylamino) pristinamycine IE
- 2"-cyclopropylaminométhyl-pyrido [2,3-5γ,5δ] (4ζ-N-méthyl, N-propylamino) (4ζ-désdiméthylamino) pristinamycine IE
- 2"-cyclopropylaminométhyl-pyrido [2,3-5γ,5δ] (4ζ-N-méthyl, N-(4-pyridylméthyl) amino) (4ζ-désdiméthylamino) pristinamycine IE
- 2"-cyclopropylaminométhyl-pyrido [2,3-5γ,5δ] (4ζ-N-méthyl, N-(3-pyridylméthyl) amino) (4ζ-désdiméthylamino) pristinamycine IE
- 2"-cyclopropylaminométhyl-pyrido [2,3-5γ,5δ] (4ζ-méthyl) (4ζ-désdiméthylamino) pristinamycine IE
- 2"-cyclopropylaminométhyl-pyrido [2,3-5γ,5δ] (4ζ-tert-butyl) (4ζ-désdiméthylamino) pristinamycine IE
- 4ε-bromo 2",3"-diméthyl-pyrido [2,3-5γ,5δ] pristinamycine IE
- 4ε-bromo 2",3 "-diméthyl-pyrido [2,3-5γ,5δ] (4ζ-méthylamino) (4ζ-désdiméthylamino) pristinamycine IE
- 4ε-allyl 2",3"-diméthyl-pyrido [2,3-5γ,5δ] pristinamycine IE
- 4ε-allyl 2",3 "-diméthyl-pyrido [2,3-5γ,5δ] (4ζ-méthylamino) (4ζ-désdiméthylamino) pristinamycine IE
- 4ε-(2-méthyl prop-ène 1-yl) 2"-,3"-diméthyl-pyrido [2,3-5γ,5δ] pristinamycine IE
- 4ε-(2-méthyl prop-ène 1-yl) 2"-,3"-diméthyl-pyrido [2,3-5γ,5δ] (4ζ-méthylamino) (4ζ-désdiméthylamino) pristinamycine IE
- 2",3 "-diméthyl-pyrido [2,3-5γ,5δ] (4ζ-diéthylamino) (4ζ-désdiméthylamino) pristinamycine IE
- 2",3"-diméthyl-pyrido [2,3-5γ,5δ] (4ζ-N-méthyl, N-allylamino) (4ζ-désdiméthylamino) pristinamycine IE
- 2",3"-diméthyl-pyrido [2,3-5γ,5δ] (4ζ-N-méthyl, N-éthylamino) (4ζ-désdiméthylamino) pristinamycine IE
- 2",3"-diméthyl-pyrido [2,3-5γ,5δ] (4ζ-N-méthyl, N-propylamino) (4ζ-désdiméthylamino) pristinamycine IE
- 2",3"-diméthyl-pyrido [2,3-5γ,5δ] (4ζ-N-méthyl, N-(4-pyridylméthyl) amino) (4ζ-désdiméthylamino) pristinamycine IE
- 2",3"-diméthyl-pyrido [2,3-5γ,5δ] (4ζ-N-méthyl, N-(3-pyridylméthyl) amino) (4ζ-désdiméthylamino) pristinamycine IE
- 2",3"-diméthyl-pyrido [2,3-5γ,5δ] (4ζ-méthyl) (4ζ-désdiméthylamino) pristinamycine IE
- 2",3"-diméthyl-pyrido [2,3-5γ,5δ] (4ζ-tert-butyl) (4ζ-désdiméthylamino) pristinamycine IE
- 4ε-bromo 2"-éthoxycarbonyl-pyrido [2,3-5γ,5δ] pristinamycine IE
- 4ε-bromo 2"-éthoxycarbonyl-pyrido [2,3-5γ,5δ] (4ζ-méthylamino) (4ζ-désdiméthylamino) pristinamycine IE
- 4ε-allyl 2"-éthoxycarbonyl-pyrido [2,3-5γ,5δ] pristinamycine IE
- 4ε-allyl 2"-éthoxycarbonyl-pyrido [2,3-5γ,5δ] (4ζ-méthylamino) (4ζ-désdiméthylamino) pristinamycine IE
- 4ε-(2-méthyl prop-ène 1-yl) 2 "-éthoxycarbonyl-pyrido [2,3-5γ,5δ] pristinamycine IE
- 4ε-(2-méthyl prop-ène 1-yl) 2"-éthoxycarbonyl-pyrido [2,3-5γ,5δ] (4ζ-méthylamino) (4ζ-désdiméthylamino) pristinamycine IE
- 2"-éthoxycarbonyl-pyrido [2,3-5γ,5δ] (4ζ-diéthylamino) (4ζ-désdiméthylamino) pristinamycine IE
- 2"-éthoxycarbonyl-pyrido [2,3-5γ,5δ] (4ζ-N-méthyl, N-allylamino) (4ζ-désdiméthylamino) pristinamycine IE
- 2"-éthoxycarbonyl-pyrido [2,3-5γ,5δ] (4ζ-N-méthyl, N-éthylamino) (4ζ-désdiméthylamino) pristinamycine IE
- 2"-éthoxycarbonyl-pyrido [2,3-5γ,5δ] (4ζ-N-méthyl, N-propylamino) (4ζ-désdiméthylamino) pristinamycine IE
- 2"-éthoxycarbonyl-pyrido [2,3-5γ,5δ] (4ζ-N-méthyl, N-(4-pyridylméthyl) amino) (4ζ-désdiméthylamino) pristinamycine IE
- 2"-éthoxycarbonyl-pyrido [2,3-5γ,5δ] (4ζ-N-méthyl, N-(3-pyridylméthyl) amino) (4ζ-désdiméthylamino) pristinamycine IE
- 2"-éthoxycarbonyl-pyrido [2,3-5γ,5δ] (4ζ-méthyl) (4ζ-désdiméthylamino) pristinamycine IE
- 2"-éthoxycarbonyl-pyrido [2,3-5γ,5δ] (4ζ-tert-butyl) (4ζ-désdiméthylamino) pristinamycine IE
- 4ε-bromo 2"-(N-diéthylaminométhyl)-pyrido [2,3-5γ,5δ] pristinamycine IE
- 4ε-bromo 2"-(N-diéthylaminométhyl)-pyrido [2,3-5γ,5δ] (4ζ-méthylamino) (4ζ-désdiméthylamino) pristinamycine IE
- 4ε-allyl2''-(N-diéthylaminométhyl)-pyrido [2,3-5γ,5δ] pristinamycine IE
- 4ε-allyl 2"-(N-diéthylaminométhyl)-pyrido [2,3-5γ,5δ] (4ζ-méthylamino) (4ζ-désdiméthylamino) pristinamycine IE
- 4ε-(2-méthyl prop-ène 1-yl) 2" -(N-diéthylaminométhyl)-pyrido [2,3-5γ,5δ] pristinamycine IE
- 4ε-(2-méthyl prop-ène 1-yl) 2"-(N-diéthylaminométhyl)-pyrido [2,3-5γ,5δ] (4ζ-méthylamino) (4ζ-désdiméthylamino) pristinamycine IE
- 2"-(N-diéthylaminométhyl)-pyrido [2,3-5γ,5δ] (4ζ-diéthylamino) (4ζ-désdiméthylamino) pristinamycine IE
- 2"-(N-diéthylaminométhyl)-pyrido [2,3-5γ,5δ] (4ζ-N-méthyl, N-allylamino) (4ζ-désdiméthylamino)- pristinamycine IE
- 2"-(N-diéthylaminométhyl)-pyrido [2,3-5γ,5δ] (4ζ-N-méthyl, N-éthylamino) (4ζ-désdiméthylamino) pristinamycine IE
- 2"-(N-diéthylaminométhyl)-pyrido [2,3-5γ,5δ] (4ζ-N-méthyl, N-propylamino) (4ζ-désdiméthylamino) pristinamycine IE
- 2"-(N-diéthylaminométhyl)-pyrido [2,3-5γ,5δ] (4ζ-N-méthyl, N-(4-pyridylméthyl) amino) (4ζ-désdiméthylamino) pristinamycine IE
- 2"-(N-diéthylaminométhyl)-pyrido [2,3-5γ,5δ] (4ζ-N-méthyl, N-(3-pyridylméthyl) amino) (4ζ-désdiméthylamino) pristinamycine IE
- 2"-(N-diéthylaminométhyl)-pyrido [2,3-5γ,5δ] (4ζ-méthyl) (4ζ-désdiméthylamino) pristinamycine IE
- 2"-(N-diéthylaminométhyl)-pyrido [2,3-5γ,5δ] (4ζ-tert-butyl) (4ζ-désdiméthylamino) pristinamycine IE
- 4ε-bromo 2"-carbamoyl-pyrido [2,3-5γ,5δ] pristinamycine IE
- 4ε-bromo 2"-carbamoyl-pyrido [2,3-5γ,5δ] (4ζ-méthylamino) (4ζ-désdiméthylamino) pristinamycine IE
- 4ε-allyl 2"-carbamoyl-pyrido [2,3-5γ,5δ] pristinamycine IE
- 4ε-allyl 2"-carbamoyl-pyrido [2,3-5γ,5δ] (4ζ-méthylamino) (4ζ-désdiméthylamino) pristinamycine IE
- 4ε-(2-méthyl prop-ène 1-yl) 2"-carbamoyl-pyrido [2,3-5γ,5δ] pristinamycine IE
- 4ε-(2-méthyl prop-ène 1-yl) 2"-carbamoyl-pyrido [2,3-5γ,5δ] (4ζ-méthylamino) (4ζ-désdiméthylamino) pristinamycine IE
- 2"-carbamoyl-pyrido [2,3-5γ,5δ] (4ζ-diéthylamino) (4ζ-désdiméthylamino) pristinamycine IE
- 2"-carbamoyl-pyrido [2,3-5γ,5δ] (4ζ-N-méthyl, N-allylamino) (4ζ-désdiméthylamino) pristinamycine IE
- 2"-carbamoyl-pyrido [2,3-5γ,5δ] (4ζ-N-méthyl, N-éthylamino) (4ζ-désdiméthylamino) pristinamycine IE
- 2"-carbamoyl-pyrido [2,3-5γ,5δ] (4ζ-N-méthyl, N-propylamino) (4ζ-désdiméthylamino) pristinamycine IE
- 2 "-carbamoyl-pyrido [2,3-5γ,5δ] (4ζ-N-méthyl, N-(4-pyridylméthyl) amino) (4ζ-désdiméthylamino) pristinamycine IE
- 2"-carbamoyl-pyrido [2,3-5γ,5δ] (4ζ-N-méthyl, N-(3-pyridylméthyl) amino) (4ζ-désdiméthylamino) pristinamycine IE
- 2"-carbamoyl-pyrido [2,3-5γ,5δ] (4ζ-méthyl) (4ζ-désdiméthylamino) pristinamycine IE
- 2"-carbamoyl-pyrido [2,3-5γ,5δ] (4ζ-tert-butyl) (4ζ-désdiméthylamino) pristinamycine IE
- 2"-méthoxy-pyrimido [4,5-5γ,5δ] (4ζ-méthylamino) (4ζ-désdiméthylamino) pristinamycine IE
- 2"-méthoxy-pyrimido [4,5-5γ,5δ] 4ε-chloro pristinamycine IE
- 2"-méthoxy-pyrimido [4,5-5γ,5δ] (4ζ-méthylamino) (4ζ-désdiméthylamino) 4ε-chloro pristinamycine IE
- 2"-(4-pyridyl)-pyrimido [4,5-5γ,5δ] (4ζ-méthylamino) (4ζ-désdiméthylamino) pristinamycine IE
- 2"-(4-pyridyl)-pyrimido[4,5-5γ,5δ] 4ε-chloro pristinamycine IE
- 2"-(4-pyridyl)-pyrimido [4,5-5γ,5δ] (4ζ-méthylamino) (4ζ-désdiméthylamino) 4ε-chloro pristinamycine IE
- 2"- méthylthio -pyrimido [4,5-5γ,5δ] 4ε-chloro pristinamycine IE
- 2"-(3-aminophényl)-pyrimido [4,5-5γ,5δ] (4ζ-méthylamino) (4ζ-désdiméthylamino) pristinamycine IE
- 2"-(3-aminophényl)-pyrimido [4,5-5γ,5δ] 4ε-chloro pristinamycine IE
- 2"-(3-aminophényl)-pyrimido [4,5-5γ,5δ] (4ζ-méthylamino) (4ζ-désdiméthylamino) 4ε-chloro pristinamycine IE
- 2 "-(méthylthio)-pyrimido [4,5-5γ,5δ] (4ζ-méthylamino) (4ζ-désdiméthylamino) 4ε-chloro pristinamycine IE
- 2"-(1-pyrrolidinyl)-pyrimido [4,5-5γ,5δ] 4ε-chloro pristinamycine IE
- 2"-(1-azétidinyl)-pyrimido [4,5-5γ,5δ] (4ζ-méthylamino) (4ζ-désdiméthylamino) pristinamycine IE
- 2"-(1-azétidinyl)-pyrimido [4,5-5γ,5δ] 4ε-chloro pristinamycine IE
- 2"-(1-azétidinyl)-pyrimido [4,5-5γ,5δ] (4ζ-méthylamino) (4ζ-désdiméthylamino) 4ε-chloro pristinamycine IE
- 2"-(3-pyridyl)-pyrimido [4,5-5γ,5δ] pristinamycine IE
- 2"-(3-pyridyl)-pyrimido [4,5-5γ,5δ] (4ζ-méthylamino) (4ζ-désdiméthylamino) pristinamycine IE
- 2"-(3-pyridyl)-pyrimido [4,5-5γ,5δ] 4ε-chloro pristinamycine IE
- 2"-(3-pyridyl)-pyrimido [4,5-5γ,5δ] (4ζ-méthylamino) (4ζ-désdiméthylamino) 4ε-chloro pristinamycine IE
- 2" -(2-pyridyl)-pyrimido [4,5-5γ,5δ] 4ε-chloro pristinamycine IE
- 2"-(1-pyrrolidinyl)-pyrimido [4,5-5γ,5δ] (4ζ-méthylamino) (4ζ-désdiméthylamino) 4ε-chloro pristinamycine IE
- 2"-méthyl-pyrimido [4,5-5γ,5δ] (4ζ-méthylamino) (4ζ-désdiméthylamino) pristinamycine IE
- 2"-méthyl-pyrimido [4,5-5γ,5δ] 4ε-chloro pristinamycine IE
- 2"-méthyl-pyrimido [4,5-5γ,5δ] (4ζ-méthylamino) (4ζ-désdiméthylamino) 4ε-chloro pristinamycine IE
- 2"-(2-pyridyl)-pyrimido [4,5-5γ,5δ] (4ζ-méthylamino) (4ζ-désdiméthylamino) 4ε-chloro pristinamycine IE
- 2"-(2-pyrazinyl)-pyrimido [4,5-5γ,5δ] (4ζ-méthylamino) (4ζ-désdiméthylamino) pristinamycine IE
- 2"-(2-pyrazinyl)-pyrimido [4,5-5γ,5δ] 4ε-chloro pristinamycine IE
- 2"-(2-pyrazinyl)-pyrimido [4,5-5γ,5δ] (4ζ-méthylamino) (4ζ-désdiméthylamino) 4ε-chloro pristinamycine IE
- 2"-(2-morpholinoéthylthio)-pyrimido [4,5-5γ,5δ] (4ζ-méthylamino) (4ζ-désdiméthylamino) pristinamycine IE
- 2"-(2-morpholinoéthylthio)-pyrimido [4,5-5γ,5δ] 4ε-chloro pristinamycine IE
- 2"-(2-morpholinoéthylthio)-pyrimido [4,5-5γ,5δ] (4ζ-méthylamino) (4ζ-désdiméthylamino) 4ε-chloro pristinamycine IE
- 2"-amino-pyrimido [4,5-5γ,5δ] (4ζ-méthylamino) (4ζ-désdiméthylamino) pristinamycine IE
- 2"-amino-pyrimido [4,5-5γ,5δ] 4ε-chloro pristinamycine IE
- 2"-amino-pyrimido [4,5-5γ,5δ] (4ζ-méthylamino) (4ζ-désdiméthylamino) 4ε-chloro pristinamycine IE
- 2"-(1-pyrazol)-pyrimido [4,5-5γ,5δ] (4ζ-méthylamino) (4ζ-désdiméthylamino) pristinamycine IE
- 2"-(1-pyrazol)-pyrimido [4,5-5γ,5δ] 4ε-chloro pristinamycine IE
- 2"-(1-pyrazol)-pyrimido [4,5-5γ,5δ] (4ζ-méthylamino) (4ζ-désdiméthylamino) 4ε-chloro pristinamycine IE
- 2"-(diéthylaminoéthylthio)-pyrimido [4,5-5γ,5δ] (4ζ-méthylamino) (4ζ-désdiméthylamino) pristinamycine IE
- 2"-(diéthylaminoéthylthio)-pyrimido [4,5-5γ,5δ] 4ε-chloro pristinamycine IE
- 2"-(diéthylaminoéthylthio)-pyrimido [4,5-5γ,5δ] (4ζ-méthylamino) (4ζ-désdiméthylamino) 4ε-chloro pristinamycine IE
- 2"-méthylamino-pyrimido [4,5-5γ,5δ] (4ζ-méthylamino) (4ζ-désdiméthylamino) pristinamycine IE
- 2"-méthylamino-pyrimido [4,5-5γ,5δ] (4ε-chloro pristinamycine IE
- 2"-méthylamino-pyrimido [4,5-5γ,5δ] (4ζ-méthylamino) (4ζ-désdiméthylamino) 4ε-chloro pristinamycine IE
- 2"-méthylsulfonyl-pyrimido [4,5-5γ,5δ] (4ζ-méthylamino) (4ζ-désdiméthylamino) pristinamycine IE
- 2"-méthylsulfonyl-pyrimido [4,5-5γ,5δ] 4ε-chloro pristinamycine IE
- 2"-méthylsulfonyl-pyrimido [4,5-5γ,5δ] (4ζ-méthylamino) (4ζ-désdiméthylamino) 4ε-chloro pristinamycine IE
- 2"-(4-aminophényl)-pyrimido [4,5-5γ,5δ] pristinamycine IE
- 2"-(4-aminophényl)-pyrimido [4,5-5γ,5δ] (4ζ-méthylamino) (4ζ-désdiméthylamino) pristinamycine IE
- 2"-(4-aminophényl)-pyrimido [4,5-5γ,5δ] 4ε-chloro pristinamycine IE
- 2"-(4-aminophényl)-pyrimido [4,5-5γ,5δ] (4ζ-méthylamino) (4ζ-désdiméthylamino) 4ε-chloro pristinamycine IE
- 2"-trifluorométhyl-pyrimido [4,5-5γ,5δ] pristinamycine IE
- 2"- trifluorométhyl -pyrimido [4,5-5γ,5δ] (4ζ-méthylamino) (4ζ-désdiméthylamino) pristinamycine IE
- 2"- trifluorométhyl -pyrimido [4,5-5γ,5δ] 4ε-chloro pristinamycine IE
- 2"- trifluorométhyl -pyrimido [4,5-5γ,5δ] (4ζ-méthylamino) (4ζ-désdiméthylamino) 4ε-chloro pristinamycine IE
- 2"-cyclopropyl-pyrimido [4,5-5γ,5δ] pristinamycine IE
- 2"- cyclopropyl -pyrimido [4,5-5γ,5δ] (4ζ-méthylamino) (4ζ-désdiméthylamino) pristinamycine IE
- 2"- cyclopropyl -pyrimido [4,5-5γ,5δ] 4ε-chloro pristinamycine IE
- 2"- cyclopropyl -pyrimido [4,5-5γ,5δ] (4ξ-méthylamino) (4ξ-désdiméthylamino) 4ε-chloro pristinamycine IE
- 2"-morpholinométhyl-pyrimido [4,5-5γ,5δ] pristinamycine IE
- 2"- morpholinométhyl -pyrimido [4,5-5-5γ,5δ] (4ζ-méthylamino) (4ζ-désdiméthylamino) pristinamycine IE
- 2"- morpholinométhyl -pyrimido [4,5-5γ,5δ] 4ε-chloro pristinamycine IE
- 2"- morpholinométhyl -pyrimido [4,5-5γ,5δ] (4ζ-méthylamino) (4ζ-désdiméthylamino) 4ε-chloro pristinamycine IE
- 2"-éthyl-pyrimido [4,5-5γ,5δ] pristinamycine IE
- 2"- éthyl -pyrimido [4,5-5γ,5δ] (4ζ-méthylamino) (4ζ-désdiméthylamino) pristinamycine IE
- 2"- éthyl -pyrimido [4,5-5γ,5δ] 4ε-chloro pristinamycine IE
- 2"-éthyl -pyrimido [4,5-5γ,5δ] (4ζ-méthylamino) (4ζ-désdiméthylamino) 4ε-chloro pristinamycine IE
- 2"-propyl-pyrimido [4,5-5γ,5δ] pristinamycine IE
- 2"- propyl -pyrimido [4,5-5γ,5δ] (4ζ-méthylamino) (4ζ-désdiméthylamino) pristinamycine IE
- 2"-propyl-pyrimido [4,5-5γ,5δ] 4ε-chloro pristinamycine IE
- 2"- propyl-pyrimido [4,5-5γ,5δ] (4ζ-méthylamino) (4ζ-désdiméthylamino) 4ε-chloro pristinamycine IE
- 2"-isopropyl-pyrimido [4,5-5γ,5δ] pristinamycine IE
- 2"- isopropyl -pyrimido [4,5-5γ,5δ] (4ζ-méthylamino) (4ζ-désdiméthylamino) pristinamycine IE
- 2"- isopropyl -pyrimido [4,5-5γ,5δ] 4ε-chloro pristinamycine IE
- 2"- isopropyl -pyrimido [4,5-5γ,5δ] (4ζ-méthylamino) (4ζ-désdiméthylamino) 4ε-chloro pristinamycine IE
- 2"-cyclopropylaminométhyl-pyrimido [4,5-5γ,5δ] pristinamycine IE
- 2"- cyclopropylaminométhyl -pyrimido [4,5-5γ,5δ] (4ζ-méthylamino) (4ζ-désdiméthylamino) pristinamycine IE
- 2"- cyclopropylaminométhyl -pyrimido [4,5-5γ,5δ] 4ε-chloro pristinamycine IE
- 2"- cyclopropylaminométhyl -pyrimido [4,5-5γ,5δ] (4ζ-méthylamino) (4ζ-désdiméthylamino) 4ε-chloro pristinamycine IE
- 4ε-bromo 2"-méthoxy-pyrimido [4,5-5γ,5δ] pristinamycine IE
- 4ε-bromo 2"-méthoxy-pyrimido [4,5-5γ,5δ] (4ζ-méthylamino) (4ζ-désdiméthylamino) pristinamycine IE
- 4ε-allyl 2"-méthoxy-pyrimido [4,5-5γ,5δ] pristinamycine IE
- 4ε-allyl 2"-méthoxy-pyrimido [4,5-5γ,5δ] (4ζ-méthylamino) (4ζ-désdiméthylamino) pristinamycine IE
- 4ε-(2-méthyl prop-ène 1-yl) 2"-méthoxy-pyrimido [4,5-5γ,5δ] pristinamycine IE
- 4ε-(2-méthyl prop-ène 1-yl) 2"-méthoxy-pyrimido [4,5-5γ,5δ] (4ζ-méthylamino) (4ζ-désdiméthylamino) pristinamycine IE
- 2"-méthoxy-pyrimido [4,5-5γ,5δ] (4ζ-diéthylamino) (4ζ-désdiméthylamino) pristinamycine IE
- 2"-méthoxy-pyrimido [4,5-5γ,5δ] (4ζ-N méthyl, N-allylamino) (4ζ-désdiméthylamino) pristinamycine IE
- 2"-méthoxy-pyrimido [4,5-5γ,5δ] (4ζ-N méthyl, N-éthylamino) (4ζ-désdiméthylamino) pristinamycine IE
- 2"-méthoxy-pyrimido [4,5-5γ,5δ] (4ζ-N méthyl, N-propylamino) (4ζ-désdiméthylamino) pristinamycine IE
- 2"-méthoxy-pyrimido [4,5-5γ,5δ] (4ζ-N méthyl, N-(4-pyridylméthyl)amino) (4ζ-désdiméthylamino) pristinamycine IE
- 2"-méthoxy-pyrimido [4,5-5γ,5δ] (4ζ-N méthyl, N-(3-pyridylméthyl)amino) (4ζ-désdiméthylamino) pristinamycine IE
- 2"-méthoxy-pyrimido [4,5-5γ,5δ] (4ζ-méthyl) (4ζ-désdiméthylamino) pristinamycine IE
- 2"-méthoxy-pyrimido [4,5-5γ,5δ] (4ζ-tert-butyl) (4ζ-désdiméthylamino) pristinamycine IE
- 4ε-bromo 2"-(4-pyridyl)-pyrimido [4,5-5γ,5δ] pristinamycine IE
- 4ε-bromo 2"-(4-pyridyl)-pyrimido [4,5-5γ,5δ] (4ζ-méthylamino) (4ζ-désdiméthylamino) pristinamycine IE
- 4ε-allyl 2"-(4-pyridyl)-pyrimido [4,5-5γ,5δ] pristinamycine IE
- 4ε-allyl 2"-(4-pyridyl)-pyrimido [4,5-5γ,5δ] (4ζ-méthylamino) (4ζ-désdiméthylamino) pristinamycine IE
- 4ε-(2-méthyl prop-ène 1-yl) 2"-(4-pyridyl)-pyrimido [4,5-5γ,5δ] pristinamycine IE
- 4ε-(2-méthyl prop-ène 1-yl) 2"-(4-pyridyl)-pyrimido [4,5-5γ,5δ] (4ζ-méthylamino) (4ζ-désdiméthylamino) pristinamycine IE
- 2"-(4-pyridyl)-pyrimido [4,5-5γ,5δ] (4ζ-diéthylamino) (4ζ-désdiméthylamino) pristinamycine IE
- 2"-(4-pyridyl)-pyrimido [4,5-5γ,5δ] (4ζ-N méthyl, N-allylamino) (4ζ-désdiméthylamino) pristinamycine IE
- 2"-(4-pyridyl)-pyrimido [4,5-5γ,5δ] (4ζ-N méthyl, N-éthylamino) (4ζ-désdiméthylamino) pristinamycine IE
- 2"-(4-pyridyl)-pyrimido [4,5-5γ,5δ] (4ζ-N méthyl, N-propylamino) (4ζ-désdiméthylamino) pristinamycine IE
- 2"-(4-pyridyl)-pyrimido [4,5-5γ,5δ] (4ζ-N méthyl, N-(4-pyridylméthyl)amino) (4ζ-désdiméthylamino) pristinamycine IE
- 2"-(4-pyridyl)-pyrimido [4,5-5γ,5δ] (4ζ-N méthyl, N-(3-pyridylméthyl)amino) (4ζ-désdiméthylamino) pristinamycine IE
- 2"-(4-pyridyl)-pyrimido [4,5-5γ,5δ] (4ζ-méthyl) (4ζ-désdiméthylamino) pristinamycine IE
- 2"-(4-pyridyl)-pyrimido [4,5-5γ,5δ] (4ζ-tert-butyl) (4ζ-désdiméthylamino) pristinamycine IE
- 4ε-bromo 2"-méthylthio-pyrimido [4,5-5γ,5δ] pristinamycine IE
- 4ε-bromo 2"-méthylthio-pyrimido [4,5-5γ,5δ] (4ζ-méthylamino) (4ζ-désdiméthylamino) pristinamycine IE
- 4ε-allyl 2"-méthylthio-pyrimido [4,5-5γ,5δ] pristinamycine IE
- 4ε-allyl 2"-méthylthio-pyrimido [4,5-5γ,5δ] (4ζ-méthylamino) (4ζ-désdiméthylamino) pristinamycine IE
- 4ε-(2-méthyl prop-ène 1-yl) 2"-méthylthio-pyrimido [4,5-5γ,5δ] pristinamycine IE
- 4ε-(2-méthyl prop-ène 1-yl) 2"-méthylthio-pyrimido [4,5-5γ,5δ] (4ζ-méthylamino) (4ζ-désdiméthylamino) pristinamycine IE
- 2"-méthylthio-pyrimido [*4,5-5γ,5δ] (4ζ-diéthylamino) (4ζ-désdiméthylamino) pristinamycine IE
- 2"-méthylthio-pyrimido [4,5-5γ,5δ] (4ζ-N méthyl, N-allylamino) (4ζ-désdiméthylamino) pristinamycine IE
- 2"-méthylthio-pyrimido [4,5-5γ,5δ] (4ζ-N méthyl, N-éthylamino) (4ζ-désdiméthylamino) pristinamycine IE
- 2"-méthylthio-pyrimido [4,5-5γ,5δ] (4ζ-N méthyl, N-propylamino) (4ζ-désdiméthylamino) pristinamycine IE
- 2"-méthylthio-pyrimido [4,5-5γ,5δ] (4ζ-N méthyl, N-(4-pyridylméthyl)amino) (4ζ-désdiméthylamino) pristinamycine IE
- 2"-méthylthio-pyrimido [4,5-5γ,5δ (4ζ-N méthyl, N-(3-pyridylméthyl)amino) (4ζ-désdiméthylamino) pristinamycine IE
- 2"-méthylthio-pyrimido [4,5-5γ,5δ] (4ζ-méthyl) (4ζ-désdiméthylamino) pristinamycine IE
- 2"-méthylthio-pyrimido [4,5-5γ,5δ] (4ζ-tert-butyl) (4ζ-désdiméthylamino) pristinamycine IE
- 4ε-bromo 2"-(3-aminophényl)-pyrimido [4,5-5γ,5δ] pristinamycine IE
- 4ε-bromo 2"-(3-aminophényl)-pyrimido [4,5-5γ,5δ] (4ζ-méthylamino) (4ζ-désdiméthylamino) pristinamycine IE
- 4ε-allyl 2"-(3-aminophényl)-pyrimido [4,5-5γ,5δ] pristinamycine IE
- 4ε-allyl 2"-(3-aminophényl)-pyrimido [4,5-5γ,5δ] (4ζ-méthylamino) (4ζ-désdiméthylamino) pristinamycine IE
- 4ε-(2-méthyl prop-ène 1-yl) 2"-(3-aminophényl)-pyrimido [4,5-5γ,5δ] pristinamycine IE
- 4ε-(2-méthyl prop-ène 1-yl) 2"-(3-aminophényl)-pyrimido [4,5-5γ,5δ] (4ζ-méthylamino) (4ζ-désdiméthylamino) pristinamycine IE
- 2"-(3-aminophényl)-pyrimido [4,5-5γ,5δ] (4ζ-diéthylamino) (4ζ-désdiméthylamino) pristinamycine IE
- 2"-(3-aminophényl)-pyrimido [4,5-5γ,5δ] (4ζ-N méthyl, N-allylamino) (4ζ-désdiméthylamino) pristinamycine IE
- 2"-(3-aminophényl)-pyrimido [4,5-5γ,5δ] (4ζ-N méthyl, N-éthylamino) (4ζ-désdiméthylamino) pristinamycine IE
- 2"-(3-aminophényl)-pyrimido [4,5-5γ,5δ] (4ζ-N méthyl, N-propylamino) (4ζ-désdiméthylamino) pristinamycine IE
- 2"-(3-aminophényl)-pyrimido [4,5-5γ,5δ] (4ζ-N méthyl, N-(4-pyridylméthyl)amino) (4ζ-désdiméthylamino) pristinamycine IE
- 2"-(3-aminophényl)-pyrimido [4,5-5γ,5δ] (4ζ-N méthyl, N-(3-pyridylméthyl)amino) (4ζ-desdimethylamino) pristinamycine IE
- 2"-(3-aminophényl)-pyrimido [4,5-5γ,5δ] (4ζ-méthyl) (4ζ-désdiméthylamino) pristinamycine IE
- 2"-(3-aminophényl)-pyrimido [4,5-5γ,5δ] (4ζ-tert-butyl) (4ζ-désdiméthylamino) pristinamycine IE
- 4ε-bromo 2"-(1-pyrrolidinyl)-pyrimido [4,5-5γ,5δ] (4ζ-méthylamino) (4ζ-désdiméthylamino) pristinamycine IE
- 4ε-allyl 2"-(1-pyrrolidinyl)-pyrimido [4,5-5γ,5δ] pristinamycine IE
- 4ε-allyl 2"-(1-pyrrolidinyl)-pyrimido [4,5-5γ,5δ] (4ζ-méthylamino) (4ζ-désdiméthylamino) pristinamycine IE
- 4ε-(2-méthyl prop-ène 1-yl) 2"-(1-pyrrolidinyl)pyrimido [4,5-5γ,5δ] pristinamycine IE
- 4ε-(2-méthyl prop-ène 1-yl) 2"-(1-pyrrolidinyl)-pyrimido [4,5-5γ,5δ] (4ζ-méthylamino) (4ζ-désdiméthylamino) pristinamycine IE
- 2"-(1-pyrrolidinyl)-pyrimido [4,5-5γ,5δ] (4ζ-diéthylamino) (4ζ-désdiméthylamino) pristinamycine IE
- 2"-(1-pyrrolidinyl)-pyrimido [4,5-5γ,5δ] (4ζ-N méthyl, N-allylamino) (4ζ-désdiméthylamino) pristinamycine IE
- 2"-(1-pyrrolidinyl)-pyrimido [4,5-5γ,5δ] (4ζ-N méthyl, N-éthylamino) (4ζ-désdiméthylamino) pristinamycine IE
- 2"-(1-pyrrolidinyl)-pyrimido [4,5-5γ,5δ] (4ζ-N méthyl, N-propylamino) (4ζ-désdiméthylamino) pristinamycine IE
- 2"-(1-pyrrolidinyl)-pyrimido [4,5-5γ,5δ] (4ζ-N méthyl, N-(4-pyridylméthyl)amino) (4ζ-désdiméthylamino) pristinamycine IE
- 2"-(1-pyrrolidinyl)-pyrimido [4,5-5y,5δ] (4ζ-N méthyl, N-(3-pyridylméthyl)amino) (4ζ-désdiméthylamino) pristinamycine IE
- 2"-(1-pyrrolidinyl)-pyrimido [4,5-5γ,5δ] (4ζ-méthyl) (4ζ-désdiméthylamino) pristinamycine IE
- 2"-(1-pyrrolidinyl)-pyrimido [4,5-5γ,5δ] (4ζ-tert-butyl) (4ζ-désdiméthylamino) pristinamycine IE
- 4ε-bromo 2"-(1-azétidinyl)-pyrimido [4,5-5γ,5δ] pristinamycine IE
- 4ε-bromo 2"-(1-azétidinyl)-pyrimido [4,5-5γ,5δ] (4ζ-raéthylamino) (4ζ-désdiméthylamino) pristinamycine IE
- 4ε-allyl 2''-(1-azétidinyl)-pyrimido [4,5-5γ,5δ] pristinamycine IE
- 4ε-allyl 2"-(1-azétidinyl)-pyrimido [4,5-5γ,5δ] (4ζ-méthylamino) (4ζ-désdiméthylamino) pristinamycine IE
- 4ε-(2-méthyl prop-ène 1-yl) 2"-(1-azétidinyl)-pyrimido [4,5-5γ,5δ] pristinamycine IE
- 4ε-(2-méthyl prop-ène 1-yl) 2"-(1-azétidinyl)-pyrimido [4,5-5γ,5δ] (4ζ-méthylamino) (4ζ-désdiméthylamino) pristinamycine IE
- 2"-(1-azétidinyl)-pyrimido [4,5-5γ,5δ] (4ζ-diéthylamino) (4ζ-désdiméthylamino) pristinamycine IE
- 2"-(1-azétidinyl)-pyrimido [4,5-5γ,5δ] (4ζ-N méthyl, N-allylamino) (4ζ-désdiméthylamino) pristinamycine IE
- 2"-(1-azétidinyl)-pyrimido [4,5-5γ,5δ] (4ζ-N méthyl, N-éthylamino) (4ζ-désdiméthylamino) pristinamycine IE
- 2''-(1-azétidinyi)-pyrimido [4,5-5γ,5δ] (4ζ-N méthyl, N-propylamino) (4ζ-désdiméthylamino) pristinamycine IE
- 2"-(1-azétidinyl)-pyrimido [4,5-5γ,5δ] (4ζ-N méthyl, N-(4-pyridylméthyl)amino) (4ξ-désdiméthylamino) pristinamycine IE
- 2"-(1-azétidinyl)-pyrimido [4,5-5γ,5δ] (4ζ-N méthyl, N-(3-pyridylméthyl)amino) (4ζ-désdiméthylamino) pristinamycine IE
- 2"-(1-azétidinyl)-pyrimido [4,5γ,5δ] (4ζ-méthyl) (4ζ-désdiméthylamino) pristinamycine IE
- 2"-(1-azétidinyl)-pyrimido [4,5-5γ,5δ] (4ζ-tert-butyl) (4ζ-désdiméthylamino) pristinamycine IE
- 4ε-bromo 2"-(3-pyridyl)-pyrimido [4,5-5γ,5δ] pristinamycine IE
- 4ε-bromo 2"-(3-pyridyl)-pyrimido [4,5-5γ,5δ] (4ζ-méthylamino) (4ζ-désdiméthylamino) pristinamycine IE
- 4ε-ally] 2"-(3-pyridyl)-pyrimido [4,5-5γ,5δ] pristinamycine IE
- 4ε-allyl 2"-(3-pyridyl)-pyrimido (4,5-5γ,5δ] (4ξ-méthylamino) (4ζ-désdiméthylamino) pristinamycine IE
- 4ε-(2-méthyl prop-éne 1-yl) 2"-(3-pyridyl)-pyrimido [4,5-5γ,5δ] pristinamycine IE
- 4ε-(2-méthyl prop-ène 1-yl) 2"-(3-pyridyl)-pyrimido [4,5-5γ,5δ] (4ζ-méthylamino) (4ζ-désdiméthylamino) pristinamycine IE
- 2"-(3-pyridyl)-pyrimido [4,5-5δ,5δ] (4ζ-diéthylamino) (4ζ-désdiméthylamino) pristinamycine IE
- 2"-(3-pyridyl)-pyrimido [4,5-5γ,5δ] (4ζ-N méthyl, N-allylamino) (4ζ-désdiméthylamino) pristinamycine IE
- 2"-(3-pyridyl)-pyrimido [4,5-5γ,5δ] (4ζ-N méthyl, N-éthylamino) (4ζ-désdiméthylamino) pristinamycine IE
- 2"-(3-pyridyl)-pyrimido [4,5-5γ,5δ] (4ζ-N méthyl, N-propylamino) (4ζ-désdiméthylamino) pristinamycine IE
- 2"-(3-pyridyl)-pyrimido [4,5-5γ,5δ] (4ζ-N méthyl, N-(4-pyridylméthyl)amino) (4ζ-désdiméthylamino) pristinamycine IE
- 2"-(3-pyridyl)-pyrimido [4,5-5γ,5δ] (4ζ-N méthyl, N-(3-pyridylméthyl)amino) (4ζ-désdiméthylamino) pristinamycine IE
- 2"-(3-pyridyl)-pyrimido [4,5-5γ,5δ] (4ζ-méthyl) (4ζ-désdiméthylamino) pristinamycine IE
- 2"-(3-pyridyl)-pyrimido [4,5-5γ,5δ] (4ζ-tert-butyl) (4ζ-désdiméthylamino) pristinamycine IE
- 4ε-bromo 2"-(2-pyridyl)pyrimido (4,5-5γ,5δ] pristinamycine IE
- 4ε-bromo 2"-(2-pyridyl)-pyrimido [4,5-5γ,5δ] (4ζ-méthylamino) (4ζ-désdiméthylamino) pristinamycine IE
- 4ε-allyl 2"-(2-pyridyl)-pyrimido [4,5-5γ,5δ] pristinamycine IE
- 4ε-allyl 2"-(2-pyridyl)-pyrimido [4,5-5γ,5δ] (4ζ-méthylamino) (4ζ-désdiméthylamino) pristinamycine IE
- 4ε-(2-méthyl prop-ène 1-yl) 2"-(2-pyridyl)-pyrimido [4,5-5γ,5δ] pristinamycine IE
- 4ε-(2-méthyl prop-ène 1-yl) 2"-(2-pyridyl)-pyrimido [4,5-5γ,5δ] (4ζ-méthylamino) (4ζ-désdiméthylamino) pristinamycine IE
- 2"-(2-pyridyl)-pyrimido [4,5-5γ,5δ] (4ζ-diéthylamino) (4ζ-désdiméthylamino) pristinamycine IE
- 2"-(2-pyridyl)-pyrimido [4,5-5γ,5δ] (4ζ-N méthyl, N-allylamino) (4ζ-désdiméthylamino) pristinamycine IE
- 2"-(2-pyridyl)-pyrimido [4,5-5γ,5δ] (4ζ-N méthyl, N-éthylamino) (4ζ-désdiméthylamino) pristinamycine IE
- 2"-(2-pyridyl)-pyrimido [4,5-5γ,5δ] (4Ç-N méthyl, N-propylamino) (4ζ-désdiméthylamino) pristinamycine IE
- 2"-(2-pyridyl)-pyrimido [4,5-5γ,5δ] (4ζ-N méthyl, N-(4-pyridylméthyl)amino) (4ζ-désdiméthylamino) pristinamycine IE
- 2"-(2-pyridyl)-pyrimido [4,5-5γ,5δ] (4ζ-N méthyl, N-(3-pyridylméthyl)amino) (4ζ-désdiméthylamino) pristinamycine IE
- 2"-(2-pyridyl)-pyrimido [4,5-5γ,5δ] (4ζ-méthyl) (4ζ-désdiméthylamino) pristinamycine IE
- 2"-(2-pyridyl)-pyrimido [4,5-5γ,5δ] (4ζ-tert-butyl) (4ζ-désdiméthylamino) pristinamycine IE
- 4ε-bromo 2"-méthyl-pyrimido [4,5-5γ,5δ] pristinamycine IE
- 4ε-bromo 2"-méthyl-pyrimido [4,5-5γ,5δ] (4ζ-méthylamino) (4ζ-désdiméthylamino) pristinamycine IE
- 4ε-allyl 2"-méthyl-pyrimido (4,5-5γ,5δ] pristinamycine IE
- 4ε-allyl 2"-méthyl-pyrimido [4,5-5γ,5δ] (4ζ-méthylamino) (4ζ-désdiméthylamino) pristinamycine IE
- 4ε-(2-méthyl prop-ène 1-yl) 2"-méthyl-pyrimido [4,5-5γ,5δ] pristinamycine IE
- 4ε-(2-méthyl prop-ène 1-yl) 2"-méthyl-pyrimido [4,5-5γ,5δ] (4ζ-méthylamino) (4ζ-désdiméthylamino) pristinamycine IE
- 2"-méthyl-pyrimido [4,5-5γ,5δ] (4ζ-diéthylamino) (4ζ-désdiméthylamino) pristinamycine IE
- 2"-méthyl-pyrimido [4,5-5γ,5δ] (4ζ-N méthyl, N-allylamino) (4ζ-désdiméthylamino) pristinamycine IE
- 2"-méthyl-pyrimido [4,5-5γ,5δ] (4ζ-N méthyl, N-éthylamino) (4ζ-désdiméthylamino) pristinamycine IE
- 2"-méthyl-pyrimido [4,5-5γ,5δ] (4ζ-N méthyl, N-propylamino) (4ζ-désdiméthylamino) pristinamycine IE
- 2"-méthyl-pyrimido [4,5-5γ,5δ] (4ζ-N méthyl, N-(4-pyridylméthyl)amino) (4ζ-désdiméthylamino) pristinamycine IE
- 2"-méthyl-pyrimido [4,5-5γ,5δ] (4ζ-N méthyl, N-(3-pyridylméthyl)amino) (4ξ-désdiméthylamino) pristinamycine IE
- 2"-méthyl-pyrimido [4,5-5γ,50] (4ζ-méthyl) (4ζ-désdiméthylamino) pristinamycine IE
- 2"-méthyl-pyrimido [4,5-5γ,5δ] (4ζ-tert-butyl) (4ζ-désdiméthylamino) pristinamycine IE
- 4ε-bromo 2"-(2-pyrazinyl)-pyrimido [4,5-5γ,5δ] pristinamycine IE
- 4ε-bromo 2"-(2-pyrazinyl)-pyrimido [4,5-5γ,5δ] (4ζ-méthylamino) (4ζ-désdiméthylamino) pristinamycine IE
- 4ε-allyl 2"-(2-pyrazinyl)-pyrimido [4,5-5γ,5δ] pristinamycine IE
- 4ε-allyl 2"-(2-pyrazinyl)-pyrimido [4,5-5γ,5δ] (4ζ-méthylamino) (4ζ-désdiméthylamino) pristinamycine IE
- 4ε-(2-méthyl prop-ène 1-yl) 2"-(2-pyrazinyl)-pyrimido [4,5-5γ,5δ] pristinamycine IE
- 4ε-(2-méthyl prop-ène 1-yl) 2"-(2-pyrazinyl)-pyrimido [4,5-5γ,5δ] (4ζ-méthylamino) (4ζ-désdiméthylamino) pristinamycine IE
- 2"-(2-pyrazinyl)-pyrimido [4,5-5γ,5δ] (4ζ-diéthylamino) (4ζ-désdiméthylamino) pristinamycine IE
- 2"-(2-pyrazinyl)-pyrimido [4,5-5γ,5δ] (4ζ-N méthyl, N-allylamino) (4ζ-désdiméthylamino) pristinamycine IE
- 2"-(2-pyrazinyl)-pyrimido [4,5-5γ,5δ] (4ζ-N méthyl, N-éthylamino) (4ζ-désdiméthylamino) pristinamycine IE
- 2"-(2-pyrazinyl)-pyrimido [4,5-5γ,5δ] (4Ç-N méthyl, N-propylamino) (4ζ-désdiméthylamino) pristinamycine IE
- 2"-(2-pyrazinyl)-pyrimido [4,5-5γ,5δ] (4ζ-N méthyl, N-(4-pyridylméthyl)amino) (4ζ-désdiméthylamino) pristinamycine IE
- 2"-(2-pyrazinyl)-pyrimido [4,5-5γ,5δ] (4ζ-N méthyl, N-(3-pyridylméthyl)amino) (4ζ-désdiméthylamino) pristinamycine IE
- 2"-(2-pyrazinyl)-pyrimido [4,5-5γ,5δ] (4ζ-méthyl) (4ζ-désdiméthylamino) pristinamycine IE
- 2"-(2-pyrazinyl)-pyrimido [4,5-5γ,5δ] (4ζ-tert-butyl) (4ζ-désdiméthylamino) pristinamycine IE
- 4ε-bromo 2"-(2-morpholinoéthylthio)-pyrimido [4,5-5γ,5δ] pristinamycine IE
- 4ε-bromo 2"-(2-morpholinoéthylthio)-pyrimido [4,5-5y,58] (4ζ-méthylamino) (4ζ-désdiméthylamino) pristinamycine IE
- 4ε-allyl 2"-(2-morpholinoéthylthio)-pyrimido [4,5-5γ,5δ] pristinamycine IE
- 4ε-allyl 2"-(2-morpholinoéthylthio)-pyrimido [4,5-5γ,5δ] (4ζ-méthylamino) (4ζ-désdiméthylamino) pristinamycine IE
- 4ε-(2-méthyl prop-ène 1-yl) 2"-(2-morpholinoéthylthio)-pyrimido [4,5-5γ,5δ] pristinamycine IE
- 4ε-(2-méthyl prop-ène 1-yl) 2"-(2-morpholinoéthylthio)-pyrimido [4,5-5γ,5δ] (4ζ-méthylamino) (4ζ-désdiméthylamino) pristinamycine IE
- 2 "-(2-morpholinoéthylthio)-pyrimido [4,5-5γ,5δ] (4ζ-diéthylamino) (4ζ-désdiméthylamino) pristinamycine IE
- 2"-(2-morpholinoéthylthio)-pyrimido [4,5-5γ,5δ] (4ζ-N méthyl, N-allylamino) (4ζ-désdiméthylamino) pristinamycine IE
- 2"-(2-morpholinoéthylthio)-pyrimido [4,5-5γ,5δ] (4ζ-N méthyl, N-éthylamino) (4ζ-désdiméthylamino) pristinamycine IE
- 2"-(2-morpholinoéthylthio)-pyrimido [4,5-5γ,5δ] (4ζ-N méthyl, N-propylamino) (4ζ-désdiméthylamino) pristinamycine IE
- 2"-(2-morpholinoéthyithio)-pyrimido [4,5-5γ,5δ] (4ζ-N méthyl, N-(4-pyridylméthyl)amino) (4ζ-désdiméthylamino) pristinamycine IE
- 2"-(2-morpholinoéthylthio)-pyrimido [4,5-5γ,5δ] (4ζ-N méthyl, N-(3-pyridylméthyl)amino) (4ζ-désdiméthylamino) pristinamycine IE
- 2"-(2-morpholinoéthylthio)-pyrimido [4,5-5γ,5δ] (4ζ-méthyl) (4ζ-désdiméthylamino) pristinamycine IE
- 2"-(2-morpholinoéthylthio)-pyrimido [4,5-5γ,5δ] (4ζ-tert-butyl) (4ζ-désdiméthylamino) pristinamycine IE
- 4ε-bromo 2"-amino-pyrimido [4,5-5γ,5δ] pristinamycine IE
- 4ε-bromo 2"-amino-pyrimido [4,5-5γ,5δ] (4ζ-méthylamino) (4ζ-désdiméthylamino) pristinamycine IE
- 4ε-allyl 2"-amino-pyrimido [4,5-5γ,5δ] pristinamycine IE
- 4ε-allyl 2"-amino-pyrimido [4,5-5γ,5δ] (4ζ-méthylamino) (4ζ-désdiméthylamino) pristinamycine IE
- 4ε-(2-méthyl prop-ène 1-yl) 2"-amino-pyrimido [4,5-5γ,5δ] pristinamycine IE
- 4ε-(2-méthyl prop-ène 1-yl) 2"-amino-pyrimido [4,5-5γ,5δ] (4ζ-méthylamino) (4ζ-désdiméthylamino) pristinamycine IE
- 2 "-amino-pyrimido [4,5-5γ,5δ] (4ζ-diéthylamino) (4ζ-désdiméthylamino) pristinamycine IE
- 2 '-amino-pyrimido [4,5-5γ,5δ] (4ζ-N méthyl, N-allylamino) (4ζ-désdiméthylamino) pristinamycine IE
- 2"-amino-pyrimido [4,5-5γ,5δ] (4ζ-N méthyl, N-éthylamino) (4ζ-désdiméthylamino) pristinamycine IE
- 2"-amino-pyrimido [4,5-5γ,5δ] (4ζ-N méthyl, N-propylamino) (4ζ-désdiméthylamino) pristinamycine IE
- 2"-amino-pyrimido [4,5-5γ,5δ] (4ζ-N méthyl, N-(4-pyridylméthyl)amino) (4ζ-désdiméthylamino) pristinamycine IE
- 2"-amino-pyrimido [4,5-5γ,5δ] (4ζ-N méthyl, N-(3-pyridylméthyl)amino) (4ζ-désdiméthylamino) pristinamycine IE
- 2 "-amino-pyrimido [4,5-5γ,5δ] (4ζ-méthyl) (4ζ-désdiméthylamino) pristinamycine IE
- 2"-amino-pyrimido [4,5-5γ,5δ] (4ζ-tert-butyl) (4ζ-désdiméthylamino) pristinamycine IE
- 4ε-bromo 2"-(1-pyrazolyl)-pyrimido [4,5-5γ,5δ] pristinamycine IE
- 4ε-bromo 2"-(1-pyrazolyl)-pyrimido [4,5-5γ,5δ] (4ζ-méthylamino) (4ζ-désdiméthylamino) pristinamycine IE
- 4ε-allyl 2"-(1-pyrazolyl)-pyrimido [4,5-5γ,5δ] pristinamycine IE
- 4ε-allyl 2"-(1-pyrazolyl)-pyrimido [4,5-5γ,5δ] (4ζ-méthylamino) (4ζ-désdiméthylamino) pristinamycine IE
- 4ε-(2-méthyl prop-ène 1-yl) 2"-(1-pyrazolyl)-pyrimido [4,5-5γ,5δ] pristinamycine IE
- 4ε-(2-méthyl prop-ène 1-yl) 2"-(1-pyrazolyl)-pyrimido [4,5-5γ,5δ] (4ζ-méthylamino) (4ζ-désdiméthylamino) pristinamycine IE
- 2"-(1-pyrazolyl)-pyrimido [4,5-5γ,5δ] (4ζ-diéthylamino) (4ζ-désdiméthylamino) pristinamycine IE
- 2"-(1-pyrazolyl)-pyrimido [4,5-5γ,5δ] (4ζ-N méthyl, N-allylamino) (4ζ-désdiméthylamino) pristinamycine IE
- 2"-(1-pyrazolyl)-pyrimido [4,5-5γ,5δ] (4ζ-N méthyl, N-éthylamino) (4ζ-désdiméthylamino) pristinamycine IE
- 2''-(1-pyrazolyl)-pyrimido [4,5-5γ,5δ] (4ζ-N méthyl, N-propylamino) (4ζ-désdiméthylamino) pristinamycine IE
- 2"-(1-pyrazolyl)-pyrimido [4,5-5γ,5δ] (4ζ-N méthyl, N-(4-pyridylméthyl)amino) (4ζ-désdiméthylamino) pristinamycine IE
- 2''-(1-pyrazolyl)-pyrimido [4,5-5γ,5δ] (4ζ-N méthyl, N-(3-pyridylméthyl)amino) (4ζ-désdiméthylamino) pristinamycine IE
- 2"-(1-pyrazolyl)-pyrimido [4,5-5γ,5δ] (4ζ-méthyl) (4ζ-désdiméthylamino) pristinamycine IE
- 2"-(1-pyrazolyl)-pyrimido [4,5-5γ,5δ] (4ζ-tert-butyl) (4ζ-désdiméthylamino) pristinamycine IE
- 4ε-bromo 2"-(diéthylaminoéthylthio)-pyrimido [4,5-5γ,5δ] pristinamycine IE
- 4ε-bromo 2"-(diéthylaminoéthylthio)-pyrimido [4,5-5γ,5δ] (4ζ-méthylamino) (4ζ-désdiméthylamino) pristinamycine IE
- 4ε-allyl 2"-(diéthylaminoéthylthio)-pyrimido [4,5-5γ,5δ] pristinamycine IE
- 4ε-allyl 2"-(diéthylaminoéthylthio)-pyrimido [4,5-5γ,5δ] (4ζ-méthylamino) (4ζ-désdiméthylamino) pristinamycine IE
- 4ε-(2-méthyl prop-ène 1-yl) 2"-(diéthylaminoéthylthio)-pyrimido [4,5-5γ,5δ] pristinamycine IE
- 4ε-(2-méthyl prop-ène 1-yl) 2"-(diéthylaminoéthylthio)-pyrimido [4,5-5γ,5δ] (4ζ-méthylamino) (4ζ-désdiméthylamino) pristinamycine IE
- 2"-(1-pyrazolyl)-pyrimido [4,5-5γ,5δ] (4ζ-diéthylamino) (4ζ-désdiméthylamino) pristinamycine IE
- 2"-(diéthylaminoéthylthio)-pyrimido [4,5-5γ,5δ] (4ζ-N méthyl, N-allylamino) (4ζ-désdiméthylamino) pristinamycine IE
- 2"-(diéthylaminoéthylthio)-pyrimido [4,5-5γ,5δ] (4ζ-N méthyl, N-éthylamino) (4ζ-désdiméthylamino) pristinamycine IE
- 2"-(diéthylaminoéthylthio)-pyrimido [4,5-5γ,5δ] (4ζ-N méthyl, N-propylamino) (4ζ-désdiméthylamino) pristinamycine IE
- 2"-(diéthylaminoéthylthio)-pyrimido [4,5-5γ,5δ] (4ζ-N méthyl, N-(4-pyridylméthyl)amino) (4ζ-désdiméthylamino) pristinamycine IE
- 2"-(diéthylaminoéthylthio)-pyrimido [4,5-5γ,5δ] (4ζ-N méthyl, N-(3-pyridylméthyl)amino) (4ζ-désdiméthylamino) pristinamycine IE
- 2"-(diéthylaminoéthylthio)-pyrimido [4,5-5γ,5δ] (4ζ-méthyl) (4ζ-désdiméthylamino) pristinamycine IE
- 2"-(diéthylaminoéthylthio)-pyrimido [4,5-5γ,5δ] (4ζ-tert-butyl) (4Ç-désdiméthylamino) pristinamycine IE
- 4ε-bromo 2"-méthylamino-pyrimido [4,5-5γ,5δ] pristinamycine IE
- 4ε-bromo 2"-méthylamino-pyrimido [4,5-5γ,5δ] (4ζ-méthylamino) (4ζ-désdiméthylamino) pristinamycine IE
- 4ε-allyl 2"-méthylamino-pyrimido [4,5-5γ,5δ] pristinamycine IE
- 4ε-allyl 2"-méthylamino-pyrimido [4,5-5γ,5δ] (4ζ-méthylamino) (4ζ-désdiméthylamino) pristinamycine IE
- 4ε-(2-méthyl prop-ène 1-yl) 2"-méthylamino-pyrimido [4,5-5γ,5δ] pristinamycine IE
- 4ε-(2-méthyl prop-ène 1-yl) 2"-méthylamino-pyrimido [4,5-5γ,5δ] (4ζ-méthylamino) (4ζ-désdiméthylamino) pristinamycine IE
- 2"-méthylamino-pyrimido [4,5-5γ,5δ] (4ζ-diéthylamino) (4ζ-désdiméthylamino) pristinamycine IE
- 2"-méthylamino-pyrimido [4,5-5γ,5δ] (4ζ-N méthyl, N-allylamino) (4ζ-désdiméthylamino) pristinamycine IE
- 2"-méthylamino-pyrimido [4,5-5γ,5δ] (4ζ-N méthyl, N-éthylamino) (4Ç-désdiméthylamino) pristinamycine IE
- 2"-méthylamino-pyrimido [4,5-5γ,5δ] (4ζ-N méthyl, N-propylamino) (4ζ-désdiméthylamino) pristinamycine IE
- 2"-méthylamino-pyrimido [4,5-5γ,5δ] (4ζ-N méthyl, N-(4pyridylméthyl)amino) (4ζ-désdiméthylamino) pristinamycine IE
- 2"-méthylamino-pyrimido [4,5-5γ,5δ] (4ζ-N méthyl, N-(3-pyridylméthyl)amino) (4ζ-désdiméthylamino) pristinamycine IE
- 2"-méthylamino-pyrimido [4,5-5γ,5δ] (4ζ-méthyl) (4ζ-désdiméthylamino) pristinamycine IE
- 2"-méthylamino-pyrimido [4,5-5γ,5δ] (4ζ-tert-butyl) (4ζ-désdiméthylamino) pristinamycine IE
- 4ε-bromo 2"-méthylsulfonyl-pyrimido [4,5-5γ,5δ] pristinamycine IE
- 4ε-bromo 2"-méthylsulfonyl-pyrimido [4,5-5γ,5δ] (4ζ-méthylamino) (4ζ-désdiméthylamino) pristinamycine IE
- 4ε-allyl 2"-méthylsulfonyl-pyrimido [4,5-5γ,5δ] pristinamycine IE
- 4ε-allyl 2"-méthylsulfonyl-pyrimido [4,5-5γ,5δ] (4ζ-méthylamino) (4ζ-désdiméthylamino) pristinamycine IE
- 4ε-(2-méthyl prop-ène 1-yl) 2" -méthylsulfonyl-pyrimido [4,5-5γ,5δ] pristinamycine IE
- 4ε-(2-méthyl prop-ène 1-yl) 2"-méthylsulfonyl-pyrimido [4,5-5γ,5δ] (4ζ-méthylamino) (4ζ-désdiméthylamino) pristinamycine IE
- 2"-méthylsulfonyl-pyrimido [4,5-5γ,5δ] (4ζ-diéthylamino) (4ζ-désdiméthylamino) pristinamycine IE
- 2"-méthylsulfonyl-pyrimido [4,5-5γ,5δ] (4ζ-N méthyl, N-allylamino) (4ζ-désdiméthylamino) pristinamycine IE
- 2"-méthylsulfonyl-pyrimido [4,5-5γ,5δ] (4ζ-N méthyl, N-éthylamino) (4ζ-désdiméthylamino) pristinamycine IE
- 2"-méthylsulfonyl-pyrimido [4,5-5γ,5δ] (4ζ-N méthyl, N-propylamino) (4ζ-désdiméthylamino) pristinamycine IE
- 2"-méthylsulfonyl-pyrimido [4,5-5γ,5δ] (4ζ-N méthyl, N-(4-pyridylméthyl)amino) (4ζ -désdiméthylamino) pristinamycine IE
- 2"-méthylsulfonyl-pyrimido [4,5-5γ,5δ] (4ζ-N méthyl, N-(3-pyridylméthyl)amino) (4ζ-désdiméthylamino) pristinamycine IE
- 2"-méthylsulfonyl-pyrimido [4,5-5γ,5δ] (4ζ-méthyl) (4ζ-désdiméthylamino) pristinamycine IE
- 2"-méthylsulfonyl-pyrimido [4,5-5γ,5δ] (4ζ,-tert-butyl) (4ζ-désdiméthylamino) pristinamycine IE
- 4ε-bromo 2"-(4-aminophényl)-pyrimido [4,5-5γ,5δ] pristinamycine IE
- 4ε-bromo 2"-(4-aminophényl)-pyrimido [4,5-5γ,5δ] (4ζ-methytamino) (4ζ-désdiméthylamino) pristinamycine IE
- 4ε-allyl 2"-(4-aminophényl)-pyrimido [4,5-5γ,5δ] pristinamycine IE
- 4ε-allyl 2"-(4-aminophényl)-pyrimido [4,5-5γ,5δ] (4ζ-méthylamino) (4ζ-désdiméthylamino) pristinamycine IE
- 4ε-(2-méthyl prop-ène 1-yl) 2"-(4-aminophényl)-pyrimido [4,5-5γ,5δ] pristinamycine IE
- 4ε-(2-méthyl prop-ène 1-yl) 2"-(4-aminophényl)-pyrimido [4,5-5γ,5δ] (4ζ-méthylamino) (4ζ-désdiméthylamino) pristinamycine IE
- 2"-(4-aminophényl)-pyrimido [4,5-5γ,5δ] (4ζ-diéthylamino) (4ζ-désdiméthylamino) pristinamycine IE
- 2"-(4-aminophényl)-pyrimido [4,5-5γ,5δ] (4ζ-N méthyl, N-allylamino) (4ζ-désdiméthylamino) pristinamycine IE
- 2"-(4-aminophényl)-pyrimido [4,5-5γ,5δ] (4ζ-N méthyl, N-éthylamino) (4ζ-désdiméthylamino) pristinamycine IE
- 2"-(4-aminophényl)-pyrimido [4,5-5γ,5δ] (4ζ-N méthyl, N-propylamino) (4ζ-désdiméthylamino) pristinamycine IE
- 2"-(4-aminophényl)-pyrimido [4,5-5γ,5δ] (4ζ-N méthyl, N-(4-pyridylméthyl)amino) (4ζ-désdiméthylamino) pristinamycine IE
- 2"-(4-aminophényl)-pyrimido [4,5-5γ,5δ] (4ζ-N méthyl, N-(3-pyridylméthyl)amino) (4ζ-désdiméthylamino) pristinamycine IE
- 2"-(4-aminophényl)-pyrimido [4,5-5γ,5δ] (4ζ-méthyl) (4ζ-désdiméthylamino) pristinamycine IE
- 2"-(4-aminophényl)-pyrimido [4,5-5γ,5δ] (4ζ-tert-butyl) (4ζ-désdiméthylamino) pristinamycine IE
- 4ε-bromo 2"-trifluorométhyl-pyrimido [4,5-5γ,5δ] pristinamycine IE
- 4ε-bromo 2"-trifluorométhyl-pyrimido [4,5-5γ,5δ] (4ζ-méthylamino) (4ζ-désdiméthylamino) pristinamycine IE
- 4ε-allyl 2"-trifluorométhyl-pyrimido [4,5-5γ,5δ] pristinamycine IE
- 4ε-allyl 2"-trifluorométhyl-pyrimido [4,5-5γ,5δ] (4ζ-méthylamino) (4ζ-désdiméthylamino) pristinamycine IE
- 4ε-(2-méthyl prop-ène 1-yl) 2"-trifluorométhyl-pyrimido [4,5-5γ,5δ] pristinamycine IE
- 4ε-(2-méthyl prop-ène 1-yl) 2"-trifluorométhyl-pyrimido [4,5-5γ,5δ] (4ζ-méthylamino) (4ζ-désdiméthylamino) pristinamycine IE
- 2"-trifluorométhyl-pyrimido [4,5-5γ,5δ] (4ζ-diéthylamino) (4ζ-désdiméthylamino) pristinamycine IE
- 2"-trifluorométhyl-pyrimido [4,5-5γ,5δ] (4ζ-N méthyl, N-allylamino) (4ζ-désdiméthylamino) pristinamycine IE
- 2"-trifluorométhyl-pyrimido [4,5-5γ,5δ] (4ζ-N méthyl, N-éthylamino) (4ζ-désdiméthylamino) pristinamycine IE
- 2"-trifluorométhyl-pyrimido [4,5-5γ,5δ] (4ζ-N méthyl, N-propylamino) (4ζ-désdiméthylamino) pristinamycine IE
- 2"-trifluorométhyl-pyrimido [4,5-5γ,5δ] (4ζ-N méthyl, N-(4-pyridylméthyl)amino) (4ζ-désdiméthylamino) pristinamycine IE
- 2"-trifluorométhyl-pyrimido [4,5-5γ,5δ] (4ζ-N méthyl, N-(3-pyridylméthyl)amino) (4ζ-désdiméthylamino) pristinamycine IE
- 2"-trifluorométhyl-pyrimido [4,5-5γ,5δ] (4ζ-méthyl) (4ζ-désdiméthylamino) pristinamycine IE
- 2"-trifluorométhyl-pyrimido [4,5-5γ,5δ] (4ζ-tert-butyl) (4ζ-désdiméthylamino) pristinamycine IE
- 4ε-bromo 2"-cyclopropyl-pyrimido [4,5-5γ,5δ] pristinamycine IE
- 4ε-bromo 2"-cyclopropyl-pyrimido [4,5-5γ,5δ] (4ζ-méthylamino) (4ζ-désdiméthylamino) pristinamycine IE
- 4ε-allyl 2"-cyclopropyl-pyrimido [4,5-5γ,5δ] pristinamycine IE
- 4ε-allyl 2"-cyclopropyl-pyrimido [4,5-5γ,5δ] (4ζ-méthylamino) (4ζ-désdiméthylamino) pristinamycine IE
- 4ε-(2-méthyl prop-ène 1-yl) 2"-cyclopropyl-pyrimido [4,5-5γ,5δ] pristinamycine IE
- 4ε-(2-méthyl prop-éne 1-yl) 2"-cyclopropyl-pyrimido [4,5-5γ,5δ] (4ζ-méthylamino) (4ζ-désdiméthylamino) pristinamycine IE
- 2"-cyclopropyl-pyrimido [4,5-5γ,5δ] (4ζ-diéthylamino) (4ζ-désdiméthylamino) pristinamycine IE
- 2"-cyclopropyl-pyrimido [4,5-5γ,5δ] (4ζ-N méthyl, N-allylamino) (4ζ-désdiméthylamino) pristinamycine IE
- 2"-cyclopropyl-pyrimido [4,5-5γ,5δ] (4ζ-N méthyl, N-éthylamino) (4ζ-désdiméthylamino) pristinamycine IE
- 2"-cyclopropyl-pyrimido [4,5-5γ,5δ] (4ζ-N méthyl, N-propylamino) (4ζ-désdiméthylamino) pristinamycine IE
- 2"-cyclopropyl-pyrimido [4,5-5y,58] (4ζ-N méthyl, N-(4-pyridylméthyl)amino) (4ζ-désdiméthylamino) pristinamycine IE
- 2"-cyclopropyl-pyrimido [4,5-5γ,5δ] (4ζ-N méthyl, N-(3-pyridylméthyl)amino) (4ζ-désdiméthylamino) pristinamycine IE
- 2"-cyclopropyl-pyrimido [4,5-5γ,5δ] (4ζ-méthyl) (4ζ-désdiméthylamino) pristinamycine IE
- 2"-cyclopropyl-pyrimido [4,5-5γ,5δ] (4ζ-tert-butyl) (4ζ-désdiméthylamino) pristinamycine IE
- 4ε-bromo 2"-morpholinométhyl-pyrimido [4,5-5γ,5δ] pristinamycine IE
- 4ε-bromo 2"-morpholinométhyl-pyrimido [4,5-5γ,5δ] (4ζ-méthylamino) (4ζ-désdiméthylamino) pristinamycine IE
- 4ε-allyl 2"-morpholinométhyl-pyrimido [4,5-5γ,5δ] pristinamycine IE
- 4ε-allyl 2"-morpholinométhyl-pyrimido [4,5-5γ,5δ] (4ζ-méthylamino) (4ζ-désdiméthylamino) pristinamycine IE
- 4ε-(2-méthyl prop-ène 1-yl) 2"-morpholinométhyl-pyrimido [4,5-5γ,5δ] pristinamycine IE
- 4ε-(2-méthyl prop-ène 1-yl) 2"-morpholinométhyl-pyrimido [4,5-5γ,5δ] (4ζ-méthylamino) (4ζ-désdiméthylamino) pristinamycine IE
- 2"-morpholinométhyl-pyrimido [4,5-5γ,5δ] (4ζ-diéthylamino) (4ζ-désdiméthylamino) pristinamycine IE
- 2"-morpholinométhyl-pyrimido [4,5-5γ,5δ] (4ζ-N méthyl, N-allylamino) (4ζ-désdiméthylamino) pristinamycine IE
- 2"-morpholinométhyl-pyrimido [4,5-5γ,5δ] (4ζ-N méthyl, N-éthylamino) (4ζ-désdiméthylamino) pristinamycine IE
- 2"-morpholinométhyl-pyrimido [4,5-5γ,5δ] (4ζ-N méthyl, N-propylamino) (4ζ-désdiméthylamino) pristinamycine IE
- 2"-morpholinométhyl-pyrimido [4,5-5γ,5δ] (4ζ-N méthyl, N-(4-pyridylméthyl)amino) (4ζ-désdiméthylamino) pristinamycine IE
- 2"-morpholinométhyl-pyrimido [4,5-5γ,5δ] (4ζ-N méthyl, N-(3-pyridylméthyl)amino) (4ζ-désdiméthylamino) pristinamycine IE
- 2"-morpholinométhyl-pyrimido [4,5-5γ,5δ] (4ζ-méthyl) (4ζ-désdiméthylamino) pristinamycine IE
- 2"-morpholinométhyl-pyrimido [4,5-5γ,5δ] (4ζ-tert-butyl) (4ζ-désdiméthylamino) pristinamycine IE
- 4ε-bromo 2"-éthyl-pyrimido [4,5-5γ,5δ] pristinamycine IE
- 4ε-bromo 2"-éthyl-pyrimido [4,5-5γ,5δ] (4ζ-méthylamino) (4ζ-désdiméthylamino) pristinamycine IE
- 4ε-allyl 2"-éthyl-pyrimido [4,5-5γ,5δ] pristinamycine IE
- 4ε-allyl 2"-éthyl-pyrimido [4,5-5γ,5δ] (4ζ-méthylamino) (4ζ-désdiméthylamino) pristinamycine IE
- 4ε-(2-méthyl prop-ène 1-yl) 2"-éthyl-pyrimido [4,5-5γ,5δ] pristinamycine IE
- 4ε-(2-méthyl prop-ène 1-yl) 2"-éthyl-pyrimido [4,5-5γ,5δ] (4ζ-méthylamino) (4ζ-désdiméthylamino) pristinamycine IE
- 2"-éthyl-pyrimido [4,5-5γ,5δ] (4ζ-diéthylamino) (4ζ-désdiméthylamino) pristinamycine IE
- 2"-éthyl-pyrimido [4,5-5γ,5δ] (4ζ-N méthyl, N-allylamino) (4ζ-désdiméthylamino) pristinamycine IE
- 2"-éthyl-pyrimido [4,5-5γ,5δ] (4ζ-N méthyl, N-éthylamino) (4ζ-désdiméthylamino) pristinamycine IE
- 2"-éthyl-pyrimido [4,5-5γ,5δ] (4ζ-N méthyl, N-propylamino) (4ζ-désdiméthylamino) pristinamycine IE
- 2"-éthyl-pyrimido (4,5-5γ,5δ] (4ζ-N méthyl, N-(4-pyridylméthyl)amino) (4ζ-désdiméthylamino) pristinamycine IE
- 2"-éthyl-pyrimido [4,5-5γ,5δ] (4ζ-N méthyl, N-(3-pyridylméthyl)amino) (4ζ-désdiméthylamino) pristinamycine IE
- 2"-éthyl-pyrimido [4,5-5γ,5δ] (4ζ-méthyl) (4ζ-désdiméthylamino) pristinamycine IE
- 2"-éthyl-pyrimido [4,5-5γ,5δ] (4ζ-tert-butyl) (4ζ-désdiméthylamino) pristinamycine IE
- 4ε-bromo 2"-propyl-pyrimido [4,5-5γ,5δ] pristinamycine IE
- 4ε-bromo 2"-propyl-pyrimido [4,5-5γ,5δ] (4ζ-méthylamino) (4ζ-désdiméthylamino) pristinamycine IE
- 4ε-allyl 2"-propyl-pyrimido [4,5-5γ,5δ] pristinamycine IE
- 4ε-allyl 2"-propyl-pyrimido [4,5-5γ,5δ] (4ζ-méthylamino) (4ζ-désdiméthylamino) pristinamycine IE
- 4ε-(2-méthyl prop-ène 1-yl) 2"-propyl-pyrimido [4,5-5γ,5δ] pristinamycine IE
- 4ε-(2-méthyl prop-ène 1-yl) 2"-propyl-pyrimido [4,5-5γ,5δ] (4ζ-méthylamino) (4ζ-désdiméthylamino) pristinamycine IE
- 2"-propyl-pyrimido [4,5-5γ,5δ] (4ζ-diéthylamino) (4ζ-désdiméthylamino) pristinamycine IE
- 2"-propyl-pyrimido [4,5-5γ,5δ] (4ζ-N méthyl, N-allylamino) (4ζ-désdiméthylamino) pristinamycine IE
- 2 "-propyl-pyrimido [4,5-5γ,5δ] (4ζ-N méthyl, N-éthylamino) (4ζ-désdiméthylamino) pristinamycine IE
- 2"-propyl-pyrimido [4,5-5γ,5δ] (4ζ-N méthyl, N-propylamino) (4ζ-désdiméthylamino) pristinamycine IE
- 2"-propyl-pyrimido [4,5-5γ,5δ] (4ζ-N méthyl, N-(4-pyridylméthyl)amino) (4ζ-désdiméthylamino) pristinamycine IE
- 2"-propyl-pyrimido [4,5-5γ,5δ] (4ζ-N méthyl, N-(3-pyridylméthyl)amino) (4ζ-désdiméthylamino) pristinamycine IE
- 2"-propyl-pyrimido [4,5-5γ,5δ] (4ζ-méthyl) (4ζ-désdiméthylamino) pristinamycine IE
- 2"-propyl-pyrimido [4,5-5γ,5δ] (4ζ-tert-butyl) (4ζ-désdiméthylamino) pristinamycine IE
- 4ε-bromo 2"-isopropyl-pyrimido [4,5-5γ,5δ] pristinamycine IE
- 4ε-bromo 2"-isopropyl-pyrimido [4,5-5γ,5δ] (4ζ-méthylamino) (4ζ-désdiméthylamino) pristinamycine IE
- 4ε-allyl 2"-isopropyl-pyrimido [4,5-5γ,5δ] pristinamycine IE
- 4ε-allyl 2"-isopropyl-pyrimido [4,5-5γ,5δ] (4ζ-méthylamino) (4ζ-désdiméthylamino) pristinamycine IE
- 4ε-(2-méthyl prop-ène 1-yl) 2 "-isopropyl-pyrimido [4,5-5γ,5δ] pristinamycine IE
- 4ε-(2-méthyl prop-ène 1-yl) 2"-isopropyl-pyrimido [4,5-5γ,5δ] (4ζ-méthylamino) (4ζ-désdiméthylamino) pristinamycine IE
- 2"-isopropyl-pyrimido [4,5-5γ,5δ] (4ζ-diéthylamino) (4ζ-désdiméthylamino) pristinamycine IE
- 2"-isopropyl-pyrimido [4,5-5γ,5δ] (4ζ-N méthyl, N-allylamino) (4ζ-désdiméthylamino) pristinamycine IE
- 2"-isopropyl-pyrimido [4,5-5γ,5δ] (4ζ-N méthyl, N-éthylamino) (4ζ-désdiméthylamino) pristinamycine IE
- 2"-isopropyl-pyrimido [4,5-5γ,5δ] (4ζ-N méthyl, N-propylamino) (4ζ-désdiméthylamino) pristinamycine IE
- 2"-isopropyl-pyrimido [4,5-5γ,5δ] (4ζ-N méthyl, N-(4-pyridylméthyl)amino) (4ζ-désdiméthylamino) pristinamycine IE
- 2"-isopropyl-pyrimido [4,5-5γ,5δ] (4ζ-N méthyl, N-(3-pyridylméthyl)amino) (4ζ-désdiméthylamino) pristinamycine IE
- 2"-isopropyl-pyrimido [4,5-5γ,5δ] (4ζ-méthyl) (4ζ-désdiméthylamino) pristinamycine IE
- 2"-isopropyl-pyrimido [4,5-5γ,5δ] (4ζ-tert-butyl) (4ζ-désdiméthylamino) pristinamycine IE
- 4ε-bromo 2"-cyclopropylaminométhyl-pyrimido [4,5-5γ,5δ] pristinamycine IE
- 4ε-bromo 2"-cyclopropylaminométhyl-pyrimido [4,5-5γ,5δ] (4ζ-méthylamino) (4ζ-désdiméthylamino) pristinamycine IE
- 4ε-allyl 2"-cyclopropylaminométhyl-pyrimido [4,5-5γ,5δ] pristinamycine IE
- 4ε-allyl 2"-cyclopropylaminométhyl-pyrimido [4,5-5γ,5δ] (4ζ-méthylamino) (4ζ-désdiméthylamino) pristinamycine IE
- 4ε-(2-méthyl prop-ène 1-yl) 2"-cyclopropylaminométhyl-pyrimido [4,5-5γ,5δ] pristinamycine IE
- 4ε-(2-méthyl prop-ène 1-yl) 2"-cyclopropylaminométhyl-pyrimido [4,5-5γ,5δ] (4ζ-méthylamino) (4ζ-désdiméthylamino) pristinamycine IE
- 2"-cyclopropylaminométhyl-pyrimido [4,5-5γ,5δ] (4ζ-diéthylamino) (4ζ-désdiméthylamino) prisunamycine IE
- 2"-cyclopropylaminométhyl-pyrimido [4,5-5γ,5δ] (4ζ-N méthyl, N-allylamino) (4ζ-désdiméthylamino) pristinamycine IE
- 2"-cyclopropylaminométhyl-pyrimido [4,5-5γ,5δ] (4ζ-N méthyl, N-éthylamino) (4ζ-désdiméthylamino) pristinamycine IE
- 2"-cyclopropylaminométhyl-pyrimido [4,5-5γ,5δ] (4ζ-N méthyl, N-propylamino) (4ζ-désdiméthylamino) pristinamycine IE
- 2"-cyclopropylaminométhyl-pyrimido [4,5-5γ,5δ] (4ζ-N méthyl, N-(4pyridylméthyl)amino) (4ζ-désdiméthylamino) pristinamycine IE
- 2"-cyclopropylaminométhyl-pyrimido [4,5-5γ,5δ] (4-ζ-N méthyl, N-(3-pyridylméthyl)amino) (4-ζ-désdiméthylamino) pristinamycine IE
- 2"-cyclopropylaminométhyl-pyrimido [4,5-5γ,5δ] (4-Ç-méthyl) (4-Ç-désdiméthylamino) pristinamycine IE
- 2"-cyclopropylaminométhyl-pyrimido [4,5-5γ,5δ] (4-ζ-tert-butyl) (4-ζ-désdiméthylamino) pristinamycine IE

### PREPARATION DES INTERMEDIAIRES

### Exemple A

### Méthode a

On place dans un tricol maintenu sous atmosphère d'azote 170 mg de pristinamycine IB en solution dans 0,5 cm3 de diméthylformamide sec puis on ajoute 0,026 cm3 de bromure de 3,3-diméthylallyle en solution dans 0,2 cm3 de diméthylformamide sec. Après 3 heures d'agitation à température ambiante le mélange réactionnel est dilué par 10 cm3 d'eau distillée puis lavé par 2 fois 20 cm3 d'acétate d'éthyle. La phase organique est décantée, lavée à l'eau, séchée sur sulfate de magnésium, filtrée puis concentrée sous pression réduite (2,7 kPa) pour donner un solide qui est repris dans l'éther éthylique puis séché. Ce solide est purifié par chromatographie flash (éluant : dichlorométhane : méthanol 97/3) pour donner 57 mg de 4-N-(2-méthyl 2-butène 4-yl) pristinamycine IB sous forme d'un solide jaune pâle fondant à 170°C.

Spectre de R.M.N. ¹H (400 MHz, CDCl₃, δ en ppm) : 1,70 (s, 6H : CH₃) ; 2,85 (s, 3H : ArNCH₃) ; 2,87 (mt, 1H : 1H du CH₂ en 4 β) ; 3,23 (s, 3H : NCH₃) ; 3,30 (t, J = 12 Hz, 1H : l'autre H du CH₂ en 4 β) ; 3,82 et 3,91 (2 dd, J = 16,5 et 5 Hz, 1H chacun : ArNCH₂) ; 5,08 (mt, 1H : CH=) ; 5,18 (dd, J = 12 et 4 Hz, 1H : 4 α) ; 6,62 (d, J = 8,5 Hz, 2H : H aromatiques en 4ε) ; 7,03 (d, J = 8,5 Hz, 2H : H aromatiques en 4δ).

### Méthode b

On place dans un tricol maintenu sous atmosphère d'azote 660 mg de pristinamycine IB en solution dans 3,3 cm3 de chloroforme sec (sur amylène) puis on ajoute 65 mg de NaHCO₃ en poudre. Après une heure d'agitation, on additionne 0,09 cm3 de bromure de 3,3-diméthylallyle en solution dans 0,9 cm3 de chloroforme sec (sur amylène). Après 18 heures d'agitation à température ambiante le mélange réactionnel est dilué par 20 cm3 de chloroforme puis lavé par 3 fois 5 cm3 d'eau distillée. La phase organique est décantée, séchée sur sulfate de magnésium, filtrée puis concentrée sous pression réduite (2,7 kPa) pour donner un solide qui est repris dans 20 cm3 d'éther puis séché. Ce solide est purifié par chromatographie flash (éluant : dichlorométhane : méthanol 97/3) pour donner 360 mg de 4-N-(2-méthyl 2-butène 4-yl) pristinamycine IB sous forme d'un solide jaune pâle fondant à 170°C.

### Exemple B

On place dans un tricol maintenu sous atmosphère d'azote 1,7 g de pristinamycine IB dans 5,1 cm3 de diméthylformamide sec puis on ajoute 310 mg de bromure de crotyle. Le mélange est agité 22 heures à température ambiante. Le mélange réactionnel est dilué par 50 cm3 d'eau distillée sous agitation puis extrait par 2 fois 20 cm3 d'acétate d'éthyle. La phase aqueuse est décantée et la phase organique lavée par 2 fois 10 cm3 d'eau distillée, décantée, séchée sur sulfate de magnésium, filtrée puis concentrée sous pression réduite (2,7 kPa) pour donner 1,1 g d'une huile jaune qui est purifiée par chromatographie flash (éluant : dichlorométhane : méthanol 97/3) pour donner 0,62 g de 4-N-(2-butènyl) pristinamycine IB sous forme d'un solide blanc fondant à 180°C.

Spectre de R.M.N. ¹H (400 MHz, CDCl₃, δ en ppm) : 1,70 (d, J = 6 Hz, 3H : CH₃); de 2,85 à 2,90 (mt, 1H : 1H du CH₂ en 4 β) ; 2,90 (s, 3H : ArNCH₃) ; 3,28 (s, 3 H : NCH₃) ; 3,32 (t, J = 12 Hz, 1H : l'autre H du CH₂ en 4 β) ; 3,81 et 3,91 (2 d larges, J = 18 Hz, 1H chacun : ArNCH₂) ; 5,22 (dd, J = 12 et 4 Hz, 1H : 4 α) ; 5,43 et 5,57 (respectivement d mt et dq, J = 14 Hz et J = 14 et 6 Hz, 1H chacun : CH=CH) ; 6,62 (d, J = 8 Hz, 2H : H aromatiques en 4ε) ; 7,05 (d, J = 8 Hz, 2H : H aromatiques en 4δ).

### Exemple C

En opérant comme à l'exemple A mais à partir de 2,5 g de pristinamycine IB, de 400 mg d'acide bromo acétique dans 8 cm3 de diméthylformamide sec, on obtient après 48 heures d'agitation à température ambiante, 2,1 g d'un solide blanc qui est purifié par chromatographie flash (éluants successifs : dichlorométhane-méthanol, 95/5 puis 90/10 puis 80/20) pour donner 1,1 g d'une huile qui est reprise au dichlorométhane, acidifiée à pH 4 par de l'acide acétique puis lavée à l'eau distillée. La phase organique est décantée, séchée sur sulfate de magnésium, filtrée puis concentrée sous pression réduite (2,7 kPa), puis reprise dans l'éther diéthylique pour donner 750 mg de 4-N-(carboxyméthyl) pristinamycine IB sous forme d'un solide blanc fondant à 230°C.

Spectre de R.M.N. ¹H (400 MHz, CDCl₃, δ en ppm) : 2,85 (dd, J = 12 et 4 Hz, 1H: 1H du CH₂ en 4 β) ; 3,03 (s, 3H : ArNCH₃) ; de 3,10 à 3,40 (mt, 1H : l'autre H du CH₂ en 4 β) ; 3,25 (s, 3H : NCH₃) ; 4,04 (AB limite, J = 18 Hz, 2H : ArNCH₂) ; 5,25 (dd, J = 12 et 4 Hz, 1H : 4 α) ; 6,62 (d, J = 8 Hz, 2H : H aromatiques en 4ε) ; 7,07 (d, J = 8 Hz, 2H : H aromatiques en 4δ).

### Exemple D

En opérant comme à l'exemple A mais à partir de 1 g de pristinamycine IB, de 0,1 ml de bromure d'allyle dans 3 cm3 de diméthylformamide sec, on obtient après 72 heures d'agitation à température ambiante, 620 mg d'un solide blanc qui est purifié par chromatographie flash (éluant dichlorométhane-méthanol, 97/3) pour donner 290 mg de 4-N-allyl pristinamycine IB sous forme d'un solide jaune blanc fondant à 208°C.

Spectre de R.M.N. ¹H (400 MHz, CDCl₃, δ en ppm) : 2,88 (dd, J = 12 et 4 Hz, 1H : 1H du CH₂ en 4 β) ; 2,90 (s, 3H : ArNCH₃) ; 3,21 (s, 3H : NCH₃) ; 3,29 (t, J = 12 Hz, 1H : l'autre H du CH₂ en 4 β) ; 3,85 et 3,95 (2 d larges, J = 18 Hz, 1H chacun : ArNCH₂) ; 5,10 et 5,17 (2 d, respectivement J = 17 Hz et J = 11,5 Hz, 1H chacun : =CH₂) ; 5,20 (dd, J = 12 et 4 Hz, 1H : 4 α) ; 5,78 (mt, 1H : CH=) ; 6,60 (d, J = 8 Hz, 2H : H aromatiques en 4ε) ; 7,02 (d, J = 8 Hz, 2H : H aromatiques en 4δ).

### Exemple E

En opérant comme à l'exemple A, mais à partir de 1 g de pristinamycine IB dans 3 cm3 de diméthylformamide sec et de 230 mg de bromure de cinnamyle, on obtient après 72 heures à température ambiante, 0,8 g d'un solide blanc qui est purifié par chromatographie flash (éluant : dichlorométhane : méthanol 97/3) pour donner 0,31 g de 4-N-cinnamyl pristinamycine IB sous forme d'un solide blanc fondant à 204°C.

Spectre de R.M.N. ¹ H (600 MHz, CDCl₃, δ en ppm) : 2,90 (dd, J = 12,5 et 4 Hz, 1 H : 1H du CH₂ en 4 β) ; 2,97 (s, 3H : ArNCH₃) ; 3,24 (s, 3H : NCH₃); 3,33 (t, J = 12,5 Hz, 1H : l'autre H du CH₂ en 4 β) ; 4,70 (AB limite, J = 18 et 5,5 Hz, 2H : ArNCH₂) ; 5,20 (dd, J = 12,5 et 4 Hz, 1H : 4 α) ; 6,23 et 6,52 (respectivement dt et d large, J = 16,5 et 5,5 Hz et J = 16,5 Hz, 1 H chacun : CH=CH) ; 6,68 (d, J = 8 Hz, 2H : H aromatiques en 4ε) ; 7,07 (d, J = 8 Hz, 2H : H aromatiques en 4δ) ; de 7,25 à 7,40 (mt, 5H : H aromatiques du phényle).

### Exemple F

En opérant comme à l'exemple A, mais à partir de 1 g de pristinamycine IB dans 3 cm3 de diméthylformamide sec et de 240 mg de bromure de benzyle, on obtient après 72 heures à température ambiante, 0,85 g d'un solide blanc qui est purifié par chromatographie flash (éluant : dichlorométhane : méthanol 97/3) pour donner 0,64 g de 4-N-benzyl pristinamycine IB sous forme d'un solide blanc fondant à une température supérieure à 260°C.

Spectre de R.M.N. ¹ H (400 MHz, CDCl₃, δ en ppm) : 2,86 (dd, J = 12,5 et 4 Hz, 1H : 1H du CH₂ en 4 β) ; 3,10 (s, 3H : ArNCH₃) ; 3,26 (s, 3H : NCH₃) ; 3,32 (t, J = 12,5 Hz, 1H : l'autre H du CH₂ en 4 β) ; 4,52 et 4,69 (2 d, J = 18 Hz, 1H chacun : ArNCH₂) ; 5,16 (dd, J = 12,5 et 4 Hz, 1H : 4 α) ; 6,59 (d, J = 8 Hz, 2H :H aromatiques en 4ε) ; 7,01 (d, J = 8 Hz, 2H : H aromatiques en 4δ) ; 7,18 (mt, 2H : H en ortho du benzyl) ; 7,28 (mt, 2H : H en méta du benzyl) . 7,40 (t, J = 7,5 Hz, 1H : H en para du benzyl).

### Exemple G

En opérant comme à l'exemple A, mais à partir de 1 g de pristinamycine IB dans 3 cm3 de diméthylformamide sec et de 200 mg de iodure d'éthyle, on obtient après 5 heures à 60°C puis 72 heures à température ambiante et après ajout de 20 mg de iodure d'éthyle supplémentaire et chauffage à 60°C pendant 4 heures, 0,65 g d'un solide jaune pâle qui est purifié par chromatographie flash (éluant : dichlorométhane : méthanol 97/3) pour donner 0,37 g de 4-N-éthyl pristinamycine IB sous forme d'un solide blanc fondant à une température supérieure à 260°C.

Spectre de R.M.N. ¹H (400 MHz, CDCl₃, δ en ppm) : 1,10 (t, J = 7 Hz, 3H : CH₃ de l'éthyle) ; 2,87 (s, 3H : ArNCH₃) ; 2,90 (dd, J = 12,5 et 4 Hz, 1H : 1H du CH₂ en 4 β); 3,25 (s, 3H : NCH₃) ; 3,32 (t, J = 12,5 Hz, 1H : l'autre H du CH₂ en 4 β) ; 3,39 (mt, 2H : ArNCH₂) ; 5,21 (dd, J = 12,5 et 4 Hz, 1H : 4 α) ; 6,60 (d, J = 8 Hz, 2H : H aromatiques en 4ε) ; 7,04 (d, J = 8 Hz, 2H : H aromatiques en 4δ.

### Exemple H

On place dans un tricol maintenu sous atmosphère d'azote 1 g de pristinamycine IB dans 3 cm3 de diméthylformamide sec puis on ajoute 175 mg d'un mélange d'environ 20 % de 4-bromo-1-butène de 15 % de bromométhylcyclopropane et de 65 % de bromocyclobutane et 195 mg d'iodure de sodium. Le mélange est agité 72 heures à température ambiante puis chauffé 7 heures à 60°C. On ajoute à nouveau 175 mg de ce mélange, puis l'agitation est poursuivie 48 heures. Le mélange réactionnel est dilué par 50 cm3 d'eau distillée sous agitation puis extrait par 2 fois 20 cm3 d'acétate d'éthyle. La phase aqueuse est décantée puis la phase organique lavée par 2 fois 10 cm3 d'eau distillée, décantée, séchée sur sulfate de magnésium, filtrée puis concentrée sous pression réduite (2,7 kPa) pour donner 800 mg d'une poudre blanche qui est purifiée par chromatographie flash (éluant : dichlorométhane : méthanol 98/2) puis par chromatographie liquide haute performance (CLHP) pour donner 220 mg de 4-N-(but-2-ényl) pristinamycine IB sous forme d'un solide blanc fondant à 190°C.

Spectre de R.M.N. ¹ H (300 MHz, CDCl₃, δ en ppm) : 2,29 (mt, 2H : CH₂); 2,88 (dd, J = 12 et 4 Hz, 1H : 1H du CH₂ en 4 β) ; 2,90 (s, 3H : ArNCH₃) ; 3,25 (s, 3H : NCH₃) ; 3,31 (t, J = 12 Hz, 1H : l'autre H du CH₂ en 4 β) ; 3,38 (mt, 2H : ArNCH₂) ; 5,05 et 5,10 (2 dd, respectivement J = 10,5 et 2 Hz et J = 16,5 et 2 Hz, 1H chacun : =CH₂) ; 5,20 (dd, J = 12 et 4 Hz, 1H : 4 α) ; 5,78 (mt, 1H : CH=) ; 6,62 (d, J = 8 Hz, 2H : H aromatiques en 4ε) ; 7,04 (d, J= 8 Hz, 2H : H aromatiques en 4δ).

### Exemple I

On place dans un tricol maintenu sous atmosphère d'azote 1g de pristinamycine IB dans 3 cm3 de diméthylformamide sec puis on ajoute 175 mg d'un mélange d'environ 20 % de 4-bromo-1-butène de 15 % de bromométhylcyclopropane et de 65 % de bromocyclobutane et 195 mg d'iodure de sodium. Le mélange est agité 72 heures à température ambiante puis chauffé 7 heures à 60°C. On ajoute à nouveau 175 mg de ce mélange, puis l'agitation est poursuivie 48 heures. Le mélange réactionnel est dilué par 50 cm3 d'eau distillée sous agitation puis extrait par 2 fois 20 cm3 d'acétate d'éthyle. La phase aqueuse est décantée puis la phase organique lavée par 2 fois 10 cm3 d'eau distillée, décantée, séchée sur sulfate de magnésium, filtrée puis concentrée sous pression réduite (2,7 kPa) pour donner 800 mg d'une poudre blanche qui est purifiée par chromatographie flash (éluant : dichlorométhane : méthanol 98/2) puis par chromatographie CLHP pour donner 222 mg de 4-N-cyclopropylméthyl pristinamycine IB sous forme d'un solide blanc fondant à 190°C.

Spectre de R.M.N. ¹H (300 MHz, CDCl₃, δ en ppm) : 0,20 et 0,53 (2 mts, 2H chacun : CH₂ du cyclopropane) ; 0,92 (mt, 1H : CH du cyclopropane) ; 2,90 (dd, J = 12,5 et 4 Hz, 1H : 1H du CH₂ en 4β) ; 2,93 (s, 3H : ArNCH₃) ; 3,13 et 3,25 (respectivement dd et mt, J = 15 et 7 Hz, 1H chacun : ArNCH₂) ; 3,25 (s, 3H : NCH₃) ; 3,32 (t, J = 12,5 Hz, 1H : l'autre H du CH₂ en 4 β) ; 5,20 (dd, J = 12,5 et 4 Hz, 1H : 4 α) ; 6,67 (d, J = 8 Hz, 2H : H aromatiques en 4ε) ; 7,04 (d, J = 8 Hz, 2H: H aromatiques en 4δ).

### Exemple J

On place dans un tricol maintenu sous atmosphère d'azote 2 g de pristinamycine IB dans 10 cm3 de diméthylformamide sec puis on ajoute 460 mg de chlorydrate de 4-chlorométhyl pyridine et 350 mg d'iodure de sodium. Le mélange est agité 5 heures à 60°C. Le mélange réactionnel est versé sur 150 cm3 d'eau distillée puis extrait par 3 fois 100 cm3 d'acétate d'éthyle. La phase aqueuse est décantée puis la phase organique séchée sur sulfate de magnésium, filtrée puis concentrée sous pression réduite (2,7 kPa) pour donner 2,6 g d'une huile jaune qui est purifiée par 2 chromatographies flash (éluant : dichlorométhane : méthanol 97/3) pour donner 130 mg de 4-N-(4-pyridyl méthyl) pristinamycine IB sous forme d'un solide blanc fondant à 260°C.

Spectre de R.M.N. ¹H (300 MHz, CDCl₃, δ en ppm) : 2,90 (dd, J = 12,5 et 4 Hz, 1H : 1H du CH₂ en 4 β) ; 3,07 (s, 3H : ArNCH₃); 3,27 (s, 3H : NCH₃) ; 3,32 (t, J = 12,5 Hz, 1H : l'autre H du CH₂ en 4 β); 4,50 et 4,63 (2 d, J = 17 Hz, 1H chacun : ArNCH₂) ; 5,16 (dd, J = 12,5 et 4 Hz, 1H : 4 α) ; 6,59 (d, J = 8 Hz, 2H : H aromatiques en 4ε) ; 1,01 (d, J = 8 Hz, 2H : H aromatiques en 4δ) ; 7,13 (d, J = 5,5 Hz; 2H : H en β de la pyridine) ; 8,60 (d, J = 5,5 Hz; 2H : H en a de la pyridine).

### Exemple K

En opérant comme à l'exemple A, mais à partir de 1 g de pristinamycine IB dans 3 cm3 de diméthylformamide sec et de 237 mg de iodobutane, on obtient après 48 heures à 60°C puis 72 heures à température ambiante, 0,94 g d'un solide jaune pâle qui est purifié par chromatographie flash (éluant : dichloro-méthane : méthanol 98/2) pour donner 0,23 g de 4-N-butyl pristinamycine IB sous forme d'un solide blanc fondant à 170°C.

Spectre de R.M.N. ¹H (300 MHz, CDCl₃, δ en ppm) : 0,95 (t, J = 7,5 Hz, 3H : CH₃ du butyle) ; 1,35 et 1,55 (2 mts, 2H chacun : CH₂CH₂ du butyle) ; 2,90 (s, 3H : ArNCH₃) ; 2,90 (dd, J = 12,5 et 4 Hz, 1H : 1H du CH₂ en 4 β) ; de 3,20 à 3,40 (mt, 3H : l'autre H du CH₂ en 4 β et ArNCH₂) ; 3,28 (s, 3H : NCH₃) ; 5,21 (dd, J = 12,5 et 4 Hz, 1H : 4 α) ; 6,60 (d, J = 8 Hz, 2H : H aromatiques en 4ε); 7,05 (d, J = 8 Hz, 2H : H aromatiques en 4δ).

### Exemple L

En opérant comme à l'exemple A mais à partir de 3 g de pristinamycine IB dans 15 cm3 de diméthylformamide sec et de 720 mg de iodopropane, on obtient, après 22 heures à 50°C, 2,07 g d'une huile jaune pâle qui est purifiée par 2 chromatographies flash (éluant : dichlorométhane : méthanol 98/2 et dichlorométhane : méthanol 99/1) pour donner 0,49 g de 4-N-propyl pristinamycine IB sous forme d'un solide blanc fondant à 220°C (déc.).

Spectre de R.M.N. ¹H (300 MHz, CDCl₃, δ en ppm) : 0,95 (t, J = 7,5 Hz, 3H : CH₃ du propyle) ; 1,58 (mt, 2H : CH₂ du propyle) ; 2,88 (mt, 1H : 1H du CH₂ en 4 β) ; 2,90 (s, 3H : ArNCH₃) ; de 3,15 à 3,40 (mt, 3H : l'autre H du CH₂ en 4 β et ArNCH₂); 3,25 (s, 3H : NCH₃) ; 5,20 (dd, J = 12,5 et 4 Hz, 1H : 4 α) ; 6,60 (d, J = 8 Hz, 2H : H aromatiques en 4ε) ; 7,03 (d, J = 8 Hz, 2H : H aromatiques en 4δ).

### Exemple M

En opérant comme à l'exemple A, mais à partir de 2 g de pristinamycine IB dans 5 cm3 de diméthylformamide sec et de 480 mg de 2-iodopropane, on obtient après 6 heures à 60°C puis 17 heures à température ambiante, 2,07 g d'une huile jaune pâle qui est purifiée par chromatographie flash (éluant : dichlorométhane : méthanol 97/3) pour donner 0,19 g de 4-N-isopropyl pristinamycine IB sous forme d'un solide blanc fondant à 220°C (déc.).

Spectre de R.M.N. ¹H (300 MHz, CDCl₃, δ en ppm) : 1,14 et 1,17 (2 d, J = 6.5 Hz, 6H : CH₃ de l'isopropyle) ; 2,68 (s, 3H : ArNCH₃) ; 2,88 (dd, J = 12 et 4 Hz, 1H : 1H du CH₂ en 4 β) ; 3,23 (s, 3H : NCH₃) ; 3,30 (t, J= 12 Hz, 1H : l'autre H du CH₂ en 4 β) ; 3,90 (mt, 1H : ArNCH) ; 5,20 (dd, J = 12 et 4 Hz, 1H : 4 α) ; 6,68 (d, J = 8 Hz, 2H : H aromatiques en 4ε) ; 7,03(d, J = 8 Hz, 2H : H aromatiques en 4δ).

### Exemple N

En opérant comme à l'exemple A, mais à partir de 3 g de pristinamycine IB dans 15 cm3 de diméthylformamide sec et de 780 mg de iodure de 3-méthyl 2-propane, on obtient après 70 heures à température ambiante puis ajout de 160 mg de iodure de 3-méthyl 2-propane supplémentaires et chauffage à 50°C pendant 24 heures, 3.86 g d'un solide qui est purifié par chromatographie flash (éluant : dichlorométhane : méthanol 98/2) pour donner 690 mg de 4-N-isobutyl pristinamycine IB sous forme d'un solide blanc fondant à 190°C (déc.).

Spectre de RM.N. ¹H (300 MHz, CDCl₃, δ en ppm) : 0,93 (d, J = 7 Hz, 6H : CH₃ de l'isobutyle) ; 2,05 (mt, 1 H : CH de l'isobutyle) ; 2,92 (dd, J = 12,5 et 4 Hz, 1H : 1H du CH₂ en 4 β) ; 2,98 (s, 3H : ArNCH₃) ; 3,10 et 3,18 (2 dd, J = 15 et 7,5 Hz, 1H chacun : ArNCH₂) ; 3,30 (s, 3H : NCH₃) ; 3,35 (t, J = 12,5 Hz, 1H : l'autre H du CH₂ en 4 β) ; 5,20 (dd, J = 12,5 et 4 Hz, 1H : 4 α) ; 6,60 (d, J = 8 Hz, 2H : H aromatiques en 4ε) ; 7,03(d, J = 8 Hz, 2H : H aromatiques en 4δ).

### Exemple O

On place dans un tricol maintenu sous atmosphère d'azote 3 g de pristinamycine IB dans 15 cm3 de diméthylformamide sec puis on ajoute 690 mg de chlorydrate de 3-chlorométhyl pyridine et 350 mg d'iodure de sodium. Le mélange est agité 24 heures à 60°C puis 48 heures à température ambiante. Le mélange réactionnel est versé sur 50 cm3 d'eau distillée additionnée de bicarbonate de sodium puis extrait par 3 fois 50 cm3 d'acétate d'éthyle. La phase aqueuse est décantée puis la phase organique séchée sur sulfate de magnésium, filtrée puis concentrée sous pression réduite (2,7 kPa) pour donner 2,96 g d'un solide jaune qui est purifié par chromatographie flash (éluant : dichlorométhane : méthanol 98/2) pour donner 409 mg de 4-N-(3-pyridyl méthyl) pristinamycine IB sous forme d'un solide blanc fondant à 186°C.

Spectre de R.M.N. ¹H (400 MHz, CDCl₃, δ en ppm) : 2,87 (dd, J = 12,5 et 4 Hz, 1 H : 1H du CH₂ en 4 β) ; 3,05 (s, 3H : ArNCH₃) ; 3,23 (s, 3H : NCH₃) ; 3,29 (t, J = 12,5 Hz, 1H : l'autre H du CH₂ en 4β) ; 4,50 et 4,65 (2 d, J = 18 Hz, 1H chacun : ArNCH₂) ; 5,15 (dd, J = 12,5 et 4 Hz, 1H : 4 α) ; 6,62 (d, J = 8 Hz, 2H : H aromatiques en 4ε) ; 7,05 (d, J = 8 Hz, 2H : H aromatiques en 4δ) ; 7,35 (mt; 1H : H en 5 de la pyridine) ; 7,42 (d large, J = 8 Hz, 1H : H en 4 de la pyridine) ; 8,45 (d large, J = 5 Hz, 1H : H en 6 de la pyridine) ; 8,58 (s large; 1H : H en 2 de la pyridine).

### Exemple P

On place dans un tricol maintenu sous atmosphère d'azote 3 g de pristinamycine IB dans 15 cm3 de diméthylformamide sec puis on ajoute 690 mg de chlorydrate de 2-chlorométhyl pyridine et 70 mg d'iodure de sodium. Le mélange est agité 2 heures à 60°C puis on ajoute 0,48 g d'iodure de sodium supplémentaire et l'agitation est maintenue 23 heures à 60°C. Le mélange réactionnel est versé sur 150 cm3 d'eau distillée additionnée de bicarbonate de sodium puis extrait par 3 fois 100 cm3 d'acétate d'éthyle. La phase aqueuse est décantée puis les phases organiques réunies puis lavées par une solution aqueuse de sulfite de sodium. La phase organique est décantée, séchée sur sulfate de magnésium, filtrée puis concentrée sous pression réduite (2,7 kPa) pour donner 3,34 g d'un solide jaune qui est purifié par chromatographie flash (éluant : dichlorométhane : méthanol 98/2) pour donner 1,16 g de 4-N-(2-pyridyl méthyl) pristinamycine IB sous forme d'un solide blanc fondant à 190°C.

Spectre de R.M.N. ¹H (400 MHz, CDCl₃, δ en ppm) : 2,85 (dd, J = 12,5 et 4 Hz, 1H : 1H du CH₂ en 4β) ; 3,15 (s, 3H : ArNCH₃) ; 3,24 (s, 3H : NCH₃) ; 3,29 (t, J = 12,5 Hz, 1H : l'autre H du CH₂ en 4 β) ; 4,55 et 4,83 (2 d, J = 18 Hz, 1H chacun : ArNCH₂) ; 5,10 (dd, J = 12,5 et 4 Hz, 1H : 4 α) ; 6,57 (d, J = 8 Hz, 2H : H aromatiques en 4ε) ; 6,99 (mt, 1H : H en 3 de la pyridine) ; 7,00 (d, J = 8 Hz, 2H : H aromatiques en 4δ) ; 7,08 (dd, J = 7,5 et 5 Hz; 1H : H en 5 de la pyridine) ; 7,80 (dt, J = 7,5 et 1 Hz, 1H : H en 4 de la pyridine) ; 8,57 (d large, J = 5 Hz; 1H : H en 6 de la pyridine).

### Exemple O

On place dans un tricol maintenu sous atmosphère d'azote 5 g de pristinamycine IB dans 7 cm3 de diméthylformamide sec puis on ajoute 0,66 g de 1-chloro 3-hydroxypropane, 50 mg d'iodure de sodium et 580 mg de bicarbonate de potassium. Le mélange est agité 22 heures à 70°C. Le mélange réactionnel est refroidi, versé sur 30 cm3 d'eau distillée puis extrait par 3 fois 40 cm3 d'acétate d'éthyle. La phase aqueuse est décantée puis la phase organique séchée sur sulfate de magnésium, filtrée puis concentrée sous pression réduite (2,7 kPa) pour donner 5,41 g d'un solide qui est purifié par chromatographie flash (éluant : dichlorométhane : méthanol 98/2) pour donner 0,28 g de 4-N-(3-hydroxy 3-propyl) pristinamycine IB sous forme d'un solide blanc fondant à 186°C.

Spectre de R.M.N. ¹ H (400 MHz, CDCl₃, δ en ppm) : 1,75 (mt, 2H : CH₂ cental du propyle) ; 2,88 (mt, 1H : 1H du CH₂ en 4β) ; 2,90 (s, 3H : ArNCH₃) ; 3,24 (s, 3H : NCH₃) ; 3,30 (t, J = 12,5 Hz, 1H : l'autre H du CH₂ en 4 β) ; 3,43 et 3,62 (2 mts, 2H chacun : ArNCH₂ et CH₂O) ; 5,20 (dd, J = 12,5 et 4 Hz, 1H: 4 α) ; 6,68 (mf, 2H : H aromatiques en 4ε) ; 7,03 (d, J = 8 Hz, 2H : H aromatiques en 4δ).

### Exemple R

En opérant comme à l'exemple Q, mais à partir de 4 g de pristinamycine IB, de 1,7 cm3 de 3-(dioxo-1,2-éthylène) bromopropane dans 12 cm3 de diméthylformamide sec, on obtient après chauffage 24 heures à 60°C, 3,8 g d'un solide jaune qui est purifié par chromatographie-flash (éluant dichlorométhane / méthanol 97/3) pour donner 0,81 g de 4-N-[2-(1,3-dioxolan-2-yl) éthyl] pristinamycin IB sous forme d'un solide blanc fondant à une température supérieure à 260°C.

Spectre de R.M.N. ¹H (400 MHz, CDCl₃, δ en ppm) : 1,91 (mt, 2H : CH₂ central) ; 2,87 (s, 3H : ArNCH₃) ; 2,88 (dd, J = 12 et 4 Hz, 1H : 1H du CH₂ en 4 β) ; 3,25 (s, 3H : NCH₃) ; 3,29 (t, J = 12 Hz, 1H : l'autre H du CH₂ en 4 β) ; 3,35 à 3,55 (mt, 2H : ArNCH₂) ; 3,87 et 3,97 (2 mts, 2H chacun : OCH₂CH₂O) ; 4,92 (t, J = 4 Hz, 1H : OCHO) ; 5,21 (dd, J = 12 et 4 Hz, 1H : 4 α) ; 6,64 (d, J = 8 Hz, 2H : H aromatiques en 4ε) ; 7,04 (d, J = 8 Hz, 2H : H aromatiques en 4δ).

### Exemple S

En opérant comme à l'exemple A mais à partir de 0,53 g de 4ε-chloro pristinamycine IB, de 0,082 cm3 de bromure d'allyle, dans 3 cm3 de diméthylformamide sec, on obtient après 7 heures à 50°C puis ajout de 0,5 cm3 de bromure d'allyle supplémentaire et chauffage 2 heures 30 minutes, un solide qui est purifié par chromatographie flash (éluant : dichlorométhane : méthanol 98/2) pour donner 77 mg de 4-N-allyl 4ε-chloro pristinamycine IB sous forme d'un solide jaune très clair fondant à 175°C (déc.).

Spectre de R.M.N. ¹ H (300 MHz, CDCl₃, δ en ppm) : 2,71 (s, 3H : ArNCH₃) ; 2,93 (dd, J = 12 et 4 Hz, 1H : 1H du CH₂ en 4β) ; 3,21 (s, 3H : NCH₃) ; 3,33 (t, J = 12 Hz, 1H : l'autre H du CH₂ en 4β) ; 3,58 (d, J = 6 Hz, 2H : ArNCH₂) ; 5,20 et 5,27 (2 dd, respectivement J = 11 et 1 Hz et J = 16 et 1 Hz, 1H chacun : =CH₂) ; 5,30 (dd, J = 12 et 4 Hz, 1H : 4 α) ; de 5,75 à 5,95 (mt, 1H : CH=) ; 6,95 (d, J = 8 Hz, 1H : H aromatique en 4ε) ; 7,03 (dd, J = 8 et 1,5 Hz, 1H : H aromatique en 4δ) ; 7,18 (d, J = 1,5 Hz, 1 H : H aromatique en 4δ et en ortho du Cl).

La 4ε-chloro pristinamycine IB peut être préparée comme décrit dans la demande de brevet EP 772630.

### Exemple T

On place dans un ballon 0,3 g de 4-N-éthoxycarbonylméthyl pristinamycine IB dans 3,5 cm3 de dichlorométhane puis on ajoute 51 mg de N-chloro-succinimide. Le mélange est agité 5 jours à température ambiante. Le mélange réactionnel est concentré à sec sous pression réduite (2,7 kPa) à 30°C. Le solide obtenu est agité 3 fois dans 5 cm3 d'eau distillée, filtré, lavé par 3 fois 3 cm3 d'éther pour donner un solide jaune qui est recristallisé dans 4 cm3 d'éthanol. Après filtration des cristaux et séchage sous pression réduite (135 Pa) à 50°C on obtient 0,15 g de 4ε-chloro (4-N-éthoxycarbonylméthyl) pristinamycine IB sous forme de cristaux beige clair fondant à 176°C.

Spectre de R.M.N. ¹ H (300 MHz, CDCl₃, δ en ppm) : 1,34 (t, J = 7 Hz, 3H : CH₃ de l'éthyle) ; 2,95 (dd, J = 12 et 4 Hz, 1H : 1H du CH₂ en 4 β) ; 3,05 (s, 3H : ArNCH₃) ; 3,32 (s, 3H : NCH₃ 4) ; 3,38 (t, J = 12 Hz, 1H : l'autre H du CH₂ en 4 β); 3,85 et 4,19 (2 d, J = 17,5 Hz, 1H chacun : ArNCH₂) ; 4,22 (q, J = 7 Hz, 2H : CH₂ de l'éthyle) ; 5,29 (dd, J = 12 et 4 Hz, 1H : 4 α) ; 7,10 (d, J = 8,5 Hz, 1H : H aromatique en 4ε) ; 7,25 (mt, 2H : H aromatiques en 4δ).

La 4-N-éthoxycarbonylméthyl pristinamycine IB peut être préparée comme décrit ci-après à l'exemple AD.

### Exemple U

En opérant comme à l'exemple T mais à partir de 0,3 g de 4-N-éthyl pristinamycine IB et de 0,545 g de N-chlorosuccinimide dans 3,5 cm3 de dichlorométhane, on obtient après une semaine d'agitation à température ambiante, 0,33 g d'un solide qui est recristallisé dans 6 cm3 d'éthanol. Après filtration des cristaux et séchage sous pression réduite (135 Pa) à 50°C on obtient 0,15g de 4ε-chloro 4-N-éthyl pristinamycine IB sous forme de cristaux beige clair fondant >à 260°C.

Spectre de R.M.N. ¹H (400 MHz, CDCl₃, δ en ppm) : 1,16 (t, J = 7 Hz, 3H : CH₃ de l'éthyle) ; 2,70 (s, 3H : ArNCH₃) ; 2,92 (dd, J = 12 et 4 Hz, 1H : 1H du CH₂ en 4β) ; 3,00 (q, J = 7 Hz, 2H : NCH₂ de l'éthyle) ; 3,22 (s, 3H : NCH₃) ; 3,33 (t, J = 12 Hz, 1H : l'autre H du CH₂ en 4 β) ; 5,22 (dd, J = 12 et 4 Hz, 1H: 4 α) ; 6,95 (d, J = 8 Hz, 1H : H aromatique en 4ε) ; 7,03 (dd, J = 8 et 1,5 Hz, 1H : H aromatique en 4δ) ; 7,23 (d, J = 1,5 Hz, 1H : H aromatique en 4δ et en ortho du Cl).

### Exemple V

En opérant comme à l'exemple T, mais à partir de 200 mg de 4-N-isobutyl pristinamycine IB, de 44 mg de N-chlorosuccinimide et de 3 cm3 de dichlorométhane on obtient après 36 heures d'agitation à température ambiante puis 40 minutes de reflux, 99 mg d'un solide blanc qui est agité dans 10 cm3 d'eau, filtré puis rincé pour donner 690 mg de 4-N-isobutyl pristinamycine IB sous forme d'un solide blanc fondant à 190°C (déc.).

Spectre de R.M.N. ¹H (400 MHz, CDCl₃, δ en ppm) : 0,88 (d, J = 7 Hz, 6H : CH₃ de l'isobutyle) ; 1,80 (mt, 1H : CH de l'isobutyle) ; 2.69 (s, 3H : ArNCH₃) ; 2,75 (AB limite, 2H : ArNCH₂) ; 2,95 (dd, J = 12 et 4 Hz, 1H : 1H du CH₂ en 4 β) ; 3,25 (s, 3H : NCH₃) ; 3,34 (t, J = 12 Hz, 1H : l'autre H du CH₂ en 4 β) ; 5,27 (dd, J = 12 et 4 Hz, 1H : 4 α) ; 6,99 (d, J = 8 Hz, 1H : H aromatique en 4ε) ; 7,06 (d large, J = 8 Hz, 1H : H aromatique en 4δ) ; de 7,25 à 7,40 (mt, 1H : H aromatique en 4δ et en ortho du CI).

### Exemple W

En opérant comme à l'exemple T, mais à partir de 224 mg de 4-N-(4-pyridyl méthyl) pristinamycine IB, de 32 mg de N-chlorosuccinimide et de 3 cm3 d'acétonitrile on obtient après 2 heures d'agitation à 65°C, un solide beige qui est agité dans 10 cm3 d'eau, filtré puis rincé puis purifié par chromatographie flash (éluant : dichlorométhane : méthanol 98/2) pour donner 190 mg de 4ε-chloro 4-N-(4-pyridyl méthyl) pristinamycine IB sous forme d'un solide jaune pâle blanc fondant à 232°C (déc.).

Spectre de R.M.N. ¹H (300 MHz, CDCl₃, δ en ppm) : 2,67 (s, 3H : ArNCH₃) ; 2,97 (dd, J = 12,5 et 4 Hz, 1H : 1H du CH₂ en 4 β) ; 3,24 (s, 3H : NCH₃) ; 3,32 (t, J = 12,5 Hz, 1H : l'autre H du CH₂ en 4 β) ; 4,10 (s, 2H : ArNCH₂) ; 5,29 (dd, J = 12,5 et 4 Hz, 1H : 4 α) ; 6,99 (d, J = 8 Hz, 1H : H aromatique en 4ε) ; 7,06 (dd, J = 8 et 1,5 Hz, 1H : H aromatique en 4δ) ; de 7,15 à 7,40 (mt, 1H : H aromatique en 4δ et en ortho du Cl) ; 7,37 (d, J = 6 Hz; 2H : H en β de la pyridine) ; 8,57 (d, J = 6 Hz; 2H : H en a de la pyridine.

### Exemple X

En opérant comme à l'exemple T, mais à partir de 260 mg de 4-N-(3-pyridyl méthyl) pristinamycine IB, de 37 mg de N-chlorosuccinimide et de 3 cm3 d'acétonitrile on obtient après 20 heures d'agitation à 65°C, 270 mg d'un solide blanc qui est agité dans 10 cm3 d'eau, filtré puis rincé pour donner 120 mg de 4ε-chloro 4-N-(3-pyridyl méthyl) pristinamycine IB sous forme d'un solide blanc fondant à 258°C (déc.).

Spectre de R.M.N. ¹H (300 MHz, CDCl₃, δ en ppm) : 2,65 (s, 3H : ArNCH₃) ; 2,98 (dd, J = 12 et 4 Hz, 1H : 1H du CH₂ en 4 β) ; 3,23 (s, 3H : NCH₃) ; 3,33 (t, J = 12 Hz, 1 H : l'autre H du CH₂ en 4 β) ; 4,13 (s, 2H : ArNCH₂) ; 5,19 (dd, J = 12 et 4 Hz, 1H : 4 α) ; 7,00 (d, J = 8 Hz, 1H : H aromatique en 4ε) ; 7,08 (dd, J = 8 et 1,5 Hz, 1H : H aromatique en 4δ) ; de 7,15 à 7,40 (mt, 2H : H aromatique en 4δ et en ortho du Cl et H en 5 de la pyridine) ; 7,80 (mt, 1H : H en 4 de la pyridine) ; 8,55 (d large, J = 6 Hz; 1H : H en 6 de la pyridine) ; 8,65 (s large, 1H : H en 2 de la pyridine).

### Exemple Y

On place dans un tricol maintenu sous atmosphère d'azote 2 g de pristinamycine IB dans 6 cm3 de diméthylformamide sec puis on ajoute 1,46 g de bromhydrate de bromoacétate de 4-pyridylméthyle et 0,33 cm3 de triéthylamine. Le mélange est agité 18 heures à 60°C. Le mélange réactionnel est refroidi est versé sur 100 cm3 d'eau distillée puis extrait par 4 fois 30 cm3 d'acétate d'éthyle. La phase aqueuse est décantée puis la phase organique relavée par 3 fois 10 cm3 d'eau distillée, décantée puis séchée sur sulfate de magnésium, filtrée puis concentrée sous pression réduite (2,7 kPa) pour donner 1,2 g d'un solide jaune pâle qui est purifié par chromatographie flash (éluant : dichlorométhane : méthanol 97/3) pour donner 0,53 g d'un solide qui est repurifié par CLHP pour donner 197 mg de (4-N-pyridylméthoxycarbonylméthyl) pristinamycine IB sous forme d'une poudre blanche fondant à 252°C.

Le bromhydrate de bromoacétate de 4-pyridylméthyle peut être préparé de la manière suivante :

On place dans un tricol maintenu sous atmosphère d'azote, 1,09 g de 4-hydroxyméthyl pyridine en solution dans 20 cm3 de chloroforme (sec sur amylène), puis on ajoute en 1 heure à température ambiante, 0,88 cm3 de bromure de bromo acétyle en solution dans 2 cm3 de chloroforme. Après 24 heures d'agitation, on ajoute 10 % bromure de bromo acétyle supplémentaires puis l'agitation est poursuivie 24 heures. Le mélange réactionnel est filtré, repris au chloroforme puis à l'éther. Le solide résultant est séché sous pression réduite pour donner 2,1 g d'un solide qui est utilisé tel quel dans l'étape suivante.

Spectre de R.M.N. 1H (400 MHz, CDCl₃, δ en ppm) : 2,92 (dd, J = 12 et 4 Hz, 1H : 1H du CH₂ en 4 β) ; 3,08 (s, 3H : ArNCH₃) ; 3,27 (s, 3H : NCH₃) ; 3,33 (t, J = 12 Hz, 1H : l'autre H du CH₂ en 4β) ; 4,17 (s, 2H : ArNCH₂) ; 5,19 (s, 2H : COOCH₂) ; 5,25 (dd, J = 12 et 4 Hz, 1H : 4 α) ; 6,67 (d, J = 8,5 Hz, 2H : H aromatiques en 4ε) ; 7,07 (d, J = 8,5 Hz, 2H : H aromatiques en 4δ) ; 7,22 (d, J = 5,5 Hz, 2H : H β de la pyridine) ; 8,59 (d, J = 5,5 Hz, 2H : H α de la pyridine).

### Exemple Z

En opérant comme à l'exemple Y mais à partir de 1,5 g de pristinamycine **IB** et de 640 mg de bromhydrate de N-méthyl N-(1-méthylpipérid-4-yl) bromoacétamide dans 4,5 cm3 de diméthylformamide sec et après 72 heures d'agitation à température ambiante, on obtient après évaporation d'une partie du diméthylformamide à 50°C sous pression partielle, une solution qui est reprise par 15 cm3 d'eau distillée. Le mélange réactionnel est lavé par 2 fois 15 cm3 d'acétate d'éthyle. La phase aqueuse est décantée, ajustée à pH 5-6, relavée à l'acétate d'éthyle puis alcalinisée à pH 8 par soude 0,1N. La phase aqueuse est additionnée de chlorure de sodium puis extraite par 15 cm3 de chlorure de méthylène. La phase organique est lavée par 2 cm3 d'eau, décantée, séchée sur sulfate de magnésium, filtrée puis concentrée sous pression réduite (2,7 kPa) pour donner 1,1 g d'un solide jaune pâle qui est mis en solution dans 30 cm3 d'un mélange chlorure de méthylène / méthanol / ammoniaque concentré (70/20/1 en volumes) puis additionné de 5,5 g de silice. Après 45 minutes d'agitation, le mélange est filtré, rincé par 2 fois le même volume de mélange de solvants puis concentré à sec. Le produit obtenu est concrété dans 15 cm3 d'éther puis filtré pour donner 680 mg de [N-(1-(méthyl pipérid-4yl) N-méthyl amino carbonylméthyl] pristinamycine IB sous forme d'un solide blanc fondant à 210°C.

Spectre de R.M.N. ¹ H (400 MHz, CDCl₃, δ en ppm) : de 1,60 à 2,10 et de 2,75 à 3,00 (2 mts, respectivement 6H et 2H : CH₂CH₂N de la pipéridine) ; 2,30 (s, 3H : NCH₃ de la pipéridine) ; 2,85 (s, 3H : CONCH₃) ; de 2,80 à 3,00 (mt, 1H : 1H du CH₂ en 4 β) ; 3,00 (s, 3H : ArNCH₃) ; 3,22 (s, 3H : NCH₃) ; 3,28 (t, J = 12 Hz, 1H : l'autre H du CH₂ en 4 β) ; 4,04 (s, 2H : ArNCH₂) ; 4.45 (mt, 1H : CONCH) ; 5,25 (dd, J = 12 et 4 Hz, 1H : 4 α) ; 6,60 (d, J = 8,5 Hz, 2H : H aromatiques en 4ε) ; 7,00 (d, J = 8,5 Hz, 2H : H aromatiques en 4δ).

Le bromhydrate de N-méthyl N-(1-méthylpipérid-4-yl) bromoacétamide peut être obtenu de la manière suivante :

On place dans un tricol maintenu sous azote à 5°C 1,45 g de 1-méthyl 4-méthyl amino pipéridine dans 30 cm3 de diméthylformamide sec puis on ajoute en 1 heure, 0,95 g de bromure de bromoacétyle en solution dans 10 cm3 de chloroforme. Après 18 heures à température ambiante, le mélange réactionnel est concentré, le résidu repris avec 30 cm3 d'éther puis agité 3 heures. Le solide résultant est filtré, lavé à l'éther puis séché sous pression réduite (2,7 kPa) pour donner 3,2 g de bromhydrate de N-méthyl N-(1-méthylpipérid-4-yl) bromoacétamide sous forme d'un solide jaune pâle qui est utilisé tel quel.

### Exemple AA

En opérant comme à l'exemple Y mais à partir de 2,4 g de pristinamycine IB et de 0,91 g de (1-éthoxycarbonylpipérid-4-yl) bromoacétamide dans 7,5 cm3 de diméthylformamide sec et après 96 heures d'agitation à température ambiante, on obtient une solution qui est diluée par 80 cm3 d'eau distillée. Le mélange est ajusté à pH 8 par du bicarbonate de sodium, additionné de chlorure de sodium puis extrait par 2 fois 20 cm3 d'acétate d'éthyle. La phase aqueuse est décantée puis réextraite par 2 fois 20 cm3 d'acétate d'éthyle. Les phases organiques sont réunies, séchées sur sulfate de magnésium, filtrées puis concentrées sous pression réduite (2,7 kPa) pour donner un solide jaune pâle qui est repris dans l'éther pour donner après filtration et séchage 2,6 g d'une poudre jaune pâle qui est purifiée par chromatographie flash (éluant : dichlorométhane : méthanol 97/3) pour donner 1,1 g de [N-(1-éthoxycarbonyl pipérid-4-yl) amino carbonylméthyl] pristinamycine IB sous forme d'un solide blanc fondant à 195°C.

Spectre de R.M.N. ¹H (400 MHz, CDCl₃, δ en ppm) : 1,23 (t, J = 7 Hz, 3H : CH₃ de l'éthyle) ; de 1,20 à 1,50 et de 1,70 à 1,95 (2 mts, 2H chacun : CH₂ de la pipéridine) ; 2,85 et de 3,90 à 4,15 (respectivement mt et mf, respectivement 2H et 3H : NCH₂ et NCH de la pipéridine) ; 2,95 (dd, J = 12,5 et 4 Hz, 1H : 1H du CH₂ en 4 β) ; 2,97 (s, 3H : ArNCH₃) ; 3,25 (s, 3H : NCH₃); 3,34 (t, J = 12,5 Hz, 1H : l'autre H du CH₂ en 4 β) ; 3,79 et 3,90 (2 d, J = 18 Hz, 1H chacun : ArNCH₂) ; 4,20 (q, J = 7 Hz, 2H : COOCH₂ de l'éthyle) ; 5,19 (dd, J = 12,5 et 4 Hz, 1 H : 4 α) ; 6,55 (mt, 1H : CONH) ; 6,63 (d, J = 8 Hz, 2H: H aromatiques en 4ε) ; 7,15 (d, J = 8 Hz, 2H : H aromatiques en 4δ).

La (1-éthoxycarbonylpipérid-4-yl) bromoacétamide peut être préparée de la manière suivante :

On place dans un tricol maitenu sous azote, 860 mg de 1-éthoxycarbonyl 4-aminopipéridine, puis 15 cm3 de chloroforme sec (sur amylène) et 0,84 cm3 de triéthylamine. Le mélange est refroidi à 5°C puis on ajoute en 45 minutes, 0,48 cm3 de bromure de bromoacétyle en solution dans 2 cm3 de chloroforme sec et l'agitation est poursuivie 5 heures à température ambiante. Le chloroforme est évaporé sous pression réduite et le mélange repris par 20 cm3 d'acétate d'éthyle et 120 cm3 d'eau distillée. La phase organique est décantée, lavée par 2 fois 5 cm3 d'eau, puis séchée sur sulfate de magnésium, filtrée, concentrée sous pression réduite (2,7 kPa) pour donner un solide jaune pâle qui est repris dans l'éther pour donner après filtration et séchage 970 mg de (1-éthoxycarbonylpipérid-4-yl) bromoacétamide sous forme d'une poudre blanche qui est utilisée telle quelle.

### Exemple AB

En opérant comme à l'exemple Y mais à partir de 3 g de pristinamycine IB et de 1,53 g de bromhydrate de N-(1-benzylpipérid-4-yl) bromoacétamide dans 9 cm3 de diméthylformamide sec et après 72 heures d'agitation à température ambiante, on obtient une solution qui est diluée par 120 cm3 d'eau distillée. Le mélange est ajusté à pH 8, puis extrait par 3 fois 30 cm3 d'acétate d'éthyle. La phase organique est décantée, lavée par 30 cm3 d'eau, puis séchée sur sulfate de magnésium, filtrée puis concentrée sous pression réduite (2,7 kPa) pour donner un solide jaune pâle qui est repris dans l'éther pour donner après filtration et séchage 3,3 g d'une poudre blanche qui est purifiée par chromatographie flash (éluant : dichlorométhane : méthanol 97/3) pour donner 0,95 g de [(1-benzyl pipérid-4-yl) amino carbonylméthyl] pristinamycine IB sous forme d'un solide blanc fondant à 195°C.

Spectre de R.M.N. ¹ H (300 MHz, CDCl₃, δ en ppm) : de 1,35 à 1,65 et de 1,95 à 2,20 (2 mts, 2H chacun : CH₂ de la pipéridine) ; de 2,70 à 2,85 et de 3,25 à 3,40 (2 mts, 2H : NCH₂ de la pipéridine) ; 2,95 (dd, J = 12 et 4 Hz, 1H : 1H du CH₂ en 4 β) ; 2,97 (s, 3H : ArNCH₃) ; 3,26 (s, 3H : NCH₃) ; 3,35 (t, J = 12 Hz, 1H : l'autre H du CH₂ en 4 β) ; 3,47 (s, 2H : NCH₂Ar) ; 3,80 et 3,90 (2 d, J = 18 Hz, 1H chacun : ArNCH₂) ; de 3,75 à 3,95 (mt, 1H : NCH de la pipéridine) ; 5,25 (dd, J = 12 et 4 Hz, 1H : 4 α) ; 6,50 (d, J = 7,5 Hz, 1H : CONH) ; 6,65 (d, J = 8,5 Hz, 2H : H aromatiques en 4ε) ; 7,15 (d, J = 8,5 Hz, 2H : H aromatiques en 4δ) ; de 7,15 à 7,40 (mt, 5H : H aromatiques du benzyle).

Le bromhydrate de N-(1-benzylpipérid-4-yl) bromoacétamide peut être obtenu de la manière suivante :

On place dans un tricol maintenu sous azote, 950 mg de 4-amino 1-benzyl pipéridine, puis 15 cm3 de chloroforme sec (sur amylène). Le mélange est refroidi à 5°C puis on ajoute en 45 minutes, 0,47 cm3 de bromure de bromoacétyle en solution dans 5 cm3 de chloroforme sec et l'agitation est poursuivie 30 minutes à 5°C. Le chloroforme est évaporé sous pression réduite et le mélange est repris par 15 cm3 d'éther pour donner après filtration et séchage 2 g de bromhydrate de N-(1-benzylpipérid-4-yl) bromoacétamide sous forme d'une poudre blanche qui est utilisée telle quelle.

### Exemple AC

On place dans un tricol maintenu sous azote 605 mg de [(1-benzyl pipérid-4-yl) aminocarbonylméthyl] pristinamycine IB dans 12 cm3 de méthanol et 6 cm3 de dichlorométhane, 120 mg de palladium sur charbon à 10%, puis 0,22 cm3 d'éther chlorhydrique 2,5N. Le mélange est placé sous atmosphère d'hydrogène à 18°C puis chauffé à 33°C. Après 3 jours, le mélange est purgé à l'azote, filtré sur Clarcel®, concentré sous pression réduite puis repris par 15 cm3 d'eau. La solution est ajustée à pH 8 par de la soude 1N, additionnée de chlorure de sodium, puis extraite au dichlorométhane. La phase organique est décantée, lavée avec de l'eau saturée en chlorure de sodium, séchée sur sulfate de magnésium, filtrée puis concentrée sous pression réduite (2,7 kPa) pour donner un solide qui est agité 18 heures dans 11,2 cm3 d'acide chlorhydrique 0,1N. Le milieu est ajusté à pH 8 par addition de 11,2 cm3 de soude 0,1N puis additionné de 3,6 g de chlorure de sodium. Après 2 heures d'agitation, le précipité est filtré, rincé avec un minimum d'eau glacée, puis repris dans l'éther. Le solide est repris au dichlorométhane, séché sur sulfate de manésium, filtré puis séché à 35°C sous pression réduite (90Pa) pour donner 270 mg de [(4-pipéridinyl) amino carbonylméthyl] pristinamycine IB sous forme d'un solide crème fondant à 230°C.

Spectre de R.M.N. ¹H (400 MHz, CDCl₃, δ en ppm) : de 1,45 à 1,65 et de 1,80 à 2,00 (2 mts, 2H chacun : CH₂ de la pipéridine) ; de 2,65 à 2,85 et de 3,05 à 3,25 (2 mts, 2H chacun : NCH₂ de la pipéridine) ; 2,95 (dd, J = 12 et 4 Hz, 1H : 1 H du CH₂ en 4 β) ; 2,98 (s, 3H : ArNCH₃) ; 3,27 (s, 3H : NCH₃) ; 3,32 (t, J = 12 Hz, 1 H : l'autre H du CH₂ en 4 β) ; 3,80 et 3,88 (2 d, J = 18 Hz, 1H chacun : ArNCH₂) ; 3,95 (mt, 1H : CONCH de la pipéridine) ; 5,22 (dd, J = 12 et 4 Hz, 1H : 4 α) ; 6,63 (d, J = 8,5 Hz, 2H : H aromatiques en 4ε) ; 6,68 (d, J = 8 Hz, 1H : CONH) ; 7,10 (d, J = 8,5 Hz, 2H : H aromatiques en 4δ).

### Exemple AD

On place dans un tricol maintenu sous atmosphère d'azote 15 g de pristinamycine IA dans 30 cm3 de diméthylformamide sec puis on ajoute 2,2 cm3 de bromoacétate d'éthyle. Le mélange est agité 22 heures à 80°C. Après refroidissement, le mélange réactionnel est dilué par 300 cm3 d'eau distillée puis agité. Le précipité formé est filtré, rincé par 3 fois 50 cm3 d'eau distillée puis à l'éther. Le solide résultant est solubilisé dans l'acétate d'éthyle, filtré puis lavé en ampoule à décanter par 3 fois 50 cm3 d'eau distillée. La phase organique est décantée, séchée sur sulfate de magnésium,filtrée puis concentrée sous pression réduite (2,7 kPa) pour donner 7,2 g d'une huile brune qui est purifiée par chromatographie flash (éluant : dichlorométhane / méthanol 98/2) pour donner 3,2 g 4-N-(éthoxycarbonylméthyl) pristinamycine IB sous forme d'un solide blanc fondant à 244°C.

Spectre de R.M.N. ¹H (400 MHz, CDCl₃, δ en ppm) : 1,28 (t, J = 7 Hz, 3H : CH₃ de l'éthyle) ; 2,90 (dd, J = 12,5 et 4 Hz, 1 H : 1H du CH₂ en 4β) ; 3,05 (s, 3H : ArNCH₃); 3,26 (s, 3H : NCH₃) ; 3,34 (t, J = 12,5 Hz, 1H : l'autre H du CH₂ en 4 β) ; 4,02 et 4,08 (2 d, J = 18 Hz, 1H chacun : ArNCH₂) ; 4,20 (q, J = 7 Hz, 2H : CH₂ de l'éthyle) ; 5,22 (dd, J = 12,5 et 4 Hz, 1 H : 4 α) ; 6,62 (d, J = 8,5 Hz, 2H : H aromatiques en 4ε) ; 7,07 (d, J = 8,5 Hz, 2H : H aromatiques en 4δ).

### Exemple AE

En opérant comme à l'exemple AD, mais à partir de 1,5 g de pristinamycine IA dans 3 cm3 de diméthylformamide sec et de 240 mg de bromo acétonitrile, on obtient après 6 heures à 80°C, 0,8 g d'un solide blanc qui est purifié par chromatographie flash (éluant : dichlorométhane / méthanol 97/3) pour donner 0,48 g de 4-N-cyanométhyl pristinamycine IB sous forme d'un solide blanc fondant à 258°C.

Spectre de R.M.N. ¹H (300 MHz, CDCl₃, δ en ppm) : 2,95 (dd, J = 12 et 4 Hz, 1H : 1H du CH₂ en 4 β) ; 2,97 (s, 3H : ArNCH₃) ; 3,20 (s, 3H : NCH₃) ; 3,32 (t, J = 12 Hz, 1H : l'autre H du CH₂ en 4β) ; 4,10 (AB limite, J = 18 Hz, 2H : ArNCH₂) ; 5,23 (dd, J = 12 et 4 Hz, 1H : 4 α) ; 6,75 (d, J = 8 Hz, 2H : H aromatiques en 4ε) ; 7,09 (d, J = 8 Hz, 2H : H aromatiques en 4δ).

### Exemple AF

La 5δ-méthylène pristinamycine I_{B} peut être obtenue de la manière suivante.

On place dans un tricol maintenu sous atmosphère d'azote, 10 cm3 de méthanol et 1 cm3 de morpholine puis on ajoute lentement 0,6 cm3 d'acide méthanesulfonique en maintenant la température en dessous de 20°C. On ajoute ensuite sous agitation 0,17g de polyoxyméthylène puis 1 g de pristinamycine I_{B}. La suspension laiteuse obtenue est chauffée 4 heures à 40°C puis agitée 12 heures à température ambiante. Le mélange est concentré à sec, repris par 20 cm3 d'acétate d'éthyle et 20 cm3 d'eau distillée, filtré sur Clarcel® puis décanté. La phase aqueuse est extraite par 2 fois 10 cm3 d'acétate d'éthyle puis les phases organiques sont réunies, lavées par 30 cm3 d'une solution aqueuse de chlorure de sodium, décantées, séchées sur sulfate de sodium puis concentrées sous pression réduite (2,7 kPa) juqu'à un volume de 50 cm3. La phase organique de 50 cm3 ainsi concentrée est ajoutée dans un tricol sous agitation à 35 cm3 d'eau distillée, 1,3 cm3 d'acide acétique et 0,16 g d'acétate de sodium trihydraté. Le mélange est chauffé 3 heures à 40-45°C puis après refroidissement on ajoute une solution saturée de bicarbonate de sodium jusqu'à un pH de 5-6. La phase aqueuse est décantée, extraite par 20 cm3 d'acétate d'éthyle puis les phases organiques sont rassemblées et lavées par 30 cm3 d'eau distillée bicarbonatée. La phase aqueuse est décantée puis extraite par 20 cm3 d'acétate d'éthyle. Toutes les phases organiques sont réunies, lavées par une solution aqueuse saturée en chlorure de sodium, séchées sur sulfate de sodium, filtrées et concentrées à sec sous pression réduite pour donner 1,03 g d'un solide qui est purifié par deux chromatographies flash successives (éluant: chlorure de méthylène / méthanol 96/4) pour donner 0,21 g d'un produit qui est concrété dans 5 cm3 d'éther diéthylique. Après filtration et séchage à 50°C sous pression réduite (90 Pa). On obtient 169 mg de 5δ-méthylène pristinamycine IB sous forme d'un solide blanc cassé fondant à 210°C (peu net).

Spectre de R.M.N. ¹H (300 MHz, CDCl₃, δ en ppm). 0,66 (dd, J = 16,5 et 6 Hz, 1H : 1H du CH₂ en 5 β) ; 0,91 (t, J = 7,5 Hz, 3H : CH₃ en 2γ) ; de 1,15 à 1,35 (mt, 2H: 1H du CH₂ en 3 β et 1H du CH₂ en 3 γ) ; 1,33 (d, J = 7 Hz, 3H : CH₃ en 1 γ) ; de 1,50 à 1,85 (mt : 3H correspondant à l'autre H du CH₂ en 3 γ et au CH₂ en 2 β) ; 2,03 (mt, 1H : l'autre H du CH₂ en 3 β) ; 2,50 (d, J = 16,5 Hz, 1H : l'autre H du CH₂ en 5 β) ; 2,81 (s, 3H : ArNCH₃) ; 2,88 (dd, J = 12 et 4,5 Hz, 1H : 1H du CH₂ en 4 β); de 3,20 à 3,35 (mt, 2H : 1 H du CH₂ en 3 δ et l'autre H du CH₂ en 4 β) ; 3,26 (s, 3H : NCH₃) ; 3,52 (mt, 1H : l'autre H du CH₂ en 3 δ) ; 3,59 (d large, J = 16,5 Hz, 1H : 1H du CH₂ en 5 ε) ; de 3,65 à 3,90 (mf étalé, 1H : ArNH) ; 4,60 (dd, J = 9 et 6 Hz, 1H : CH en 3 α) ; 4,82 (mt, 1H : CH en 2 α) ; 4,88 (dd, J = 10 et 1 Hz, 1H : CH en 1α) ; 5,05 (dd, J = 12 et 4,5 Hz, 1H : CH en 4 α) ; 5,28 (d large, J = 16,5 Hz, 1H : l'autre H du CH₂ en 5 ε) ; 5,28 (d, J = 6 Hz, 1H : CH en 5 α) ; 5,35 et 6,17 (2 s larges, 1H chacun : =CH₂) ; 5,84 (d, J = 9 Hz, 1 H : CH en 6 α) ; 5,90 (dq, J = 7 et 1 Hz, 1H : CH en 1β) ; 6,46 (d, J = 8 Hz, 2H : H aromatiques en 4 ε) ; 6,50 (d, J = 10 Hz, 1H : CONH en 2) ; 6,91 (d, J = 8 Hz, 2H : H aromatiques en 4 δ); de 7,15 à 7,35 (mt : les 5H aromatiques en 6) ; 7,47 (AB limite, 2H : 1' H₄ et 1' H₅) ; 7,82 (dd, J = 4 et 2 Hz, 1H : 1' H₆) ; 8,38 (d, J = 10 Hz, 1H : CONH en 1); 8,73 (d, J = 9 Hz, 1H : CONH en 6) ; 11,60 (s, 1H : OH).

Les produits des exemples ci-dessus peuvent être traités par analogie avec les méthodes décrites dans les exemples 1 à 33 pour préparer des dérivés de streptogramine de formule générale (I).

La présente invention concerne également les compositions pharmaceutiques contenant au moins un dérivé de streptogramine selon l'invention, à l'état pur, associé à au moins un dérivé de streptogramine du groupe A, le cas échéant sous forme de sel, et/ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 1 et 3 g de produit actif en 2 ou 3 prises par jour, par voie orale pour un adulte.

L'exemple suivant illustre une composition selon l'invention.

### EXEMPLE

On prépare selon la technique habituelle des comprimés dosés à 250 mg de produit actif, ayant la composition suivante :
- 2"-méthyl-pyrido [2,3-5γ,5δ] pristinamycine IE 75 mg
- pristinamycine II_{B} 175 mg
- excipient : amidon, silice hydratée, dextrine,
   gélatine, stéarate de magnésium : qsp 500 mg

## Revendications

1. Un dérivé du groupe B des streptogramines de formule générale : 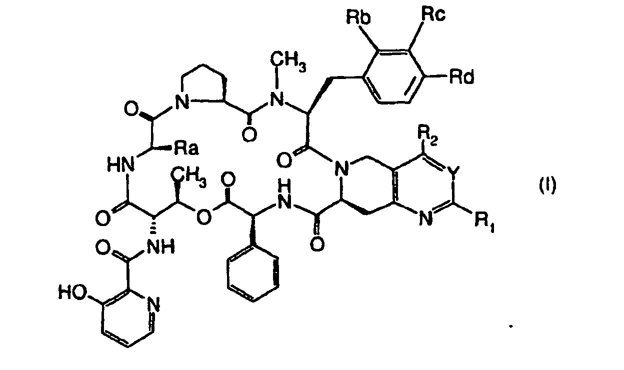 dans laquelle
Y est un atome d'azote ou un radical =CR₃-,
R₁ est un atome d'hydrogène, un radical alcoyle de 1 à 8 carbones, alcényle de 2 à 8 carbones, cycloalcoyle de 3 à 8 carbones, hétérocyclyle saturé ou insaturé de 3 à 8 chaînons, phényle, phényle substitué - par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino - ou un radical NR'R", R' et R" identiques ou différents pouvant être des atomes d'hydrogène ou des radicaux alcoyle de 1 à 3 carbones, ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué - par un radical alcoyle, alcényle de 2 à 8 carbones, cycloalcoyle de 3 à 6 carbones, hétérocyclyle saturé ou insaturé de 4 à 6 chaînons, benzyle, phényle ou phényle substitué tel que défini ci-dessus pour la définition de R₁ -,
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R", n étant un entier de 1 à 4 et R' et R" étant définis comme ci-dessus, ou bien si R₃ est un atome d'hydrogène, R₁ peut être aussi formyle, carboxy, alcoyloxycarbonyle, ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle, ou un radical NH et R° est un radical alcoyle de 1 à 8 carbones, cycloalcoyle de 3 à 6 carbones, hétérocyclyle saturé ou insaturé de 3 à 8 chaînons, hétérocyclylméthyle de 3 à 8 chaînons dont la partie hétérocyclyle est attachée au radical méthyle par un atome de carbone, phényle, phényle substitué - par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino - ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle de 1 à 3 carbones,
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle de 3 à 5C, et Rd est un radical -NMe-R"' pour lequel R"' représente un radical alcoyle, hydroxyalcoyle de 2 à 4C, ou alcényle de 2 à 8C éventuellement substitué par phényle, cycloalcoyl de 3 à 6C méthyle, benzyle, benzyle substitué - par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino- hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué - par un radical alcoyle, alcényle de 2 à 8 carbones, cycloalcoyle de 3 à 6 carbones, hétérocyclyle saturé ou insaturé de 4 à 6 chaînons ; phényle, phényle substitué tel que défini ci-avant pour la définition de R₁ ou benzyle - ou bien R"' représente un radical cyanométhyle, ou -CH₂CORe pour lequel soit Re est -OR'e, R'e étant hydrogène, alcoyle de 1 à 6 carbones, alcényle de 2 à 6 carbones, benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle de 3 à 5C, si Rd est -N(CH₃)₂,
4) Rb et Rd sont des atomes d'hydrogène et Rc est un atome d'halogène, ou un radical alcoylamino ou dialcoylamino, alcoyloxy, trifluorométhoxy, thioalcoyle, alcoyle de 1 à 6C ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle de 1 à 6C, phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle de 1 à 3C, et Rd est un atome d'halogène ou un radical amino, alcoylamino ou dialcoylaxnino, alcoyloxy ou trifluorométhoxy, thioalcoyle, alcoyle de 1 à 6C ou trihalogénométhyle,
7) Rc est un atome d'hydrogène et Rb et Rd représentent un radical méthyle,
les radicaux alcoyle, alcényle ou acyle étant droits ou ramifiés et, sauf mention spéciale, les radicaux alcoyle ou acyle contenant 1 à 4 atomes de carbone, ainsi que ses sels lorsqu'ils existent.

2. Un dérivé du groupe B des streptogramines selon la revendication 1, pour lequel
Y est un atome d'azote ou un radical =CR₃-,
R₁ est un atome d'hydrogène, un radical alcoyle de 1 à 8 carbones, cycloalcoyle de 3 à 8 carbones, hétérocyclyle saturé ou insaturé de 3 à 8 chaînons, phényle, phényle substitué - par un ou plusieurs radicaux amino, alcoylamino ou dialcoylamino - ou un radical NR'R", R' et R" identiques ou différents pouvant être des atomes d'hydrogène ou des radicaux alcoyle de (1 à 3 carbones, ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué par un radical alcoyle,
ou bien lorsque Y est un radical =CR₃-, R₁ peut être aussi halogénométhyle, hydroxyméthyle, alcoylthiométhyle dont la partie alcoyle est éventuellement substituée par NR'R", alcoylsulfinylméthyle, alcoylsulfonylméthyle, acyloxyméthyle, cyclopropylaminométhyle ou -(CH₂)ₙNR'R", n étant un entier de 1 à 4 et R' et R" étant définis comme ci-dessus, ou bien si R₃ est un atome d'hydrogène, R₁ peut être aussi formyle ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle, ou un radical NH et R° est un radical alcoyle de 1 à 8 carbones, hétérocyclylméthyle de 3 à 8 chaînons dont la partie hétérocyclyle est attachée au radical méthyle par un atome de carbone, ou un radical -(CH₂)ₙNR'R" pour lequel R' et R" sont définis comme ci-dessus et n est un entier de 2 à 4,
R₂ est un atome d'hydrogène ou un radical alcoyle de 1 à 3 carbones,
R₃ est un atome d'hydrogène ou un radical carboxy ou alcoyloxycarbonyle,
Ra est un radical méthyle ou éthyle, et
Rb, Rc et Rd ont les définitions ci-après :
• Rb et Rc sont des atomes d'hydrogène et Rd est un atome d'hydrogène ou un radical méthylamino ou diméthylamino,
• Rb est un atome d'hydrogène, Rd est un radical -NHCH₃ ou -N(CH₃)₂ et Rc est un atome de chlore ou de brome,
ainsi que ses sels lorsqu'ils existent.

3. Un dérivé du groupe B des streptogramines selon la revendication 1, pour lequel
Y est un atome d'azote ou un radical =CR₃-,
R₁ est un atome d'hydrogène, un radical alcoyle de 1 à 3 carbones, cycloalcoyle de 3 à 8 carbones, hétérocyclyle saturé ou insaturé de 3 à 8 chaînons, phényle, phényle substitué par un radical amino,
ou bien lorsque Y est un radical =CR₃-, R₁ peut être aussi acyloxyméthyle,
ou bien lorsque Y est un atome d'azote, R₁ peut être aussi un radical -XR° pour lequel X est un atome d'oxygène ou de soufre ou un radical NH et R° est un radical alcoyle
de 1 à 4 carbones ou un radical -(CH₂)ₙNR'R" pour lequel R' et R" identiques ou différents pouvent être des atomes d'hydrogène ou des radicaux alcoyle de 1 à 3 carbones, ou forment ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué par un radical alcoyle, et n est un entier de 2 à 4,
R₂ est un atome d'hydrogène ou un radical alcoyle de 1 à 3 carbones,
R₃ est un atome d'hydrogène ou un radical alcoyloxycarbonyle,
Ra est un radical méthyle ou éthyle, et
Rb, Rc et Rd ont les définitions ci-après :
• Rb et Rc sont des atomes d'hydrogène et Rd est un atome d'hydrogène ou un radical méthylamino ou diméthylamino,
• Rb est un atome d'hydrogène, Rd est un radical -NHCH₃ ou -N(CH₃)₂ et Rc est un atome de chlore,
ainsi que ses sels lorsqu'ils existent.

4. Un dérivé du groupe B des streptogramines selon la revendication 1, **caractérisé en ce qu'**il s'agit de la 2"-méthyl-pyrido [2,3-5γ,5δ] pristinamycine IE.

5. Un dérivé du groupe B des streptogramines selon la revendication 1, **caractérisé en ce qu'**il s'agit de la 2"-cyclopropyl-pyrido [2,3-5γ,5δ] pristinamycine IE.

6. Un dérivé du groupe B des streptogramines selon la revendication 1, **caractérisé en ce qu'**il s'agit de la pyrido [2,3-5γ,5δ] pristinamycine IE.

7. Un dérivé du groupe B des streptogramines selon la revendication 1, **caractérisé en ce qu'**il s'agit de la 2"-éthyl-pyrido [2,3-5γ,5δ] (4ζ-méthylamino) (4ζ-dèsdiméthylamino) pristinamycine IE.

8. Un dérivé du groupe B des streptogramines selon la revendication 1, **caractérisé en ce qu'**il s'agit de la 4ε-chloro 2"-(éthyl)-pyrido [2,3-5γ,5δ] (4ζ-méthylamino) (4ζ-désdiméthylamino) pristinamycine IE.

9. Un procédé de préparation d'un dérivé de streptogramine selon la revendication 1, pour lequel Y est un radical =CR₃- et R₃ est autre qu'un radical alcoyle, **caractérisé en ce que** l'on fait agir un énamino ester de formule générale : 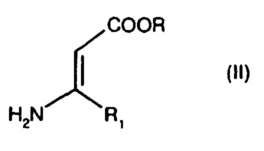 dans laquelle R₁ est défini comme précédemment et R représente le reste d'un ester facilement hydrolysable ou un radical alcoyle, sur le dérivé de méthylène-5δ pristinamycine correspondant, de formule générale : 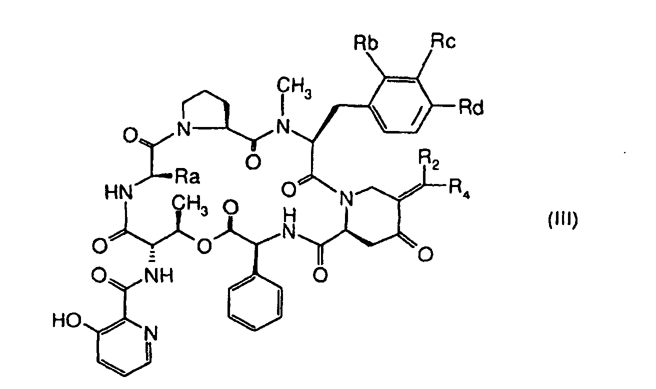 dans laquelle Ra, Rb, Rc et Rd sont définis comme pour la revendication 1, R₂ est défini comme pour la revendication 1 et R₄ est un atome d'hydrogène, ou R₂ représente un atome d'hydrogène et R₄ est un atome d'hydrogène ou un radical dialcoylamino, puis le cas échéant effectue la transformation de l'ester obtenu en un acide, puis éventuellement sa décarboxylation, ou la transformation de l'acide en un radical carbamoyle selon le dérivé selon la revendication 1 attendu, et/ou le cas échéant effectue la transformation du dérivé selon la revendication 1 pour lequel R₁ est hydroxyméthyle en un dérivé pour lequel R₁ est un radical formyle, puis le cas échéant carboxy, puis le cas échéant alcoyloxycarbonyle ou -CONR'R" et/ou éventuellement effectue la mono N-déméthylation du dérivé selon la revendication 1 pour lequel Rd est un radical diméthylamino en un dérivé pour lequel Rd est méthylamino, puis éventuellement transforme en un sel, lorsqu'ils existent.

10. Un procédé de préparation d'un dérivé de streptogramine selon la revendication 1, pour lequel Y est un radical =CR₃- et R₃ est un atome d'hydrogène ou un radical alcoyle, **caractérisé en ce que** l'on fait agir un sel de pyridinium de formule générale : 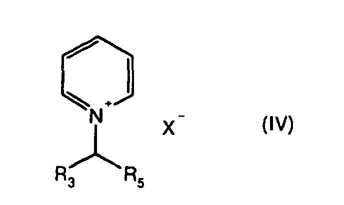 dans laquelle R₃ est défini comme ci-dessus, R₅ est le reste d'une cétone R₁-CO-pour lequel R₁ est défini comme ci-dessus à l'exception de représenter un radical -NR'R", ou représente éventuellement un radical hydroxy protégé ou un radical nitrophényle ou bien R₅ représente le radical cyano pour obtenir un dérivé de streptogramine pour lequel R₁ est un radical amino, et X⁻ est un anion, sur le dérivé de méthylène-5δ pristinamycine correspondant, de formule, générale (III) défini dans la revendication 2, dans laquelle R₄ est un atome d'hydrogène et Ra, Rb, Rc, Rd et R₂ sont définis comme pour la revendication 1, puis éventuellement libère le radical hydroxy ou le cas échéant réduit le radical nitrophényle pour obtenir un dérivé pour lequel R₁ est un radical aminophényle, ou éventuellement fait agir une amine de formule générale HNR'R" sur le dérivé de streptogramine selon la revendication 1 pour lequel R₁ est halogénométhyle, pour obtenir le dérivé correspondant pour lequel R₁ est un radical -CH₂NR'R", ou le cas échéant transforme le dérivé selon la revendication 1 pour lequel R₁ est hydroxyméthyle en un dérivé pour lequel R₁ est un radical fonmyle, puis le cas échéant carboxy, puis le cas échéant alcoyloxycarbonyle ou -CONR'R" et/ou éventuellement effectue la mono N-déméthylation du dérivé selon la revendication 1 pour lequel Rd est un radical diméthylamino en un dérivé pour lequel Rd est méthylamino, puis éventuellement transforme en un sel, lorsqu'ils existent.

11. Un procédé de préparation d'un dérivé de streptogramine selon la revendication 1, pour lequel Y est un atome d'azote, **caractérisé en ce que** l'on fait agir un sel d'une amidine ou d'un dérivé de l'isourée ou de l'isothiourée de formule générale : 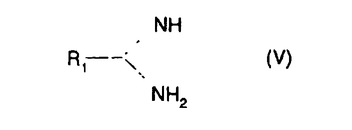 dans laquelle R₁ est défini comme pour la revendication 1, à l'exception de représenter un radical XR° pour lequel X est sulfonyle ou sulfinyle, ou un radical NR'R" autre qu'amino, sur un dérivé de streptogramine de formule générale (III) tel que défini dans la revendication 2, pour lequel R₄ est dialcoylamino, puis pour obtenir un dérivé de streptogramine selon la revendication 1, pour lequel R₁ est un radical XR° pour lequel X est sulfonyle ou sulfinyle, oxyde le dérivé correspondant pour lequel X est un atome de soufre, puis pour obtenir le dérivé de streptogramine selon la revendication 1, pour lequel R₁est un radical NR'R", substitue le dérivé sulfonyle obtenu par action de l'amine correspondante HNR'R" et/ou éventuellement pour obtenir un dérivé pour lequel Rd est méthylamino, effectue la mono N-déméthylation du dérivé selon la revendication 1, pour lequel Rd est un radical diméthylamino, puis éventuellement transforme en un sel, lorsqu'ils existent.

12. Un procédé de préparation d'un dérivé de streptogramine selon la revendication 1, pour lequel Y est un radical =CR₃-, R₁ est un atome d'hydrogène, un radical alcoyle, alcényle, cycloalcoyle, hétérocyclyle aromatique, phényle, phényle substitué, halogénométhyle, hydroxyméthyle, alcoyloxyméthyle, alcoylthiométhyle, alcoylsulfinylméthyle, alcoylsulfonylméthyle, ou - (CH₂)ₙNR'R", ou bien lorsque R₃ est un atome d'hydrogène, pour lesquels R₁ est formyle, carboxy, alcoyloxycarbonyle, ou -CONR'R" tels que définis pour la revendication 1 et R₂ est un atome d'hydrogène, **caractérisé en ce que** l'on fait agir la formyl énamine de formule générale : 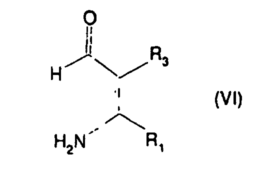 dans laquelle R₁ est un atome d'hydrogène, un radical alcoyle, alcényle, cycloalcoyle, hétérocyclyle aromatique, phényle, phényle substitué, hydroxyméthyle, alcoyloxyméthyle, alcoylthiométhyle, ou -(CH₂)ₙNR'R" et R₃ est défini comme pour la revendication 1 à l'exception de représenter carboxy, sur un dérivé de streptogramine de formule générale : 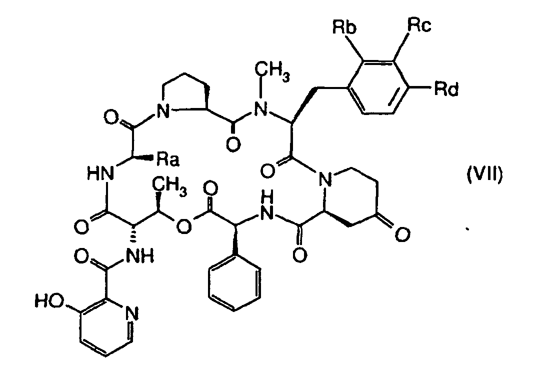 dans laquelle Ra, Rb, Rc et Rd sont définis comme dans la revendication 1, puis le cas échéant effectue la transformation du dérivé pour lequel R₃ est amide ou ester en un dérivé pour lequel R₃ est carboxy et/ou le cas échéant effectue l'oxydation du dérivé pour lequel R₁ est alcoylthiométhyle en un dérivé pour lequel R₁ est alcoylsulfinylméthyle ou alcoylsulfonylméthyle, ou le cas échéant effectue la transformation du dérivé pour lequel R₁ est un radical hydroxyméthyle en un dérivé pour lequel R₁ est halogénométhyle, puis le cas échéant effectue la transformation du dérivé pour lequel R₁ est halogénométhyle en un dérivé pour lequel R₁ est -CH₂NR'R", ou le cas échéant effectue la transformation du dérivé selon la revendication 1, pour lequel R₁ est hydroxyméthyle en un dérivé pour lequel R₁ est un radical formyle, puis le cas échéant carboxy, alcoyloxycarbonyle et/ou -CONR'R", et/ou éventuellement effectue la mono N-déméthylation du dérivé selon la revendication 1, pour lequel Rd est un radical diméthylamino en un dérivé pour lequel Rd est méthylamino, puis éventuellement transforme en un sel, lorsqu'ils existent.

13. Un procédé de préparation d'un dérivé de streptogramine selon la revendication 1, pour lequel Rd est méthylamino, **caractérisé en ce que** l'on effectue la mono N-déméthylation du dérivé selon la revendication 1, pour lequel Rd est un radical diméthylamino, puis éventuellement transforme le dérivé de streptogramine obtenu en un sel.

14. Un dérivé de streptogramine de formule générale : 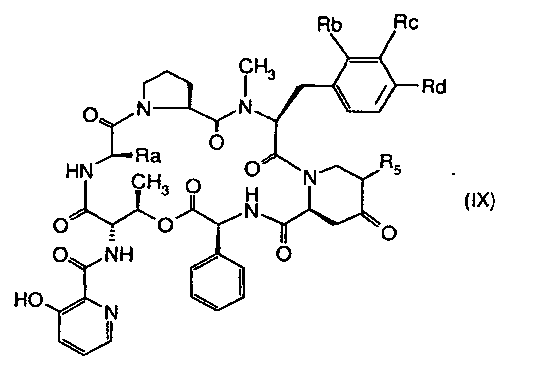 dans laquelle Ra est un radical méthyle et Rb, Rc, et Rd sont définis comme dans la revendication 1, ou Ra est un radical éthyle et Rb, Rc, et Rd sont définis comme dans la revendication 1 en 2) à 7) et R₅ représente un radical méthylènyle disubstitué de structure 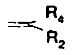 pour lequel R₂ et R₄ sont définis comme précédemment, ou bien dans laquelle Ra, Rb, Rc et Rd sont définis comme pour la revendication 1 en 2), à l'exception pour R"' de représenter éthyle si Rb et Rc sont hydrogène, et R₅ est un atome d'hydrogéne.

15. Compositions pharmaceutiques comprenant un dérivé de streptogramine du groupe B selon la revendication 1, à l'état pur ou sous forme d'association avec au moins un dérivé de streptogramine du groupe A, le cas échéant sous forme de sel, et/ou sous forme d'association avec un ou plusieurs diluants ou adjuvants compatibles et pharmaceutiquement acceptables.

16. Compositions pharmaceutiques selon la revendication 15, **caractérisées en ce que** le dérivé de la streptogramine du groupe A est choisi parmi la pristinamycine IIA, la pristinamycine IIB, la pristinamycine IIC, la pristinamycine IID, la pristinamycine IIE, la pristinamycine IIF, la pristinamycine IIG ou parmi des dérivés d'hémisynthèse connus ou parmi les dérivés de formule générale : 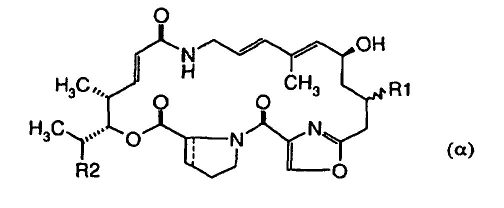 dans laquelle R₁ est un radical -NR'R" pour lequel R' est un atome d'hydrogène ou un radical méthyle, et R" est un atome d'hydrogène, un radical alcoyle, cycloalcoyle, allyle, propargyle, benzyle, ou -OR"', R"' étant un atome d'hydrogène, un radical alcoyle, cycloalcoyle, allyle, propargyle ou benzyle, ou -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double, ainsi que leurs sels.

17. Associations d'un dérivé de la streptogramine du groupe B, selon la revendication 1, avec au moins un un dérivé de la streptogramine du groupe A tel que défini dans la revendication 16.

## Patentansprüche

1. Derivat aus der Gruppe B der Streptogramine der allgemeinen Formel: 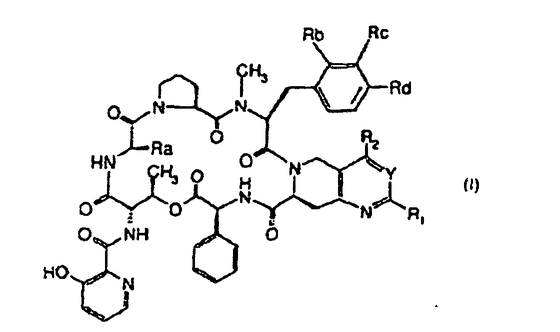 wobei
Y ein Stickstoffatom oder ein Rest =CR₃- ist,
R₁ ein Wasserstoffatom, ein Alkylrest von 1 bis 8 Kohlenstoffen, ein Alkenylrest von 2 bis 8 Kohlenstoffen, ein Cycloalkylrest von 3 bis 8 Kohlenstoffen, ein 3- bis 8-gliedriger gesättigter oder ungesättigter Heterocyclylrest, ein Phenylrest, ein Phenylrest, der mit einem oder mehreren Halogen- oder Hydroxy-, Alkyl-, Alkyloxy-, Alkylthio-, Alkylsulfinyl-, Alkylsulfonyl-, Amino-, Alkylamino- oder Dialkylaminoresten substituiert ist, oder ein Rest NR' R" ist, wobei R' und R", gleich oder verschieden, Wasserstoffatome oder Alkylreste von 1 bis 3 Kohlenstoffen sein können oder zusammen mit dem Stickstoffatom, an das sie gebunden sind, einen 3- bis 8-gliedrigen Heterocyclus bilden können, der gegebenenfalls ein weiteres Heteroatom enthält, das ausgewählt ist aus Sauerstoff, Schwefel oder Stickstoff, gegebenenfalls substituiert mit einem Alkylrest, einem Alkenylrest von 2 bis 8 Kohlenstoffen, einem Cycloalkylrest von 3 bis 6 Kohlenstoffen, einem 4- bis 6-gliedrigen gesättigten oder ungesättigten Heterocyclylrest, Benzylrest, Phenylrest oder Phenylrest, der wie zuvor für die Definition R₁ definiert substituiert ist,
oder wenn Y ein Rest =CR₃- ist, R₁ auch Halogenmethyl, Hydroxymethyl, Alkyloxymethyl, Alkylthiomethyl, dessen Alkylteil gegebenenfalls mit NR'R" substituiert ist, Alkylsulfinylmethyl, Alkylsulfonylmethyl, Acyloxymethyl, Benzoyloxymethyl, Cyclopropylaminomethyl oder - (CH₂)ₙNR'R" sein kann, wobei n eine ganze Zahl von 1 bis 4 ist und R' und R" wie vorstehend definiert sind, oder wenn R₃ ein Wasserstoffatom ist, R₁ auch Formyl, Carboxy, Alkyloxycarbonyl oder -CONR'R" sein kann, wobei R' und R" wie zuvor definiert sind,
oder wenn Y ein Stickstoffatom ist, R₁ auch ein Rest -XR⁰, wobei X ein Sauerstoffatom oder Schwefelatom, ein Sulfinyl- oder Sulfonylrest oder ein Rest NH ist und R⁰ ein Alkylrest von 1 bis 8 Kohlenstoffen, ein Cycloalkylrest von 3 bis 6 Kohlenstoffen, ein 3- bis 8-gliedriger gesättigter oder ungesättigter Heterocyclylrest, ein 3- bis 8- gliedriger Heterocyclylmethylrest, dessen Heterocyclylteil über ein Kohlenstoffatom an den Methylrest gebunden ist, ein Phenylrest, ein Phenylrest, der mit einem oder mehreren Halogenatomen oder Hydroxy-, Alkyl-, Alkyloxy-, Alkylthio-, Alkylsulfinyl-, Alkylsulfonyl-, Amino-, Alkylamino-, oder Dialkylaminoresten substituiert ist, oder ein Rest -(CH₂)ₙNR'R" sein kann, wobei R' und R" wie zuvor definiert sind und n eine ganze Zahl von 2 bis 4 ist, oder wenn X NH ist, R⁰ auch ein Wasserstoffatom darstellen kann,
R₂ ein Wasserstoffatom oder ein Alkylrest mit 1 bis 3 Kohlenstoffen ist,
R₃ ein Wasserstoffatom oder ein Alkyl-, Carboxy-, Alkyloxycarbonyl-, oder Carbamoylrest der Struktur -CO-NR'R" ist, wobei R' und R " wie zuvor definiert sind,
Ra ein Methyl- oder Ethylrest ist, und
Rb, Rc und Rd die nachstehend gegebenen Definitionen besitzen:
1) Rb und Rd sind Wasserstoffatome, und Rd ist ein Wasserstoffatom oder ein Methylamino- oder Dimethylaminorest,
2) Rb ist ein Wasserstoffatom, Rc ist ein Wasserstoffatom, Chloratom oder Bromatom oder stellt einen C₃-C₅-Alkenylrest dar, und Rd ist ein Rest - NMe-R''' , wobei R''' einen Alkylrest, C₂-C₄-Hydroxyalkylrest oder einen C₂-C₈-Alkenylrest, gegebenenfalls substituiert mit Phenyl, C₃-C₆-Cycloalkyl, Methyl, Benzyl, Benzyl, substituiert mit einem oder mehreren Halogenatomen oder Hydroxy-, Alkyl-, Alkyloxy-, Alkylthio-, Alkylsulfinyl-, Alkylsulfonyl-, Amino-, Alkylamino- oder Dialkylaminoresten, Heterocyclylmethyl- oder Heterocyclylethylrest, dessen Heterocyclylteil gesättigt oder ungesättigt ist und 5 bis 6 Kettenglieder und 1 oder 2 Heteroatome enthält, ausgewählt aus Schwefel, Sauerstoff und Stickstoff, gegebenenfalls substituiert mit einem Alkylrest, Alkenylrest von 2 bis 8 Kohlenstoffen, Cycloalkylrest von 3 bis 6 Kohlenstoffen, einem 4- bis 6-gliedrigen gesättigten oder ungesättigten Heterocyclylrest, Phenylrest, Phenylrest, substituiert wie zuvor für die Definition von R₁ definiert, oder Benzyl, oder R"' einen Cyanomethylrest oder einen Rest - CH₂CORe darstellt, wobei entweder Re -OR'e ist,
wobei R'e Wasserstoff, Alkyl mit 1 bis 6 Kohlenstoffen, Alkenyl mit 2 bis 6 Kohlenstoffen, Benzyl oder Heterocyclylmethyl ist, dessen Heterocyclylteil 5 bis 6 Kettenglieder und 1 oder 2 Heteroatome enthält, ausgewählt aus Schwefel, Sauerstoff oder Stickstoff, oder Re ein Alkylamino-, Alkylmethylamino-, Heterocyclylamino- oder Heterocyclylmethylaminorest ist, dessen Heterocyclylteil gesättigt ist und 5 bis 6 Kettenglieder und 1 oder 2 Heteroatome enthält, ausgewählt aus Schwefel, Sauerstoff oder Stickstoff, gegebenenfalls substituiert mit einem Alkyl-, Benzyl-, oder Alkyloxycarbonylrest,
3) Rb ist ein Wasserstoffatom, Rd ist ein Rest - NHCH₃ oder -N(CH₃)₂, und Rc ist ein Chlor- oder Bromatom oder stellt einen C₃-C₅-Alkenylrest dar, wenn Rd -N(CH₃)₂ ist,
4) Rb und Rd sind Wasserstoffatome, und Rc ist ein Halogenatom oder ein Alkylamino- oder Dialkylamino-, Alkyloxy-, Trifluormethoxy-, Thioalkyl-, C₁-C₆-Alkylrest oder ein Trihalogenmethylrest,
5)Rb und Rc sind Wasserstoffatome und Rd ist ein Halogenatom oder ein Ethylamino-, Diethylamino- oder Methylethylamino-, Alkyloxy-, oder Trifluormethoxy-, Alkylthio-, Alkylsulfinyl-, Alkylsulfonyl-, ein C₁-C₆-Alkylrest, ein Phenyl- oder Trihalogenmethylrest,
6) Rb ist ein Wasserstoffatom, und Rc ist ein Halogenatom oder ein Alkylamino- oder Dialkylamino-, Alkyloxy- oder Trifluormethoxy-, Thioalkyl-, C₁-C₃-Alkylrest, und Rd ist ein Halogenatom oder ein Amino-, Alkylamino- oder Dialkyl
amino-, Alkyloxy- oder Trifluormethoxy-, Thioalkyl-, C₁-C₆-Alkyl- oder ein Trihalogenmethylrest,
7) Rc ist ein Wasserstoffatom und Rb und Rd stellen einen Methylrest dar,
wobei die Alkyl-, Alkenyl- oder Acylreste geradkettig oder verzweigt sind und, wenn nicht anders angegeben, die Alkyl- oder Acylreste 1 bis 4 Kohlenstoffatome enthalten,
sowie ihre Salze, sofern sie existieren.

2. Derivat der Gruppe B der Streptogramine nach Anspruch 1, wobei
Y ein Stickstoffatom oder ein Rest =CR₃- ist,
R₁ ein Wasserstoffatom, ein Alkylrest von 1 bis 8 Kohlenstoffen, ein Cycloalkylrest von 3 bis 8 Kohlenstoffen, ein 3- bis 8-gliedriger gesättigter oder ungesättigter Heterocyclylrest, ein Phenylrest, ein Phenylrest, der mit einem oder mehreren Amino-, Alkylamino- oder Dialkylaminoresten substituiert ist, oder ein Rest NR' R'' ist, wobei R' und R" , gleich oder verschieden, Wasserstoffatome oder Alkylreste von 1 bis 3 Kohlenstoffen sein können oder zusammen mit dem Stickstoffatom, an das sie gebunden sind, einen 3- bis 8-gliedrigen Heterocyclus bilden können, der gegebenenfalls ein weiteres Heteroatom enthält, das aus Sauerstoff, Schwefel oder Stickstoff ausgewählt ist, der gegebenenfalls mit einem Alkylrest substituiert ist,
oder wenn Y ein Rest =CR₃- ist, R₁ auch Halogenmethyl, Hydroxymethyl, Alkylthiomethyl sein kann, dessen Alkylteil gegebenenfalls substituiert ist mit NR'R", Alkylsulfinylmethyl, Alkylsulfonylmethyl, Acyloxymethyl, Cyclopropylaminomethyl oder -(CH₂)ₙNR'R", wobei n eine ganze Zahl von 1 bis 4 ist und R' und R" wie vorstehend definiert sind, oder wenn R' ein Wasserstoffatom ist, R₁ auch Formyl oder
-CONR'R" sein kann, wobei R' und R" wie zuvor definiert sind,
oder wenn Y ein Stickstoffatom ist, R₁ auch ein Rest -XR⁰ sein kann, wobei X ein Sauerstoffatom oder Schwefelatom, ein Sulfinyl- oder Sulfonylrest oder ein Rest NH ist und R⁰ ein Alkylrest von 1 bis 8 Kohlenstoffen, ein Cycloalkylrest von 3 bis 6 Kohlenstoffen, ein 3- bis 8-gliedriger Heterocyclylmethylrest, dessen Heterocyclylteil über ein Kohlenstoffatom an den Methylrest gebunden ist, oder ein Rest
-(CH₂)ₙNR'R" ist, wobei R' und R" wie zuvor definiert sind und n eine ganze Zahl von 2 bis 4 ist,
R₂ ein Wasserstoffatom oder ein Alkylrest mit 1 bis 3 Kohlenstoffen ist,
R₃ ein Wasserstoffatom oder ein Carboxy- oder Alkyloxycarbonylrest ist,
Ra ein Methyl- oder Ethylrest ist, und
Rb, Rc und Rd die nachstehend gegebenen Definitionen besitzen:
• Rb und Rc sind Wasserstoffatome, und Rd ist ein Wasserstoffatom oder ein Methylamino- oder Dimethylaminorest,
• Rb ist ein Wasserstoffatom, Rd ist ein Rest - NHCH₃ oder -N(CH₃)₂, und Rc ist ein Chlor- oder Bromatom,
sowie ihre Salze, sofern sie existieren.

3. Derivat der Gruppe B der Streptogramine nach Anspruch 1, wobei
Y ein Stickstoffatom oder ein Rest =CR₃- ist,
R₁ ein Wasserstoffatom, ein Alkylrest mit 1 bis 3 Kohlenstoffen, ein Cycloalkylrest mit 3 bis 8 Kohlenstoffen, ein 3- bis 8-gliedriger gesättigter oder ungesättigter Heterocyclylrest, Phenylrest, Phenylrest, substituiert mit einem Aminorest, ist
oder wenn Y ein Rest =CR₃- ist, R₁ auch Acyloxymethyl sein kann,
oder wenn Y ein Stickstoffatom ist, R₁ auch ein Rest -XR⁰ sein kann, wobei X ein Sauerstoffatom oder Schwefelatom oder ein Rest NH ist, und R⁰ ein Alkylrest mit 1 bis 4 Kohlenstoffen oder ein Rest - (CH₂)ₙNR'R'' ist, wobei R' und R", gleich oder verschieden, Wasserstoffatome oder Alkylreste mit 1 bis 3 Kohlenstoffen sein können oder zusammen mit dem Stickstoffatom, an das sie gebunden sind, einen 3- bis 8-gliedrigen Heterocyclus bilden, der gegebenenfalls ein weiteres Heteroatom enthält, ausgewählt aus Sauerstoff, Schwefel oder Stickstoff, der gegebenenfalls mit einem Alkylrest substituiert ist, und n eine ganze Zahl von 2 bis 4 ist,
R₂ ein Wasserstoffatom oder ein Alkylrest mit 1 bis 3 Kohlenstoffen ist,
R₃ ein Wasserstoffatom oder ein Alkyloxycarbonylrest ist,
Ra ein Methyl- oder Ethylrest ist, und
Rb, Rc und Rd die nachstehend gegebenen Definitionen besitzen:
• Rb und Rc sind Wasserstoffatome, und Rd ist ein Wasserstoffatom oder ein Methylamino- oder Dimethylaminorest,
• Rb ist ein Wasserstoffatom, Rd ist ein Rest - NHCH₃ oder -N(CH₃)₂, und Rc ist ein Chloratom,
sowie ihre Salze, falls sie existieren.

4. Derivat der Gruppe B der Streptogramine nach Anspruch 1, **dadurch gekennzeichnet, dass** es sich um 2"-Methyl-pyrido[2,3-5γ,5δ]pristinamycin IE handelt.

5. Derivat der Gruppe B der Streptogramine nach Anspruch 1, **dadurch gekennzeichnet, dass** es sich um 2"-Cyclopropyl-pyrido[2,3-5γ,5δ]pristinamycin IE handelt.

6. Derivat der Gruppe B der Streptogramine nach Anspruch 1, **dadurch gekennzeichnet, dass** es sich um Pyrido[2,3-5γ,5δ]pristinamycin IE handelt.

7. Derivat der Gruppe B der Streptogramine nach Anspruch 1, **dadurch gekennzeichnet, dass** es sich um 2"-Ethyl-pyrido [2,3-5γ,5δ] (4ξ-methylamino) (4ξ-desdimethylamino)pristinamycin IE handelt.

8. Derivat der Gruppe B der Streptogramine nach Anspruch 1, **dadurch gekennzeichnet, dass** es sich um 4ε-Chlor-2"-(ethyl)-pyrido [2,3-5γ, 5δ] (4ξ-methylamino) (4ξ-desdimethylamino)pristinamycin IE handelt.

9. Verfahren zur Herstellung eines Streptogramin-Derivats nach Anspruch 1, wobei Y ein Rest =CR₃-ist und R₃ etwas anderes als ein Alkylrest ist, **dadurch gekennzeichnet, dass** man einen Enaminester der allgemeinen Formel: 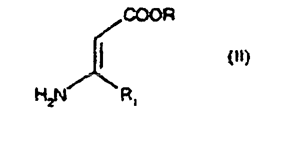 wobei R₁ wie zuvor definiert ist und R den Rest eines leicht hydrolysierbaren Esters oder einen Alkylrest darstellt, mit dem Methylen-5δ-pristinamycin-Derivat, das der allgemeinen Formel 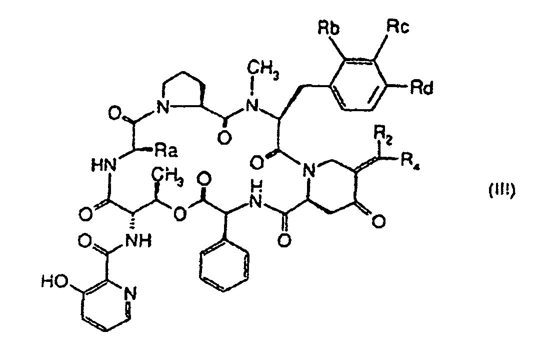 entspricht, wobei Ra, Rb, Rc und Rd wie für Anspruch 1 definiert sind, R₂ wie für Anspruch 1 definiert ist und R₄ ein Wasserstoffatom ist, oder R₂ ein Wasserstoffatom darstellt und R₄ ein Wasserstoffatom oder einen Dialkylaminorest darstellt, reagieren läßt, anschließend gegebenenfalls die Umwandlung des erhaltenen Esters in eine Säure, anschließend gegebenenfalls ihre Decarboxylierung, oder die Umwandlung der Säure in einen Carbamoylrest, je nach erwartetem Derivat nach Anspruch 1, durchführt, und/oder gegebenenfalls die Umwandlung des Derivats nach Anspruch 1, wobei R₁ Hydroxymethyl ist, in ein Derivat durchführt, wobei R₁ ein Formylrest, gegebenenfalls ein Carboxyrest, gegebenenfalls ein Alkyloxycarbonylrest oder ein Rest
-CONR'R" ist, und/oder gegebenenfalls die Mono-N-Demethylierung des Derivats nach Anspruch 1, wobei Rd ein Dimethylaminorest ist, in ein Derivat, wobei Rd Methylamino ist, durchführt, und anschließend gegebenenfalls in ein Salz, sofern es existiert, überführt.

10. Verfahren zur Herstellung eines Streptogramin-Derivats nach Anspruch 1, wobei Y ein Rest =CR₃-ist und R₃ ein Wasserstoffatom oder ein Alkylrest ist, **dadurch gekennzeichnet, dass** man ein Pyridiniumsalz der allgemeinen Formel: 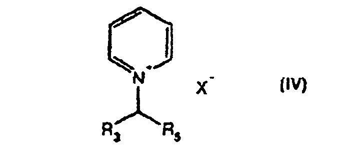 wobei R₃ wie vorstehend definiert ist, R₅ der Rest eines Ketons R₁-CO- ist, wobei R₁ wie zuvor definiert ist, mit Ausnahme der Darstellung eines Restes -NR'R", oder gegebenenfalls einen geschützten Hydroxyrest oder einen Nitrophenylrest darstellt oder R₅ einen Cyanorest darstellt, unter Erhalt eines Streptogramin-Derivats, wobei R₁ ein Aminorest ist und X⁻ ein Anion ist, mit dem Methylen-5δ-pristinamycin-Derivat entsprechend der allgemeinen Formel (III), die in Anspruch 2 definiert ist, wobei R₄ ein Wasserstoffatom ist und Ra, Rb, Rc, Rd und R₂ wie für Anspruch 1 definiert sind, reagieren lässt, anschließend gegebenenfalls den Hydroxyrest freisetzt oder gegebenenfalls den Nitrophenylrest unter Erhalt eines Derivats reduziert, wobei R₁ ein Aminophenylrest ist, oder gegebenenfalls ein Amin der allgemeinen Formel NHR'R" mit dem Streptogramin-Derivat nach Anspruch 1, wobei R₁ Halogenomethyl ist, unter Erhalt des entsprechenden Derivats, wobei R₁ ein Rest -CH₂NR'R" ist, reagieren lässt oder gegebenenfalls das Derivat nach Anspruch 1, wobei R₁ Hydroxymethyl ist, in ein Derivat überführt, wobei R₁ ein Formylrest, gegebenenfalls Carboxy, gegebenenfalls Alkyloxycarbonyl oder -CONR'R" ist, und/oder gegebenenfalls die Mono-N-Demethylierung des Derivats nach Anspruch 1, wobei Rd ein Dimethylaminorest ist, in ein Derivat durchführt,
wobei Rd Methylamino ist, und man anschließend gegebenenfalls in ein Salz überführt, sofern es existiert.

11. Verfahren zur Herstellung eines Streptogramin-Derivats nach Anspruch 1, wobei Y ein Stickstoffatom ist, **dadurch gekennzeichnet, dass** man ein A-midinsalz oder ein Isoharnstoffderivat oder ein Isothioharnstoffderivat der allgemeinen Formel: 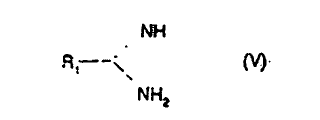 wobei R₁ wie für Anspruch 1 definiert ist, mit Ausnahme der Darstellung eines Restes XR⁰, wobei X Sulfonyl oder Sulfinyl, oder ein Rest NR'R" ist, der anders ist als Amino, mit einem Streptogramin-Derivat der allgemeinen Formel (III), wie in Anspruch 2 definiert, wobei R₄ Dialkylamino ist, reagieren lässt, anschließend, um ein Streptogramin-Derivat nach Anspruch 1 zu erhalten, wobei R₁ ein Rest XR⁰ ist, wobei X Sulfonyl oder Sulfinyl ist, das entsprechende Derivat, wobei X ein Schwefelatom ist, oxidiert, anschließend, um das Streptogramin-Derivat nach Anspruch 1 zu erhalten, wobei R₁ ein Rest NR'R" ist, das Sulfonyl-Derivat substituiert, das durch Einwirkung des entsprechenden Amins HNR'R" erhalten wird, und/oder gegebenenfalls, um das Derivat zu erhalten, wobei Rd Methylamino ist, die Mono-N-Demethylierung des Derivats nach Anspruch 1, wobei Rd ein Dimethylaminorest ist, durchführt, und man anschließend gegebenenfalls in ein Salz, sofern es existiert, überführt.

12. Verfahren zur Herstellung eines Streptogramin-Derivats nach Anspruch 1, wobei Y ein Rest =CR₃-ist, R₁ ein Wasserstoffatom, ein Alkyl-, Alkenyl-, Cycloalkyl-, aromatischer Heterocyclyl-, Phenyl, subsituierter Phenyl-, Halogenmethyl-, Hydroxymethyl-, Alkyloxymethyl-, Alkylthiomethyl-, Alkylsulfinylmethyl-, Alkylsulfonylmethylrest, oder ein Rest - (CH₂)ₙNR'R" ist, oder wenn R₃ ein Wasserstoffatom ist, R₁ Formyl, Carboxy, Alkyloxycarbonyl oder -CONR'R", wie für Anspruch 1 definiert, ist und R₂ ein Wasserstoffatom ist, **dadurch gekennzeichnet, dass** man das Formylenamin der allgemeinen Formel: 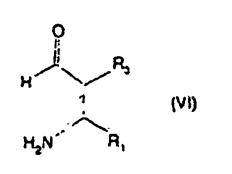 wobei R₁ ein Wasserstoffatom, ein Alkyl-, Alkenyl-, Cycloalkyl-, aromatischer Heterocyclyl-, Phenyl, substituierter Phenyl-, Hydroxymethyl, Alkyloxymethyl, Alkylthiomethylrest oder ein Rest - (CH₂)ₙNR'R" ist und R₃ wie für Anspruch 1 definiert ist, mit der Ausnahme der Darstellung von Carboxy, mit einem Streptogramin-Derivat der allgemeinen Formel: 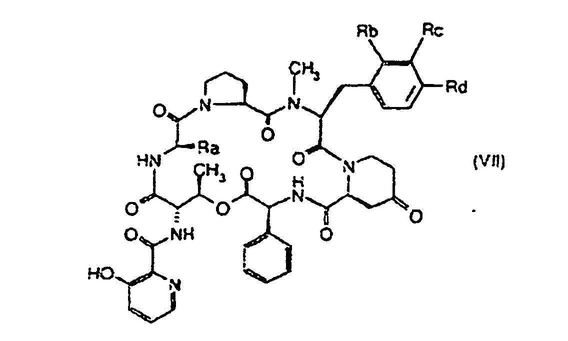 wobei Ra, Rb, Rc und Rd wie für Anspruch 1 definiert sind, reagieren lässt, anschließend gegebenenfalls die Umwandlung des Derivats, wobei R₃ Amid oder Ester ist, in ein Derivat durchführt, wobei R₃ Carboxy ist, und/oder gegebenenfalls die Oxidation des Derivats, wobei R₁ Alkylthiomethyl ist, in ein Derivat, wobei R₁ Alkylsulfinylmethyl oder Alkylsulfonylmethyl ist, durchführt oder gegebenenfalls die Umwandlung des Derivats, wobei R₁ ein Hydroxymethylrest ist, in ein Derivat, wobei R₁ Halogenomethyl ist, durchführt und anschließend gegebenenfalls die Umwandlung des Derivats, wobei R₁ Halogenmethyl ist, in ein Derivat durchführt, wobei R₁
-CH₂NR'R" ist, oder gegebenenfalls die Umwandlung des Derivats nach Anspruch 1, wobei R₁ Hydroxymethyl ist, in ein Derivat, wobei R₁ ein Formylrest, gegebenenfalls Carboxy, Alkyloxycarbonyl und/oder -CONR'R" ist, durchführt und/oder gegebenenfalls die Mono-N-Demethylierung des Derivats nach Anspruch 1, wobei Rd ein Dimethylaminorest ist, in ein Derivat, wobei Rd Methylamino ist, durchführt und man anschließend gegebenenfalls in ein Salz, sofern es existiert, überführt.

13. Verfahren zur Herstellung eines Streptogramin-Derivats nach Anspruch 1, wobei Rd Methylamino ist, **dadurch gekennzeichnet, dass** man die Mono-N-Demethylierung des Derivats nach Anspruch 1, wobei Rd ein Dimethylaminorest ist, durchführt und anschließend gegebenenfalls das erhaltene Streptogramin-Derivat in ein Salz überführt.

14. Streptogramin-Derivat der allgemeinen Formel: 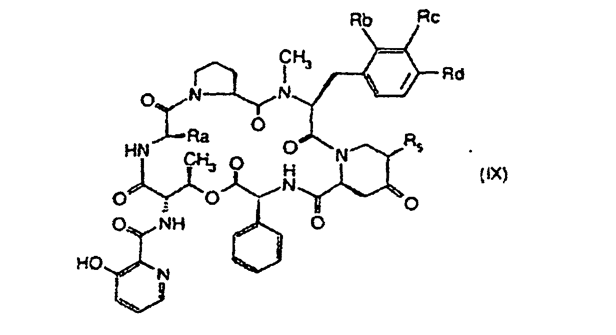 wobei Ra ein Methylrest ist und Rb, Rc und Rd wie in Anspruch 1 definiert sind oder Ra ein Ethylrest ist und Rb, Rc und Rd wie in Anspruch 1 unter 2) bis 7) definiert sind, und R₅ ein disubstituierter
Methylenylrest der Struktur 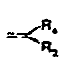 ist, wobei R₂ und R₄ wie zuvor definiert sind oder wobei Ra, Rb, Rc und Rd wie für Anspruch 1 unter 2) definiert sind, mit der Ausnahme, dass R"' Ethyl darstellt, wenn Rb und Rc Wasserstoff sind, und R₅ ein Wasserstoffatom ist.

15. Pharmazeutische Zubereitung, umfassend ein Streptogramin-Derivat der Gruppe B nach Anspruch 1 in reinem Zustand oder in kombinierter Form mit mindestens einem Streptogramin-Derivat der Gruppe A, gegebenenfalls in Form von Salz und/oder in Kombinationsform mit einem oder mehreren kompatiblen und pharmazeutisch verträglichen Verdünnungsmitteln oder Adjuvantien.

16. Pharmazeutische Zubereitung nach Anspruch 15, **dadurch gekennzeichnet, dass** das Streptogramin-Derivat der Gruppe A ausgewählt ist aus Pristinamycin IIA, Pristinamycin IIB, Pristinamycin IIC, Pristinamycin IID, Pristinamycin IIE, Pristinamycin IIF, Pristinamycin IIG oder aus den bekannten Hemisynthese-Derivaten oder aus den Derivaten der allgemeinen Formel: 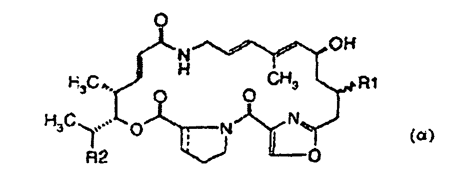 wobei R₁ ein Rest -NR' R" ist, wobei R' ein Wasserstoffatom oder ein Methylrest ist und R " ein Wasserstoffatom, ein Alkylrest, Cycloalkylrest, Allylrest, Propargylrest, Benzylrest oder -OR" ' ist, wobei R " ' ein Wasserstoffatom, ein Alkyl-, Cycloalkyl-, Allyl-, Propargyl- oder Benzylrest oder -NR₃R₄ ist, wobei R₃ und R₄ einen Methylrest darstellen können oder zusammen mit dem Stickstoffatom, an das sie gebunden sind, einen 4 bis 5-gliedrigen gesättigten oder ungesättigten Heterocyclus bilden können, der ferner ein weiteres Heteroatom enthalten kann, ausgewählt aus Stickstoff, Sauerstoff oder Schwefel, R₂ ein Wasserstoffatom oder ein Methylrest oder Ethylrest ist, und die Bindung --- eine Einfachbindung oder eine Doppelbindung darstellt, sowie aus ihren Salzen.

17. Kombination eines Derivats des Streptogramins der Gruppe B nach Anspruch 1 mit mindestens einem Derivat des Streptogramins der Gruppe A, wie in Anspruch 16 definiert.

## Claims

1. Derivative of group B streptogramins of general formula: 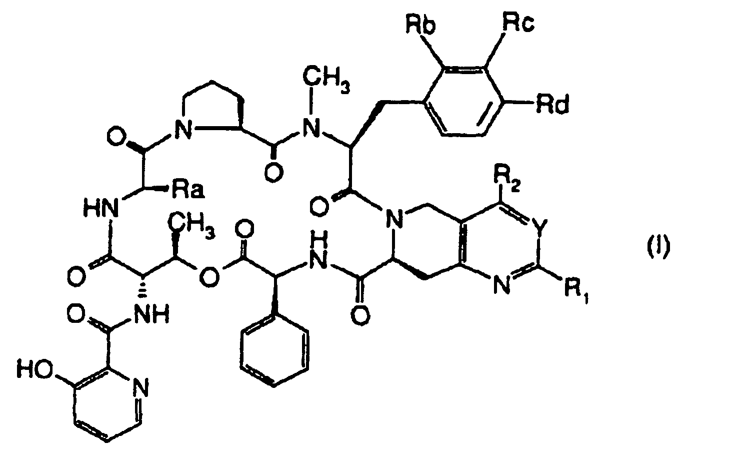 in which
Y is a nitrogen atom or a radical =CR₃-,
R₁ is a hydrogen atom, a radical alkyl having 1 to 8 carbons, alkenyl having 2 to 8 carbons, cycloalkyl having 3 to 8 carbons, heterocyclyl which is saturated or unsaturated having 3 to 8 members, phenyl, phenyl which is substituted - with one or more halogen atoms or hydroxyl, alkyl, alkyloxy, alkylthio, alkylsulphinyl, alkylsulphonyl, amino, alkylamino or dialkylamino radicals - or a radical NR'R", R' and R", which are identical or different, being capable of being hydrogen atoms or alkyl radicals having 1 to 3 carbons, or being capable of forming together with the nitrogen atom to which they are attached a 3- to 8- membered heterocycle optionally containing another heteroatom chosen from oxygen, sulphur or nitrogen which is optionally substituted - with a radical alkyl, alkenyl having 2 to 8 carbons, cycloalkyl having 3 to 6 carbons, heterocyclyl which is saturated or unsaturated having 4 to 6 members, benzyl, phenyl or phenyl which is substituted as defined above for the definition of R₁ -,
or alternatively when Y is a radical =CR₃-, R₁ may also be halomethyl, hydroxymethyl, alkyloxymethyl, alkylthiomethyl in which the alkyl portion is optionally substituted with NR'R", alkylsulphinylmethyl, alkylsulphonylmethyl, acyloxymethyl, benzoyloxymethyl, cyclopropylaminomethyl or - (CH₂)ₙNR'R", n being an integer from 1 to 4 and R' and R" being defined as above, or alternatively if R₃ is a hydrogen atom, R₁ may also be formyl, carboxyl, alkyloxycarbonyl, or -CONR'R" for which R' and R" are defined as above,
or alternatively when Y is a nitrogen atom, R₁ may also be a radical -XR° for which X is an oxygen or sulphur atom, a sulphinyl or sulphonyl radical, or an NH radical and R° is a radical alkyl having 1 to 8 carbons, cycloalkyl having 3 to 6 carbons, heterocyclyl which is saturated or unsaturated having 3 to 8 members, heterocyclylmethyl having 3 to 8 members in which the heterocyclyl portion is attached to the methyl radical by a carbon atom, phenyl, phenyl which is substituted - with one or more halogen atoms or hydroxyl, alkyl, alkyloxy, alkylthio, alkylsulphinyl, alkylsulphonyl, amino, alkylamino or dialkylamino radicals - or a radical -(CH₂)ₙNR'R" for which R' and R" are defined as above and n is an integer from 2 to 4, or alternatively if X represents NH, R° may also represent the hydrogen atom,
R₂ is a hydrogen atom or an alkyl radical having 1 to 3 carbons,
R₃ is a hydrogen atom or an alkyl, carboxyl, alkyloxycarbonyl or carbamoyl radical having the structure -CO-NR'R" in which R' and R" are defined as above,
Ra is a methyl or ethyl radical, and
Rb, Rc and Rd have the definitions below:
1) Rb and Rc are hydrogen atoms and Rd is a hydrogen atom or a methylamino or dimethylamino radical,
2) Rb is a hydrogen atom, Rc is a hydrogen, chlorine or bromine atom, or represents an alkenyl radical having 3 to 5C, and Rd is a radical -NMe-R"' for which R"' represents a radical alkyl, hydroxyalkyl having 2 to 4C or alkenyl having 2 to 8C which is optionally substituted with phenyl, cycloalkyl having 3 to 6C, methyl, benzyl, benzyl which is substituted - with one or more halogen atoms or hydroxyl, alkyl, alkyloxy, alkylthio, alkylsulphinyl, alkylsulphonyl, amino, alkylamino or dialkylamino radicals - heterocyclylmethyl or heterocyclylethyl in which the heterocyclyl portion is saturated or unsaturated and contains 5 to 6 members and 1 or 2 heteroatoms chosen from sulphur, oxygen or nitrogen which is optionally substituted - with a radical alkyl, alkenyl having 2 to 8 carbons, cycloalkyl having 3 to 6 carbons, heterocyclyl which is saturated or unsaturated having 4 to 6 members, phenyl, phenyl which is substituted as defined above for the definition of R₁ or benzyl - or alternatively R"' represents a radical cyanomethyl, or -CH₂CORe for which either Re is -OR'e, R'e being hydrogen, alkyl having 1 to 6 carbons, alkenyl having 2 to 6 carbons, benzyl or heterocyclylmethyl in which the heterocyclyl portion contains 5 to 6 members and 1 or 2 heteroatoms chosen from sulphur, oxygen or nitrogen, or Re is an alkylamino, alkylmethylamino, heterocyclylamino or heterocyclylmethylamino radical in which the heterocyclyl portion is saturated and contains 5 to 6 members and 1 or 2 heteroatoms chosen from sulphur, oxygen or nitrogen which is optionally substituted with an alkyl, benzyl or alkyloxycarbonyl radical,
3) Rb is a hydrogen atom, Rd is a radical -NHCH₃ or -N (CH₃)₂ and Rc is a chlorine or bromine atom, or represents an alkenyl radical having 3 to 5C, if Rd is -N (CH₃)₂,
4) Rb and Rd are hydrogen atoms and Rc is a halogen atom, or an alkylamino or dialkylamino, alkyloxy, trifluoromethoxy, thioalkyl, alkyl having 1 to 6C or trihalomethyl radical,
5) Rb and Rc are hydrogen atoms and Rd is a halogen atom, or an ethylamino, diethylamino or methylethylamino, alkyloxy or trifluoromethoxy, alkylthio, alkylsulphinyl, alkylsulphonyl, alkyl having 1 to 6C, phenyl or trihalomethyl radical,
6) Rb is a hydrogen atom and Rc is a halogen atom Hzor an alkylamino or dialkylamino, alkyloxy or trifluoromethoxy, thioalkyl or alkyl having 1 to 3C radical, and Rd is a halogen atom or an amino, alkylamino or dialkylamino, alkyloxy or trifluoromethoxy, thioalkyl, alkyl having 1 to 6C or trihalomethyl radical,
7) Rc is a hydrogen atom and Rb and Rd represent a methyl radical,
the alkyl, alkenyl or acyl radicals being straight or branched and, unless otherwise stated, the alkyl or acyl radicals containing 1 to 4 carbon atoms, as well as its salts when they exist.

2. Derivative of group B streptogramins according to Claim 1, for which
Y is a nitrogen atom or a radical =CR₃-,
R₁ is a hydrogen atom, a radical alkyl having 1 to 8 carbons, cycloalkyl having 3 to 8 carbons, heterocyclyl which is saturated or unsaturated having 3 to 8 members, phenyl, phenyl which is substituted - with one or more amino, alkylamino or dialkylamino radicals - or a radical NR'R", R' and R", which are identical or different, being capable of being hydrogen atoms or alkyl radicals having 1 to 3 carbons, or being capable of forming together with the nitrogen atom to which they are attached a 3- to 8-membered heterocycle optionally containing another heteroatom chosen from oxygen, sulphur or nitrogen which is optionally substituted with an alkyl radical,
or alternatively when Y is a radical =CR₃-, R₁ may also be halomethyl, hydroxymethyl, alkylthiomethyl in which the alkyl portion is optionally substituted with NR'R", alkylsulphinylmethyl, alkylsulphonylmethyl, acyloxymethyl, cyclopropylaminomethyl or -(CH₂)ₙNR'R", n being an integer from 1 to 4 and R' and R" being defined as above, or alternatively if R₃ is a hydrogen atom, R₁ may also be formyl or -CONR'R" for which R' and R" are defined as above,
or alternatively when Y is a nitrogen atom, R₁ may also be a radical -XR° for which X is an oxygen or sulphur atom, a sulphinyl or sulphonyl radical, or an NH radical and R° is a radical alkyl having 1 to 8 carbons, heterocyclylmethyl having 3 to 8 members in which the heterocyclyl portion is attached to the methyl radical by a carbon atom, or a radical - (CH₂) ₙNR' R" for which R' and R" are defined as above and n is an integer from 2 to 4,
R₂ is a hydrogen atom or an alkyl radical having 1 to 3 carbons,
R₃ is a hydrogen atom or a carboxyl or alkyloxycarbonyl radical,
Ra is a methyl or ethyl radical, and
Rb, Rc and Rd have the definitions below:
· Rb and Rc are hydrogen atoms and Rd is a hydrogen atom or a methylamino or dimethylamino radical,
· Rb is a hydrogen atom, Rd is a radical -NHCH₃ or -N(CH₃)₂ and Rc is a chlorine or bromine atom,
as well as its salts when they exist.

3. Derivative of group B streptogramins according to Claim 1, for which
Y is a nitrogen atom or a radical =CR₃-,
R₁ is a hydrogen atom, a radical alkyl having 1 to 3 carbons, cycloalkyl having 3 to 8 carbons, heterocyclyl which is saturated or unsaturated having 3 to 8 members, phenyl, phenyl which is substituted with an amino radical,
or alternatively when Y is a radical =CR₃-, R₁ may also be acyloxymethyl,
or alternatively when Y is a nitrogen atom, R₁ may also be a radical -XR° for which X is an oxygen or sulphur atom or a radical NH and R° is an alkyl radical having 1 to 4 carbons or a radical -(CH₂)ₙNR'R" for which R' and R" which are identical or different may be hydrogen atoms or alkyl radicals having 1 to 3 carbons, or form together with the nitrogen atom to which they are attached a 3- to 8-membered heterocycle optionally containing another heteroatom chosen from oxygen, sulphur or nitrogen optionally substituted with an alkyl radical, and n is an integer from 2 to 4,
R₂ is a hydrogen atom or an alkyl radical having 1 to 3 carbons,
R₃ is a hydrogen atom or an alkyloxycarbonyl radical,
Ra is a methyl or ethyl radical, and
Rb, Rc and Rd have the definitions below:
· Rb and Rc are hydrogen atoms and Rd is a hydrogen atom or a methylamino or dimethylamino radical,
· Rb is a hydrogen atom, Rd is a radical -NHCH₃ or -N(CH₃)₂ and Rc is a chlorine atom,
as well as its salts when they exist.

4. Derivative of group B streptogramins according to Claim 1, **characterized in that** it is 2"-methylpyrido[2,3-5γ,5δ]pristinamycin I_{E}.

5. Derivative of group B streptogramins according to Claim 1, **characterized in that** it is 2"-cyclopropylpyrido[2,3-5γ,5δ]pristinamycin I_{E}.

6. Derivative of group B streptogramins according to Claim 1, **characterized in that** it is pyrido[2,3-5γ,5δ)pristinamycin I_{E}.

7. Derivative of group B streptogramins according to Claim 1, **characterized in that** it is 2"-ethylpyrido[2,3-5γ,5δ] (4ζ-methylamino)-(4ζ-dedimethylamino)pristinamycin I_{E}.

8. Derivative of group B streptogramins according to Claim 1, **characterized in that** it is 4ε-chloro-2"-(ethyl)-pyrido[2,3-5γ,5δ] (4ζ-methylamino)-(4ζ-dedimethylamino)pristinamycin I_{E}.

9. Process for the preparation of a streptogramin derivative according to Claim 1, for which Y is a radical =CR₃- and R₃ is other than an alkyl radical, **characterized in that** an enamino ester of general formula: 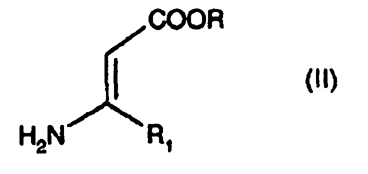 in which R₁ is defined as above and R represents the residue of an easily hydrolysable ester or an alkyl radical, is reacted with the corresponding 5δ-methylenepristinamycin derivative of general formula: 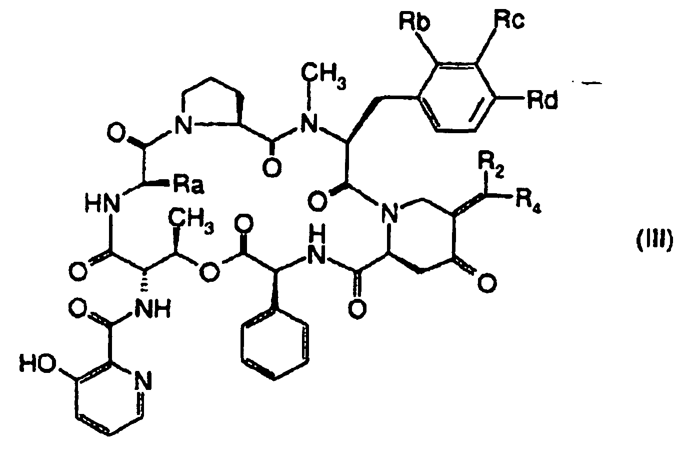 in which Ra, Rb, Rc and Rd are defined as for Claim 1, R₂ is defined as for Claim 1 and R₄ is a hydrogen atom, or R₂ represents a hydrogen atom and R₄ is a hydrogen atom or a dialkylamino radical, followed where appropriate by the conversion of the ester obtained to an acid, and then optionally by its decarboxylation, or by the conversion of the acid to a carbamoyl radical according to the derivative according to Claim 1 desired, and/or followed where appropriate by the conversion of the derivative according to Claim 1 for which R₁ is hydroxymethyl to a derivative for which R₁ is a radical formyl, and then where appropriate carboxyl, and then where appropriate alkyloxycarbonyl or -CONR'R" and/or optionally followed by the mono-N-demethylation of the derivative according to Claim 1 for which Rd is a dimethylamino radical to a derivative for which Rd is methylamino, and then optionally followed by the conversion to a salt when they exist.

10. Process for the preparation of a streptogramin derivative according to Claim 1, for which Y is a radical =CR₃- and R₃ is a hydrogen atom or an alkyl radical, **characterized in that** a pyridinium salt of general formula: 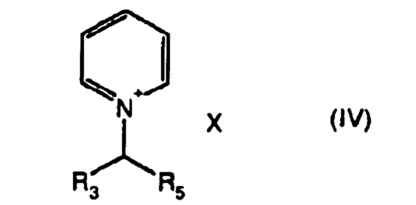 in which R₃ is defined as above, R₅ is the residue of a ketone R₁-CO- for which R₁ is defined as above with the exception of representing a radical -NR'R", or optionally represents a protected hydroxyl radical or a nitrophenyl radical or alternatively R₅ represents the cyano radical so as to obtain a streptogramin derivative for which R₁ is an amino radical, and X⁻ is an anion, is reacted with the corresponding 5δ-methylenepristinamycin derivative of general formula (III) defined in Claim 2, in which R₄ is a hydrogen atom and Ra, Rb, Rc, Rd and R₂ are defined as for Claim 1, optionally followed by the liberation of the hydroxyl radical or where appropriate the reduction of the nitrophenyl radical so as to obtain a derivative for which R₁ is an aminophenyl radical, or optionally followed by the reaction of an amine of general formula HNR'R" with the streptogramin derivative according to Claim 1, for which R₁ is halomethyl, so as to obtain the corresponding derivative for which R₁ is a radical -CH₂NR'R", or where appropriate by the conversion of the derivative according to Claim 1 for which R₁ is hydroxymethyl to a derivative for which R₁ is a radical formyl, and then where appropriate carboxyl, and then where appropriate alkyloxycarbonyl or -CONR'R" and/or optionally the mono-N-demethylation of the derivative according to Claim 1 for which Rd is a dimethylamino radical to a derivative for which Rd is methylamino, and then optionally followed by the conversion to a salt, when they exist.

11. Process for the preparation of a streptogramin derivative according to Claim 1, for which Y is a nitrogen atom, **characterized in that** an amidine salt or a derivative of isourea or of isothiourea of general formula: 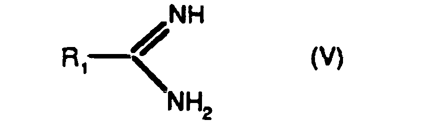 in which R₁ is defined as for Claim 1, with the exception of representing a radical XR° for which X is sulphonyl or sulphinyl, or a radical NR'R" other than amino, is reacted with a streptogramin derivative of general formula (III) as defined in Claim 2, for which R₄ is dialkylamino, and then in order to obtain a streptogramin derivative according to Claim 1, for which R₁ is a radical XR° for which X is sulphonyl or sulphinyl, the corresponding derivative for which X is a sulphur atom is oxidized, and then in order to obtain the streptogramin derivative according to Claim 1, for which R₁ is a radical NR'R", the sulphonyl derivative obtained is substituted by the action of the corresponding amine HNR'R" and/or optionally in order to obtain a derivative for which Rd is methylamino, the mono-N-demethylation of the derivative according to Claim 1, for which Rd is a dimethylamino radical is carried out, and then optionally converted to a salt, when they exist.

12. Process for the preparation of a streptogramin derivative according to Claim 1, for which Y is a radical =CR₃-, R₁ is a hydrogen atom, an alkyl, alkenyl, cycloalkyl, aromatic heterocyclyl, phenyl, substituted phenyl, halomethyl, hydroxymethyl, alkyloxymethyl, alkylthiomethyl, alkylsulphinylmethyl, alkylsulphonylmethyl or -(CH₂)ₙNR'R" radical, or alternatively when R₃ is a hydrogen atom, for which R₁ is formyl, carboxyl, alkyloxycarbonyl or -CONR'R" as defined for Claim 1 and R₂ is a hydrogen atom,
**characterized in that** the formyl enamine of general formula: 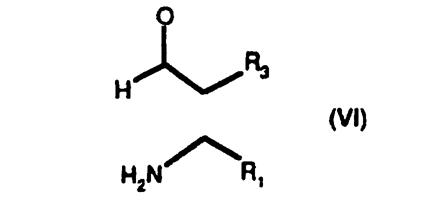 in which R₁ is a hydrogen atom, an alkyl, alkenyl, cycloalkyl, aromatic heterocyclyl, phenyl, substituted phenyl, hydroxymethyl, alkyloxymethyl, alkylthiomethyl or -(CH₂)ₙNR' R" radical and R₃ is defined as for Claim 1, with the exception of representing carboxyl, is reacted with a streptogramin derivative of general formula: 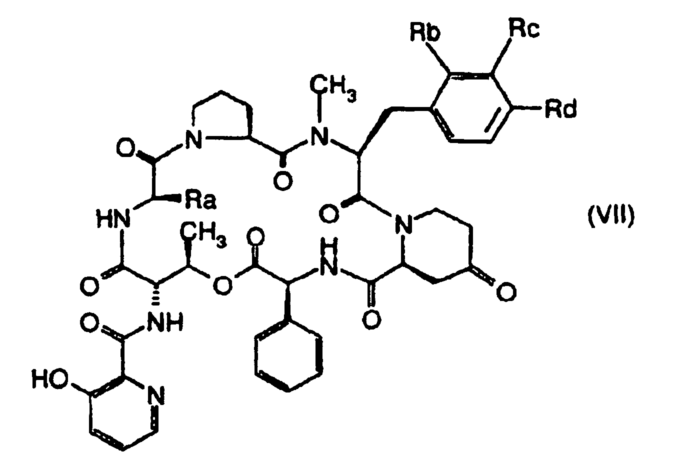 in which Ra, Rb, Rc and Rd are defined as for Claim 1, followed where appropriate by the conversion of the derivative for which R₃ is amide or ester to a derivative for which R₃ is carboxyl and/or where appropriate the oxidation of the derivative for which R₁ is alkylthiomethyl to a derivative for which R₁ is alkylsulphinylmethyl or alkylsulphonylmethyl, or where appropriate the conversion of the derivative for which R₁ is a hydroxymethyl radical to a derivative for which R₁ is halomethyl, and then where appropriate the conversion of the derivative for which R₁ is halomethyl to a derivative for which R₁ is -CH₂NR'R", or where appropriate the conversion of the derivative according to Claim 1, for which R₁ is hydroxymethyl to a derivative for which R₁ is a radical formyl, and then where appropriate carboxyl, alkyloxycarbonyl and/or -CONR'R", and/or optionally the mono-N-demethylation of the derivative according to Claim 1, for which Rd is a dimethylamino radical to a derivative for which Rd is methylamino, and then optionally followed by conversion to a salt, when they exist.

13. Process for the preparation of a streptogramin derivative according to Claim 1, for which Rd is methylamino, **characterized in that** the mono-N-demethylation of the derivative according to Claim 1, for which Rd is a dimethylamino radical, is carried out and then the streptogramin derivative obtained is optionally converted to a salt.

14. Streptogramin derivative of general formula: 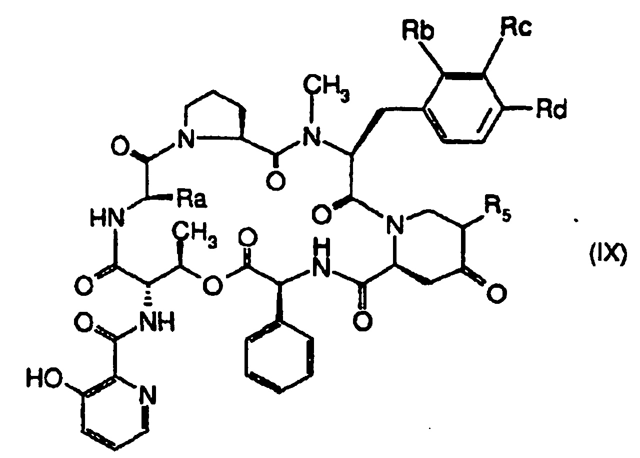 in which Ra is a methyl radical and Rb, Rc and Rd are defined as in Claim 1, or Ra is an ethyl radical and Rb, Rc and Rd are defined as in Claim 1 in 2) to 7) and R₅ represents a disubstituted methylenyl radical having the structure 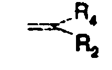 for which R₂ and R₄ are defined as above, or alternatively in which Ra, Rb, Rc and Rd are defined as for Claim 1 in 2), except for R"' representing ethyl if Rb and Rc are hydrogen, and R₅ is a hydrogen atom.

15. Pharmaceutical compositions comprising a group B streptogramin derivative according to Claim 1, in a pure state or in the form of a combination with at least one group A streptogramin derivative, where appropriate in the form of a salt, and/or in the form of a combination with one or more compatible and pharmaceutically acceptable diluents or adjuvants.

16. Pharmaceutical compositions according to Claim 15, **characterized in that** the group A streptogramin derivative is chosen from pristinamycin II_{A}, pristinamycin II_{B}, pristinamycin II_{C}, pristinamycin II_{D}, pristinamycin II_{E}, pristinamycin II_{F}, pristinamycin II_{G} or from known semisynthetic derivatives or from the derivatives of general formula: 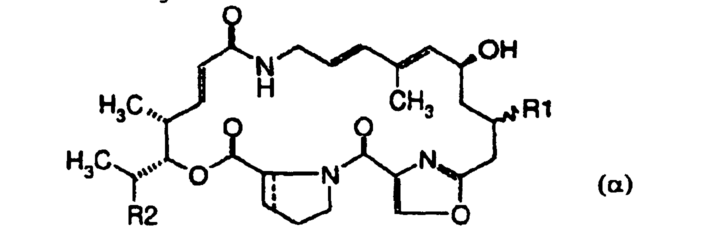 in which R₁ is a radical -NR'R" for which R' is a hydrogen atom or a methyl radical, R" is a hydrogen atom, an alkyl, cycloalkyl, allyl, propargyl, benzyl or -OR"', R"' radical being a hydrogen atom, an alkyl, cycloalkyl, allyl, propargyl or benzyl radical, or -NR₃R₄, it being possible for R₃ and R₄ to represent a methyl radical, or to form together with the nitrogen atom to which they are attached a saturated or unsaturated 4- or 5-membered heterocycle which may in addition contain another heteroatom chosen from nitrogen, oxygen or sulphur, R₂ is a hydrogen atom or a methyl or ethyl radical, and the bond --- represents a single bond or a double bond, as well as their salts.

17. Combinations of a group B streptogramin derivative according to Claim 1 with at least one group A streptogramin derivative as defined in Claim 16.
